# EUROPEAN PATENT APPLICATION

(11) **EP 4 803 523 A1**
(43) Date of publication of application: **09.09.2026**
(21) Application number: 24884800.4
(22) Date of filing: 30.10.2024
(51) Int. Cl.: C07D 491/048, C07D 519/00, A61K 31/517, A61K 31/519, A61P 35/00

(54) **FUSED HETEROARYL COMPOUND, PREPARATION METHOD THEREFOR, AND USE THEREOF IN MEDICINE**

(30) Priority: 30.10.2023 CN 202311418614; 22.12.2023 CN 202311783187; 11.01.2024 CN 202410042969; 04.02.2024 CN 202410158447; 08.04.2024 CN 202410415542; 14.06.2024 CN 202410768890; 02.08.2024 CN 202411057092
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LI, Xin, Shanghai 200245 (CN); SHEN, Feng, Shanghai 200245 (CN); GUI, Bin, Shanghai 200245 (CN); CHEN, Yang, Shanghai 200245 (CN); HE, Feng, Shanghai 200245 (CN)
(74) Representative: Regimbeau
(86) International application number: PCT/CN2024/128427
(87) International publication number: WO 2025/092798

(57) **Abstract**

The present disclosure relates to a fused heteroaryl compound, a preparation method therefor, and a use thereof in medicine. Specifically, the present disclosure relates to a fused heteroaryl compound represented by general formula (I'), a preparation method therefor, a pharmaceutical composition containing said compound, and a use thereof as a therapeutic agent, especially a use thereof in the preparation of a medicament for inhibiting KRAS amplification and/or mutant activity. Each group in general formula (I') is as defined in the description.

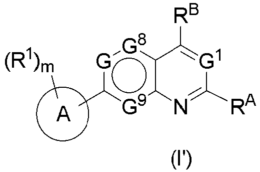

## Description

### Technical Field

The present disclosure belongs to the field of medicine and relates to a fused heteroaryl compound, a preparation method therefor and the use thereof in medicine. Specifically, the present disclosure relates to a fused heteroaryl compound represented by general formula (I'), a preparation method therefor, a pharmaceutical composition containing said compound, and a use thereof in the preparation of a medicament for inhibiting KRAS amplification and/or mutant activity.

### Background Art

The KARS gene encodes the KRAS protein, a small GTPase that belongs to the RAS superfamily of proteins. Within cells, the KRAS protein switches between inactive and active states. It is in an inactive state when bound to guanosine diphosphate (GDP) and becomes active when bound to guanosine triphosphate (GTP), leading to the activation of downstream signalling pathways. KRAS can be activated by upstream growth factor-stimulated tyrosine kinases (e.g., EGFR). Once activated, KRAS commonly triggers downstream pathways like the RAS-RAF-MEK-ERK and PI3K-AKT-mTOR signalling pathways, which regulate cell proliferation and growth.

KRAS is one of the most frequently mutated oncogenes in solid tumours, with mutations occurring in approximately 19% of all cancers. This includes about 90% of pancreatic cancers, about 50% of colourectal cancers, about 30% of lung adenocarcinomas, etc. Other types of cancer such as bile duct, cervical, bladder, liver and breast cancers also occur in some proportion. The most frequent sites for mutations are codons 12, 13, and 61, with mutations at codon 12 being the most prevalent. KRAS mutations cause RAS to predominantly remain in the GTP-bound active state, thereby activating downstream pathways. Additionally, KRAS amplification/overexpression or upstream activation in tumours can also lead to persistent activation of RAS downstream pathways, resulting in tumourigenesis.

Due to the lack of traditional small-molecule binding sites on the KRAS protein surface and its extremely high affinity for guanine nucleotides, which makes it difficult to be competitively inhibited by small molecules, KRAS has long been considered an "undruggable" drug target. However, given the crucial role and high prevalence of aberrant KRAS activation in cancer progression, KRAS has remained and continues to be a highly prioritized target in drug development. Currently, only inhibitors targeting KRAS G12C and G12D have been approved or are in clinical stages. Therefore, there remains a need to develop pan-KRAS inhibitors for the treatment of tumours with various KRAS mutations or KRAS-dependent tumours.

Currently disclosed relevant patent applications include WO 2021041671 A1, WO 2020146613 A1, WO 2017172979 A1, WO 2020238791 A1, WO 2022132200 A1, WO 2022188729 A1, WO 2022194245 A1, WO 2022199587 A1, WO 2022216762 A1, WO 2021000885 A1, WO 2023183585 A1, etc.

### Summary of the Invention

The objective of the present disclosure is to provide a compound represented by general formula (I') or a pharmaceutically acceptable salt thereof: 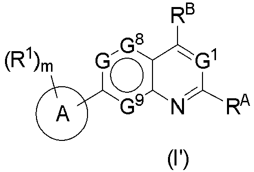 wherein:
R^{A} is selected from the group consisting of 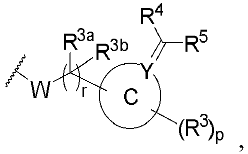 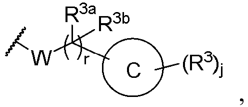 hydrogen atom, halogen, alkyl, alkoxy, haloalkyl, haloalkoxy, cyano, NR¹¹R¹², hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R³;
Y is carbon atom; ring C is selected from the group consisting of cycloalkyl, heterocyclyl, aryl and heteroaryl;
ring A is selected from the group consisting of cycloalkyl, heterocyclyl, aryl and heteroaryl;
G¹ is CR^{G1} or N; G is selected from the group consisting of C, CR^{G} and N;
G⁸ is CR⁷ or N; G⁹ is CR² or N;
R^{B} is selected from the group consisting of hydrogen atom, halogen, alkyl, alkoxy, haloalkyl, haloalkoxy, cyano, NR¹¹R¹², hydroxyl, hydroxyalkyl, alkenyl, alkynyl, OR¹⁴, C(O)OR¹⁴, S(O)ᵥR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁶;
R², R^{G1}, R^{G} and R⁷ are the same or different, and are each independently selected from the group consisting of hydrogen atom, halogen, alkyl, alkoxy, haloalkyl, haloalkoxy, cyano, hydroxyl, hydroxyalkyl, alkenyl, alkynyl, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR¹⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, G and R⁷, together with the carbon atoms to which they are attached, form ring B; ring B is optionally substituted with one or more R⁸;
ring B is selected from the group consisting of cycloalkyl, heterocyclyl, aryl and heteroaryl;
R⁴ and R⁵ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, NR¹¹R¹², C(O)R¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, R⁴ and R⁵, together with the carbon atoms to which they are attached, form cycloalkyl or heterocyclyl, wherein the cycloalkyl and heterocyclyl are each independently and optionally substituted with one or more R³;
W is selected from the group consisting of CR^{3a}R^{3b}, O, S and NR^{w};
R^{3a} and R^{3b} are the same or different, and are each independently selected from the group consisting of hydrogen atom, halogen, alkyl, alkoxy, haloalkyl, haloalkoxy, cyano, amino, hydroxyl and hydroxyalkyl; or, R^{3a} and R^{3b}, together with the carbon atoms to which they are attached, form cycloalkyl or heterocyclyl, wherein the cycloalkyl or heterocyclyl is optionally substituted with one or more R⁰;
R^{w} is selected from the group consisting of hydrogen atom, alkyl, alkoxy, haloalkyl, haloalkoxy, cyano, amino, hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R⁰;
each R¹, R³, R⁶, R⁰¹, R⁸ and R⁰ is the same or different, and is independently selected from the group consisting of oxo, halogen, alkyl, haloalkyl, alkoxy, haloalkoxy, hydroxyl, hydroxyalkyl, alkoxyalkyl, alkenyl, alkynyl, cyano, nitro, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR¹⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, =CR¹⁵R¹⁶, =NR¹³, cycloalkyl, heterocyclyl, aryl and heteroaryl, wherein the alkyl, alkoxy, alkenyl, alkynyl, alkylene, cycloalkyl, heterocyclyl, aryl and heteroaryl are each independently and optionally substituted with one or more R*;
each R¹¹, R¹², R¹³ and R¹⁴ is the same or different, and is independently selected from the group consisting of hydrogen atom, alkyl, alkoxy, hydroxyl, alkenyl, alkynyl, NR²⁰R²¹, C(O)NR²⁰R²¹, alkylene NR²⁰R²¹, alkylene C(O) NR²⁰R²¹, NR²²C(O)R²³, NR²²C(O)NR²⁰R²¹, C(O)R²³, C(O)OR²³, OC(O)R²³, OC(O)OR²³, OR²³, S(O)ᵥR²³, S(O)ᵥNR²⁰R²¹, cycloalkyl, heterocyclyl, aryl and heteroaryl, wherein the alkyl, alkylene, alkoxy, alkenyl, alkynyl, cycloalkyl, heterocyclyl, aryl and heteroaryl are each independently and optionally substituted with one or more R*; or, R¹¹ and R¹², together with the nitrogen atoms to which they are attached, form heterocyclyl, wherein the heterocyclyl is optionally substituted with one or more R*;
R¹⁵ and R¹⁶ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R*;
each R²⁰, R²¹, R²² and R²³ is the same or different, and is independently selected from the group consisting of hydrogen atom, alkyl, alkoxy, haloalkyl, haloalkoxy, hydroxyl, hydroxyalkyl, alkoxyalkyl, alkenyl, alkynyl, NR^{a}R^{b}, C(O)NR^{a}R^{b}, C(O)R^{c}, cycloalkyl, heterocyclyl, cycloalkylalkyl, heterocyclylalkyl, cycloalkyloxy, heterocyclyloxy, aryl and heteroaryl, wherein the alkyl, alkoxy, alkenyl, alkynyl, cycloalkyl, heterocyclyl, aryl and heteroaryl are each independently and optionally substituted with one or more R*; or, R²⁰ and R²¹, together with the nitrogen atoms to which they are attached, form heterocyclyl, wherein the heterocyclyl is substituted with one or more R*;
each R* is the same or different, and is independently selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, C(O)NR^{a}R^{b}, C(O)R^{c}, alkylene NR^{a}R^{b}, alkylene C(O)NR^{a}R^{b}, nitro, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy, wherein the alkyl, alkoxy, alkenyl, alkynyl, alkylene, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy are each independently and optionally substituted with one or more substituents selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, NR^{a}OR^{c}, NR^{a}C(O)NR^{a}R^{b}, NR^{a}C(O)OR^{c}, C(O)NR^{a}R^{b}, C(O)R^{c}, C(O)OR^{c}, alkylene NR^{a}R^{b}, alkylene C(O)NR^{a}R^{b}, S(O)ᵥR^{c}, S(O)ᵥOR^{c}, S(O)ᵥNR^{a}R^{b}, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy;
R^{a}, R^{b} and R^{c} are the same or different, and are each independently selected from the group consisting of hydrogen atom, alkyl, cycloalkyl and heterocyclyl;
r is 0, 1, 2, 3, 4 or 5; v is 0, 1 or 2;
m is 0, 1, 2, 3, 4, 5 or 6;
p is 0, 1, 2, 3, 4, 5 or 6; and j is 0, 1, 2, 3, 4, 5 or 6.

The objective of the present disclosure is to provide a compound represented by general formula (I'A) or a pharmaceutically acceptable salt thereof: 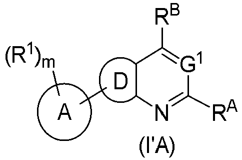 wherein: ring D is selected from the group consisting of cycloalkyl, heterocyclyl, aryl and heteroaryl, and ring D is optionally substituted with one or more R⁰; R⁰, ring A, R¹, m, G¹, R^{A} and R^{B} are as defined in general formula (I').

The objective of the present disclosure is to provide a compound represented by general formula (I) or a pharmaceutically acceptable salt thereof: 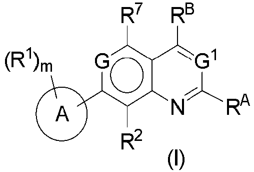 wherein: ring A, R¹, m, G, G¹, R², R⁷, R^{A} and R^{B} are as defined in general formula (I').

The objective of the present disclosure is to provide a compound represented by general formula (I) or a pharmaceutically acceptable salt thereof: 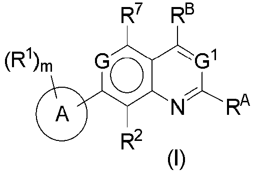 wherein:
R^{A} is selected from the group consisting of 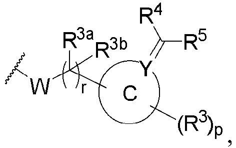 hydrogen atom, halogen, alkyl, alkoxy, haloalkyl, haloalkoxy, cyano, NR¹¹R¹², hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R³;
Y is carbon atom; ring C is selected from the group consisting of cycloalkyl, heterocyclyl, aryl and heteroaryl;
ring A is selected from the group consisting of cycloalkyl, heterocyclyl, aryl and heteroaryl;
G¹ is CR^{G1} or N; G is selected from the group consisting of C, CR^{G} and N;
R^{B} is selected from the group consisting of hydrogen atom, halogen, alkyl, alkoxy, haloalkyl, haloalkoxy, cyano, NR¹¹R¹², hydroxyl, hydroxyalkyl, alkenyl, alkynyl, OR¹⁴, C(O)OR¹⁴, S(O)ᵥR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁶;
R², R^{G1}, R^{G} and R⁷ are the same or different, and are each independently selected from the group consisting of hydrogen atom, halogen, alkyl, alkoxy, haloalkyl, haloalkoxy, cyano, hydroxyl, hydroxyalkyl, alkenyl, alkynyl, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR¹⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, G and R⁷, together with the carbon atoms to which they are attached, form ring B; ring B is optionally substituted with one or more R⁸;
ring B is selected from the group consisting of cycloalkyl, heterocyclyl, aryl and heteroaryl;
R⁴ and R⁵ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, NR¹¹R¹², C(O)R¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, R⁴ and R⁵, together with the carbon atoms to which they are attached, form cycloalkyl or heterocyclyl, wherein the cycloalkyl and heterocyclyl are each independently and optionally substituted with one or more R³;
W is selected from the group consisting of CR^{3a}R^{3b}, O, S and NR^{w};
R^{3a} and R^{3b} are the same or different, and are each independently selected from the group consisting of hydrogen atom, halogen, alkyl, alkoxy, haloalkyl, haloalkoxy, cyano, amino, hydroxyl and hydroxyalkyl; or, R^{3a} and R^{3b}, together with the carbon atoms to which they are attached, form cycloalkyl or heterocyclyl, wherein the cycloalkyl or heterocyclyl is optionally substituted with one or more R⁰;
R^{w} is selected from the group consisting of hydrogen atom, alkyl, alkoxy, haloalkyl, haloalkoxy, cyano, amino, hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R⁰;
each R¹, R³, R⁶, R⁰¹, R⁸ and R⁰ is the same or different, and is independently selected from the group consisting of oxo, halogen, alkyl, haloalkyl, alkoxy, haloalkoxy, hydroxyl, hydroxyalkyl, alkoxyalkyl, alkenyl, alkynyl, cyano, nitro, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR¹⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, =CR¹⁵R¹⁶, =NR¹³, cycloalkyl, heterocyclyl, aryl and heteroaryl, wherein the alkyl, alkoxy, alkenyl, alkynyl, alkylene, cycloalkyl, heterocyclyl, aryl and heteroaryl are each independently and optionally substituted with one or more R*;
each R¹¹, R¹², R¹³ and R¹⁴ is the same or different, and is independently selected from the group consisting of hydrogen atom, alkyl, alkoxy, hydroxyl, alkenyl, alkynyl, NR²⁰R²¹, C(O)NR²⁰R²¹, alkylene NR²⁰R²¹, alkylene C(O)NR²⁰R²¹, NR²²C(O)R²³, NR²²C(O)NR²⁰R²¹, C(O)R²³, C(O)OR²³, OC(O)R²³, OC(O)OR²³, OR²³, S(O)ᵥR²³, S(O)ᵥNR²⁰R²¹, cycloalkyl, heterocyclyl, aryl and heteroaryl, wherein the alkyl, alkylene, alkoxy, alkenyl, alkynyl, cycloalkyl, heterocyclyl, aryl and heteroaryl are each independently and optionally substituted with one or more R*; or, R¹¹ and R¹², together with the nitrogen atoms to which they are attached, form heterocyclyl, wherein the heterocyclyl is optionally substituted with one or more R*;
R¹⁵ and R¹⁶ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R*;
each R²⁰, R²¹, R²² and R²³ is the same or different, and is independently selected from the group consisting of hydrogen atom, alkyl, alkoxy, haloalkyl, haloalkoxy, hydroxyl, hydroxyalkyl, alkoxyalkyl, alkenyl, alkynyl, NR^{a}R^{b}, C(O)NR^{a}R^{b}, C(O)R^{c}, cycloalkyl, heterocyclyl, cycloalkylalkyl, heterocyclylalkyl, cycloalkyloxy, heterocyclyloxy, aryl and heteroaryl, wherein the alkyl, alkoxy, alkenyl, alkynyl, cycloalkyl, heterocyclyl, aryl and heteroaryl are each independently and optionally substituted with one or more R*; or, R²⁰ and R²¹, together with the nitrogen atoms to which they are attached, form heterocyclyl, wherein the heterocyclyl is substituted with one or more R*;
each R* is the same or different, and is independently selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, C(O)NR^{a}R^{b}, C(O)R^{c}, alkylene NR^{a}R^{b}, alkylene C(O)NR^{a}R^{b}, nitro, cycloalkyl, heterocyclyl, cycloalkylalkyl, heterocyclylalkyl, cycloalkyloxy, heterocyclyloxy, aryl and heteroaryl;
R^{a}, R^{b} and R^{c} are the same or different, and are each independently selected from the group consisting of hydrogen atom, alkyl, cycloalkyl and heterocyclyl;
r is 0, 1, 2, 3, 4 or 5; v is 0, 1 or 2;
m is 0, 1, 2, 3, 4, 5 or 6;
p is 0, 1, 2, 3, 4, 5 or 6.

In some embodiments of the present disclosure, provided is the compound represented by general formula (I), (I'), or (I'A) or the pharmaceutically acceptable salt thereof, wherein R^{A} is 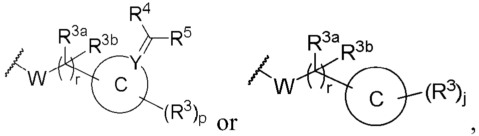 wherein W, R^{3a}, R^{3b}, r, Y, ring C, R³, R⁴, R⁵, p and j are as defined in general formula (I'); in some embodiments, R^{A} is 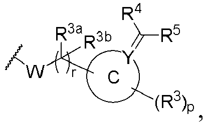 wherein W, R^{3a}, R^{3b}, r, Y, ring C, R³, R⁴, R⁵ and p are as defined in general formula (I); in some embodiments, R^{A} is 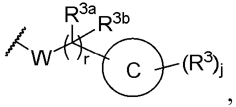 wherein W, R^{3a}, R^{3b}, r, ring C, R³ and j are as defined in general formula (I').

In some embodiments of the present disclosure, provided is the compound represented by general formula (I), (I'), (I'A), (IM), (IN), (VI), (VII-1) or (VII-2) or the pharmaceutically acceptable salt thereof, wherein R^{B} is 3- to 10-membered heterocyclyl, the 3- to 10-membered heterocyclyl is optionally substituted with one or more R⁶, and R⁶ is as defined in general formula (I); in some embodiments, R^{B} is 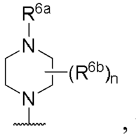 wherein R^{6a}, R^{6b} and n are as defined in general formula (II); in some embodiments, R^{B} is 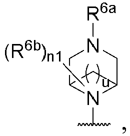 wherein R^{6a}, R^{6b}, u and n1 are as defined in general formula (II), (III), (IV) or (V);
in some embodiments, R^{B} is selected from the group consisting of hydrogen atom, C₂₋₆ alkenyl, 3- to 12-membered cycloalkyl and 3- to 12-membered heterocyclyl, wherein the 3- to 12-membered cycloalkyl and 3- to 12-membered heterocyclyl are each independently and optionally substituted with one or more R⁶, and R⁶ is as defined in general formula (I'); in some embodiments, R^{B} is hydrogen atom or 3- to 10-membered heterocyclyl, wherein the 3- to 10-membered heterocyclyl is optionally substituted with one or more R⁶, and R⁶ is as defined in general formula (I'); in some embodiments, R^{B} is NR¹¹R¹², and R¹¹ and R¹² are as defined in general formula (I');
in some embodiments, R^{B} is selected from the group consisting of hydrogen atom, 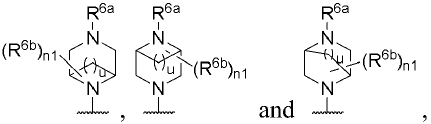 wherein R^{6a} is hydrogen atom or R⁶, R^{6b} is R⁶, and u is 0, 1 or 2; n1 is 0, 1, 2 or 3; R⁶ is as defined in general formula (I'); in some embodiments, R^{B} is hydrogen atom; in some embodiments, R^{B} is selected from the group consisting of hydrogen atom, 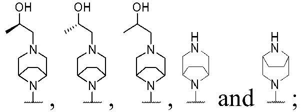 in some embodiments, R^{B} is selected from the group consisting of 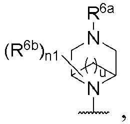 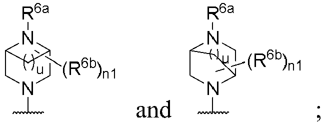 in some embodiments, R^{B} is 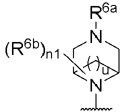 or 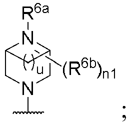 in some embodiments, R^{B} is hydrogen atom or 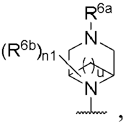 wherein u is 1 or 2, n1 is 0, 1 or 2, and R^{6a} and R^{6b} are as defined in general formula (II), (III), (IV) or (V); in some embodiments, R^{B} is 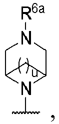 wherein u is 1 or 2, and R^{6a} is as defined in general formula (II), (III), (IV) or (V); in some embodiments, R^{B} is 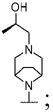 in some embodiments, R^{B} is 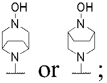
in some embodiments, R^{B} is 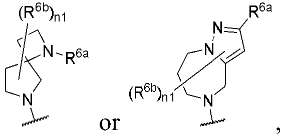 wherein R^{6a}, R^{6b} and n1 are as defined in general formula (III);
in some embodiments, R^{B} is selected from the group consisting of hydrogen atom, 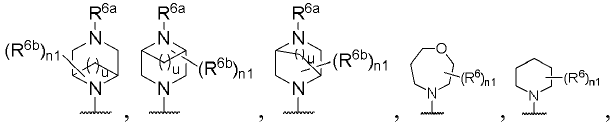 C₁₋₆ alkyl, C₂₋₆ alkenyl, C₂₋₆ alkynyl and 3- to 6-membered cycloalkyl, wherein the C₁₋₆ alkyl, C₂₋₆ alkenyl, C₂₋₆ alkynyl and 3- to 6-membered cycloalkyl are each independently and optionally substituted with one or more R⁶; R^{6a} is hydrogen atom or R⁶, R^{6b} is R⁶, and u is 0, 1 or 2; n1 is 0, 1, 2 or 3; R⁶ is as defined in general formula (I');
in some embodiments, R^{B} is selected from the group consisting of hydrogen atom, 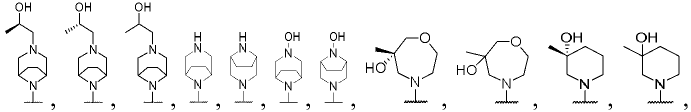 methyl, ethenyl, ethynyl, cyclopropyl and cyclobutyl;
in some embodiments, R^{B} is selected from the group consisting of hydrogen atom, C₁₋₆ alkyl, C₂₋₆ alkenyl, C₂₋₆ alkynyl, 3- to 6-membered cycloalkyl and 3- to 10-membered heterocyclyl, wherein the C₁₋₆ alkyl, C₂₋₆ alkenyl, C₂₋₆ alkynyl, 3- to 6-membered cycloalkyl and 3- to 10-membered heterocyclyl are each independently and optionally substituted with one or more substituents selected from the group consisting of halogen, cyano, hydroxyl, C₁₋₆ alkyl, C₁₋₆ alkoxy and 3- to 6-membered cycloalkyl; in some embodiments, R^{B} is selected from the group consisting of hydrogen atom, C₁₋₆ alkyl, C₂₋₆ alkenyl, C₂₋₆ alkynyl and 3- to 6-membered cycloalkyl; in some embodiments, R^{B} is selected from the group consisting of hydrogen atom, methyl, ethenyl, ethynyl, cyclopropyl and cyclobutyl;
in some embodiments, R^{B} is selected from the group consisting of hydrogen atom, 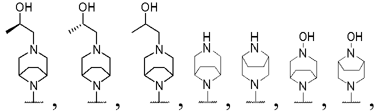 cyclopropyl, 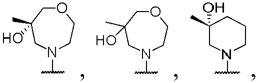 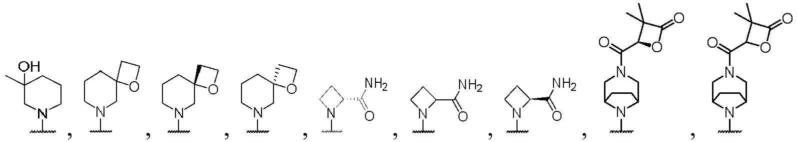 and 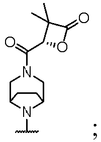 in some embodiments, R^{B} is selected from the group consisting of 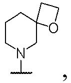 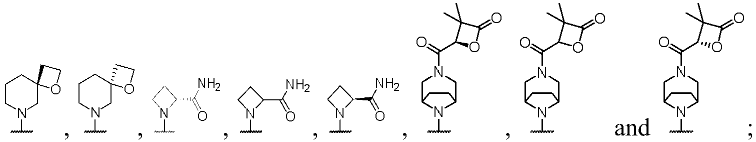
in some embodiments, R^{B} is selected from the group consisting of hydrogen atom, 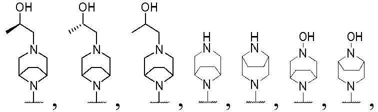 cyclopropyl, 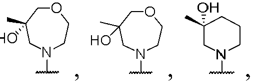 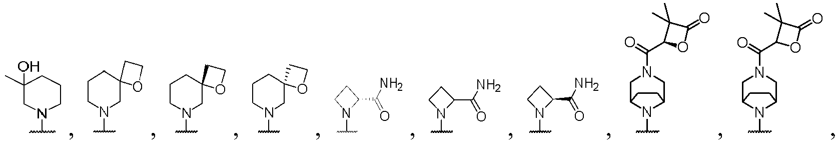 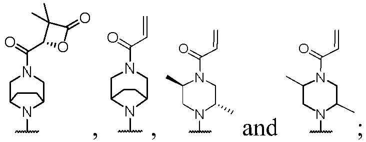 in some embodiments, R^{B} is selected from the group consisting of 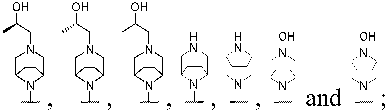 in some embodiments, R^{B} is selected from the group consisting of hydrogen atom, cyclopropyl, 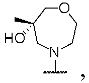 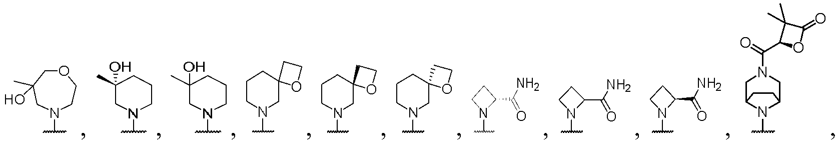 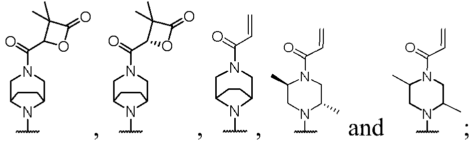
in some embodiments, R^{B} is selected from the group consisting of 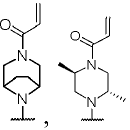 and

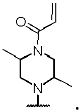

In some embodiments of the present disclosure, provided is the compound represented by general formula (I), (I') or (I'A) or the pharmaceutically acceptable salt thereof, which is a compound represented by general formula (IM) or a pharmaceutically acceptable salt thereof, 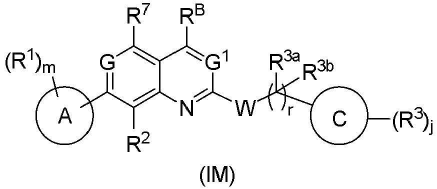 wherein
ring A, ring C, R¹, m, G, G¹, R², R⁷, R^{B}, W, R^{3a}, R^{3b}, r, R³ and j are as defined in general formula (I').

In some embodiments of the present disclosure, provided is the compound represented by general formula (I), (I') or (I'A) or the pharmaceutically acceptable salt thereof, which is a compound represented by general formula (IN) or a pharmaceutically acceptable salt thereof, 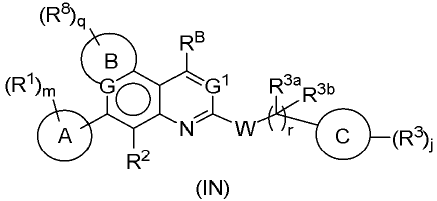 wherein, G is C or N; q is 0, 1, 2, 3 or 4;
ring A, ring B, ring C, R¹, m, R⁸, R², G¹, R^{B}, W, R^{3a}, R^{3b}, r, R³ and j are as defined in general formula (I').

In some embodiments of the present disclosure, provided is the compound represented by general formula (I), (I') or (I'A) or the pharmaceutically acceptable salt thereof, which is a compound represented by general formula (II) or a pharmaceutically acceptable salt thereof: 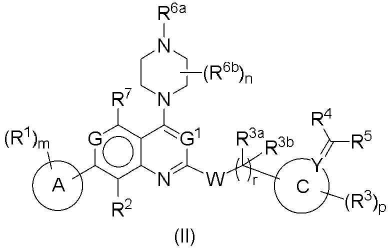
wherein, R^{6a} is hydrogen atom or R⁶;
R⁶⁶ is R⁶, or two R^{6b} groups, together with the carbon atoms to which they are attached, form CH(CR^{6c}R^{6d})ᵤCH;
R^{6c} and R^{6d} are the same or different, and are each independently hydrogen atom or R⁶;
n is 0, 1, 2, 3, 4, 5 or 6, and u is 0, 1 or 2;
ring A, ring C, R¹, m, G, G¹, R², R⁷, R⁶, W, R^{3a}, R^{3b}, r, Y, R³, R⁴, R⁵ and p are as defined in general formula (I').

In some embodiments of the present disclosure, provided is the compound represented by general formula (I), (I') or (I'A) or the pharmaceutically acceptable salt thereof, which is a compound represented by general formula (II) or a pharmaceutically acceptable salt thereof: 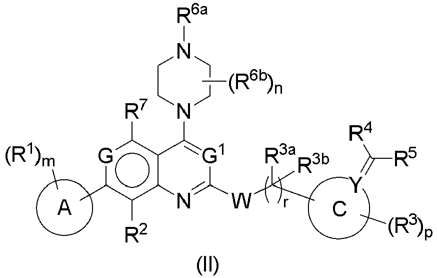 wherein
R^{6a} is selected from the group consisting of hydrogen atom, alkyl, cycloalkyl, cycloalkylalkyl, heterocyclyl, heterocyclylalkyl, aryl, heteroaryl and heteroarylalkyl, wherein the alkyl, cycloalkyl, cycloalkylalkyl, heterocyclyl, heterocyclylalkyl, aryl, heteroaryl and heteroarylalkyl are each independently and optionally substituted with one or more substituent(s) selected from the group consisiting of halogen, alkoxy, haloalkoxy, alkenyl, alkynyl, cyano, NR¹¹R¹², hydroxyl, hydroxyalkyl, OR¹⁴, C(O)R¹⁴, C(O)NR¹¹R¹², S(O)ᵥR¹⁴, S(O)ᵥNR¹¹R¹², =NR¹³, cycloalkyl, heterocyclyl, aryl and heteroaryl;
R^{6b} is R⁶, or two R^{6b} groups, together with the carbon atoms to which they are attached, form CH(CR^{6c}R^{6d})ᵤCH;
R^{6c} and R^{6d} are the same or different, and are each independently hydrogen atom or R⁶;
n is 0, 1, 2, 3, 4, 5 or 6, and u is 0, 1 or 2;
ring A, ring C, R¹, m, G, G¹, R², R⁷, R⁶, R¹¹ to R¹⁴, v, W, R^{3a}, R^{3b}, r, Y, R³, R⁴, R⁵ and p are as defined in general formula (I).

In some embodiments of the present disclosure, provided is the compound represented by general formula (I), (II), (I'), (I'A) or (IM) or the pharmaceutically acceptable salt thereof, which is a compound represented by general formula (III) or a pharmaceutically acceptable salt thereof: 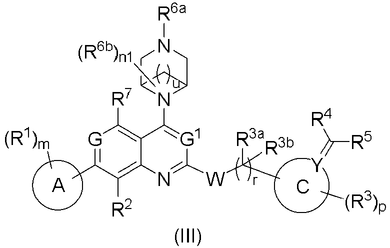 wherein
G is CR^{G} or N;
R⁷ and R^{G} are the same or different, and are each independently selected from the group consisting of hydrogen atom, halogen, alkyl, alkoxy, haloalkyl, haloalkoxy, cyano, hydroxyl, hydroxyalkyl, NR¹¹R¹², cycloalkyl, heterocyclyl, aryl and heteroaryl; n1 is 0, 1, 2 or 3;
ring A, ring C, R¹, m, G¹, R², R¹¹, R¹², R^{6a}, R^{6b}, u, W, R^{3a}, R^{3b}, r, Y, R³, R⁴, R⁵ and p are as defined in general formula (II).

In some embodiments of the present disclosure, provided is the compound represented by general formula (I), (II), (I'), (I'A) or (IN) or the pharmaceutically acceptable salt thereof, which is a compound represented by general formula (IV) or a pharmaceutically acceptable salt thereof: 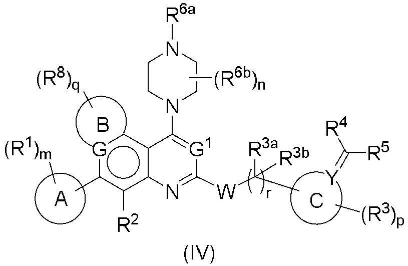 wherein
G is C or N; q is 0, 1, 2, 3 or 4;
ring A, ring B, ring C , R¹, m, R², R⁸, G¹, R^{6a}, R^{6b}, n, W, R^{3a}, R^{3b}, r, Y, R³, R⁴, R⁵ and p are as defined in general formula (II).

In some embodiments of the present disclosure, provided is the compound represented by general formula (I), (II), (IV), (I'), (I'A) or (IN) or the pharmaceutically acceptable salt thereof, which is a compound represented by general formula (V) or a pharmaceutically acceptable salt thereof: 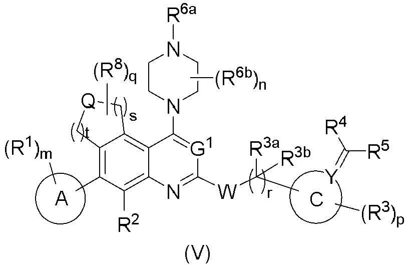 wherein
Q is selected from the group consisting of CR^{8a}R^{8b}, O, S, C(O), C(O)NR^{8c} and NR^{8c};
R^{8a} and R^{8b} are the same or different, and are each independently hydrogen atom or R⁸;
R^{8c} is selected from the group consisting of hydrogen atom, alkyl, haloalkyl, hydroxyalkyl, cycloalkyl and heterocyclyl;
s is 0, 1, 2 or 3; t is 0, 1, 2 or 3; q is 0, 1, 2, 3 or 4;
ring A, ring C, R¹, m, R², G¹, R⁸, R^{6a}, R^{6b}, n, W, R^{3a}, R^{3b}, r, Y, R³, R⁴, R⁵ and p are as defined in general formula (II).

In some embodiments of the present disclosure, provided is the compound represented by general formula (I), (I'), (I'A) or (II) or the pharmaceutically acceptable salt thereof, which is a compound represented by general formula (IIA) or a salt thereof (in some embodiments, a pharmaceutically acceptable salt thereof), 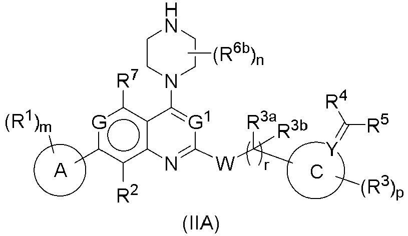 wherein
ring A, ring C, R¹, m, G, G¹, R², R⁷, R^{6b}, n, W, R^{3a}, R^{3b}, r, Y, R³, R⁴, R⁵ and p are as defined in general formula (II).

In some embodiments of the present disclosure, provided is the compound represented by general formula (I), (I'), (I'A), (II), (IM), (III) or (IIA) or the pharmaceutically acceptable salt thereof, which is a compound represented by general formula (IIIA) or a salt thereof (in some embodiments, a pharmaceutically acceptable salt thereof), 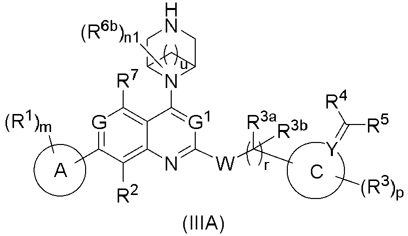 wherein
ring A, ring C, R¹, m, G, G¹, R², R⁷, R^{6b}, n1, u, W, R^{3a}, R^{3b}, r, Y, R³, R⁴, R⁵ and p are as defined in general formula (III).

In some embodiments of the present disclosure, provided is the compound represented by general formula (I), (I'), (I'A), (IN), (II), (IV) or (IIA) or the pharmaceutically acceptable salt thereof, which is a compound represented by general formula (IVA) or a salt thereof (in some embodiments, a pharmaceutically acceptable salt thereof), 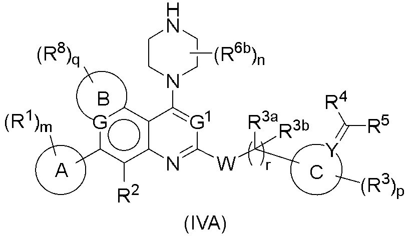 wherein
ring A, ring B, ring C, R¹, m, G, G¹, R², R⁸, q, R^{6b}, n, W, R^{3a}, R^{3b}, r, Y, R³, R⁴, R⁵ and p are as defined in general formula (IV).

In some embodiments of the present disclosure, provided is the compound represented by general formula (I), (I'), (I'A), (IN), (II), (IV), (V), (IIA) or (IVA) or the pharmaceutically acceptable salt thereof, which is a compound represented by general formula (VA) or a salt thereof (in some embodiments, a pharmaceutically acceptable salt thereof), 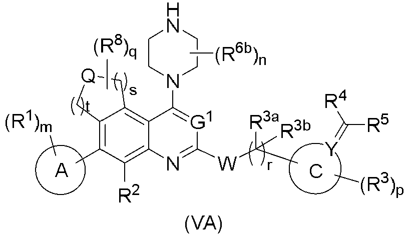 wherein
ring A, ring C, R¹, m, Q, s, t, R⁸, q, R², G¹, R^{6b}, n, W, R^{3a}, R^{3b}, r, Y, R³, R⁴, R⁵ and p are as defined in general formula (V).

In some embodiments of the present disclosure, provided is the compound represented by general formula (I), (I'A) or (I') or the pharmaceutically acceptable salt thereof, which is a compound represented by general formula (VI) or a pharmaceutically acceptable salt thereof, 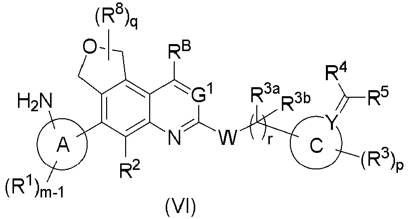
wherein, q is 0, 1, 2, 3 or 4; m is 1, 2, 3, 4, 5 or 6;
ring A, ring C, R¹, R⁸, R², G¹, R^{B}, W, R^{3a}, R^{3b}, r, Y, R³, R⁴, R⁵ and p are as defined in general formula (I).

In some embodiments of the present disclosure, provided is the compound represented by general formula (I), (I'), (I'A) or (VI) or the pharmaceutically acceptable salt thereof, which is a compound represented by general formula (VII-1) or (VII-2) or a pharmaceutically acceptable salt thereof, 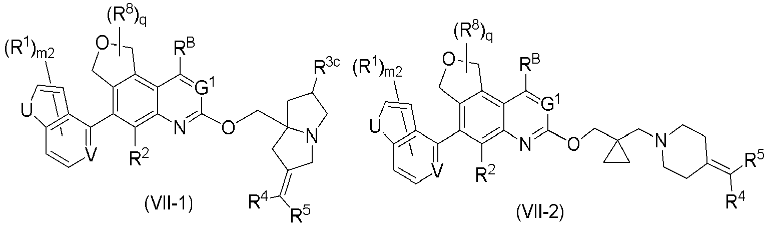 wherein
U is selected from the group consisting of NR^{1a}, O, S and Se; V is N or CR^{1b};
R^{1a} is hydrogen atom or R¹; R¹⁶ is hydrogen atom or R¹; R^{3c} is hydrogen atom or R³;
m2 is 0, 1, 2, 3 or 4; q is 0, 1, 2, 3 or 4;
R¹, R⁸, R², G¹, R^{B}, R³, R⁴ and R⁵ are as defined in general formula (I).

In some embodiments of the present disclosure, provided is the compound represented by any of general formulae (I) to (VII-2) or the pharmaceutically acceptable salt thereof, wherein U is S or Se; in some embodiments, U is S; in some embodiments, U is Se.

In some embodiments of the present disclosure, provided is the compound represented by any of general formulae (I) to (VII-2) or the pharmaceutically acceptable salt thereof, wherein V is N or CR^{1b}, and R^{1b} is selected from the group consisting of hydrogen atom, halogen, C₁₋₆ alkyl and C₁₋₆ haloalkyl; in some embodiments, V is CH or N; in some embodiments, V is CH; in some embodiments, V is N.

In some embodiments of the present disclosure, provided is the compound represented by any of general formulae (I) to (VII-2) or the pharmaceutically acceptable salt thereof, wherein U is S or Se, and/or V is CH or N; in some embodiments, U is S, and/or V is CH or N; in some embodiments, U is S, and/or V is CH; in some embodiments, U is S, and/or V is N.

In some embodiments of the present disclosure, provided is the compound represented by any of general formulae (I) to (VII-2) or the pharmaceutically acceptable salt thereof, wherein m2 is 2 or 3; in some embodiments, m2 is 3.

In some embodiments of the present disclosure, provided is the compound represented by general formula (I'A) or the pharmaceutically acceptable salt thereof, wherein ring D is aryl or heteroaryl; in some embodiments, ring D is 5- to 10-membered heteroaryl.

In some embodiments of the present disclosure, provided is the compound represented by general formula (I') or the pharmaceutically acceptable salt thereof, wherein G⁸ is CR⁷, and R⁷ is as defined in general formula (I'); in some embodiments, G⁸ is N.

In some embodiments of the present disclosure, provided is the compound represented by general formula (I') or the pharmaceutically acceptable salt thereof, wherein G⁹ is CR², and R² is as defined in general formula (I'); in some embodiments, G⁹ is CF.

In some embodiments of the present disclosure, provided is the compound represented by general formula (I), (I'), (I'A), (II) or (IIA) or the pharmaceutically acceptable salt thereof, wherein when R⁷ and G do not form a ring, R^{6a} is not hydrogen atom.

In some embodiments of the present disclosure, provided is the compound represented by general formula (III) or (IIIA) or the pharmaceutically acceptable salt thereof, wherein R^{6a} is not hydrogen atom.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R^{6a} is hydrogen atom or C₁₋₆ alkyl, wherein the C₁₋₆ alkyl is optionally substituted with one or more substituents selected from the group consisting of halogen, OR¹⁴ and 3-to 6-membered heterocyclyl; R¹⁴ is as defined in general formula (I); in some embodiments, R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; in some embodiments, R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; in some embodiments, R^{6a} is selected from the group consisting of hydrogen atom, hydroxyl and C₁₋₆ hydroxyalkyl; in some embodiments, R^{6a} is C₁₋₆ hydroxyalkyl;
in some embodiments, R^{6a} is selected from the group consisting of hydrogen atom, methyl, ethyl, 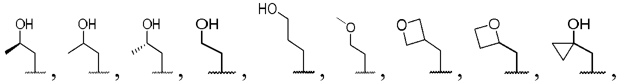 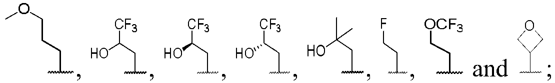 in some embodiments, R^{6a} is selected from the group consisting of methyl, ethyl, 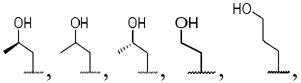 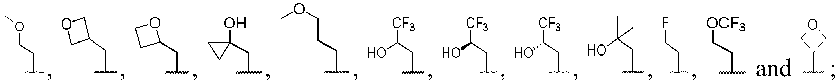 in some embodiments, R^{6a} is selected from the group consisting of 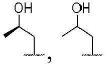 and 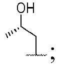 in some embodiments, R^{6a} is 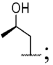
in some embodiments, R^{6a} is selected from the group consisting of hydrogen atom, C₁₋₆ alkyl, OR¹⁴ and C(O)R¹⁴, wherein the C₁₋₆ alkyl is optionally substituted with one or more substituents selected from the group consisting of halogen, OR¹⁴ and 3- to 6-membered heterocyclyl; R¹⁴ is as defined in general formula (I'); in some embodiments, R^{6a} is C(O)R¹⁴, and R¹⁴ is as defined in general formula (I'); in some embodiments, R^{6a} is OR¹⁴, and R¹⁴ is as defined in general formula (I');
in some embodiments, R^{6a} is selected from the group consisting of hydrogen atom, C₁₋₆ alkyl and OR¹⁴, wherein the C₁₋₆ alkyl is optionally substituted with one or more substituents selected from the group consisting of halogen, hydroxyl, C₁₋₆ alkoxy and 3- to 6-membered heterocyclyl; R¹⁴ is selected from the group consisting of hydrogen atom, C₁₋₆ alkyl and 3- to 6-membered cycloalkyl; in some embodiments, R^{6a} is hydrogen atom or OR¹⁴, and R¹⁴ is selected from the group consisting of hydrogen atom, C₁₋₆ alkyl and 3- to 6-membered cycloalkyl; in some embodiments, R^{6a} is hydrogen atom or OH; in some embodiments, R^{6a} is selected from the group consisting of hydrogen atom, 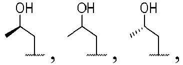 OH, 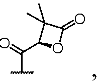 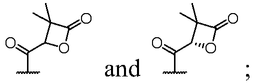 in some embodiments, R^{6a} is selected from the group consisting of 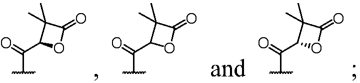 in some embodiments, R^{6a} is selected from the group consisting of hydrogen atom, 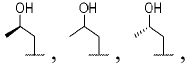 OH, 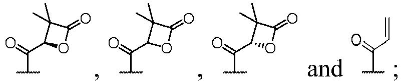 in some embodiments, R^{6a} is

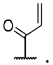

In some embodiments of the present disclosure, provided is the compound represented by general formula (I), (II), (IV), (V), (I'), (I'A), (IM), (IN), (IIA), (IIIA), (IVA), (VA), (VI), (VII-1) or (VII-2) or the pharmaceutically acceptable salt thereof, wherein 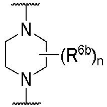 is selected from the group consisting of 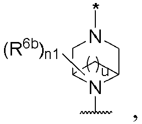 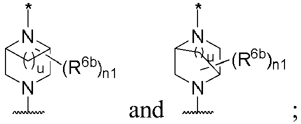 in some embodiments, 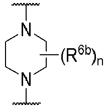 is 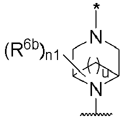 or 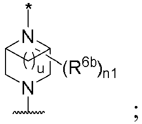 in some embodiments, 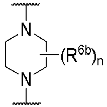 is 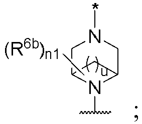 in some embodiments, 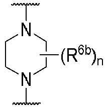 is 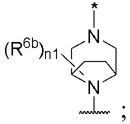 n1 is 0, 1, 2 or 3, R^{6b} is R⁶, and R⁶ and u are as defined in general formula (II); in some embodiments, 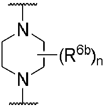 is 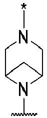 or 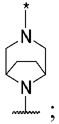 in some embodiments, 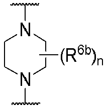 is 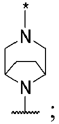 the * end is connected to R^{6a}
in some embodiments, 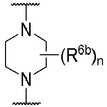 is selected from the group consisting of 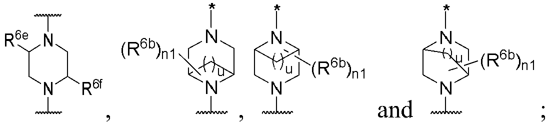 R^{6e} and R^{6f} are the same or different, and are each independently hydrogen atom or R⁶; R⁶⁶ is R⁶, n1 is 0, 1, 2 or 3, and R⁶ and u are as defined in general formula (II);
in some embodiments, 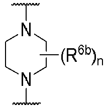 is selected from the group consisting of 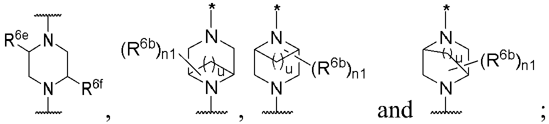 R^{6e} and R^{6f} are the same or different, and are each independently selected from the group consisting of hydrogen atom, halogen and C₁₋₆ alkyl, and each R^{6b} is the same or different, and is independently halogen or C₁₋₆ alkyl; n1 is 0, 1, 2 or 3; the * end is connected to R^{6a}.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein u is 1 or 2; in some embodiments, u is 2.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein each R^{6b} is the same or different, and is independently selected from the group consisting of halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, cyano, -NR¹¹R¹², hydroxyl and C₁₋₆ hydroxyalkyl; in some embodiments, each R^{6b} is the same or different, and is independently halogen or C₁₋₆ alkyl; in some embodiments, each R^{6b} is the same or different, and is independently halogen; in some embodiments, R^{6b} is F; in some embodiments, each R^{6b} is the same or different, and is independently C₁₋₆ alkyl; in some embodiments, R^{6b} is methyl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R^{6e} and R^{6f} are the same or different, and are each independently C₁₋₆ alkyl; in some embodiments, both R^{6e} and R^{6f} are methyl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein n is 2, 3, 4, 5 or 6; in some embodiments, n is 2, 3 or 4; in some embodiments, n is 2.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein n1 is 0, 1 or 2; in some embodiments, n1 is 0; in some embodiments, n1 is 2.

In some embodiments of the present disclosure, provided is the compound represented by general formula (I), (II), (I'), (I'A) or (IIA) or the pharmaceutically acceptable salt thereof, wherein R⁷ is selected from the group consisting of hydrogen atom, halogen, C₁₋₆ alkyl and C₁₋₆ haloalkyl, or R⁷ and G together form ring B, wherein ring B is optionally substituted with one or more R⁸; in some embodiments, R⁷ is hydrogen atom, or R⁷ and G together form ring B, wherein ring B is optionally substituted with one or more R⁸, and ring B and R⁸ are as defined in general formula (I); in some embodiments, R⁷ is selected from the group consisting of hydrogen atom, halogen, C₁₋₆ alkyl, C₁₋₆ haloalkyl, C₁₋₆ alkoxy, 3- to 6-membered cycloalkyloxy and 3- to 6-membered heterocyclyloxy; in some embodiments, R⁷ is hydrogen atom; in some embodiments, R⁷ and G together form ring B, wherein ring B is optionally substituted with one or more R⁸, and ring B and R⁸ are as defined in general formula (I); in some embodiments, R⁷ and G together form ring B, and ring B is as defined in general formula (I).

In some embodiments of the present disclosure, provided is the compound represented by general formula (I), (II), (IV), (I'), (I'A), (IN), (IIA) or (IVA) or the pharmaceutically acceptable salt thereof, wherein ring B is 5- or 6-membered heteroaryl or 4- to 7-membered heterocyclyl; in some embodiments, ring B is 5- or 6-membered heterocyclyl or 5-membered heteroaryl; in some embodiments, ring B is 5- or 6-membered heterocyclyl; in some embodiments, ring B is hydrogenated furyl; in some embodiments, ring B is 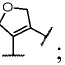 in some embodiments, ring B is 5- or 6-membered heteroaryl; in some embodiments, ring B is 5-membered heteroaryl; in some embodiments, ring B is selected from the group consisting of pyrrolyl, pyrazolyl, imidazolyl and thiazolyl; in some embodiments, ring B is pyrazolyl or imidazolyl; in some embodiments, ring B is selected from the group consisting of 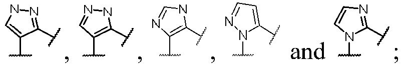 in some embodiments, ring B is 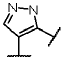 or

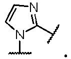

In some embodiments of the present disclosure, provided is the compound represented by general formula (I), (II), (IV), (V), (I'), (I'A), (IN), (IIA), (IVA), (VA), (VI), (VII-1) or (VII-2) or the pharmaceutically acceptable salt thereof, wherein each R⁸ is the same or different, and is independently selected from the group consisting of halogen, C₁₋₆ alkyl, C₁₋₆ haloalkyl and cyano; in some embodiments, each R⁸ is the same or different, and is independently selected from the group consisting of halogen or C₁₋₆ alkyl; in some embodiments, R⁸ is methyl.

In some embodiments of the present disclosure, provided is the compound represented by general formula (I), (II), (IV), (V), (I'), (I'A), (IN), (IIA), (IVA), (VA), (VI), (VII-1) or (VII-2) or the pharmaceutically acceptable salt thereof, wherein q is 0, 1 or 2; in some embodiments, q is 0; in some embodiments, q is 1.

In some embodiments of the present disclosure, provided is the compound represented by general formula (I), (II), (IV), (V), (I'), (I'A), (IN), (IIA), (IVA), (VA), (VI), (VII-1) or (VII-2) or the pharmaceutically acceptable salt thereof, wherein each R⁸ is the same or different, and is independently selected from the group consisting of halogen, C₁₋₆ alkyl, C₁₋₆ haloalkyl and cyano; and/or q is 0, 1 or 2.

In some embodiments of the present disclosure, provided is the compound as represented by any of general formulae (I) to (III), (I'), (I'A), (IM), (IIA) and (IIIA) or the pharmaceutically acceptable salt thereof, wherein R⁷ is selected from the group consisting of hydrogen atom, C₁₋₆ alkyl, C₁₋₆ alkoxy, 3- to 6-membered cycloalkyloxy and 3- to 6-membered heterocyclyloxy; in some embodiments, R⁷ is selected from the group consisting of hydrogen atom, halogen, C₁₋₆ alkyl and C₁₋₆ haloalkyl; in some embodiments, R⁷ is hydrogen atom or halogen; in some embodiments, R⁷ is hydrogen atom.

In some embodiments of the present disclosure, provided is the compound represented by any of general formulae (I) to (VII-2), (I'), (I'A), (IIA), (IIIA), (IVA) and (VA) or the pharmaceutically acceptable salt thereof, wherein R⁴ and R⁵ are the same or different, and are each independently selected from the group consisting of hydrogen atom, halogen, C₁₋₆ alkyl and C₁₋₆ haloalkyl, or R⁴ and R⁵, together with the carbon atoms to which they are attached, form 3- to 6-membered cycloalkyl;
in some embodiments, R⁴ and R⁵ are the same or different, and are each independently selected from the group consisting of hydrogen atom, halogen and C₁₋₆ alkyl; in some embodiments, R⁴ and R⁵ are the same or different, and are each independently hydrogen atom or halogen; in some embodiments, R⁴ and R⁵ are the same or different, and are each independently hydrogen atom or F; in some embodiments, R⁴ is hydrogen atom and R⁵ is halogen, or R⁵ is hydrogen atom, and R⁴ is halogen; in some embodiments, R⁴ and R⁵ are the same or different, and are each independently halogen; in some embodiments, R⁴ and R⁵ are F.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein W is selected from the group consisting of CH₂, NH and O; in some embodiments, W is O.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein ring C is 3- to 14-membered heterocyclyl or 3- to 14-membered cycloalkyl; in some embodiments, ring C is 3- to 8-membered heterocyclyl or 3- to 8-membered cycloalkyl; in some embodiments, ring C is 3- to 8-membered heterocyclyl.

In some embodiments of the present disclosure, provided is the compound represented by general formula (I'), (I'A), (IM) or (IN) or the pharmaceutically acceptable salt thereof, wherein j is 0, 1, 2 or 3; in some embodiments, j is 1 or 2; in some embodiments, j is 1; in some embodiments, j is 2.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein ring C is 3- to 8-membered heterocyclyl or 3- to 8-membered cycloalkyl; and/or each R³ is the same or different, and is independently selected from the group consisting of C₁₋₆ alkyl, C₁₋₆ haloalkyl, halogen, -C₁₋₆ alkylene-NR¹¹R¹² and =CR¹⁵R¹⁶, wherein R¹¹, R¹², R¹⁵ and R¹⁶ are as defined in general formula (I); in some embodiments, ring C is 3- to 8-membered heterocyclyl; and/or each R³ is the same or different, and is independently selected from the group consisting of C₁₋₆ alkyl, C₁₋₆ haloalkyl, halogen and =CR¹⁵R¹⁶, wherein R¹⁵ and R¹⁶ are the same or different, and are each independently selected from the group consisting of hydrogen atom, halogen and C₁₋₆ alkyl; in some embodiments, ring C is 3- to 8-membered heterocyclyl; and/or each R³ is the same or different, and is independently halogen or =CR¹⁵R¹⁶, and R¹⁵ and R¹⁶ are the same or different, and are each independently hydrogen atom or halogen.

In some embodiments of the present disclosure, provided is the compound represented by general formula (I'), (I'A), (IM), (IN), (IIA), (IIIA), (IVA) or (VA) or the pharmaceutically acceptable salt thereof, wherein ring C is 3- to 8-membered heterocyclyl or 3- to 8-membered cycloalkyl; and/or each R³ is the same or different, and is independently selected from the group consisting of C₁₋₆ alkyl, C₁₋₆ haloalkyl, halogen, -C₁₋₆ alkylene-NR¹¹R¹² and =CR¹⁵R¹⁶, and/or j is 0, 1, 2 or 3, wherein R¹¹, R¹², R¹⁵ and R¹⁶ are as defined in general formula (I); in some embodiments, ring C is 3- to 8-membered heterocyclyl; and/or each R³ is the same or different, and is independently selected from the group consisting of C₁₋₆ alkyl, C₁₋₆ haloalkyl, halogen and =CR¹⁵R¹⁶, wherein R¹⁵ and R¹⁶ are the same or different, and are each independently selected from the group consisting of hydrogen atom, halogen and C₁₋₆ alkyl, and/or j is 1 or 2; in some embodiments, ring C is 3- to 8-membered heterocyclyl; and/or each R³ is the same or different, and is independently halogen or =CR¹⁵R¹⁶, wherein R¹⁵ and R¹⁶ are the same or different, and are each independently hydrogen atom or halogen, and/or j is 1 or 2.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein 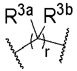 is -CH₂- or

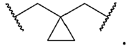

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein both R^{3a} and R^{3b} are hydrogen atoms, or R^{3a} and R^{3b}, together with the carbon atoms to which they are attached, form 3- to 6-membered cycloalkyl; in some embodiments, both R^{3a} and R^{3b} are hydrogen atom, or R^{3a} and R^{3b}, together with the carbon atoms to which they are attached, form cyclopropyl; in some embodiments, both R^{3a} and R^{3b} are hydrogen atom, or R^{3a} and R^{3b}, together with the same carbon atom to which they are attached, form cyclopropyl; in some embodiments, both R^{3a} and R^{3b} are hydrogen atoms.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein r is 1 or 3.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein both R^{3a} and R^{3b} are hydrogen atoms, or R^{3a} and R^{3b}, together with the carbon atoms to which they are attached, form cyclopropyl, and/or r is 1 or 3.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein W is O, and/or both R^{3a} and R^{3b} are hydrogen atoms, or R^{3a} and R^{3b}, together with the carbon atoms to which they are attached, form cyclopropyl, and/or r is 1 or 3.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein ring C is selected from the group consisting of cyclopropyl, azetidine, morpholinyl, 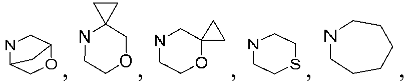 pyrrolidyl, piperidyl,

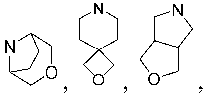

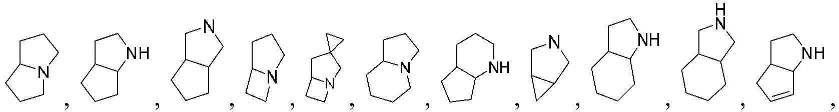

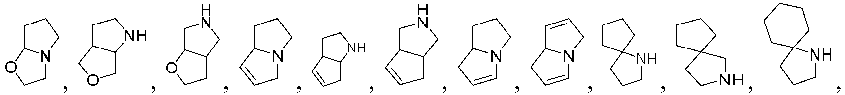

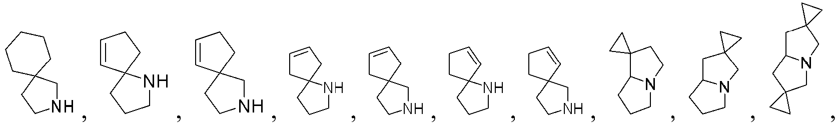

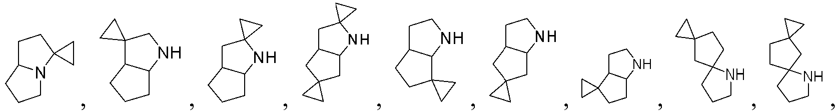

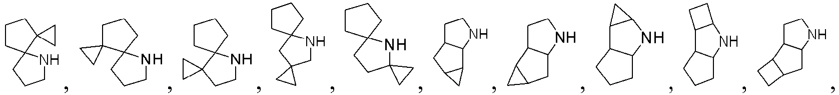

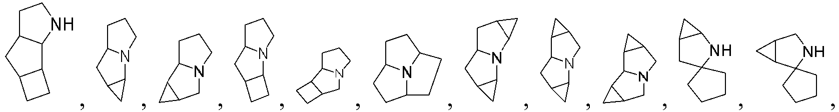

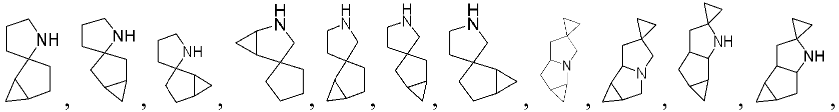

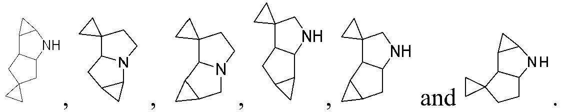

In some embodiments of the present disclosure, provided is the compound represented by any of general formulae (I) to (V), (I'), (I'A), (IM), (IN), (IIA), (IIIA), (IVA) and (VA) or the pharmaceutically acceptable salt thereof, wherein ring C is selected from the group consisting of 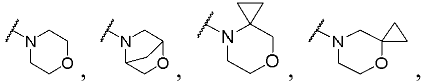 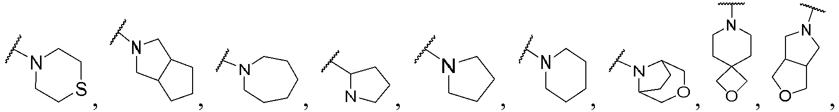 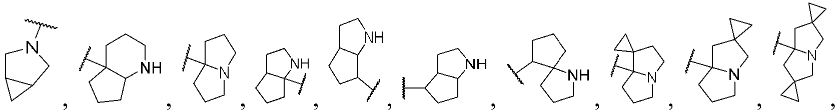 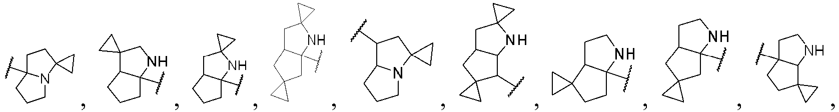 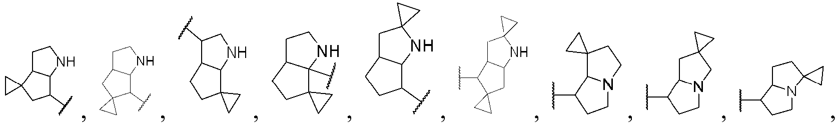 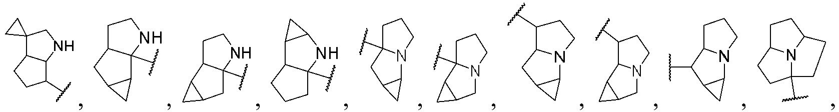 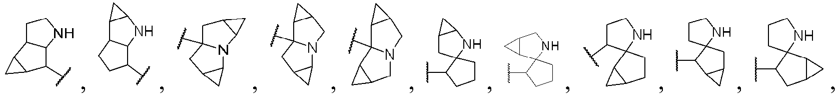 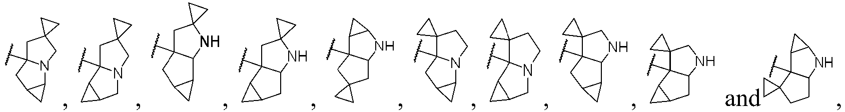 and the 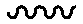 end is connected to CR^{3a}R^{3b}.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein 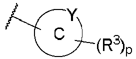 is selected from the group consisting of 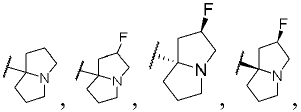 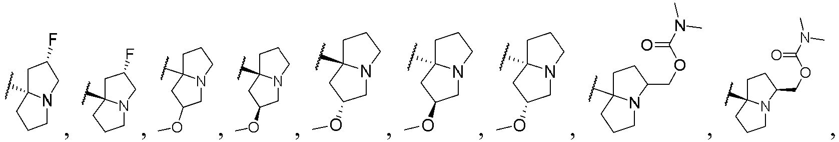 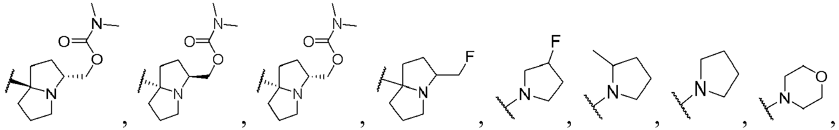 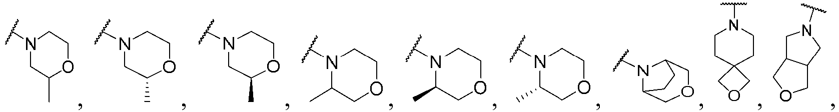 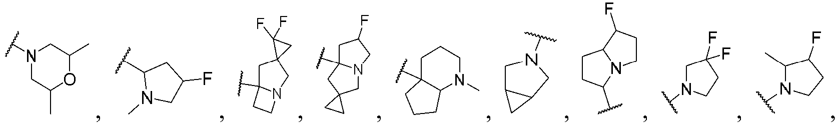 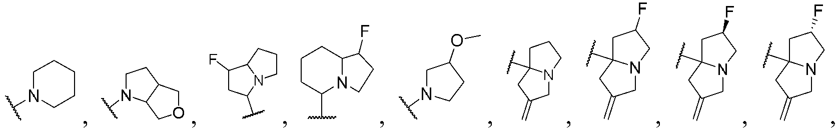 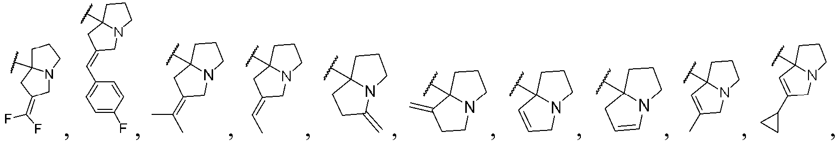 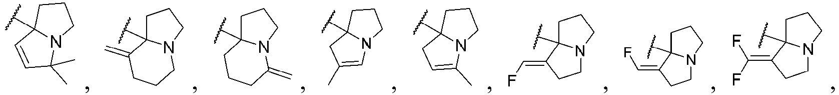 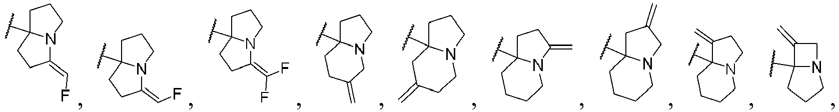 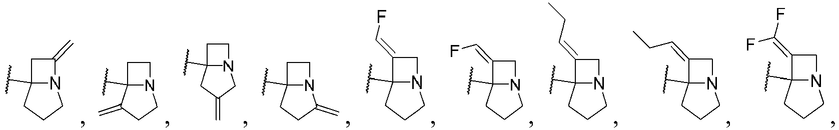 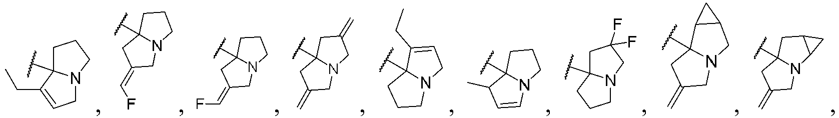 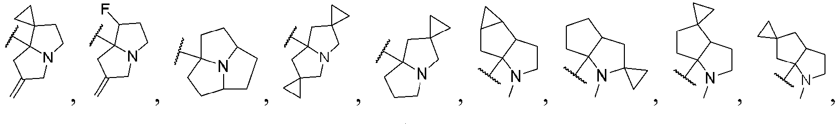 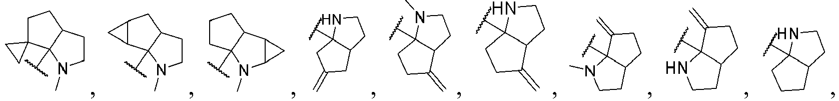 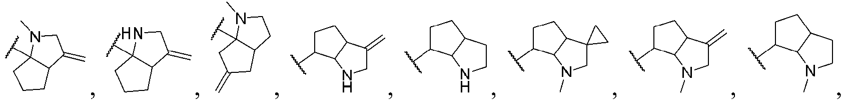 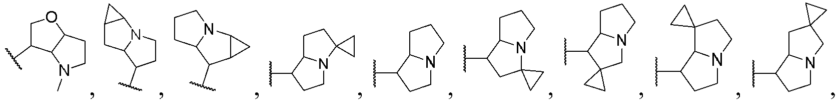 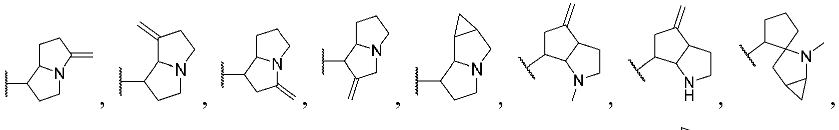 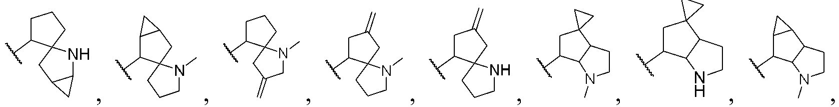 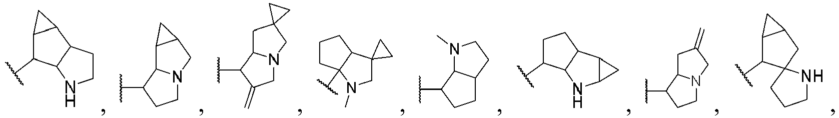 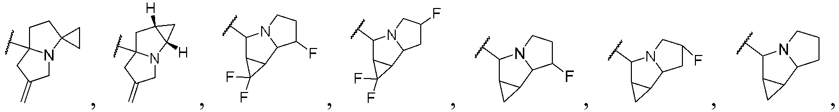 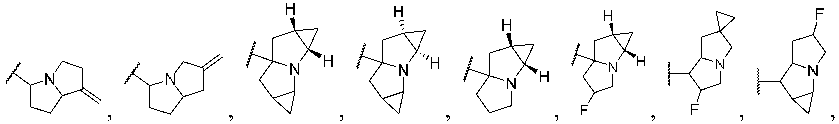 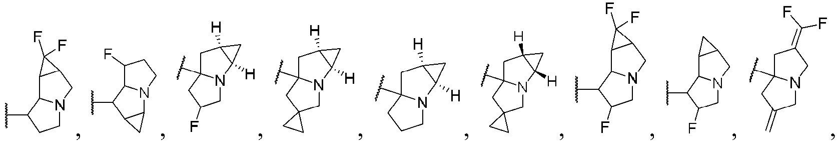 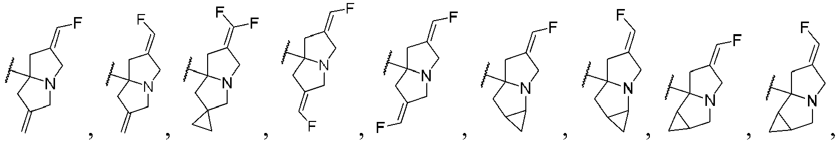 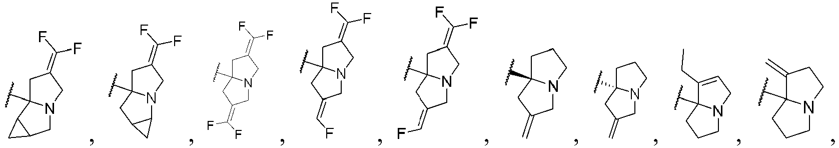 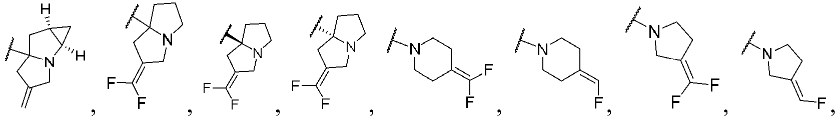 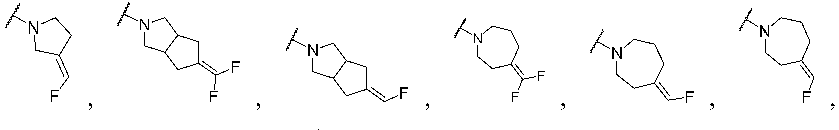 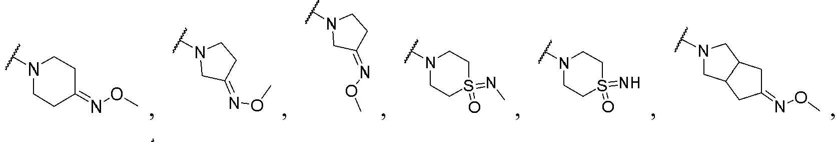 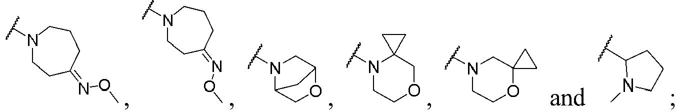 the 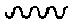 end is connected to CR^{3a}R^{3b}.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein ring C is selected from the group consisting of cyclopropyl, azetidine, 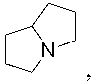 morpholinyl, 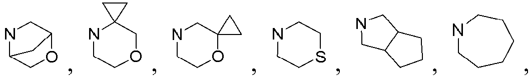 pyrrolidyl and piperidyl; in some embodiments, ring C is selected from the group consisting of cyclopropyl, azetidine, 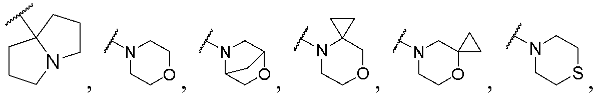 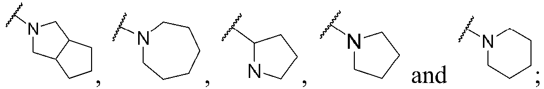 in some embodiments, ring C is selected from the group consisting of 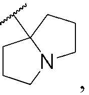 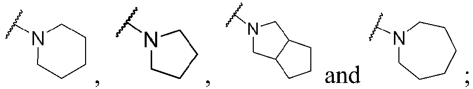 in some embodiments, ring C is selected from the group consisting of 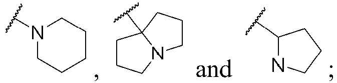 in some embodiments, ring C is 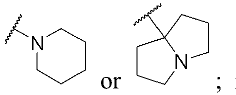 in some embodiments, ring C is 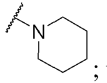 the 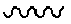 end is connected to CR^{3a}R^{3b}.

In some embodiments of the present disclosure, provided is the compound represented by any of general formulae (I) to (VI), (I'), (I'A), (IIA), (IIIA), (IVA) and (VA) or the pharmaceutically acceptable salt thereof, wherein 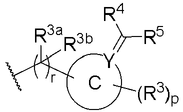 is selected from the group consisting of 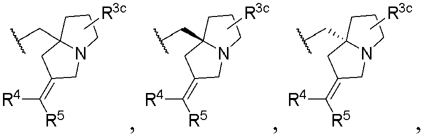 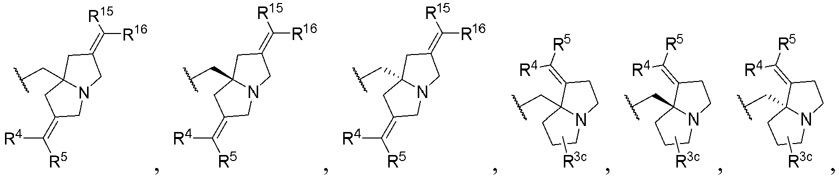 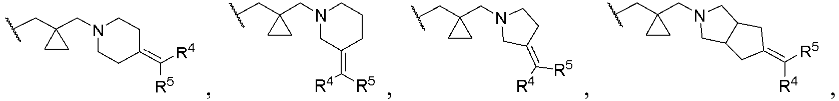 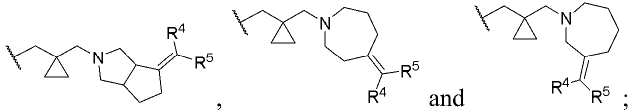
in some embodiments, 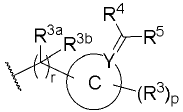 is selected from the group consisting of 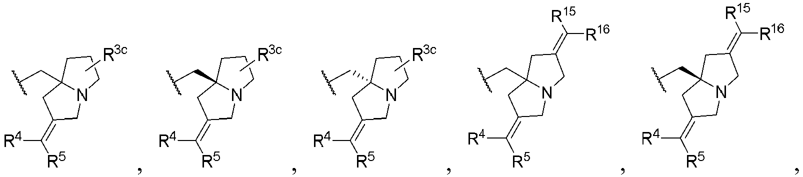 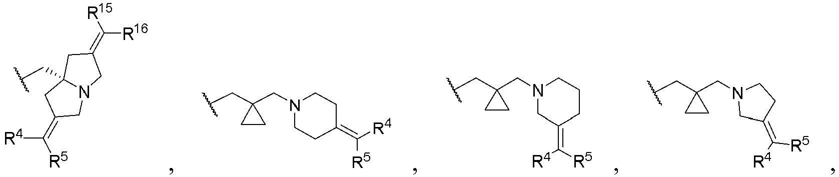 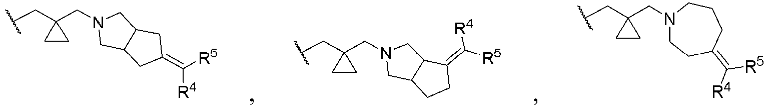 and 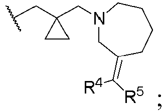 in some embodiments, 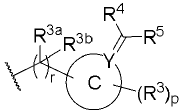 is selected from the group consisting of 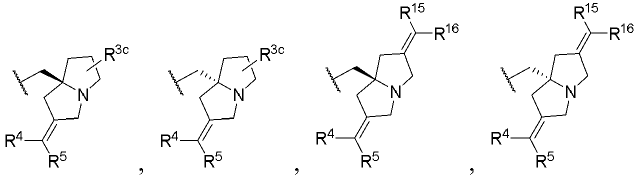 and 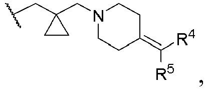 and R^{3c} is selected from the group consisting of hydrogen atom, halogen, alkyl, alkenyl, alkynyl, oxo, alkoxy, alkyl-S-, haloalkyl, haloalkoxy, cyano, NR¹¹R¹², C(O)NR¹¹R¹², S(O)₂NR¹¹R¹², -C(O) alkyl, -S(O)₂ alkyl, =CR¹⁵R¹⁶, -NR¹³, hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R*; R⁴, R⁵, R¹¹, R¹², R¹³, R¹⁵, R¹⁶ and R* are as defined in general formula (I);
in some embodiments, 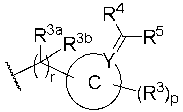 is selected from the group consisting of 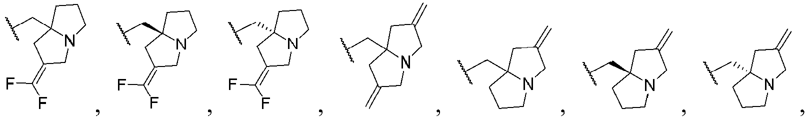 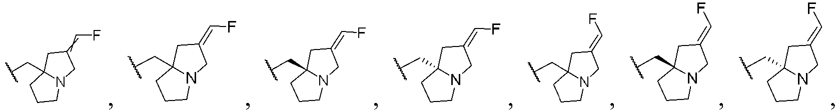 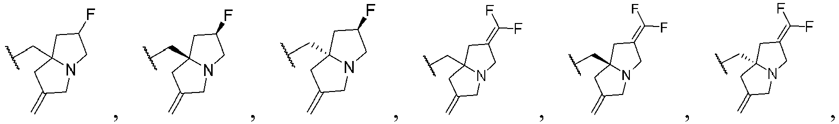 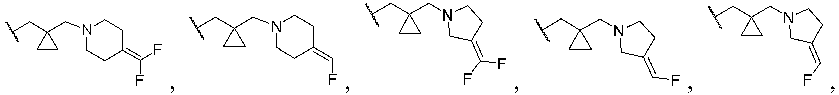 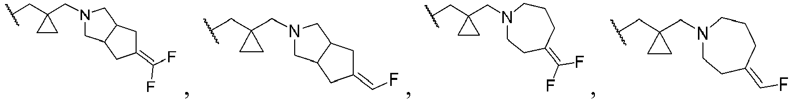 and 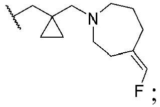 in some embodiments, 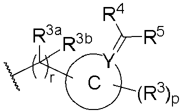 is selected from the group consisting of 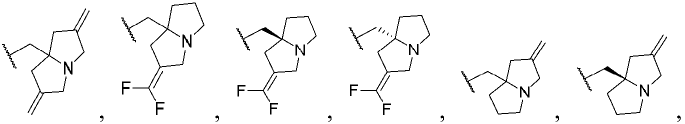 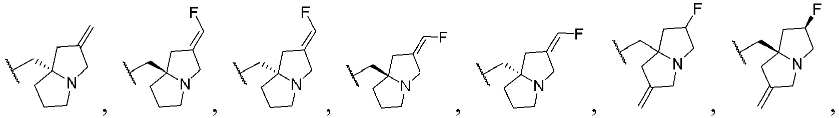 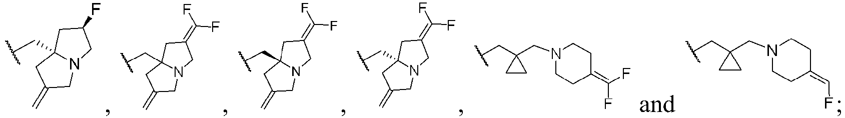 in some embodiments, 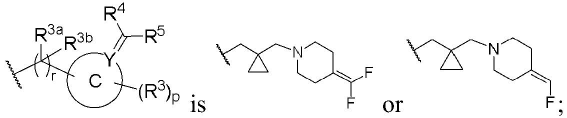
in some embodiments, 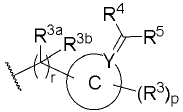 is selected from the group consisting of 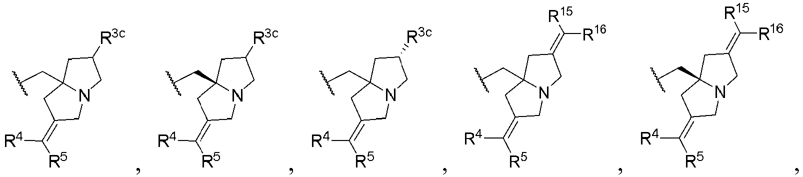 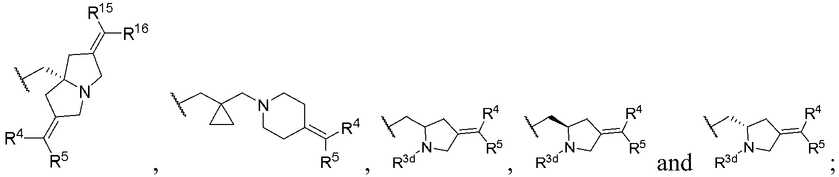 in some embodiments, 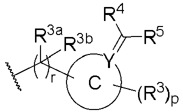 is selected from the group consisting of 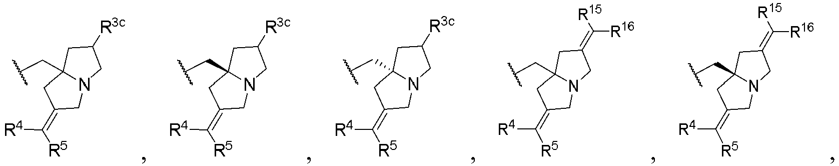 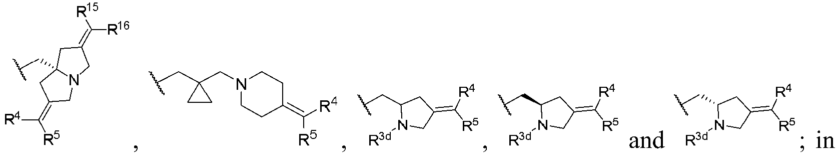 some embodiments, 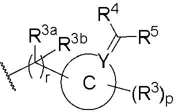 is selected from the group consisting of 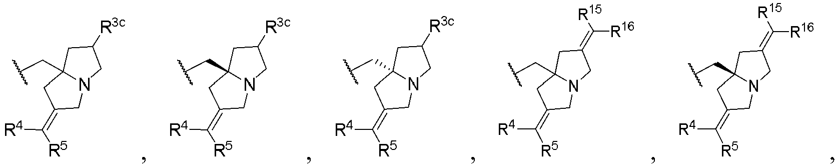 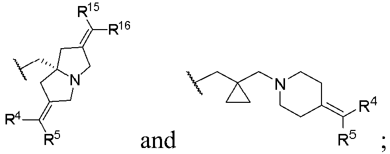 in some embodiments, 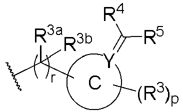 is selected from the group consisting of 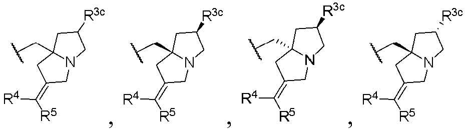 and 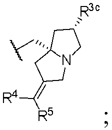 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen); in some embodiments, provided is 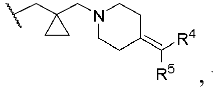 wherein R⁴ and R⁵ are as defined in general formula (I).

In some embodiments of the present disclosure, provided is the compound represented by any of general formulae (I) to (VI), (I'), (I'A), (IIA), (IIIA), (IVA) and (VA) or the pharmaceutically acceptable salt thereof, wherein 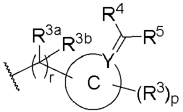 is selected from the group consisting of

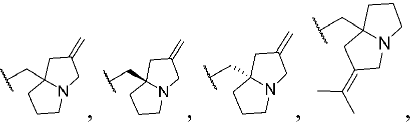

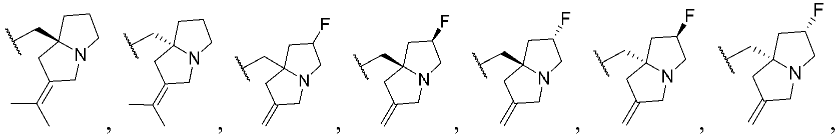

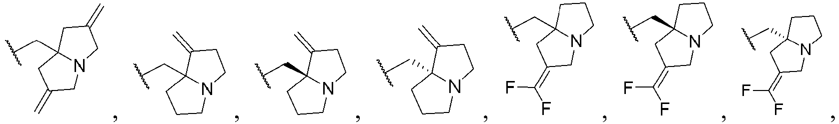

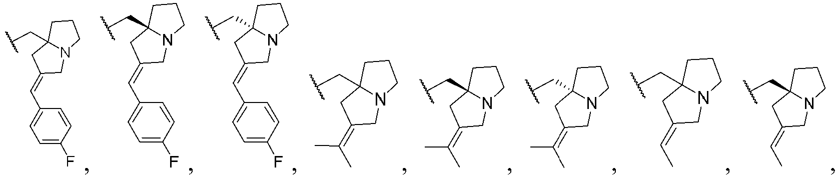

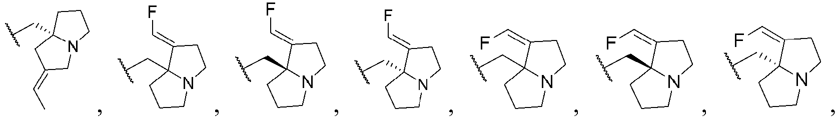

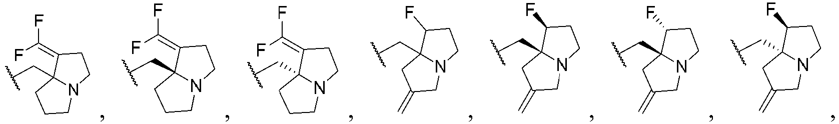

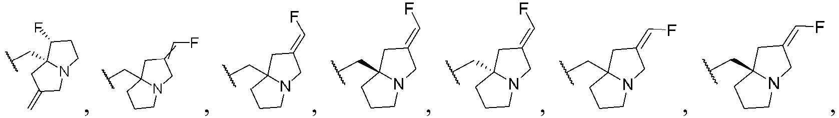

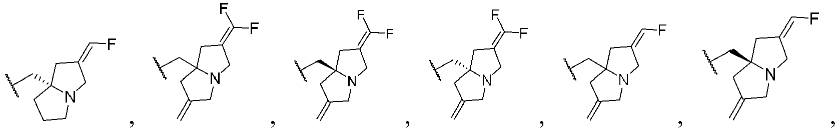

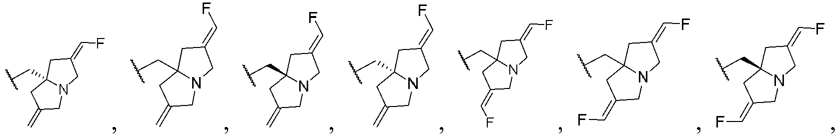

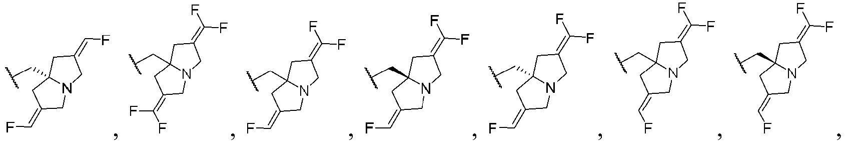

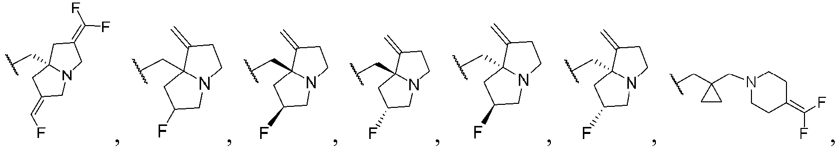

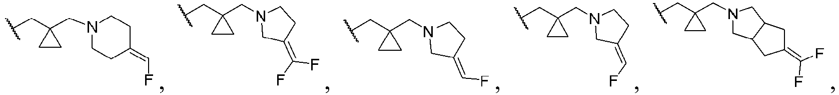

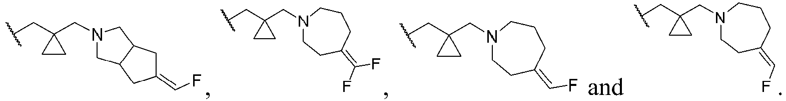

In some embodiments of the present disclosure, provided is the compound represented by general formula (I'), (I'A), (IM) or (IN) or the pharmaceutically acceptable salt thereof, wherein 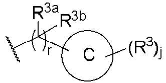 is selected from the group consisting of 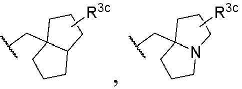 (in some embodiments, 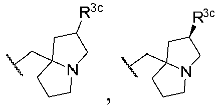 and 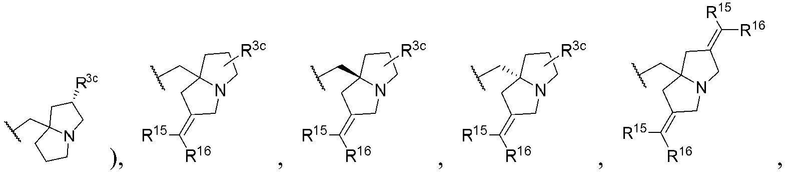 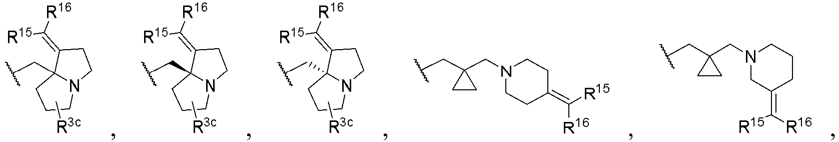 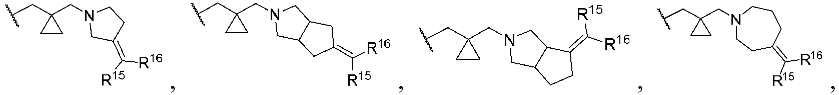 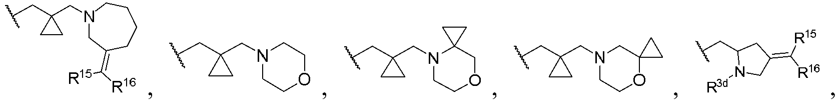 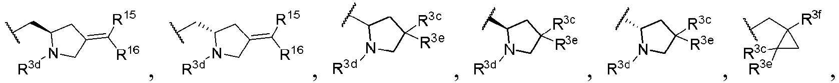 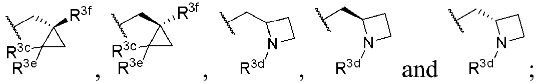 R^{3c} is hydrogen atom or R³, R^{3d} is selected from the group consisting of hydrogen atom, C₁₋₆ alkyl and C₁₋₆ haloalkyl, R^{3f} is hydrogen atom or R³, and R³, R¹⁵ and R¹⁶ are as defined in general formula (I');
in some embodiments, 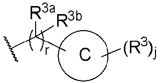 is selected from the group consisting of 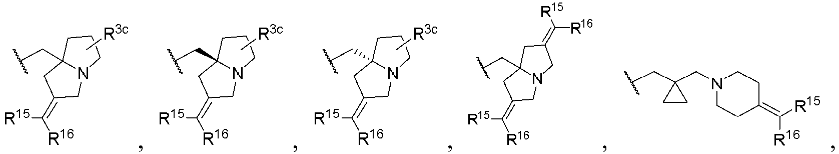 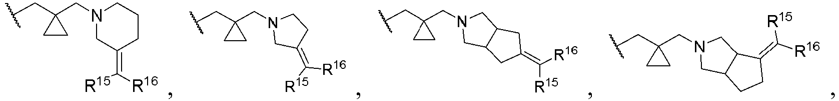 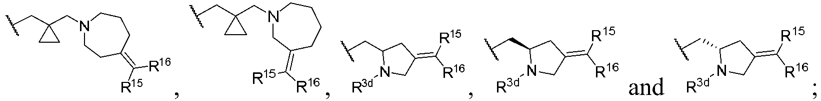
in some embodiments, 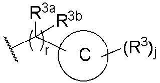 is selected from the group consisting of 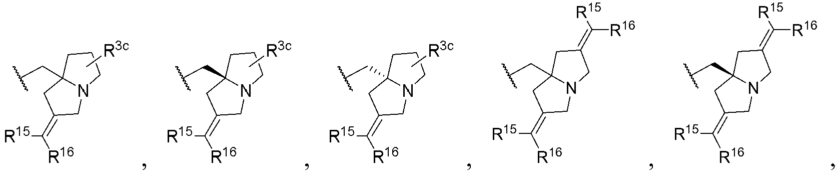 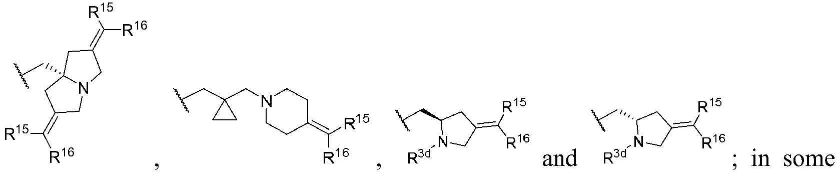 embodiments, 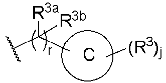 is selected from the group consisting of 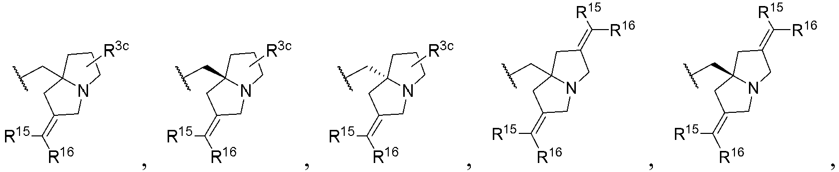 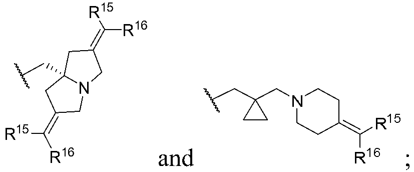 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, and R¹⁵ and R¹⁶ are the same or different, and are each independently selected from the group consisting of hydrogen atom, halogen, C₁₋₆ alkyl, C₁₋₆ haloalkyl and 3- to 6-membered cycloalkyl (in some embodiments, hydrogen atom or halogen).

In some embodiments of the present disclosure, provided is the compound represented by general formula (I'), (I'A), (IM) or (IN) or the pharmaceutically acceptable salt thereof, wherein 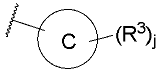 is selected from the group consisting of

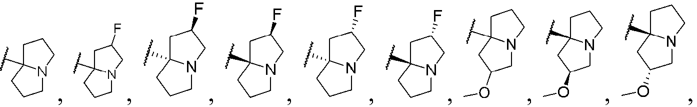

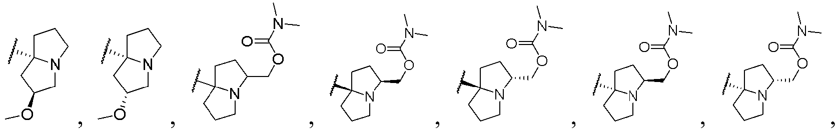

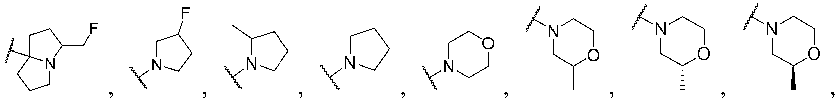

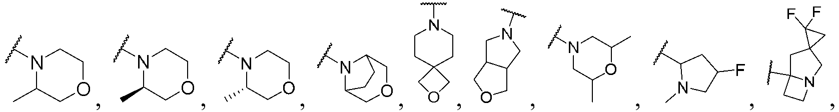

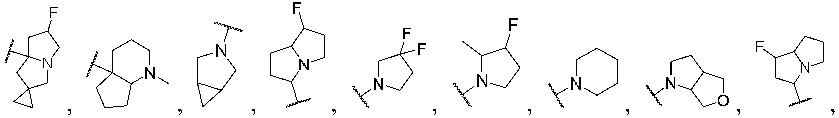

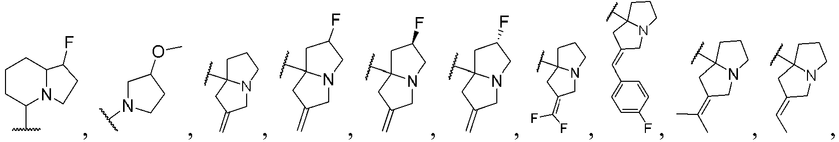

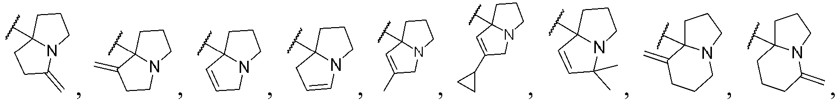

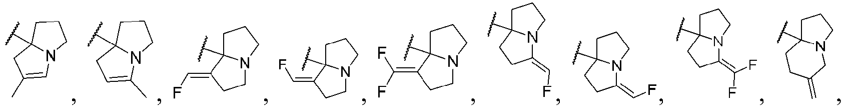

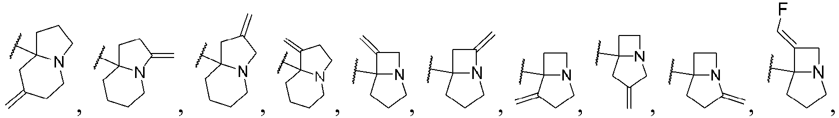

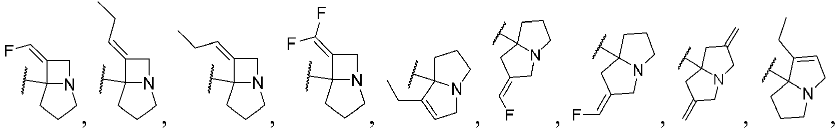

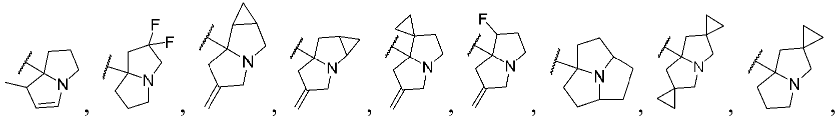

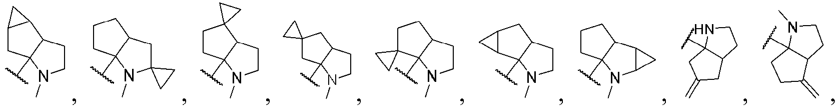

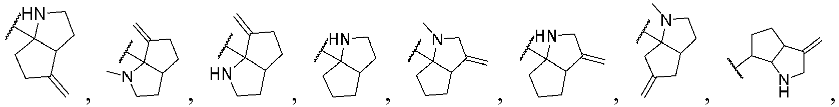

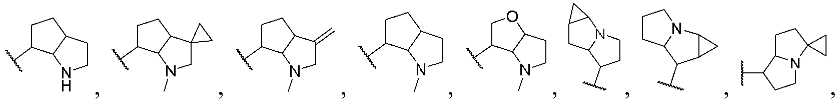

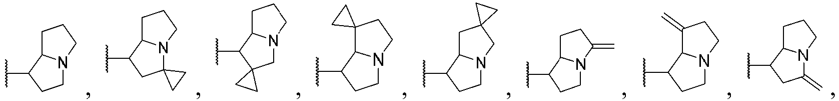

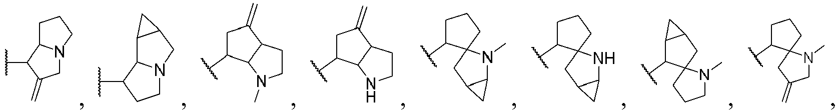

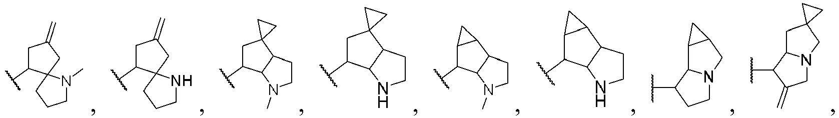

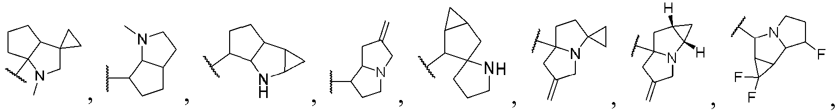

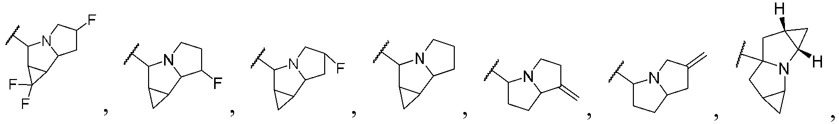

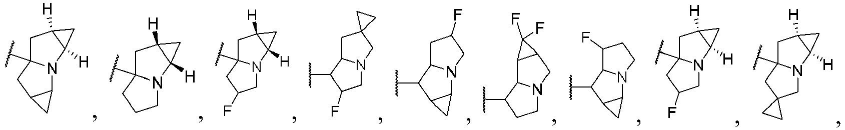

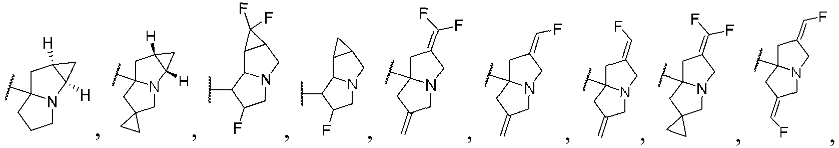

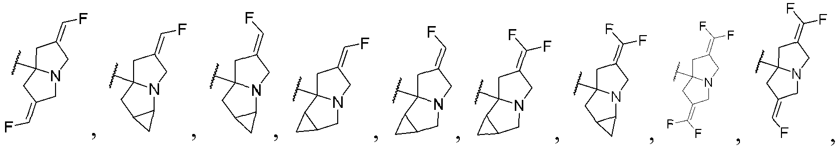

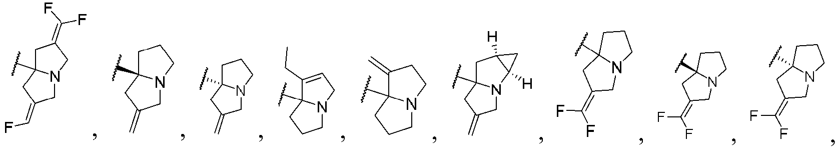

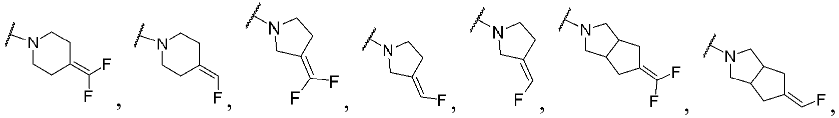

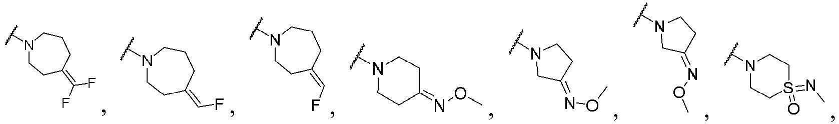

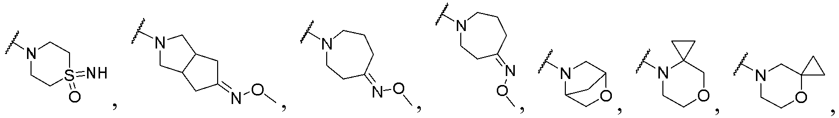

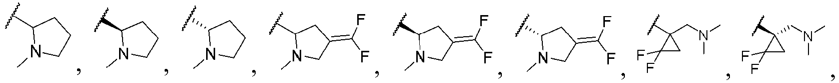

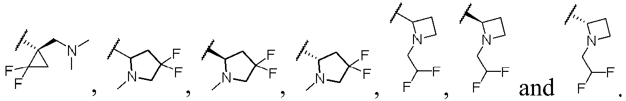

In some embodiments of the present disclosure, provided is the compound represented by general formula (I'), (I'A), (IM) or (IN) or the pharmaceutically acceptable salt thereof, wherein 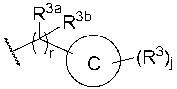 is selected from the group consisting of 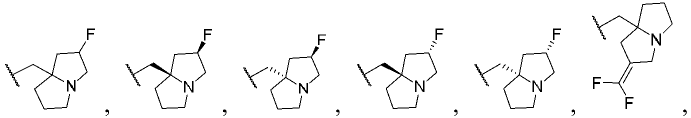 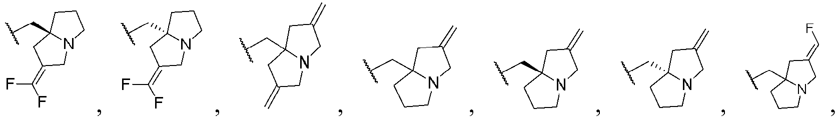 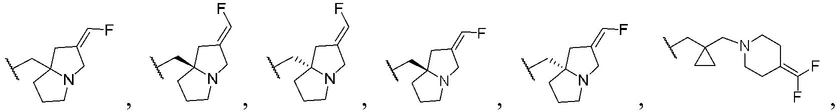 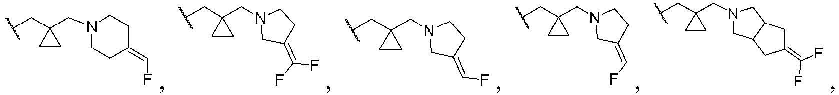 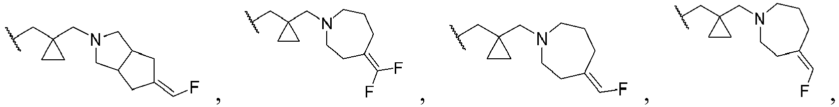 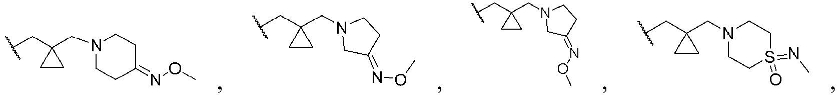 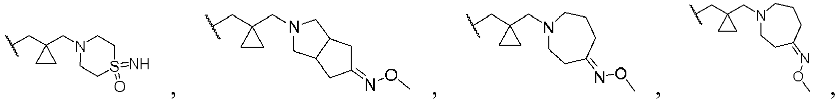 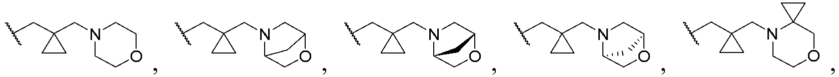 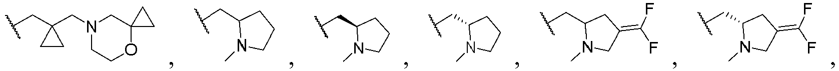 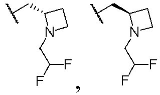 and 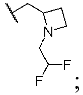 in some embodiments, 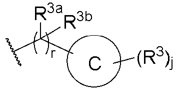 is selected from the group consisting of 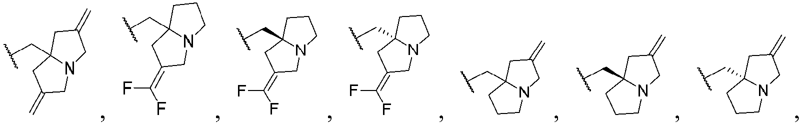 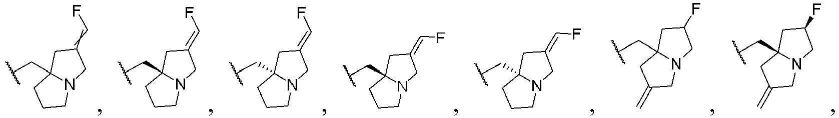 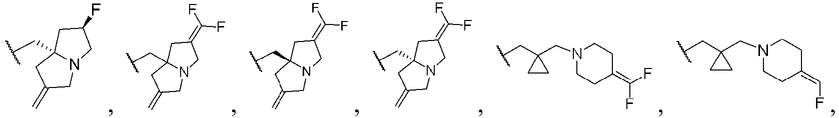 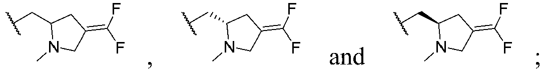 in some embodiments, 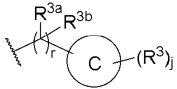 is selected from the group consisting of 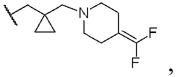 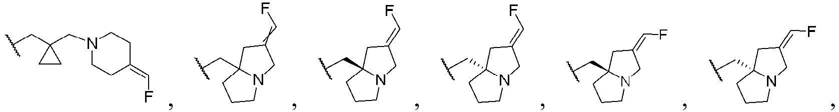 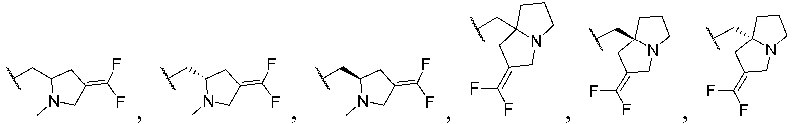 and 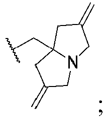 in some embodiments, provided is

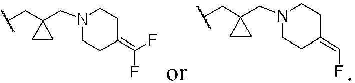

In some embodiments of the present disclosure, provided is the compound as represented by general formula (VII-1) or the pharmaceutically acceptable salt thereof, wherein 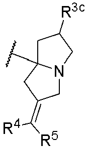 is 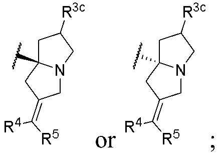 in some embodiments, provided is 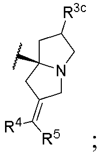 in some embodiments, provided is 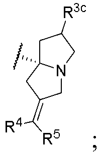 R^{3c}, R⁴ and R⁵ are as defined in general formula (V).

In some embodiments of the present disclosure, provided is the compound represented by any of general formulae (I) to (VII-1), (I'), (I'A), (IM), (IN), (IIA), (IIIA), (IVA) and (VA) or the pharmaceutically acceptable salt thereof, wherein each R³ is the same or different, and is independently selected from the group consisting of halogen, C₁₋₆ alkyl, oxo, C₁₋₆ alkoxy, C₁₋₆ haloalkyl, C₁₋₆ haloalkoxy, cyano, -C₁. ₆ alkyl NR¹¹R¹², =CR¹⁵R¹⁶, =NR¹³, hydroxyl and C₁₋₆ hydroxyalkyl, wherein R¹¹, R¹², R¹³, R¹⁵ and R¹⁶ are as defined in general formula (I); in some embodiments, each R³ is the same or different, and is independently selected from the group consisting of halogen, C₁₋₆ alkyl, C₁₋₆ haloalkyl and =CR¹⁵R¹⁶, wherein R¹⁵ and R¹⁶ are the same or different, and are each independently selected from the group consisting of hydrogen atom, halogen and C₁₋₆ alkyl; in some embodiments, each R³ is the same or different, and is independently halogen or =CR¹⁵R¹⁶, wherein R¹⁵ and R¹⁶ are the same or different, and are each independently hydrogen atom, halogen and C₁₋₆ alkyl; in some embodiments, each R³ is the same or different, and is independently selected from the group consisting of -CH₂-N(CH₃)₂, methyl,-CH₂CHF₂, F, =CH₂, =CHF and =CF₂; in some embodiments, each R³ is the same or different, and is independently selected from the group consisting of F, =CH₂, =CHF and =CF₂; in some embodiments, each R³ is the same or different, and is independently halogen; in some embodiments, R³ is F; in some embodiments, each R³ is the same or different, and is independently =CR¹⁵R¹⁶, wherein R¹⁵ and R¹⁶ are the same or different, and are each independently selected from the group consisting of hydrogen atom, halogen and C₁₋₆ alkyl; in some embodiments, each R³ is the same or different, and is independently =CR¹⁵R¹⁶, wherein R¹⁵ and R¹⁶ are the same or different, and are each independently hydrogen atom or halogen; in some embodiments, each R³ is the same or different, and is independently selected from the group consisting of =CH₂, =CHF and =CF₂.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein p is 0, 1, 2 or 3; in some embodiments, p is 0, 1 or 2; in some embodiments, p is 0 or 1; in some embodiments, p is 1; in some embodiments, p is 0.

In some embodiments of the present disclosure, provided is the compound represented by any of general formulae (I) to (VII-1), (I'), (I'A), (IM), (IN), (IIA), (IIIA), (IVA) and (VA) or the pharmaceutically acceptable salt thereof, wherein R^{3c} is selected from the group consisting of hydrogen atom, halogen, C₁₋₆ alkyl, C₁₋₆ alkoxy, C₁₋₆ haloalkyl, C₁₋₆ haloalkoxy, cyano, -C₁₋₆ alkyl NR¹¹R¹², hydroxyl, C₁₋₆ hydroxyalkyl and =CR¹⁵R¹⁶, wherein R¹¹, R¹², R¹⁵ and R¹⁶ are as defined in general formula (I);
in some embodiments, R^{3c} is selected from the group consisting of hydrogen atom, halogen, C₁₋₆ alkyl, C₁₋₆ haloalkyl and =CR¹⁵R¹⁶; in some embodiments, R^{3c} is selected from the group consisting of hydrogen atom, halogen and =CR¹⁵R¹⁶, wherein R¹⁵ and R¹⁶ are as defined in general formula (I); in some embodiments, R^{3c} is hydrogen atom or halogen; in some embodiments, R^{3c} is hydrogen atom or F; in some embodiments, R^{3c} is selected from the group consisting of hydrogen atom, F, =CH₂, =CHF and =CF₂; in some embodiments, R^{3c} is selected from the group consisting of hydrogen atom, =CH₂, =CHF and =CF₂; in some embodiments, R^{3c} is selected from the group consisting of hydrogen atom, F and =CH₂.

In some embodiments of the present disclosure, R^{3e} is selected from the group consisting of hydrogen atom, halogen, C₁₋₆ alkyl, C₁₋₆ alkoxy, C₁₋₆ haloalkyl and 3-to 6-membered cycloalkyl; in some embodiments, R^{3e} is selected from the group consisting of hydrogen atom, halogen and C₁₋₆ alkyl; in some embodiments, R^{3e} is hydrogen atom or halogen; in some embodiments, R^{3e} is halogen; in some embodiments, R^{3e} is F.

In some embodiments of the present disclosure, R^{3d} is selected from the group consisting of hydrogen atom, methyl and difluoroethyl; in some embodiments, R^{3d} is hydrogen atom or methyl; in some embodiments, R^{3d} is methyl.

In some embodiments of the present disclosure, provided is the compound represented by any of general formulae (I) to (VI), (I'), (I'A), (IM), (IN), (IIA), (IIIA), (IVA) and (VA) or the pharmaceutically acceptable salt thereof, wherein ring A is 6- to 10-membered aryl or 5- to 10-membered heteroaryl; in some embodiments, ring A is 6- to 10-membered aryl; in some embodiments, ring A is selected from the group consisting of naphthyl, phenyl, pyridyl, benzothienyl, benzothiazolyl, benzopyrazolyl, pyridothienyl, pyridothiazolyl, pyridopyrazolyl, benzoselenophenyl and pyridoselenophenyl; in some embodiments, ring A is selected from the group consisting of naphthyl, phenyl, pyridyl, benzothienyl, benzothiazolyl, benzopyrazolyl, pyridothienyl, pyridothiazolyl and pyridopyrazolyl; in some embodiments, ring A is selected from the group consisting of phenyl, benzothienyl and pyridothienyl; in some embodiments, ring A is benzoselenophenyl or pyridoselenophenyl; in some embodiments, ring A is benzoselenophenyl; in some embodiments, ring A is selected from the group consisting of 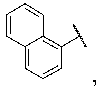 phenyl, 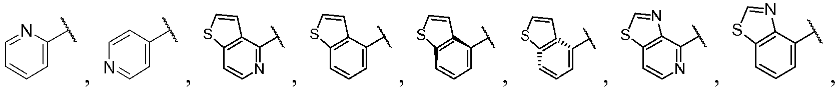 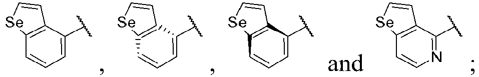 in some embodiments, ring A is selected from the group consisting of 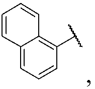 phenyl, 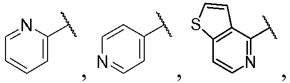 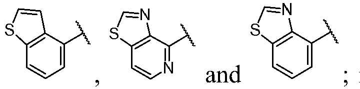 in some embodiments, ring A is selected from the group consisting of 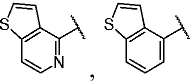 and phenyl; in some embodiments, ring A is selected from the group consisting of 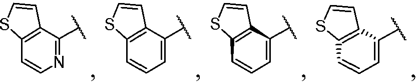 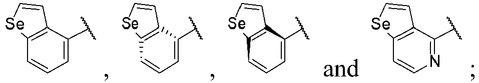 in some embodiments, ring A is selected from the group consisting of 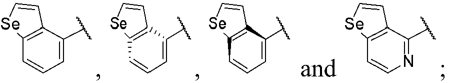 in some embodiments, ring A is 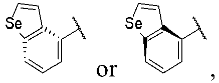 and 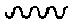 is connected to the ring in which G is located.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein each R¹ is the same or different, and is independently selected from the group consisting of halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, cyano, -NR¹¹R¹², hydroxyl, C₁₋₆ hydroxyalkyl, OR¹⁴, 3- to 8-membered cycloalkyl and C(O)NR¹¹R¹², wherein R¹¹, R¹² and R¹⁴ are as defined in general formula (I); in some embodiments, each R¹ is the same or different, and is independently selected from the group consisting of halogen, C₁₋₆ alkyl, C₂₋₆ alkynyl, C₁₋₆ haloalkyl, hydroxyl, C₁₋₆ hydroxyalkyl, cyano, -NR¹¹R¹² and 3- to 8-membered cycloalkyl, wherein R¹¹ and R¹² are as defined in general formula (I); in some embodiments, each R¹ is the same or different, and is independently selected from the group consisting of halogen, C₁₋₆ alkyl, C₂₋₆ alkynyl, C₁₋₆ haloalkyl, hydroxyl, cyano and -NR¹¹R¹², wherein R¹¹ and R¹² are the same or different, and are each independently hydrogen atom or C₁₋₆ alkyl; in some embodiments, each R¹ is the same or different, and is independently selected from the group consisting of halogen, C₁₋₆ alkyl, C₁₋₆ haloalkyl, cyano, -NH₂ and hydroxyl;
in some embodiments, each R¹ is the same or different, and is independently selected from the group consisting of halogen, C₁₋₆ alkyl and hydroxyl; in some embodiments, each R¹ is the same or different, and is independently selected from the group consisting of halogen, C₁₋₆ haloalkyl, cyano and -NR¹¹R¹², wherein R¹¹ and R¹² are as defined in general formula (I); in some embodiments, each R¹ is the same or different, and is independently selected from the group consisting of halogen, cyano and -NR¹¹R¹², wherein R¹¹ and R¹² are as defined in general formula (I); in some embodiments, each R¹ is the same or different, and is independently selected from the group consisting of halogen, cyano and amino; in some embodiments, each R¹ is the same or different, and is independently selected from the group consisting of F, Cl, CF₃, cyano, OH and amino; in some embodiments, each R¹ is the same or different, and is independently selected from the group consisting of F, CF₃, cyano and amino; in some embodiments, each R¹ is the same or different, and is independently F or cyano;
in some embodiments, each R¹ is the same or different, and is independently selected from the group consisting of halogen, C₁₋₆ alkyl, C₁₋₆ haloalkyl and cyano; in some embodiments, each R¹ is the same or different, and is independently halogen or cyano.

In some embodiments of the present disclosure, provided is the compound represented by any of general formulae (I) to (V), (I'), (I'A), (IM), (IN), (IIA), (IIIA), (IVA) and (VA) or the pharmaceutically acceptable salt thereof, wherein m is 1, 2, 3 or 4; in some embodiments, m is 1, 2 or 3; in some embodiments, m is 3 or 4; in some embodiments, m is 3.

In some embodiments of the present disclosure, provided is the compound represented by general formula (VI) or the pharmaceutically acceptable salt thereof, wherein m-1 is 0, 1, 2 or 3; in some embodiments, m-1 is 1, 2 or 3; in some embodiments, m-1 is 2 or 3; in some embodiments, m-1 is 2.

In some embodiments of the present disclosure, provided is the compound represented by any of general formulae (I) to (V), (I'), (I'A), (IM), (IN), (IIA), (IIIA), (IVA) and (VA) or the pharmaceutically acceptable salt thereof, wherein each R¹ is the same or different, and is independently selected from the group consisting of halogen, C₁₋₆ alkyl, C₂₋₆ alkynyl, C₁₋₆ haloalkyl, hydroxyl, amino and 3- to 6-membered cycloalkyl, and/or m is 1, 2 or 3; in some embodiments, each R¹ is the same or different, and is independently selected from the group consisting of halogen, C₁₋₆ haloalkyl, cyano and -NR¹¹R¹², wherein R¹¹ and R¹² are as defined in general formula (I), and/or m is 1, 2 or 3.

In some embodiments of the present disclosure, provided is the compound represented by any of general formulae (I) to (V), (I'), (I'A), (IM), (IN), (IIA), (IIIA), (IVA) and (VA) or the pharmaceutically acceptable salt thereof, wherein 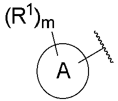 is 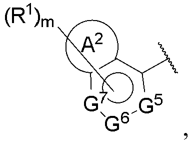 G⁵ is C or N, G⁶ is C or N, and G⁷ is C or N; ring A² is aryl or heteroaryl (in some embodiments, phenyl or 5- or 6-membered heteroaryl), and R¹ and m are as defined in general formula (I'); in some embodiments, 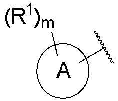 is 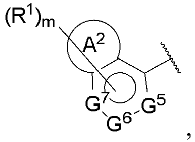 G⁵ is C or N, G⁶ is C or N, and G⁷ is C or N; ring A² is 5-membered heteroaryl, and each R¹ is the same or different, and is independently selected from the group consisting of halogen, C₁₋₆ alkyl, C₂₋₆ alkynyl, C₁₋₆ haloalkyl, hydroxyl, cyano and -NR¹¹R¹², wherein R¹¹ and R¹² are the same or different, and are each independently hydrogen atom or C₁₋₆ alkyl; m is as defined in general formula (I').

In some embodiments of the present disclosure, ring A² is 5-membered heteroaryl; in some embodiments, ring A² is selected from the group consisting of thienyl, furyl, pyrazolyl, thiazolyl, imidazolyl and pyrrolyl; in some embodiments, ring A² is selected from the group consisting of thienyl, pyrazolyl and thiazolyl; in some embodiments, ring A² is thienyl; in some embodiments, ring A² is selenophenyl.

In some embodiments of the present disclosure, G⁵ is N, G⁶ is C, and G⁷ is C; in some embodiments, G⁵ is C, G⁶ is C, and G⁷ is C.

In some embodiments of the present disclosure, provided is the compound represented by any of general formulae (I) to (V), (I'), (I'A), (IM), (IN), (IIA), (IIIA), (IVA) and (VA) or the pharmaceutically acceptable salt thereof, wherein 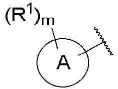 is selected from the group consisting of 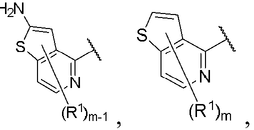 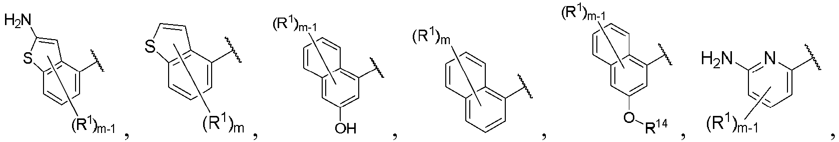 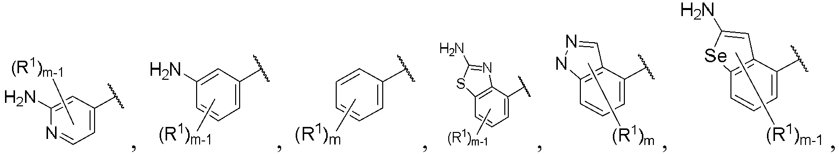 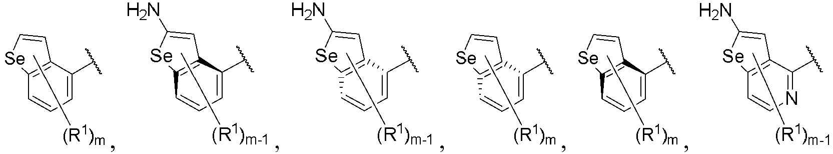 and 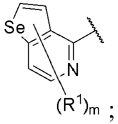
in some embodiments, 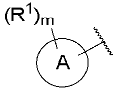 is selected from the group consisting of 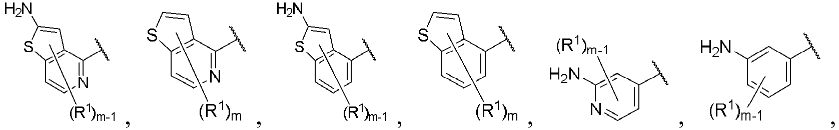 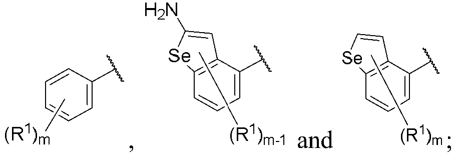
in some embodiments, 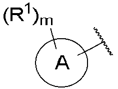 is selected from the group consisting of 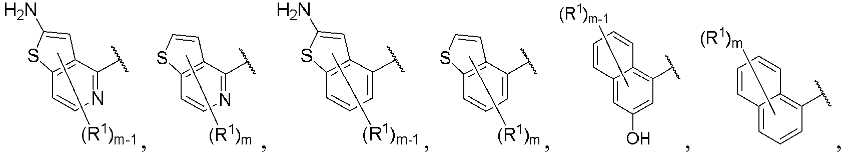 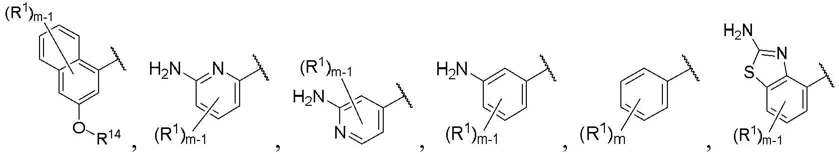 and 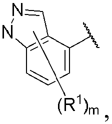 wherein R¹, R¹⁴ and m are as defined in general formula (I);
in some embodiments, 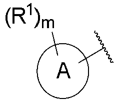 is selected from the group consisting of 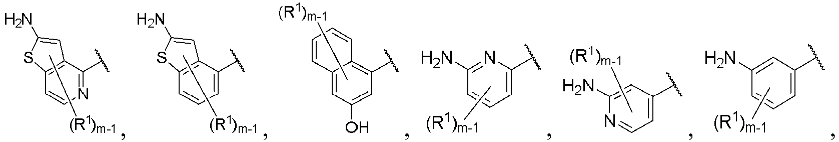 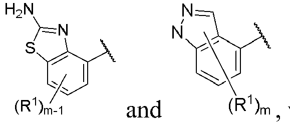 wherein R¹ and m are as defined in general formula (I);
in some embodiments, 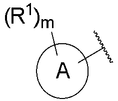 is selected from the group consisting of 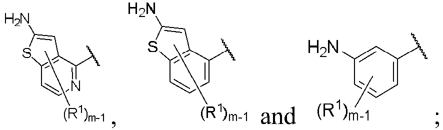 in some embodiments, 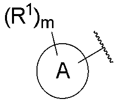 is 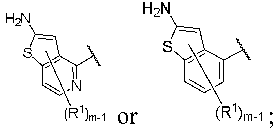 in some embodiments, 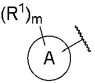 is selected from the group consisting of 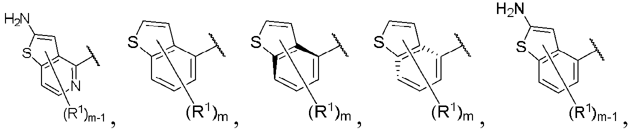 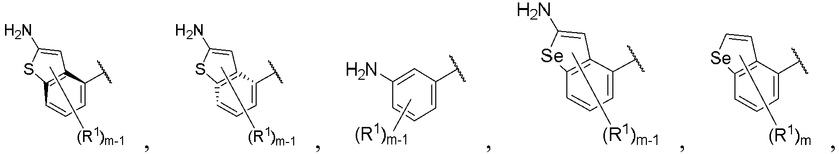 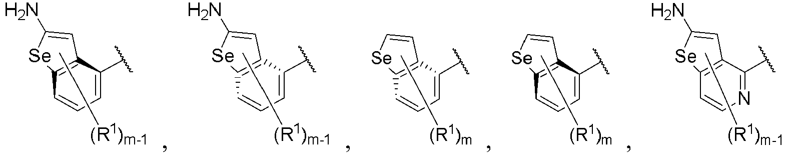 and 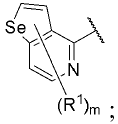 in some embodiments, 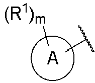 is selected from the group consisting of 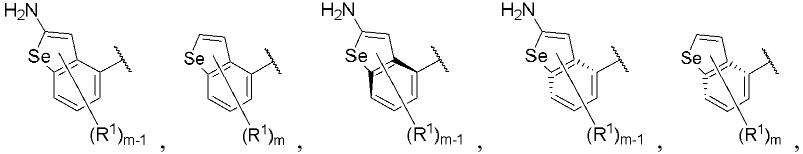 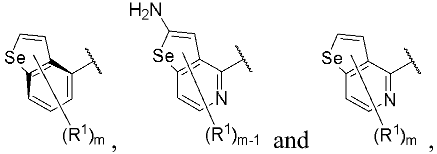 wherein R¹ and m are as defined in general formula (I); in some embodiments, 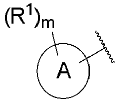 is selected from the group consisting of 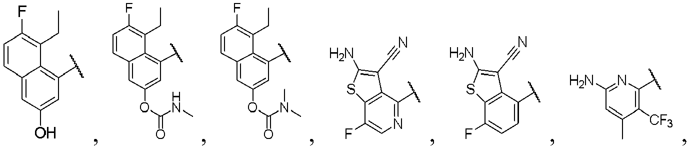 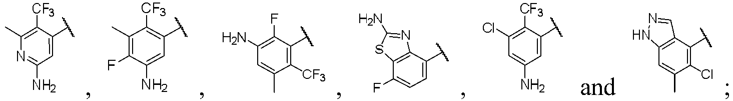 in some embodiments, 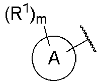 is selected from the group consisting of 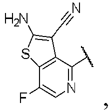 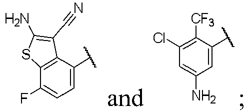 in some embodiments, 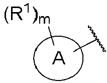 is 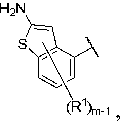 and R¹ and m are as defined in general formula (I'); in some embodiments, 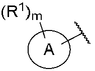 is 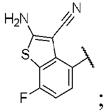 in some embodiments, 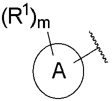 is 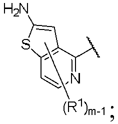 in some embodiments, 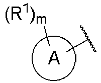 is selected from the group consisting of 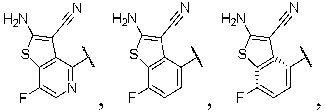 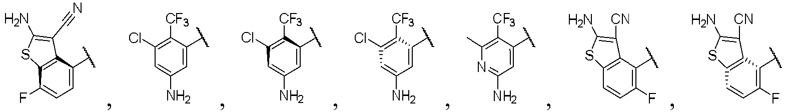 and 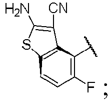 in some embodiments, 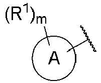 is 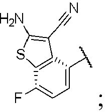 in some embodiments, 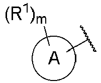 is 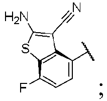 in some embodiments, 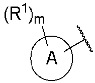 is selected from the group consisting of 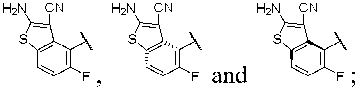 in some embodiments, provided is 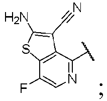
in some embodiments, 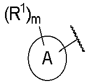 is selected from the group consisting of 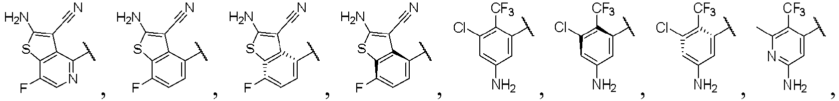 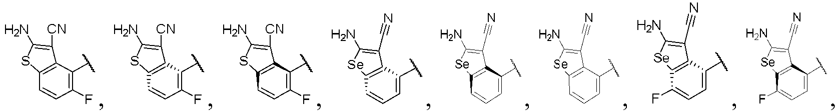 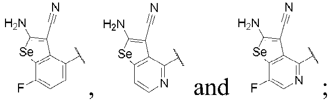 in some embodiments, 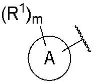 is selected from the group consisting of 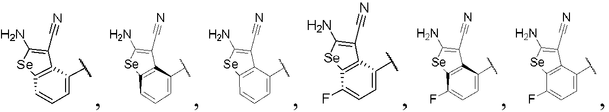 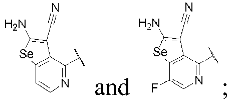 in some embodiments, 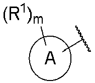 is selected from the group consisting of 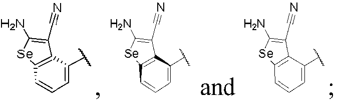 in some embodiments, 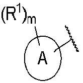 is selected from the group consisting of

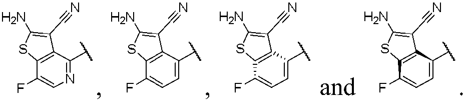

In some embodiments of the present disclosure, provided is the compound represented by general formula (VII-1) or (VII-2) or the pharmaceutically acceptable salt thereof, wherein 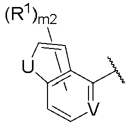 is 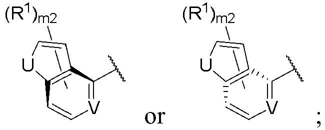 in some embodiments, 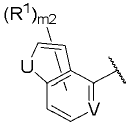 is 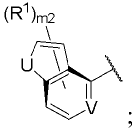 in some embodiments, 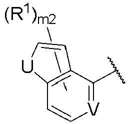 is 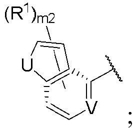 U, V, R¹ and m2 are as defined in general formula (VII-1) or (VII-2); in some embodiments, 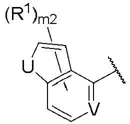 is selected from the group consisting of 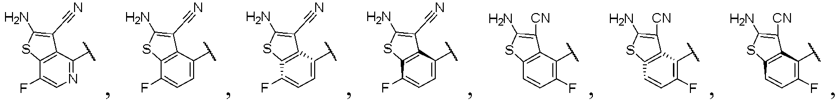 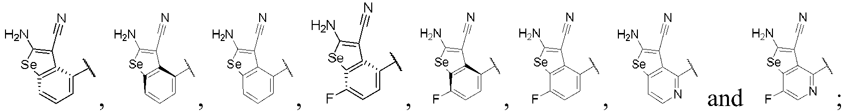 in some embodiments, 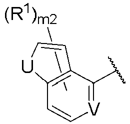 is selected from the group consisting of 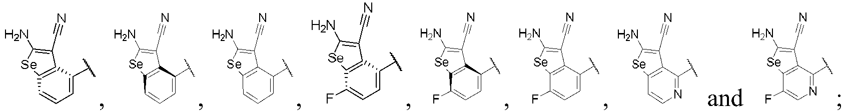 in some embodiments, 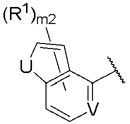 is selected from the group consisting of

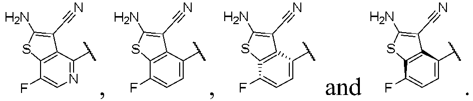

In some embodiments of the present disclosure, m-2 is 0, 1, 2 or 3; in some embodiments, m-2 is 1, 2 or 3; in some embodiments, m-2 is 3; in some embodiments, m-2 is 2.

In some embodiments of the present disclosure, provided is the compound represented by any of general formulae (I) to (VII-2) or the pharmaceutically acceptable salt thereof, wherein R^{1a} is selected from the group consisting of hydrogen atom, C₁₋₆ alkyl, 3- to 6-membered cycloalkyl and 3- to 6-membered cycloalkyl C₁₋₆ alkyl; in some embodiments, R^{1a} is hydrogen atom or C₁₋₆ alkyl; in some embodiments, R^{1a} is hydrogen atom or methyl; in some embodiments, R^{1a} is hydrogen atom.

In some embodiments of the present disclosure, provided is the compound represented by any of general formulae (I) to (VII-2) or the pharmaceutically acceptable salt thereof, wherein R^{1b} is selected from the group consisting of hydrogen atom, halogen, C₁₋₆ alkyl, C₂₋₆ alkenyl, C₂₋₆ alkynyl, C₁₋₆ alkoxy, C₁₋₆ haloalkyl, C₁₋₆ haloalkoxy, cyano, -NR¹¹R¹², hydroxyl, C₁₋₆ hydroxyalkyl, 3- to 6-membered cycloalkyl and 3- to 6-membered cycloalkyl C₁₋₆ alkyl; R¹¹ and R¹² are as defined in general formula (I); in some embodiments, R^{1b} is selected from the group consisting of hydrogen atom, halogen, C₁₋₆ alkyl, C₁₋₆ alkoxy, C₁₋₆ haloalkyl, C₁₋₆ haloalkoxy and cyano; in some embodiments, R^{1b} is selected from the group consisting of hydrogen atom, halogen and C₁₋₆ alkyl; in some embodiments, R^{1b} is hydrogen atom or halogen; in some embodiments, R^{1b} is hydrogen atom or F; in some embodiments, R^{1b} is hydrogen atom.

In some embodiments of the present disclosure, provided is the compound represented by any of general formulae (I) to (III), (IM), (IN), (IIA) and (IIIA) or the pharmaceutically acceptable salt thereof, wherein G is CR^{G} or N, R^{G} is selected from the group consisting of hydrogen atom, halogen, C₁₋₆ alkyl and C₁₋₆ haloalkyl; in some embodiments, G is CR^{G} or N, and R^{G} is hydrogen atom or halogen; in some embodiments, G is C-Cl or N; in some embodiments, G is N.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein G¹ is CR^{G1} or N, and R^{G1} is selected from the group consisting of hydrogen atom, halogen, C₁₋₆ alkyl and C₁₋₆ haloalkyl; in some embodiments, G¹ is CH or N; in some embodiments, G¹ is N.

In some embodiments of the present disclosure, provided is the compound represented by general formula (IN), (IV) or (IVA) or the pharmaceutically acceptable salt thereof, wherein G is C.

In some embodiments of the present disclosure, provided is the compound represented by any of general formulae (I) to (V), (I'), (IM), (IN), (IIA), (IIIA), (IVA) and (VA) or the pharmaceutically acceptable salt thereof, wherein G is N.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R² is selected from the group consisting of hydrogen atom, halogen, C₁₋₆ alkyl and C₁₋₆ haloalkyl; in some embodiments, R² is hydrogen atom or halogen; in some embodiments, R² is halogen; in some embodiments, R² is F; in some embodiments, R² is Cl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein G¹ is N, and/or R² is halogen.

In some embodiments of the present disclosure, provided is the compound represented by general formula (I), (IM), (II), (III), (IIA) or (IIIA) or the pharmaceutically acceptable salt thereof, wherein G¹ is N, and/or R² is halogen, and/or G is CR^{G} or N, and R^{G} is selected from the group consisting of hydrogen atom, halogen, C₁₋₆ alkyl and C₁₋₆ haloalkyl; and/or R⁷ is selected from the group consisting of hydrogen atom, C₁₋₆ alkyl, C₁₋₆ alkoxy, 3- to 6-membered cycloalkyloxy and 3- to 6-membered heterocyclyloxy; in some embodiments, G¹ is N, and/or R² is halogen, and/or G is CR^{G} or N, and R^{G} is hydrogen atom or halogen; and/or R⁷ is selected from the group consisting of hydrogen atom, C₁₋₆ alkoxy and 3- to 6-membered cycloalkyloxy.

In some embodiments of the present disclosure, provided is the compound represented by general formula (V) or the pharmaceutically acceptable salt thereof, wherein Q is NH or O; in some embodiments, Q is O.

In some embodiments of the present disclosure, provided is the compound represented by general formula (V) or the pharmaceutically acceptable salt thereof, wherein s is 1 or 2; in some embodiments, s is 1.

In some embodiments of the present disclosure, provided is the compound represented by general formula (V) or the pharmaceutically acceptable salt thereof, wherein t is 1 or 2; in some embodiments, t is 1.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein each R⁶ is the same or different, and is independently selected from the group consisting of C₁₋₆ alkyl, OR¹⁴, NR¹¹R¹², C(O)NR¹¹R¹² and C(O)R¹⁴, wherein the C₁₋₆ alkyl is optionally substituted with one or more substituents selected from the group consisting of halogen, OR¹⁴ and 3- to 6-membered heterocyclyl; R¹¹, R¹² and R¹⁴ are as defined in general formula (I'); in some embodiments, each R⁶ is the same or different, and is independently selected from the group consisting of C₁₋₆ hydroxyalkyl, C(O)NR¹¹R¹² and C(O)R¹⁴, wherein R¹¹, R¹² and R¹⁴ are as defined in general formula (I');
in some embodiments, each R⁶ is the same or different, and is independently C(O)NR¹¹R¹² or C(O)R¹⁴, wherein R¹¹, R¹² and R¹⁴ are as defined in general formula (I'); in some embodiments, each R⁶ is the same or different, and is independently C₁₋₆ hydroxyalkyl; in some embodiments, R⁶ is hydroxypropyl; in some embodiments, R⁶ is OR¹⁴, and R¹⁴ is as defined in general formula (I'); in some embodiments, R⁶ is OR¹⁴, and R¹⁴ is hydrogen atom or C₁₋₆ alkyl; in some embodiments, R⁶ is OH; in some embodiments, R⁶ is selected from the group consisting of 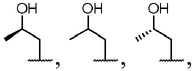 OH, C(O)NH₂, 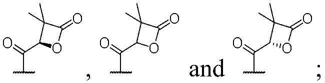 in some embodiments, R⁶ is selected from the group consisting of 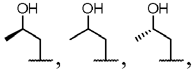 OH, methyl, C(O)NH₂, 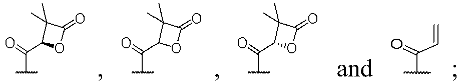 in some embodiments, R⁶ is

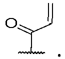

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R¹¹ is hydrogen atom or C₁₋₆ alkyl; in some embodiments, R¹¹ is hydrogen atom or methyl; in some embodiments, R¹¹ is hydrogen atom; in some embodiments, R¹¹ is hydrogen atom or C₁₋₆ alkyl, wherein the C₁₋₆ alkyl is optionally substituted with one or more substituents selected from the group consisting of hydroxyl, C₁₋₆ alkoxy, 3-to 6-membered cycloalkyl and 3- to 6-membered heterocyclyl; in some embodiments, R¹¹ is selected from the group consisting of hydrogen atom, C₁₋₆ alkyl, C₁₋₆ alkoxy C₁₋₆ alkyl and 3- to 6-membered cycloalkyl C₁₋₆ alkyl; in some embodiments, R¹¹ is selected from the group consisting of ethyl,

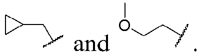

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R¹² is hydrogen atom or C₁₋₆ alkyl; in some embodiments, R¹² is hydrogen atom or methyl; in some embodiments, R¹² is hydrogen atom; in some embodiments, R¹² is hydrogen atom or C₁₋₆ alkyl, wherein the C₁₋₆ alkyl is optionally substituted with 5- or 6-membered heteroaryl, the 5- or 6-membered heteroaryl is optionally substituted with NR^{a}R^{b}, and R^{a} and R^{b} are the same or different, and are each independently hydrogen atom or C₁₋₆ alkyl; in some embodiments, R¹² is hydrogen atom or C₁₋₆ alkyl, wherein the C₁₋₆ alkyl is substituted with 6-membered heteroaryl, the 6-membered heteroaryl is substituted with NR^{a}R^{b}, and R^{a} and R^{b} are the same or different, and are each independently hydrogen atom or C₁₋₆ alkyl; in some embodiments, R¹² is hydrogen atom or C₁₋₆ alkyl, wherein the C₁₋₆ alkyl is substituted with pyridyl, and the pyridyl is substituted with NH₂;
in some embodiments, R¹² is selected from the group consisting of 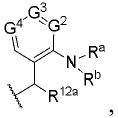 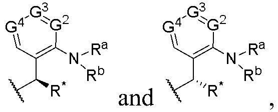 wherein G², G³ and G⁴ are the same or different, and are independently N or CR^{1*}, R^{12a} is hydrogen atom or R*, R¹* is hydrogen atom or R^{*}, and R* is as defined in general formula (I');
in some embodiments, R¹² is selected from the group consisting of 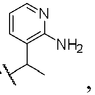 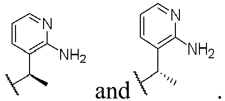
In some embodiments of the present disclosure, G², G³ and G⁴ are the same or different, and are each independently N or C R^{1*}, wherein R¹* is selected from the group consisting of hydrogen atom, halogen, C₁₋₆ alkyl, C₁₋₆ haloalkyl, C₁₋₆ alkoxy, C₁₋₆ alkoxy C₁₋₆ alkyl and 3- to 6-membered cycloalkyl; in some embodiments, G² is N, and G³ and G⁴ are CH.

In some embodiments of the present disclosure, R^{12a} is selected from the group consisting of hydrogen atom, halogen, C₁₋₆ alkyl, C₁₋₆ haloalkyl, C₁₋₆ alkoxy, C₁₋₆ alkoxy C₁₋₆ alkyl and 3- to 6-membered cycloalkyl; in some embodiments, R^{12a} is C₁₋₆ alkyl; in some embodiments, R^{12a} is methyl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R¹¹ is selected from the group consisting of hydrogen atom, C₁₋₆ alkyl, C₁₋₆ alkoxy C₁₋₆ alkyl and 3- to 6-membered cycloalkyl C₁₋₆ alkyl; and/or R¹² is C₁₋₆ alkyl, wherein the C₁₋₆ alkyl is substituted with 6-membered heteroaryl, the 6-membered heteroaryl is substituted with NR^{a}R^{b}, and R^{a} and R^{b} are the same or different, and are each independently hydrogen atom or C₁₋₆ alkyl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R¹³ is hydrogen atom or C₁₋₆ alkyl.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein R¹⁴ is selected from the group consisting of hydrogen atom, C₁₋₆ alkyl, C₂₋₆ alkenyl, 3-to 6-membered heterocyclyl and C(O)NR²⁰R²¹, wherein the C₁₋₆ alkyl and 3- to 6-membered heterocyclyl are each independently and optionally substituted with one or more substituents selected from the group consisting of oxo, halogen, hydroxyl, C₁₋₆ alkyl, C₁₋₆ alkoxy, C₁₋₆ haloalkoxy, C₁₋₆ alkoxy C₁₋₆ alkyl and C₁₋₆ hydroxyalkoxy, and R²⁰ and R²¹ are as defined in general formula (I); in some embodiments, R¹⁴ is selected from the group consisting of hydrogen atom, C₁₋₆ alkyl and C(O)NR²⁰R²¹, wherein the C₁₋₆ alkyl is optionally substituted with one or more substituents selected from the group consisting of halogen, hydroxyl, C₁₋₆ alkoxy, C₁₋₆ haloalkoxy, C₁₋₆ alkoxy C₁₋₆ alkyl and C₁₋₆ hydroxyalkoxy, and R²⁰ and R²¹ are as defined in general formula (I); in some embodiments, R¹⁴ is selected from the group consisting of hydrogen atom, C₁₋₆ alkyl, C₁₋₆ haloalkyl, C₁₋₆ hydroxyalkyl and C₁₋₆ alkoxy C₁₋₆ alkyl; in some embodiments, R¹⁴ is hydrogen atom or C₁₋₆ alkyl; in some embodiments, R¹⁴ is hydrogen atom or methyl;
in some embodiments, R¹⁴ is selected from the group consisting of hydrogen atom, C₁₋₆ alkyl, 3- to 6-membered cycloalkyl, 3- to 6-membered heterocyclyl, phenyl and 5- or 6-membered heteroaryl, wherein the C₁₋₆ alkyl, 3- to 6-membered cycloalkyl, 3- to 6-membered heterocyclyl, phenyl and 5- or 6-membered heteroaryl are each independently and optionally substituted with one or more substituents selected from the group consisting of oxo, halogen, hydroxyl, cyano, C₁₋₆ alkyl, C₁₋₆ alkoxy, C₁₋₆ haloalkyl, C₁₋₆ haloalkoxy, 3- to 6-membered cycloalkyl, C₁₋₆ alkoxy C₁₋₆ alkyl and C₁₋₆ hydroxyalkoxy; in some embodiments, R¹⁴ is selected from the group consisting of hydrogen atom, C₁₋₆ alkyl, 3- to 6-membered cycloalkyl and 5- or 6-membered heteroaryl, wherein the C₁₋₆ alkyl, 3- to 6-membered cycloalkyl and 5- or 6-membered heteroaryl are each independently and optionally substituted with one or more substituents selected from the group consisting of halogen, cyano, C₁₋₆ alkyl and C₁₋₆ haloalkyl; in some embodiments, R¹⁴ is selected from the group consisting of C₁₋₆ alkyl, 3- to 6-membered cycloalkyl and 5- or 6-membered heteroaryl, wherein the C₁₋₆ alkyl, 3- to 6-membered cycloalkyl and 5- or 6-membered heteroaryl are each independently and optionally substituted with one or more substituents selected from the group consisting of halogen, cyano and C₁₋₆ alkyl;
in some embodiments, R¹⁴ is selected from the group consisting of hydrogen atom, C₂₋₆ alkenyl, 3- to 6-membered heterocyclyl and C(O)NR²⁰R²¹, wherein the 3- to 6-membered heterocyclyl is optionally substituted with one or more substituents selected from the group consisting of oxo and C₁₋₆ alkyl, and R²⁰ and R²¹ are the same or different, and are each independently hydrogen atom or C₁₋₆ alkyl;
in some embodiments, R¹⁴ is selected from the group consisting of hydrogen atom, 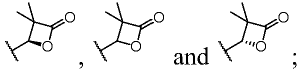 in some embodiments, R¹⁴ is selected from the group consisting of hydrogen atom, ethenyl, 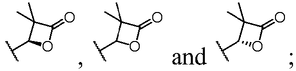 in some embodiments, R¹⁴ is ethenyl.

In some embodiments of the present disclosure, provided is the compound represented by any of general formulae (I) to (VII-1), (I'), (I'A), (IM), (IN), (IIA), (IIIA), (IVA) and (VA) or the pharmaceutically acceptable salt thereof, wherein R¹⁵ and R¹⁶ are the same or different, and are each independently selected from the group consisting of hydrogen atom, halogen and C₁₋₆ alkyl; in some embodiments, R¹⁵ and R¹⁶ are the same or different, and are each independently selected from the group consisting of hydrogen atom or halogen; in some embodiments, R¹⁵ and R¹⁶ are hydrogen atoms; in some embodiments, R¹⁵ is hydrogen atom and R¹⁶ is halogen, or R¹⁶ is hydrogen atom and R¹⁵ is halogen.

In some embodiments of the present disclosure, provided is the compound represented by any of general formulae (I) to (VII-1), (I'), (I'A), (IM), (IN), (IIA), (IIIA), (IVA) and (VA) or the pharmaceutically acceptable salt thereof, wherein R¹⁵ is hydrogen atom or halogen; in some embodiments, R¹⁵ is hydrogen atom or F; in some embodiments, R¹⁵ is hydrogen atom.

In some embodiments of the present disclosure, provided is the compound represented by any of general formulae (I) to (VII-1), (I'), (I'A), (IM), (IN), (IIA), (IIIA), (IVA) and (VA) or the pharmaceutically acceptable salt thereof, wherein R¹⁶ is hydrogen atom or halogen; in some embodiments, R¹⁶ is hydrogen atom or F; in some embodiments, R¹⁶ is hydrogen atom.

In some embodiments of the present disclosure, provided is the compound represented by general formula (I) or (V) or the pharmaceutically acceptable salt thereof, wherein R^{8c} is hydrogen atom or C₁₋₆ alkyl; in some embodiments, R^{8c} is hydrogen atom or methyl; in some embodiments, R^{8c} is hydrogen atom.

In some embodiments of the present disclosure, provided is the compound represented by general formula (I), (II), (IV), (V), (VI), (VII-1) or (VII-2) or the pharmaceutically acceptable salt thereof, wherein R^{6c} and R^{6d} are the same or different, and are each independently selected from the group consisting of hydrogen atom, halogen and C₁₋₆ alkyl; in some embodiments, R^{6c} and R^{6d} are the same or different, and are each independently hydrogen atom or halogen.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein each R²⁰, R²¹, R²² and R²³ is the same or different, and is independently hydrogen atom or C₁₋₆ alkyl; in some embodiments, each R²⁰, R²¹, R²² and R²³ is the same or different, and is independently hydrogen atom or methyl; in some embodiments, each R²⁰ or R²¹ is hydrogen atom.

In some embodiments of the present disclosure, provided is the compound represented by any of general formulae (I) to (VII-2), (I'), (I'A), (IM), (IN), (IIA), (IIIA), (IVA) and (VA) or the pharmaceutically acceptable salt thereof, wherein each R* is the same or different, and is independently selected from the group consisting of halogen, C₁₋₆ alkyl, C₁₋₆ haloalkyl and 3- to 6-membered cycloalkyl; in some embodiments, each R* is the same or different, and is independently C₁₋₆ alkyl; in some embodiments, R* is methyl.

In present disclosure, the general formulae (I) to (V) include general formulae (I), (II), (III), (IV) and (V); general formulae (I) to (VI) include general formulae (I), (II), (III), (IV), (V) and (VI); general formulae (I) to (VII-1) include general formulae (I), (II), (III), (IV), (V), (VI) and (VII-1); general formulae (I) to (VII-2) include general formulae (I), (II), (III), (IV), (V), (VI), (VII-1) and (VII-2).

In some embodiments of the present disclosure, provided is the compound represented by general formula (II) or the pharmaceutically acceptable salt thereof, wherein G¹ is CH or N; G is CR^{G} or N, and R^{G} is selected from the group consisting of hydrogen atom, halogen, C₁₋₆ alkyl and C₁₋₆ haloalkyl; R⁷ is hydrogen atom, or R⁷ and G together form ring B, ring B is optionally substituted with one or more R⁸, and ring B is 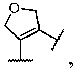 wherein each R⁸ is the same or different, and is independently selected from the group consisting of halogen, C₁₋₆ alkyl, C₁₋₆ haloalkyl and cyano; R² is selected from the group consisting of hydrogen atom, halogen, C₁₋₆ alkyl and C₁₋₆ haloalkyl; 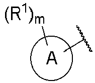 is selected from the group consisting of 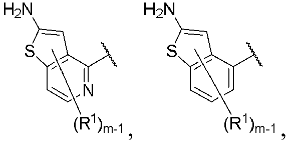 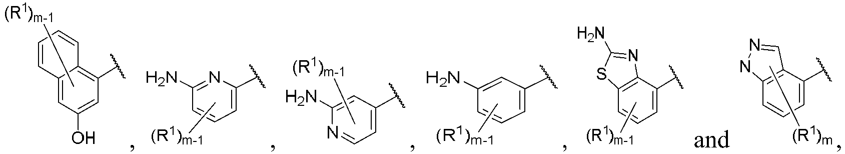 wherein each R¹ is the same or different, and is independently selected from the group consisting of halogen, C₁₋₆ alkyl, C₁₋₆ haloalkyl and cyano; m is 1, 2, 3 or 4; 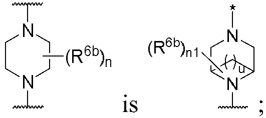 u is 1 or 2, n1 is 0, and R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 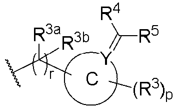 is selected from the group consisting of 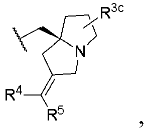 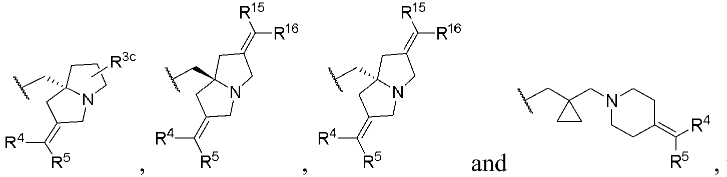 wherein R⁴ and R⁵ are the same or different, and are each independently selected from the group consisting of hydrogen atom, halogen and C₁₋₆ alkyl; R^{3c} is hydrogen atom or halogen; R¹⁵ is hydrogen atom or halogen; R¹⁶ is hydrogen atom or halogen.

In some embodiments of the present disclosure, provided is the compound represented by general formula (III) or the pharmaceutically acceptable salt thereof, wherein G is CR^{G} or N; R^{G} is selected from the group consisting of hydrogen atom, halogen, C₁₋₆ alkyl and C₁₋₆ haloalkyl; G¹ is N, and R² is selected from the group consisting of hydrogen atom, halogen, C₁₋₆ alkyl and C₁₋₆ haloalkyl; R⁷ is selected from the group consisting of hydrogen atom, halogen, C₁₋₆ alkyl and C₁₋₆ haloalkyl; u is 1 or 2; n1 is 0; R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; 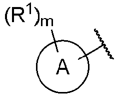 is selected from the group consisting of 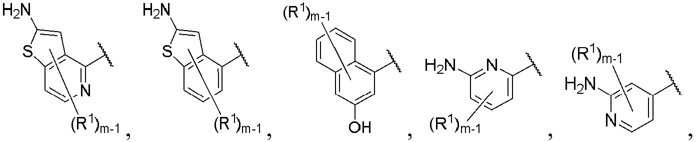 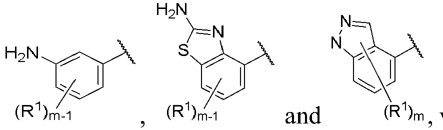 wherein each R¹ is the same or different, and is independently selected from the group consisting of halogen, C₁₋₆ alkyl, C₁₋₆ haloalkyl and cyano; m is 1, 2, 3 or 4; W is O; 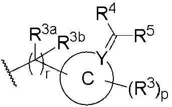 is selected from the group consisting of 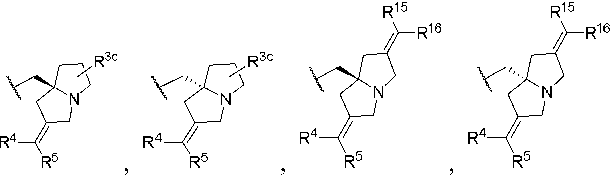 and 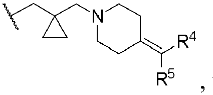 wherein R⁴ and R⁵ are the same or different, and are each independently selected from the group consisting of hydrogen atom, halogen and C₁₋₆ alkyl; R^{3c} is hydrogen atom or halogen; R¹⁵ is hydrogen atom or halogen; R¹⁶ is hydrogen atom or halogen.

In some embodiments of the present disclosure, provided is the compound represented by general formula (III) or the pharmaceutically acceptable salt thereof, wherein G is CR^{G} or N; R^{G} is selected from the group consisting of hydrogen atom, halogen, C₁₋₆ alkyl and C₁₋₆ haloalkyl; G¹ is N, and R² is selected from the group consisting of hydrogen atom, halogen, C₁₋₆ alkyl and C₁₋₆ haloalkyl; R⁷ is selected from the group consisting of hydrogen atom, halogen, C₁₋₆ alkyl and C₁₋₆ haloalkyl; u is 1 or 2; n1 is 0; R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 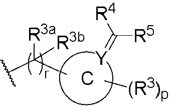 is selected from the group consisting of 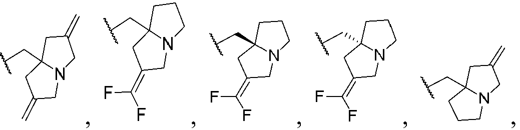 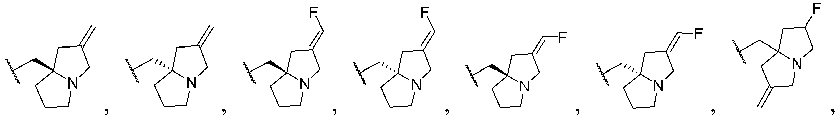 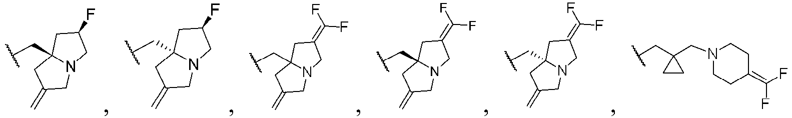 and 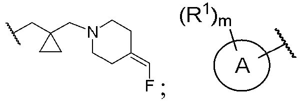 is selected from the group consisting of

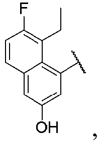

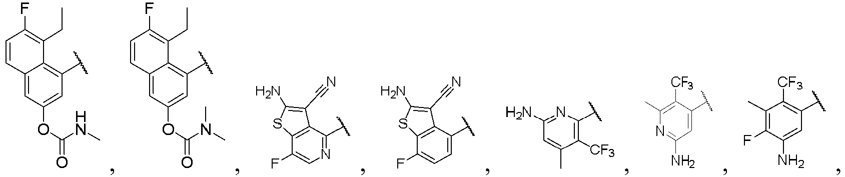

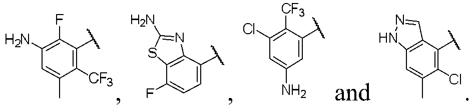

In some embodiments of the present disclosure, provided is the compound represented by general formula (V) or the pharmaceutically acceptable salt thereof, wherein Q is O; t is 1 or 2, and s is 1 or 2; R² is selected from the group consisting of hydrogen atom, halogen, C₁₋₆ alkyl and C₁₋₆ haloalkyl; q is 0; G¹ is N; 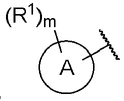 is selected from the group consisting of 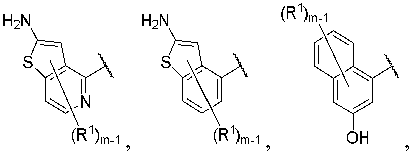 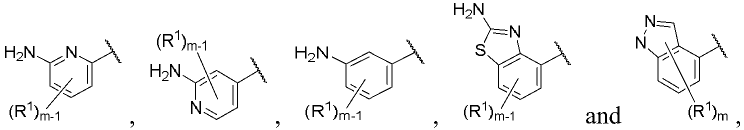 wherein each R¹ is the same or different, and is independently selected from the group consisting of halogen, C₁₋₆ alkyl, C₁₋₆ haloalkyl and cyano; m is 1, 2, 3 or 4; 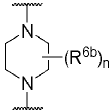 is 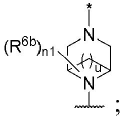 u is 1 or 2, n1 is 0, and R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 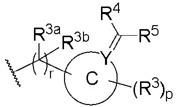 is selected from the group consisting of 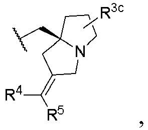 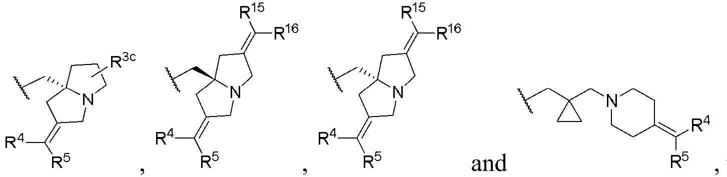 wherein R⁴ and R⁵ are the same or different, and are each independently selected from the group consisting of hydrogen atom, halogen and C₁₋₆ alkyl; R^{3c} is hydrogen atom or halogen; R¹⁵ is hydrogen atom or halogen; R¹⁶ is hydrogen atom or halogen.

In some embodiments of the present disclosure, provided is the compound represented by general formula (V) or the pharmaceutically acceptable salt thereof, wherein Q is O; t is 1 or 2, and s is 1 or 2; R² is selected from the group consisting of hydrogen atom, halogen, C₁₋₆ alkyl and C₁₋₆ haloalkyl; q is 0; G¹ is N; 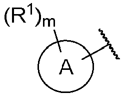 is selected from the group consisting of 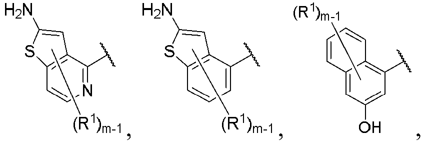 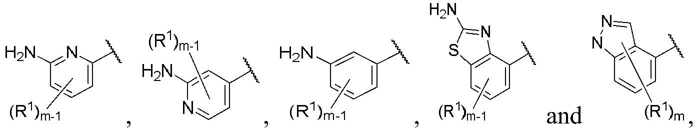 wherein each R¹ is the same or different, and is independently selected from the group consisting of halogen, C₁₋₆ alkyl, C₁₋₆ haloalkyl and cyano; m is 1, 2, 3 or 4; 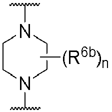 is 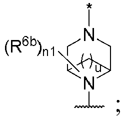 u is 1 or 2, n1 is 0, and R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 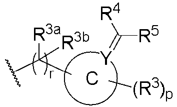 is selected from the group consisting of

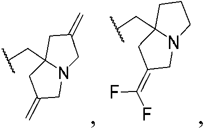

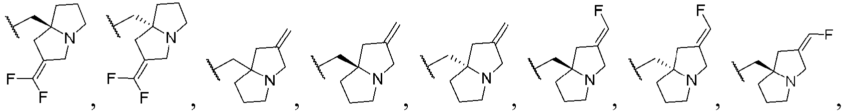

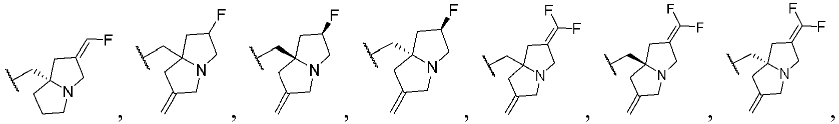

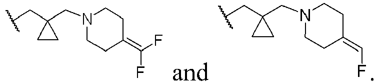

In some embodiments of the present disclosure, provided is the compound represented by general formula (II) or the pharmaceutically acceptable salt thereof, wherein G¹ is CH or N; G is CR^{G} or N, and R^{G} is selected from the group consisting of hydrogen atom, halogen, C₁₋₆ alkyl and C₁₋₆ haloalkyl; R⁷ is hydrogen atom; R² is selected from the group consisting of hydrogen atom, halogen, C₁₋₆ alkyl and C₁₋₆ haloalkyl; 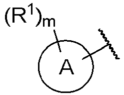 is selected from the group consisting of 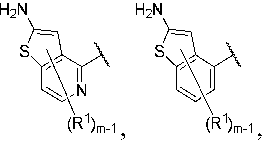 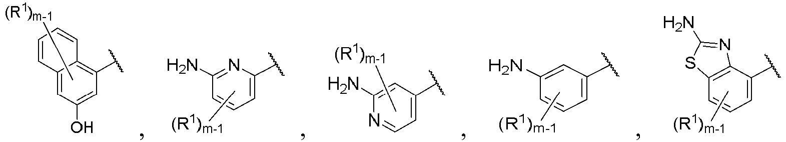 and 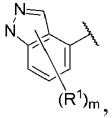 wherein each R¹ is the same or different, and is independently selected from the group consisting of halogen, C₁₋₆ alkyl, C₁₋₆ haloalkyl and cyano; m is 1, 2, 3 or 4; 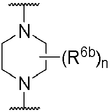 is selected from the group consisting of 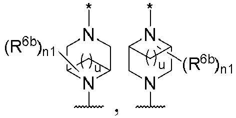 and 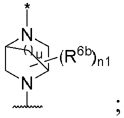 u is 1 or 2, n1 is 0, the * end is connected to R^{6a}, and R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 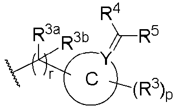 is selected from the group consisting of 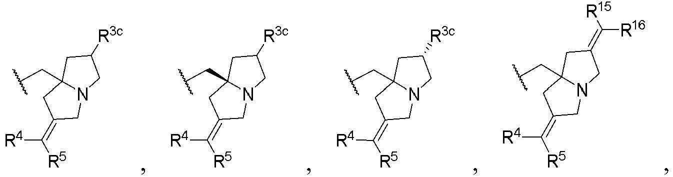 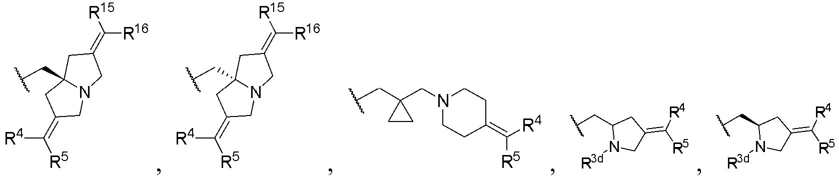 and 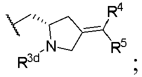 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen).

In some embodiments of the present disclosure, provided is the compound represented by general formula (V) or the pharmaceutically acceptable salt thereof, wherein Q is O; t is 1 or 2, and s is 1 or 2; R² is selected from the group consisting of hydrogen atom, halogen, C₁₋₆ alkyl and C₁₋₆ haloalkyl; q is 0; G¹ is N; 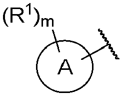 is selected from the group consisting of 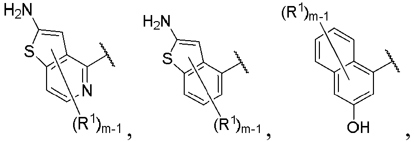 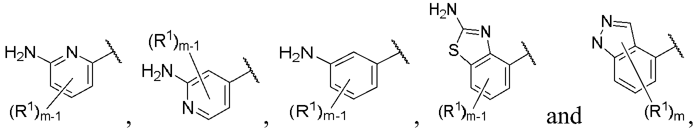 wherein each R¹ is the same or different, and is independently selected from the group consisting of halogen, C₁₋₆ alkyl, C₁₋₆ haloalkyl and cyano; m is 1, 2, 3 or 4; 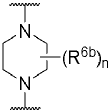 is selected from the group consisting of 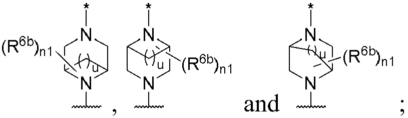 u is 1 or 2, n1 is 0, the * end is connected to R^{6a}, and R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 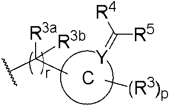 is selected from the group consisting of 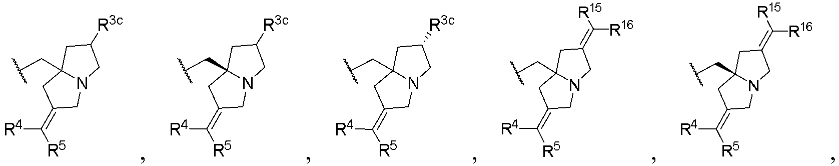 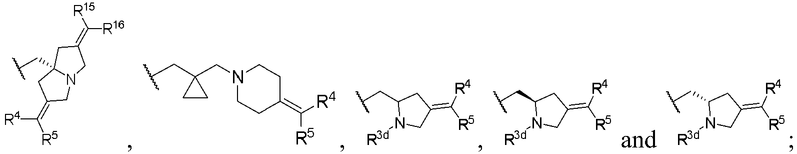 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen).

In some embodiments of the present disclosure, provided is the compound represented by general formula (V) or the pharmaceutically acceptable salt thereof, wherein Q is O; t is 1 or 2, and s is 1 or 2; R² is selected from the group consisting of hydrogen atom, halogen, C₁₋₆ alkyl and C₁₋₆ haloalkyl; q is 0; G¹ is N; 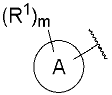 is selected from the group consisting of 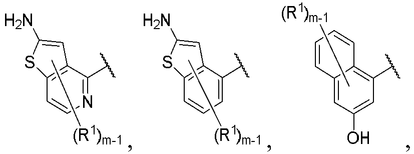 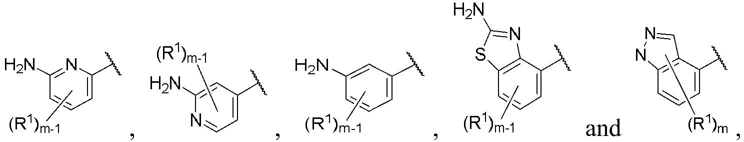 wherein each R¹ is the same or different, and is independently selected from the group consisting of halogen, C₁₋₆ alkyl, C₁₋₆ haloalkyl and cyano; m is 1, 2, 3 or 4; 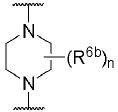 is selected from the group consisting of 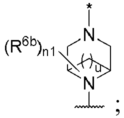 u is 1 or 2, and n1 is 0; the * end is connected to R^{6a}, and R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 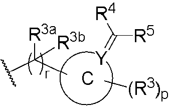 is selected from the group consisting of 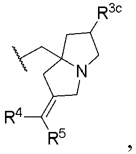 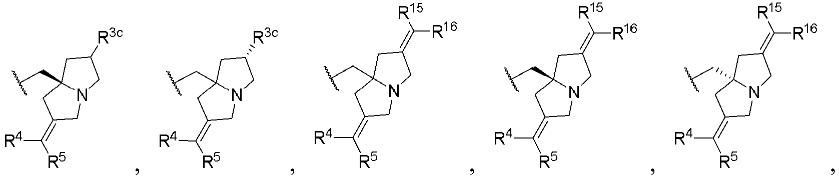 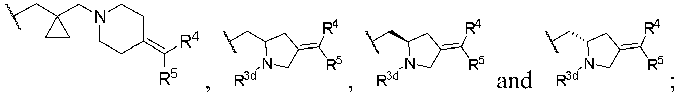 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen).

In some embodiments of the present disclosure, provided is the compound represented by general formula (V) or the pharmaceutically acceptable salt thereof, wherein Q is O; t is 1 or 2, and s is 1 or 2; R² is selected from the group consisting of hydrogen atom, halogen, C₁₋₆ alkyl and C₁₋₆ haloalkyl; q is 0; G¹ is N; 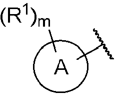 is 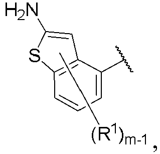 and each R¹ is the same or different, and is independently selected from the group consisting of halogen, C₁₋₆ alkyl, C₁₋₆ haloalkyl and cyano; m is 1, 2, 3 or 4; 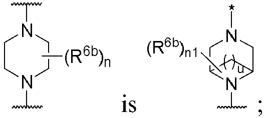 u is 1 or 2, n1 is 0, and the * end is connected to R^{6a}; R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; W is O; 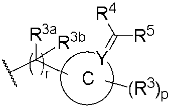 is selected from the group consisting of 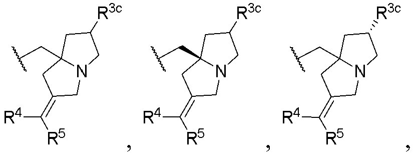 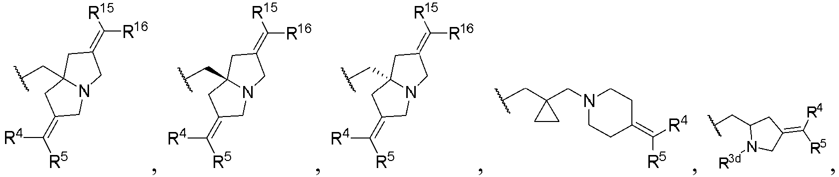 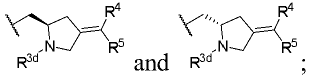 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen).

In some embodiments of the present disclosure, provided is the compound represented by general formula (V) or the pharmaceutically acceptable salt thereof, wherein Q is O; t is 1, and s is 1; R² is halogen; q is 0; G¹ is N; 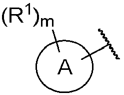 is 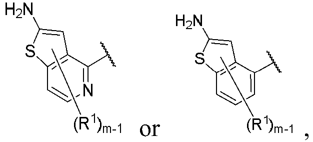 and each R¹ is the same or different, and is independently halogen or cyano; m-1 is 2; 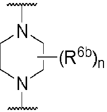 is 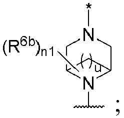 u is 2, n1 is 0, and R^{6a} is C₁₋₆ hydroxyalkyl; W is O; 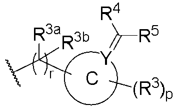 is 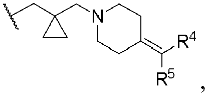 and R⁴ and R⁵ are the same or different, and are each independently hydrogen atom or halogen.

In some embodiments of the present disclosure, provided is the compound represented by general formula (IN) or the pharmaceutically acceptable salt thereof, wherein ring B is 5- or 6-membered heterocyclyl; each R⁸ is the same or different, and is independently selected from the group consisting of halogen, C₁₋₆ alkyl, C₁₋₆ haloalkyl and cyano; q is 0, 1 or 2; G is C or N; R² is selected from the group consisting of hydrogen atom, halogen, C₁₋₆ alkyl and C₁₋₆ haloalkyl; G¹ is N; 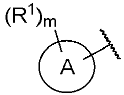 is selected from the group consisting of 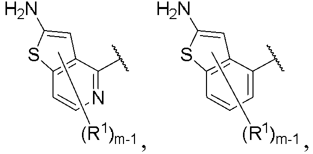 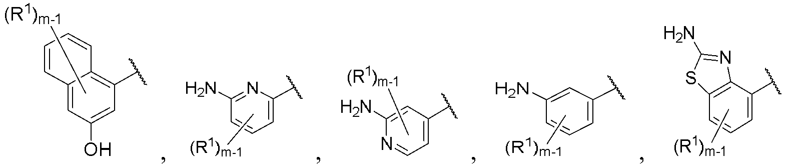 and 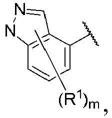 wherein each R¹ is the same or different, and is independently selected from the group consisting of halogen, C₁₋₆ alkyl, C₁₋₆ haloalkyl and cyano; m is 1, 2, 3 or 4; R^{B} is hydrogen atom or 3- to 10-membered heterocyclyl, wherein the 3- to 10-membered heterocyclyl is optionally substituted with one or more R⁶, each R⁶ is the same or different, and is independently selected from the group consisting of C₁₋₆ hydroxyalkyl, C(O)NR¹¹R¹² and C(O)R¹⁴, and R¹¹ is hydrogen atom or C₁₋₆ alkyl; R¹² is hydrogen atom or C₁₋₆ alkyl; R¹⁴ is selected from the group consisting of C₁₋₆ alkyl, 3- to 6-membered cycloalkyl and 5- or 6-membered heteroaryl, wherein the C₁₋₆ alkyl, 3- to 6-membered cycloalkyl and 5- or 6-membered heteroaryl are each independently and optionally substituted with one or more substituents selected from the group consisting of halogen, cyano and C₁₋₆ alkyl; W is O; 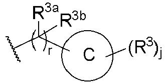 is selected from the group consisting of 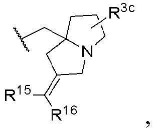 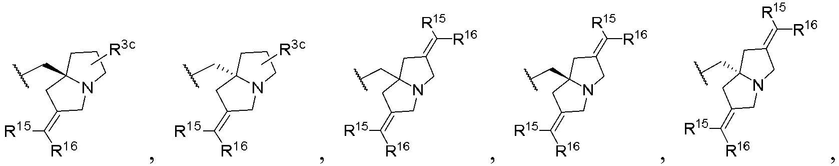 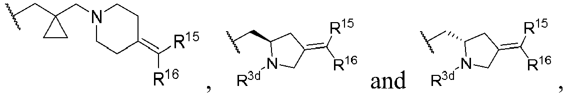 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, and R¹⁵ and R¹⁶ are the same or different, and are each independently selected from the group consisting of hydrogen atom, halogen, C₁₋₆ alkyl, C₁₋₆ haloalkyl and 3- to 6-membered cycloalkyl (in some embodiments, hydrogen atom or halogen).

In some embodiments of the present disclosure, provided is the compound represented by general formula (IN) or the pharmaceutically acceptable salt thereof, wherein ring B is hydrogenated furyl; each R⁸ is the same or different, and is independently selected from the group consisting of halogen, C₁₋₆ alkyl, C₁₋₆ haloalkyl and cyano; q is 0, 1 or 2; G is C; R² is selected from the group consisting of hydrogen atom, halogen, C₁₋₆ alkyl and C₁₋₆ haloalkyl; G¹ is N; 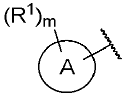 is selected from the group consisting of 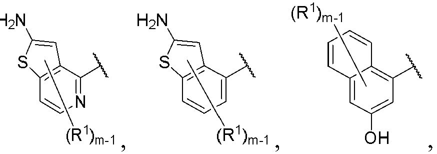 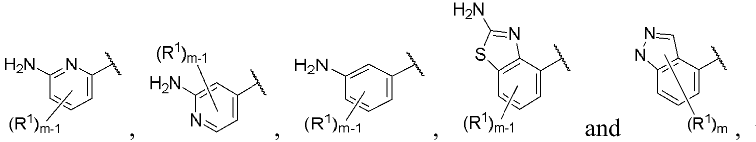 wherein each R¹ is the same or different, and is independently selected from the group consisting of halogen, C₁₋₆ alkyl, C₁₋₆ haloalkyl and cyano; m is 1, 2, 3 or 4; R^{B} is selected from the group consisting of hydrogen atom, 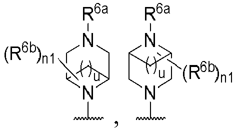 and 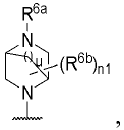 R^{6a} is hydrogen atom or R⁶, R^{6b} is R⁶, and u is 0, 1 or 2; n1 is 0, 1, 2 or 3; each R⁶ is the same or different, and is independently C₁₋₆ hydroxyalkyl; W is O; 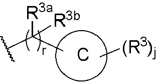 is selected from the group consisting of 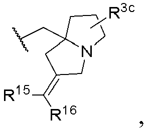 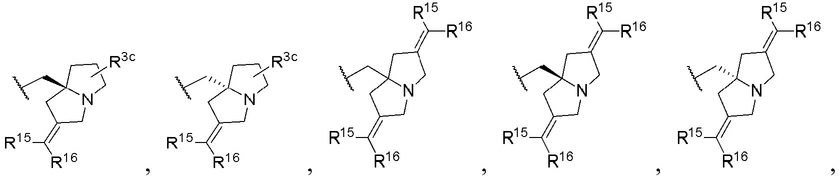 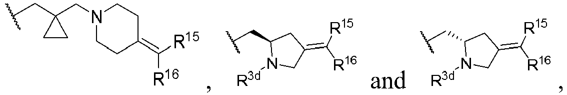 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, and R¹⁵ and R¹⁶ are the same or different, and are each independently selected from the group consisting of hydrogen atom, halogen, C₁₋₆ alkyl, C₁₋₆ haloalkyl and 3- to 6-membered cycloalkyl (in some embodiments, hydrogen atom or halogen).

In some embodiments of the present disclosure, provided is the compound represented by general formula (IN) or the pharmaceutically acceptable salt thereof, wherein ring B is hydrogenated furyl; each R⁸ is the same or different, and is independently selected from the group consisting of halogen, C₁₋₆ alkyl, C₁₋₆ haloalkyl and cyano; q is 0, 1 or 2; G is C; R² is selected from the group consisting of hydrogen atom, halogen, C₁₋₆ alkyl and C₁₋₆ haloalkyl; G¹ is N; 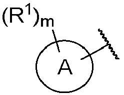 is selected from the group consisting of 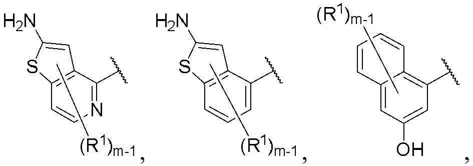 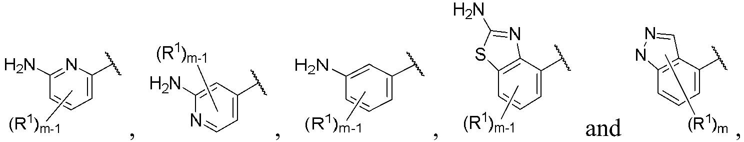 wherein each R¹ is the same or different, and is independently selected from the group consisting of halogen, C₁₋₆ alkyl, C₁₋₆ haloalkyl and cyano; m is 1, 2, 3 or 4; R^{B} is selected from the group consisting of hydrogen atom, 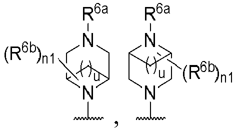 and 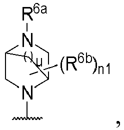 R^{6a} is hydrogen atom or R⁶, and u is 1 or 2; n1 is 0; R⁶ is OR¹⁴, and R¹⁴ is hydrogen atom or C₁₋₆ alkyl; W is O; 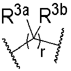 is -CH₂- or 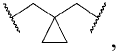 ring C is 3- to 8-membered heterocyclyl, and each R³ is the same or different, and is independently selected from the group consisting of C₁₋₆ alkyl, C₁₋₆ haloalkyl, halogen and =CR¹⁵R¹⁶, wherein R¹⁵ and R¹⁶ are the same or different, and are each independently selected from the group consisting of hydrogen atom, halogen and C₁₋₆ alkyl; j is 0, 1, 2 or 3.

In some embodiments of the present disclosure, provided is the compound represented by general formula (IN) or the pharmaceutically acceptable salt thereof, wherein ring B is hydrogenated furyl; each R⁸ is the same or different, and is independently selected from the group consisting of halogen, C₁₋₆ alkyl, C₁₋₆ haloalkyl and cyano; q is 0, 1 or 2; G is C; R² is selected from the group consisting of hydrogen atom, halogen, C₁₋₆ alkyl and C₁₋₆ haloalkyl; G¹ is N; 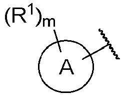 is selected from the group consisting of 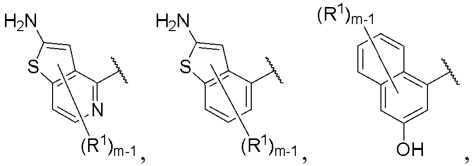 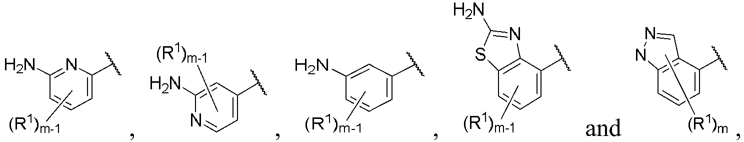 wherein each R¹ is the same or different, and is independently selected from the group consisting of halogen, C₁₋₆ alkyl, C₁₋₆ haloalkyl and cyano; m is 1, 2, 3 or 4; R^{B} is selected from the group consisting of hydrogen atom, 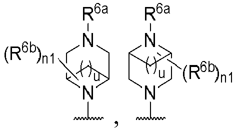 and 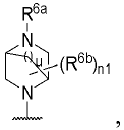 R^{6a} is hydrogen atom or R⁶, and u is 0, 1 or 2; n1 is 0; R⁶ is OR¹⁴, and R¹⁴ is hydrogen atom or C₁₋₆ alkyl; W is O; 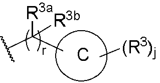 is selected from the group consisting of 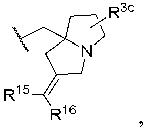 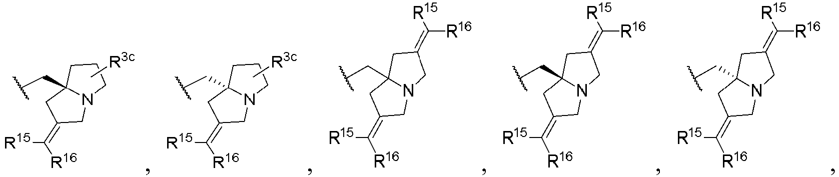 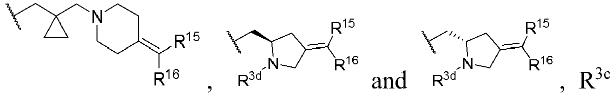 is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, and R¹⁵ and R¹⁶ are the same or different, and are each independently selected from the group consisting of hydrogen atom, halogen, C₁₋₆ alkyl, C₁₋₆ haloalkyl and 3- to 6-membered cycloalkyl (in some embodiments, hydrogen atom or halogen).

In some embodiments of the present disclosure, provided is the compound represented by general formula (IN) or the pharmaceutically acceptable salt thereof, wherein ring B is 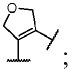 q is 0; G is C; R² is selected from the group consisting of hydrogen atom, halogen, C₁₋₆ alkyl and C₁₋₆ haloalkyl; G¹ is N; 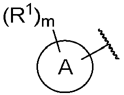 is selected from the group consisting of 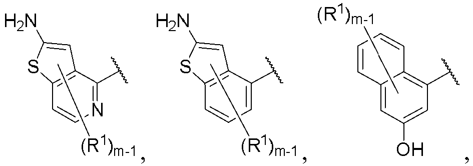 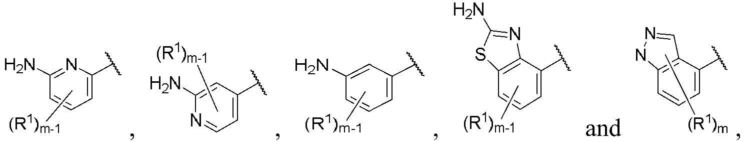 wherein each R¹ is the same or different, and is independently selected from the group consisting of halogen, C₁₋₆ alkyl, C₁₋₆ haloalkyl and cyano; m is 1, 2, 3 or 4; R^{B} is selected from the group consisting of hydrogen atom, C₁₋₆ alkyl, C₂₋₆ alkenyl, C₂₋₆ alkynyl, 3- to 6-membered cycloalkyl and 3- to 10-membered heterocyclyl, wherein the C₁₋₆ alkyl, C₂₋₆ alkenyl, C₂₋₆ alkynyl, 3- to 6-membered cycloalkyl and 3- to 10-membered heterocyclyl are each independently and optionally substituted with one or more substituents selected from the group consisting of halogen, cyano, hydroxyl, C₁₋₆ alkyl, C₁₋₆ alkoxy and 3- to 6-membered cycloalkyl, and W is O; 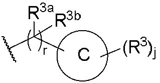 is selected from the group consisting of 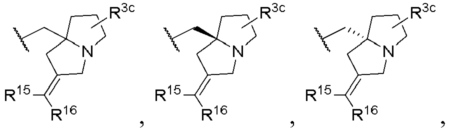 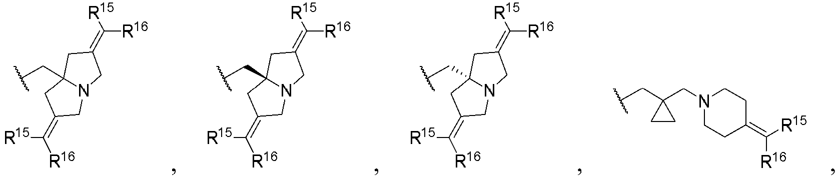 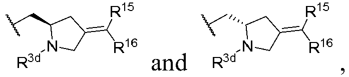 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, and R¹⁵ and R¹⁶ are the same or different, and are each independently selected from the group consisting of hydrogen atom, halogen, C₁₋₆ alkyl, C₁₋₆ haloalkyl and 3- to 6-membered cycloalkyl (in some embodiments, hydrogen atom or halogen).

In some embodiments of the present disclosure, provided is the compound represented by general formula (IN) or the pharmaceutically acceptable salt thereof, wherein ring B is 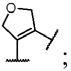 q is 0; G is C; R² is selected from the group consisting of hydrogen atom, halogen, C₁₋₆ alkyl and C₁₋₆ haloalkyl; G¹ is N; 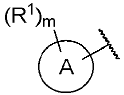 is selected from the group consisting of 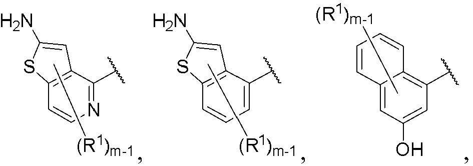 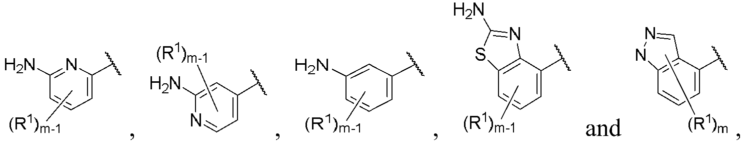 wherein each R¹ is the same or different, and is independently selected from the group consisting of halogen, C₁₋₆ alkyl, C₁₋₆ haloalkyl and cyano; m is 1, 2, 3 or 4; R^{B} is selected from the group consisting of hydrogen atom, methyl, ethenyl, ethynyl, cyclopropyl and cyclobutyl; W is O; 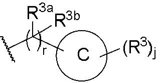 is selected from the group consisting of

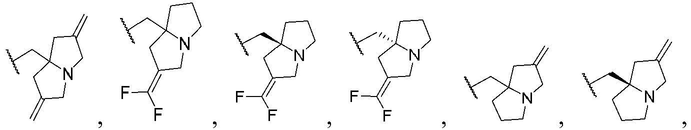

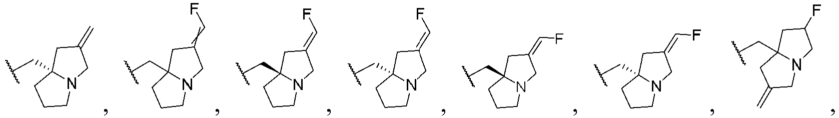

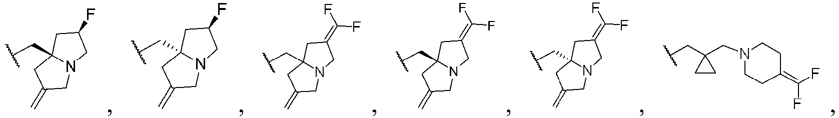

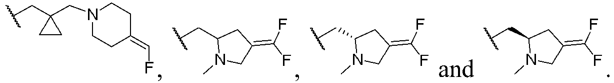

In some embodiments of the present disclosure, provided is the compound represented by general formula (I) or the pharmaceutically acceptable salt thereof, wherein R⁷ and G together form ring B, and ring B is 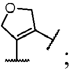 G is C; G¹ is N; R² is selected from the group consisting of hydrogen atom, halogen, C₁₋₆ alkyl and C₁₋₆ haloalkyl; R^{B} is hydrogen atom; 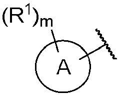 is selected from the group consisting of 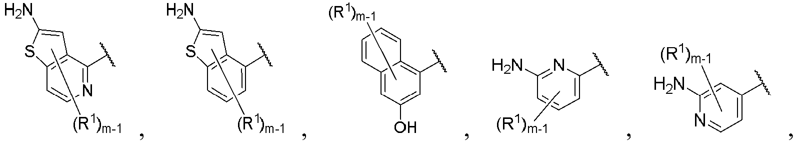 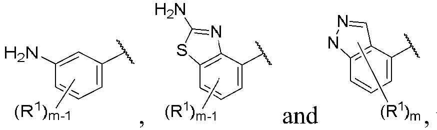 wherein each R¹ is the same or different, and is independently selected from the group consisting of halogen, C₁₋₆ alkyl, C₁₋₆ haloalkyl and cyano; m is 1, 2, 3 or 4; R^{A} is 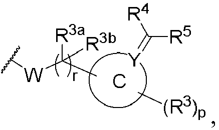 and W is O; 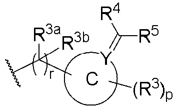 is selected from the group consisting of 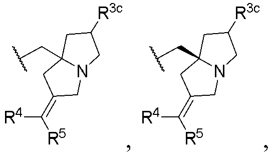 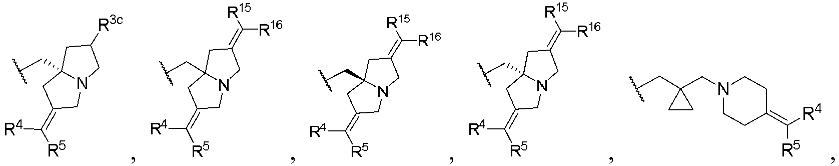 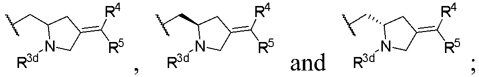 R^{3c} is selected from the group consisting of hydrogen atom, halogen, C₁₋₆ alkyl, C₁₋₆ haloalkyl and 3- to 6-membered cycloalkyl (in some embodiments, hydrogen atom or halogen), R^{3d} is hydrogen atom or C₁₋₆ alkyl, R⁴ and R⁵ are the same or different, and are each independently selected from the group consisting of hydrogen atom, halogen and C₁₋₆ alkyl (in some embodiments, hydrogen atom or halogen), and R¹⁵ and R¹⁶ are the same or different, and are each independently selected from the group consisting of hydrogen atom, halogen and C₁₋₆ alkyl (in some embodiments, hydrogen atom or halogen).

In some embodiments of the present disclosure, provided is the compound represented by general formula (I) or the pharmaceutically acceptable salt thereof, wherein R⁷ and G together form ring B, and ring B is 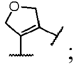 G is C; G¹ is N; R² is halogen; R^{B} is hydrogen atom; 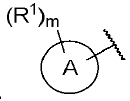 is selected from the group consisting of 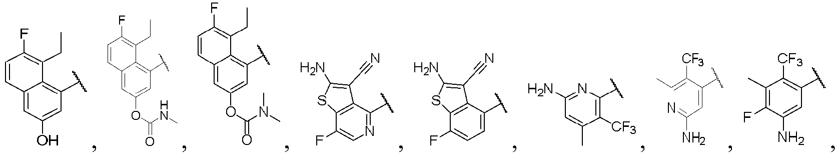 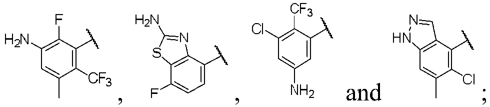 R^{A} is 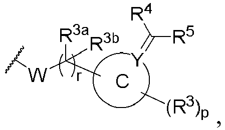 and 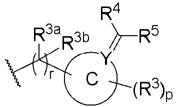 is selected from the group consisting of

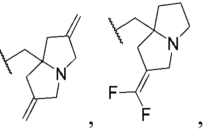

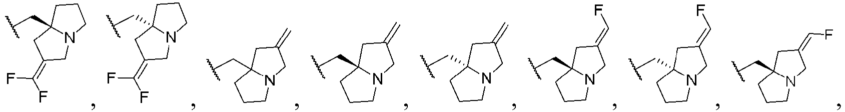

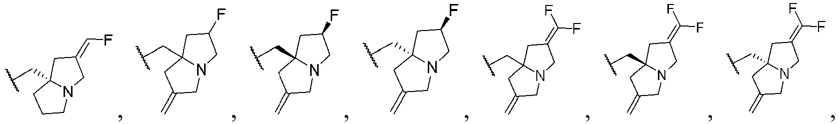

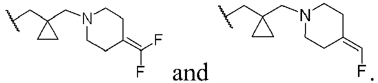

In some embodiments of the present disclosure, provided is the compound represented by general formula (IN) or the pharmaceutically acceptable salt thereof, wherein ring B is 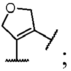 q is 0; G is C; R² is halogen; G¹ is N; 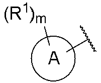 is selected from the group consisting of 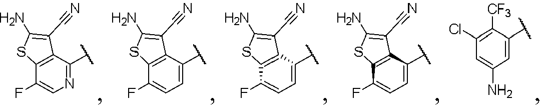 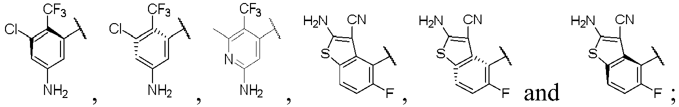 R^{B} is selected from the group consisting of 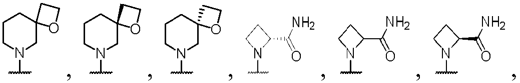 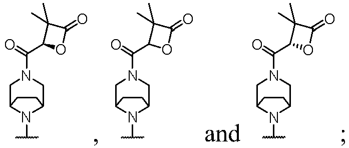 W is O; 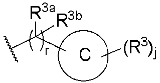 is selected from the group consisting of

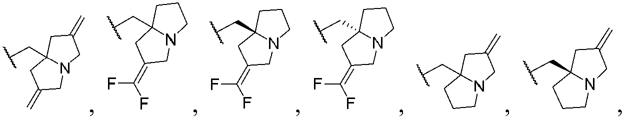

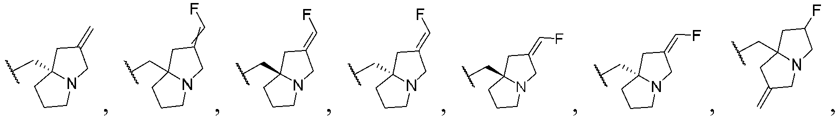

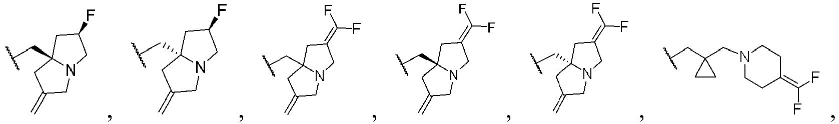

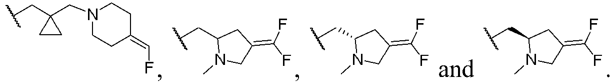

In some embodiments of the present disclosure, provided is the compound represented by general formula (VI) or the pharmaceutically acceptable salt thereof, wherein q is 0; R² is halogen; G¹ is N; m is 3 or 4; each R¹ is the same or different, and is independently selected from the group consisting of halogen, C₁₋₆ alkyl, C₁₋₆ haloalkyl and cyano; ring A is selected from the group consisting of 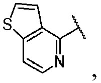 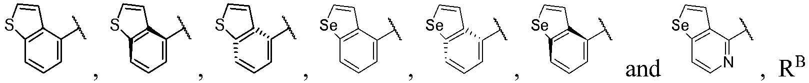 is selected from the group consisting of hydrogen atom, 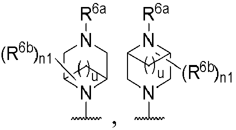 and 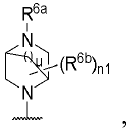 R^{6a} is C₁₋₆ hydroxyalkyl, and u is 1 or 2; n1 is 0; W is O; 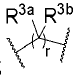 is-CH₂- or 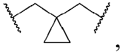 ring C is 3- to 8-membered heterocyclyl, and R⁴ and R⁵ are the same or different, and are each independently hydrogen atom or halogen; each R³ is the same or different, and is independently halogen or =CR¹⁵R¹⁶, and R¹⁵ and R¹⁶ are the same or different, and are each independently hydrogen atom or halogen; p is 0 or 1.

In some embodiments of the present disclosure, provided is the compound represented by general formula (VI) or the pharmaceutically acceptable salt thereof, wherein q is 0; R² is halogen; G¹ is N; m-1 is 2; each R¹ is the same or different, and is independently selected from the group consisting of halogen, C₁₋₆ alkyl, C₁₋₆ haloalkyl and cyano; ring A is selected from the group consisting of 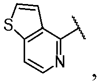 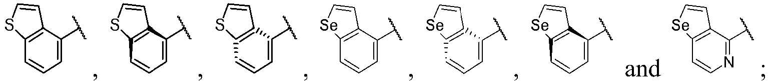 R^{B} is selected from the group consisting of hydrogen atom, 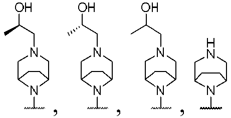 and 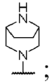 W is O; 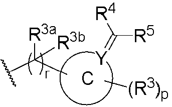 is selected from the group consisting of

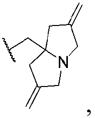

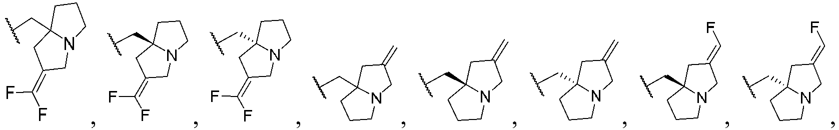

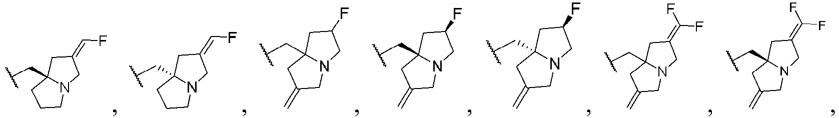

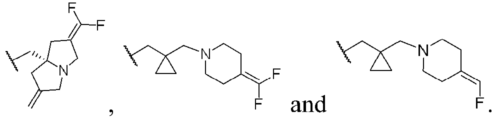

In some embodiments of the present disclosure, provided is the compound represented by general formula (VII-1) or (VII-2) or the pharmaceutically acceptable salt thereof, wherein q is 0; R² is halogen; G¹ is N; U is S or Se; V is CH or N; each R¹ is the same or different, and is independently selected from the group consisting of halogen, C₁₋₆ alkyl, C₁₋₆ haloalkyl, cyano, -NH₂ and hydroxyl; m2 is 0, 1, 2 or 3; R⁴ and R⁵ are the same or different, and are each independently hydrogen atom or halogen; R^{3c} is selected from the group consisting of hydrogen atom, halogen and =CR¹⁵R¹⁶, and R¹⁵ is hydrogen atom or halogen; R¹⁶ is hydrogen atom or halogen; R^{B} is selected from the group consisting of hydrogen atom, 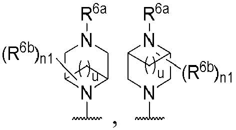 and 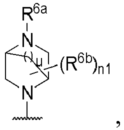 R^{6a} is C₁₋₆ hydroxyalkyl, and u is 1 or 2; n1 is 0.

In some embodiments of the present disclosure, provided is the compound represented by general formula (VII-1) or (VII-2) or the pharmaceutically acceptable salt thereof, wherein q is 0; R² is halogen; G¹ is N; R⁴ and R⁵ are the same or different, and are each independently hydrogen atom or halogen; R^{3c} is selected from the group consisting of hydrogen atom, F, =CH₂, =CHF and =CF₂; 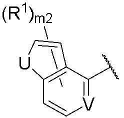 is selected from the group consisting of 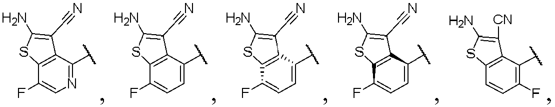 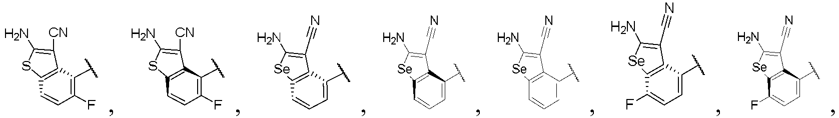 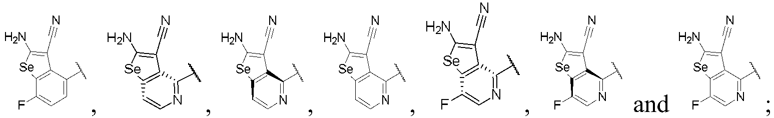 R^{B} is selected from the group consisting of hydrogen atom, 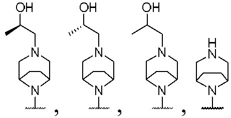 and

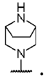

In some embodiments of the present disclosure, provided is the compound represented by general formula (VII-1) or (VII-2) or the pharmaceutically acceptable salt thereof, wherein q is 0; R² is halogen; G¹ is N; U is S or Se; V is CH or N; each R¹ is the same or different, and is independently selected from the group consisting of halogen, C₁₋₆ alkyl, C₁₋₆ haloalkyl, cyano, -NH₂ and hydroxyl; m2 is 0, 1, 2 or 3; R⁴ and R⁵ are the same or different, and are each independently hydrogen atom or halogen; R^{3c} is selected from the group consisting of hydrogen atom, F, =CH₂, =CHF and =CF₂; R^{B} is 3- to 10-membered heterocyclyl, wherein the 3- to 10-membered heterocyclyl is optionally substituted with one or more R⁶, and R⁶ is .

In some embodiments of the present disclosure, provided is the compound represented by general formula (VII-1) or (VII-2) or the pharmaceutically acceptable salt thereof, wherein q is 0; R² is halogen; G¹ is N; U is S; V is CH or N; each R¹ is the same or different, and is independently selected from the group consisting of halogen, cyano and amino; m2 is 2 or 3; R⁴ and R⁵ are the same or different, and are each independently hydrogen atom or halogen; R^{3c} is selected from the group consisting of hydrogen atom, halogen and =CR¹⁵R¹⁶, and R¹⁵ and R¹⁶ are the same or different, and are each independently hydrogen atom or halogen; R^{B} is hydrogen atom.

**Table A. Typical compounds of the present disclosure, including but not limited to:**

| Compound No. | Compound structure | Name |
|---|---|---|
| **1i** | 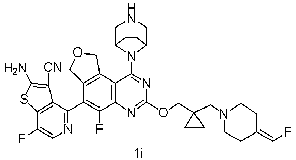 | 4-(1-(3,8-Diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-*f*]quinazolin-6-yl)-2-amino-7-fluorothieno[3,2-*c*]pyridine-3-carbonitrile **1i** |
| 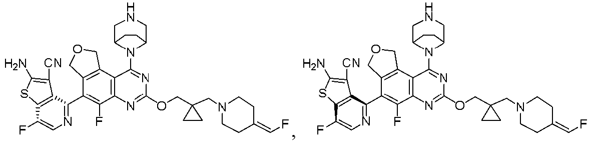 | | |
| **1** | 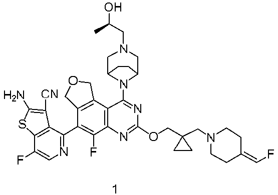 | 2-Amino-7-fluoro-4-(5-fluoro-3-((1-((4-(fluoromethylene)piperidin-1-yl)methyl)cyclopropyl)methoxy)-1-(3-((*R*)-2-hydroxypropyl)-3,8-diazabicyclo[3.2.1]oct-8-yl)-7,9-dihydrofuro[3,4-*f*]quinazolin-6-yl)thieno[3,2-*c*]pyridine-3-carbonitrile 1 |
| 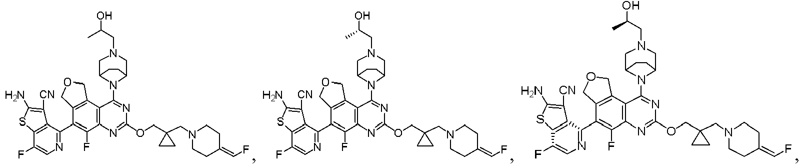 | | |
| 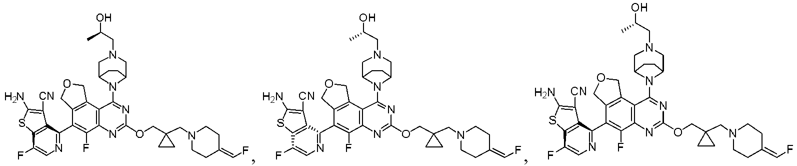 | | |
| **2** | 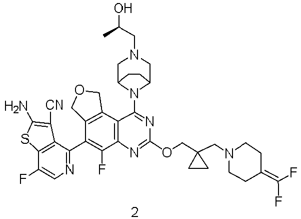 | 2-Amino-4-(3-((1-((4-(difluoromethylene)piperidin-1-yl)methyl)cyclopropyl)methoxy)-5-fluoro-1-(3-((*R*)-2-hydroxypropyl)-3,8-diazabicyclo[3.2.1]oct-8-yl)-7,9-dihydrofuro[3,4-*f*]quinazolin-6-yl)-7-fluorothieno[3,2-*c*]pyridine-3-carbonitrile **2** |
| 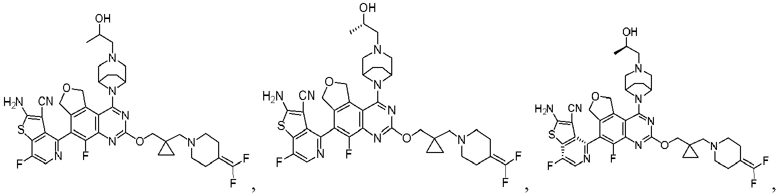 | | |
| 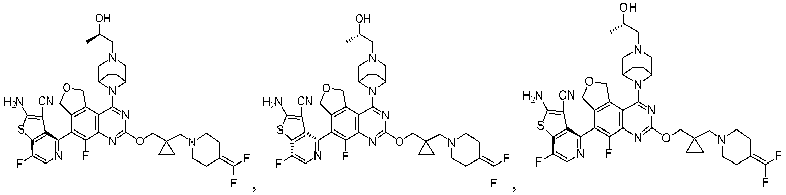 | | |
| **3** | 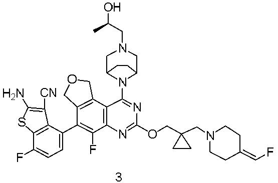 | 2-Amino-7-fluoro-4-(5-fluoro-3-((1-((4-(fluoromethylene)piperidin-1-yl)methyl)cyclopropyl)methoxy)-1-(3-((*R*)-2-hydroxypropyl)-3,8-diazabicyclo[3.2.1]oct-8-yl)-7,9-dihydrofuro[3,4-*f*]quinazolin-6-yl)benzo[*b*]thiophene-3-carbonitrile **3** |
| 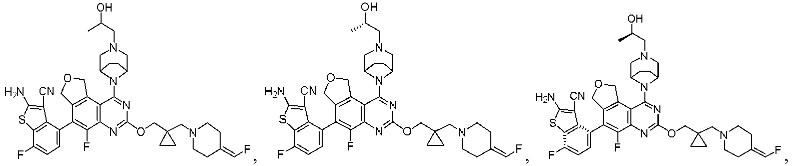 | | |
| 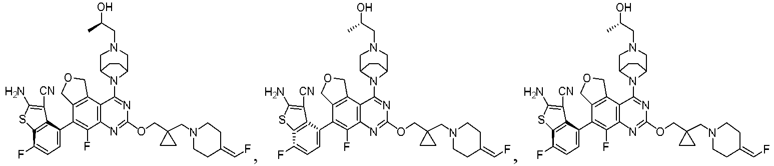 | | |
| **4** | 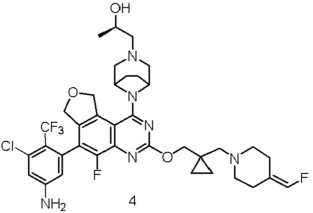 | (2R)-1-(8-(6-(5-Amino-3-chloro-2-(trifluoromethyl)phenyl)-5-fluoro-3-((1-((4-(fluoromethylene)piperidin-1-yl)methyl)cyclopropyl)methoxy)-7,9-dihydrofuro[3,4-*f*]quinazolin-1-yl)-3,8-diazabicyclo[3.2.1]oct-3-yl)propan-2-ol **4** |
| 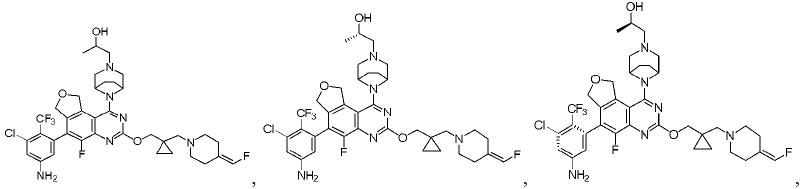 | | |
| 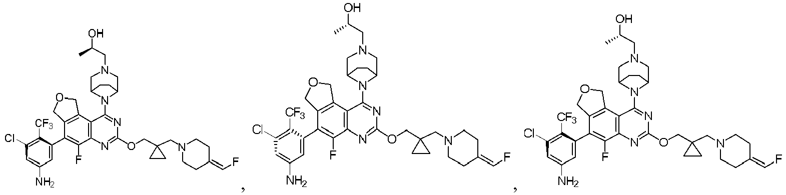 | | |
| **5** | 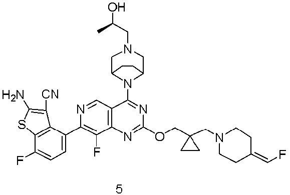 | 2-Amino-7-fluoro-4-(8-fluoro-2-((1-((4-(fluoromethylene)piperidin-1-yl)methyl)cyclopropyl)methoxy)-4-(3-((*R*)-2-hydroxypropyl)-3,8-diazabicyclo[3.2.1]oct-8-yl)pyrido[4,3-*d*]pyrimidin-7-yl)benzo[*b*]thiophene-3-carbonitrile **5** |
| 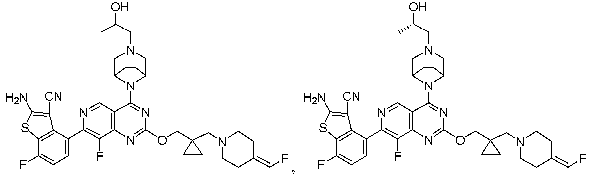 | | |
| **6** | 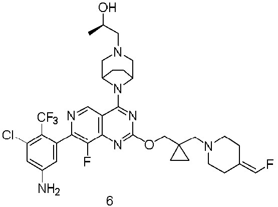 | (*R*)-1-(8-(7-(5-Amino-3-chloro-2-(trifluoromethyl)phenyl)-8-fluoro-2-((1-((4-(fluoromethylene)piperidin-1-yl)methyl)cyclopropyl)methoxy)pyrido[4,3-*d*]pyrimidin-4-yl)-3,8-diazabicyclo[3.2.1]oct-3-yl)propan-2-ol **6** |
| 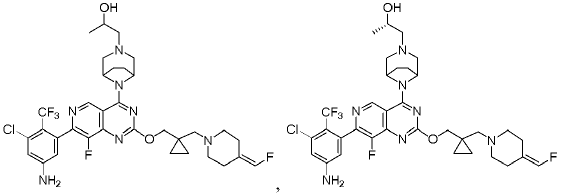 | | |
| **7** | 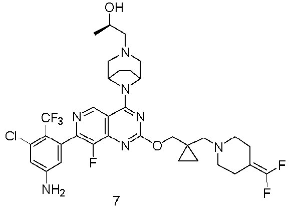 | (R)-1-(8-(7-(5-Amino-3-chloro-2-(trifluoromethyl)phenyl)-2-((1-((4-(difluoromethylene)piperidin-1-yl)methyl)cyclopropyl)methoxy)-8-fluoropyrido[4,3-*d*]pyrimidin-4-yl)-3,8-diazabicyclo[3.2.1]oct-3-yl)propan-2-ol **7** |
| 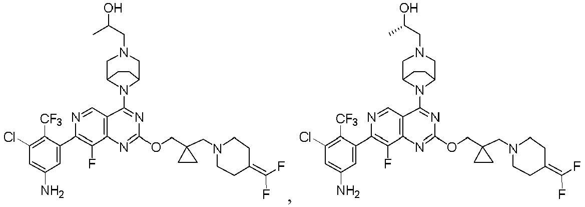 | | |
| **8** | 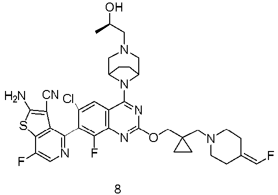 | 2-Amino-4-(6-chloro-8-fluoro-2-((1-((4-(fluoromethylene)piperidin-1-yl)methyl)cyclopropyl)methoxy)-4-(3-((*R*)-2-hydroxypropyl)-3,8-diazabicyclo[3.2.1]oct-8-yl)quinazolin-7-yl)-7-fluorothieno[3,2-c]pyridine-3-carbonitrile **8** |
| 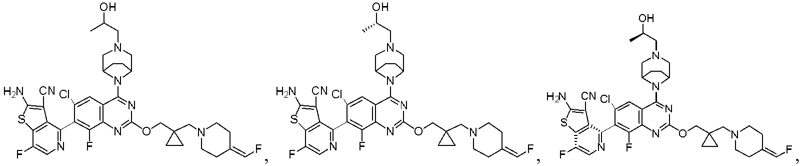 | | |
| 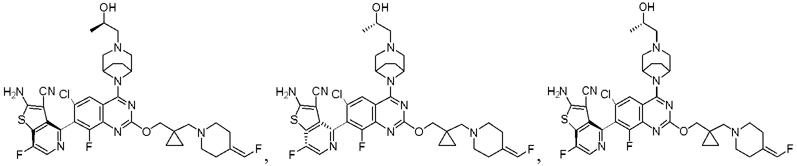 | | |
| **9** | 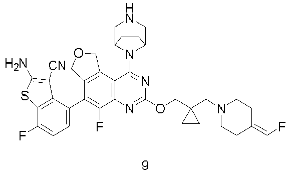 | 4-(1-(3,8-Diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorobenzo[b]thiophene-3-carbonitrile **9** |
| **9-p1** | 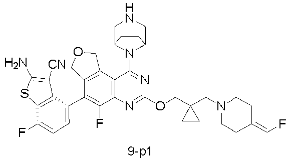 | 4-((*R*)-1-(-3,8-Diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile **9-p1** |
| **9-p2** | 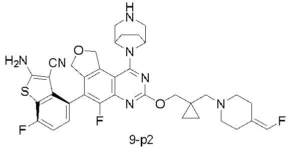 | 4-((*S*)-1-(3,8-Diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile **9-p2** |
| **10** | 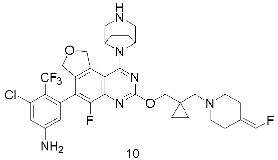 | 3-(1-(3,8-Diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-5-chloro-4-(trifluoromethyl)aniline **10** |
| 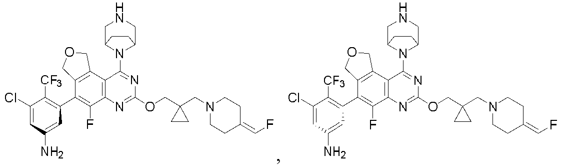 | | |
| **11** | 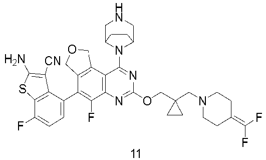 | 4-(1-(3,8-Diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorobenzo[b]thiophene-3-carbonitrile **11** |
| **11-p1** | 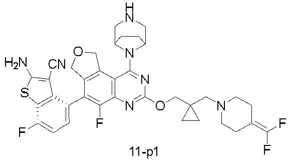 | 4-((*R*)-1-(3,8-Diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile **11-p1** |
| **11-p2** | 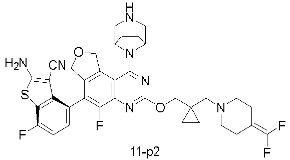 | 4-((*S*)-1-(3,8-Diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile **11-p2** |
| **12** | 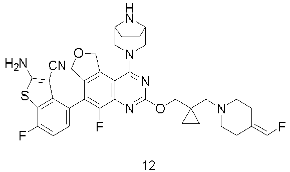 | 4-(1-(3,8-Diazabicyclo[3.2.1]oct-3-yl)-5-fluoro-3-((1-((4-(fluoromethylene)piperidin-1-yl)methyl)cyclopropyl)methoxy)-7,9-dihydrofuro[3,4-*f*]quinazolin-6-yl)-2-amino-7-fluorobenzo[*b*]thiophene-3-carbonitrile **12** |
| 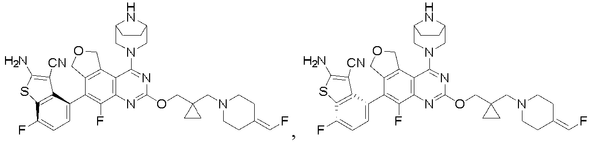 | | |
| **13** | 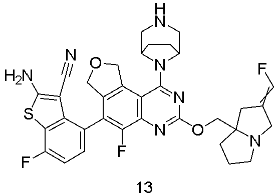 | 4-(1-(3,8-Diazabicyclo[3.2.1]oct-8-yl)-5-fluoro-3-((2-(fluoromethylene)tetrahydro-1*H-*pyrrolizin-7a(5*H*)-yl)methoxy)-7,9-dihydrofuro[3,4-*f*]quinazolin-6-yl)-2-amino-7-fluorobenzo[*b*]thiophene-3-carbonitrile **13** |
| 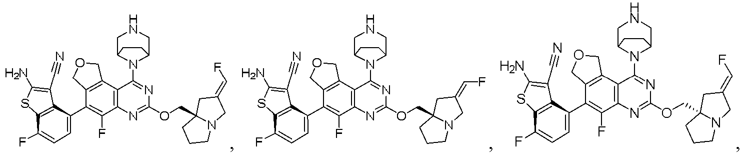 | | |
| 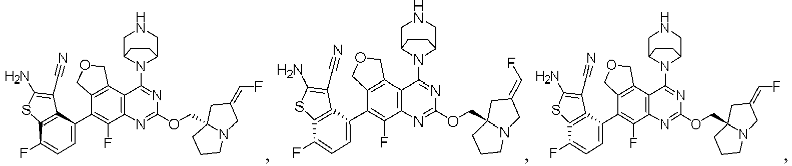 | | |
| 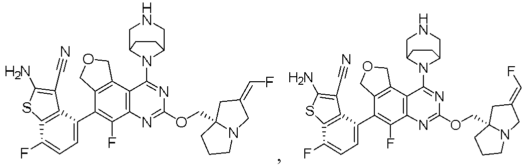 | | |
| **14** | 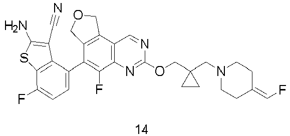 | 2-Amino-7-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)benzo[*b*]thiophene-3-carbonitrile **14** |
| **14-p1** | 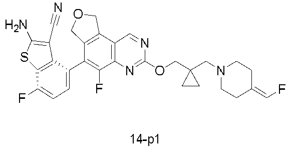 | (*R*)-2-Amino-7-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)benzo[*b*]thiophene-3-carbonitrile **14-p1** |
| **14-p2** | 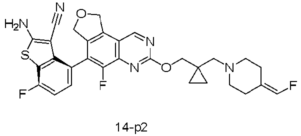 | (*S*)-2-Amino-7-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)benzo[*b*]thiophene-3-carbonitrile **14-p2** |
| **15** | 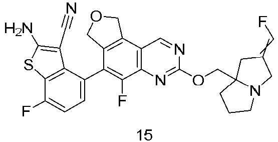 | 2-Amino-7-fluoro-4-(5-fluoro-3-((2-(fluoromethylene)tetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **15** |
| 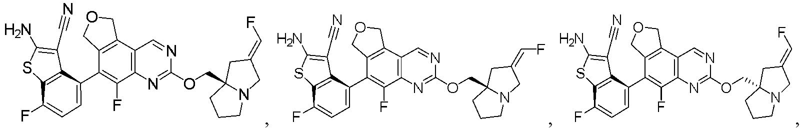 | | |
| 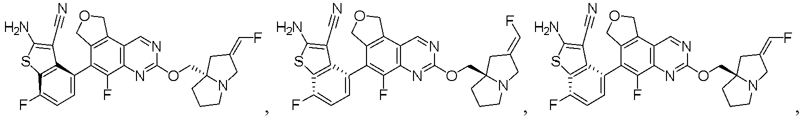 | | |
| 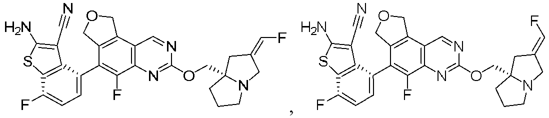 | | |
| **16** | 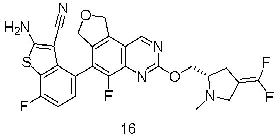 | 2-Amino-4-(3-(((*S*)-4-(difluoromethylene)-1-methylpyrrolidin-2-yl)methoxy)-5-fluoro-7,9-dihydrofuro[3,4-*f*]quinazolin-6-yl)-7-fluorobenzo[*b*]thiophene-3-carbonitrile **16** |
| 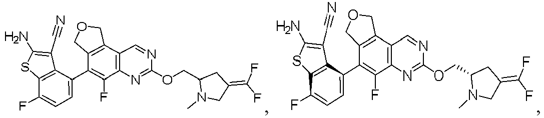 | | |
| 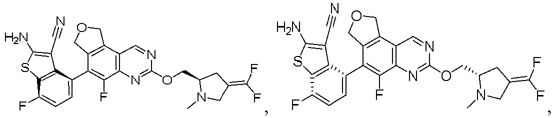 | | |
| 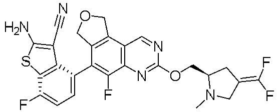 | | |
| **17** | 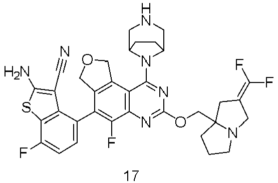 | 4-(1-(3,8-Diazabicyclo[3.2.1]oct-8-yl)-3-((2-(difluoromethylene)tetrahydro-1*H*-pyrrolizin-7*a*(5*H*)-yl)methoxy)-5-fluoro-7,9-dihydrofuro[3,4-*f*]quinazolin-6-yl)-2-amino-7-fluorobenzo[b]thiophene-3-carbonitrile **17** |
| 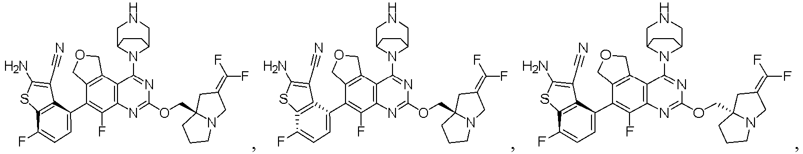 | | |
| 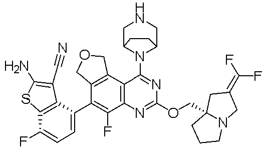 | | |
| **18** | 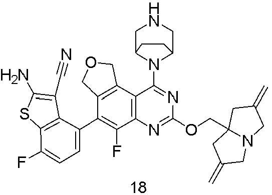 | 4-(1-(3,8-Diazabicyclo[3.2.1]oct-8-yl)-3-((2,6-dimethylenetetrahydro-1*H*-pyrrolizin-7*a*(5*H*)-yl)methoxy)-5-fluoro-7,9-dihydrofuro[3,4-*f*]quinazolin-6-yl)-2-amino-7-fluorobenzo[*b*]thiophene-3-carbonitrile **18** |
| 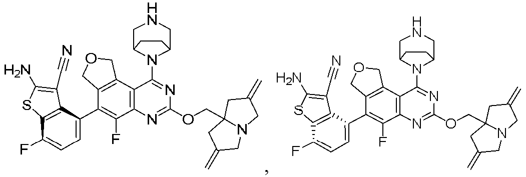 | | |
| **19** | 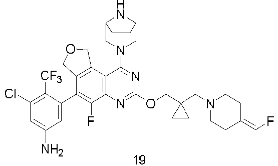 | 3-(1-(3,8-Diazabicyclo[3.2.1]oct-3-yl)-5-fluoro-3-((1-((4-(fluoromethylene)piperidin-1-yl)methyl)cyclopropyl)methoxy)-7,9-dihydrofuro[3,4-*f*]quinazolin-6-yl)-5-chloro-4-(trifluoromethyl)aniline **19** |
| 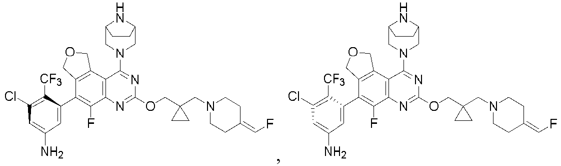 | | |
| **20** | 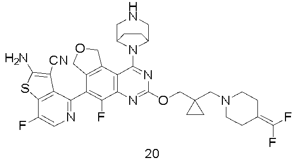 | 4-(1-(3,8-Diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-*c*]pyridine-3-carbonitrile **20** |
| **21** | 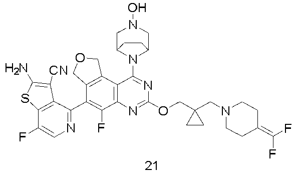 | 2-Amino-4-(3-((1-((4-(difluoromethylene)piperidin-1-yl)methyl)cyclopropyl)methoxy)-5-fluoro-1-(3-hydroxyl-3,8-diazabicyclo[3.2.1]oct-8-yl)-7,9-dihydrofuro[3,4-*f*]quinazolin-6-yl)-7-fluorothieno[3,2-*c*]pyridine-3-carbonitrile **21** |
| **22** | 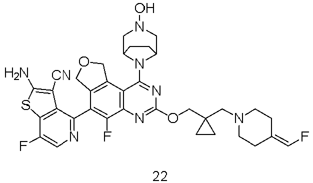 | 2-Amino-7-fluoro-4-(5-fluoro-3-((1-((4-(fluoromethylene)piperidin-1-yl)methyl)cyclopropyl)methoxy)-1-(3-hydroxyl-3,8-diazabicyclo[3.2.1]oct-8-yl)-7,9-dihydrofuro[3,4-*f*]quinazolin-6-yl)thieno[3,2-c]pyridine-3-carbonitrile **22** |
| **23** | 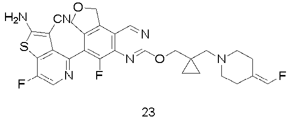 | 2-Amino-7-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)thieno[3,2-c]pyridine-3-carbonitrile **23** |
| **24** | 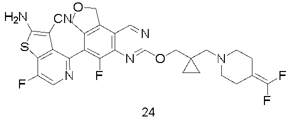 | 2-Amino-4-(3-((1-((4-(difluoromethylene)piperidin-1-yl)methyl)cyclopropyl)methoxy)-5-fluoro-7,9-dihydrofuro[3,4-*f*]quinazolin-6-yl)-7-fluorothieno[3,2-*c*]pyridine-3-carbonitrile **24** |
| **25** | 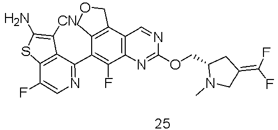 | 2-Amino-4-(3-(((S)-4-(difluoromethylene)-1-methylpyrrolidin-2-yl)methoxy)-5-fluoro-7,9-dihydrofuro[3,4-f]quinazolin-6-yl)-7-fluorothieno[3,2-*c*]pyridine-3-carbonitrile **25** |
| 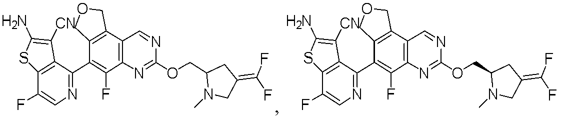 | | |
| **26** | 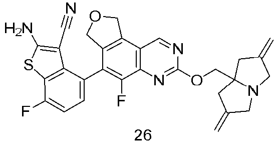 | 2-Amino-4-(3-((2,6-dimethylenetetrahydro-1*H-*pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **26** |
| **26-p1** | 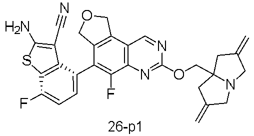 | (*R*)-2-Amino-4-(3-((2,6-dimethylenetetrahydro-1*H*-pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **26-p1** |
| **26-p2** | 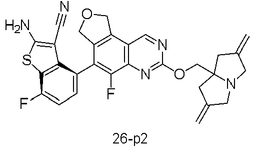 | (*S*)-2-Amino-4-(3-((2,6-dimethylenetetrahydro-1*H*-pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **26-p2** |
| **27** | 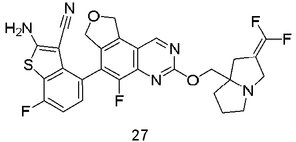 | 2-Amino-4-(3-((2-(difluoromethylene)tetrahydro-1*H*-pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **27** |
| 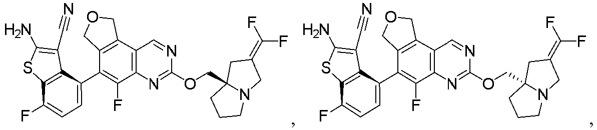 | | |
| 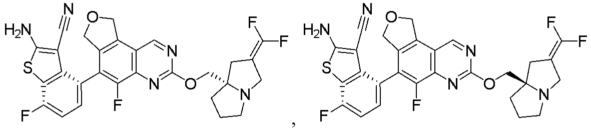 | | |
| **28** | 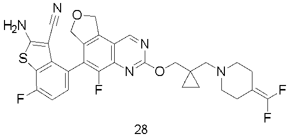 | 2-Amino-7-fluoro-4-(5-fluoro-3-((1-((4-(difluoromethylene)piperidin-1-yl)methyl)cyclopropyl)methoxy)-7,9-dihydrofuro[3,4-*f*]quinazolin-6-yl)benzo[*b*]thiophene-3-carbonitrile **28** |
| **28-p1** | 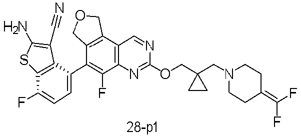 | (*R*)-2-Amino-4-(3-((1-((4-(difluoromethylene)piperidin-1-yl)methyl)cyclopropyl)methoxy)-5-fluoro-7,9-dihydrofuro[3,4-*f*]quinazolin-6-yl)-7-fluoro-benzo[*b*]thiophene-3-carbonitrile **28-p1** |
| **28-p2** | 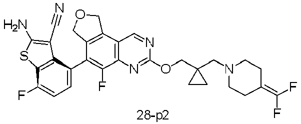 | (*S*)-2-Amino-4-(3-((1-((4-(difluoromethylene)piperidin-1-yl)methyl)cyclopropyl)methoxy)-5-fluoro-7,9-dihydrofuro[3,4-*f*]quinazolin-6-yl)-7-fluoro-benzo[*b*]thiophene-3-carbonitrile **28-p2** |
| **29** | 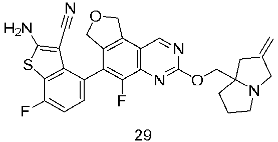 | 2-Amino-7-fluoro-4-(5-fluoro-3-((2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **29** |
| 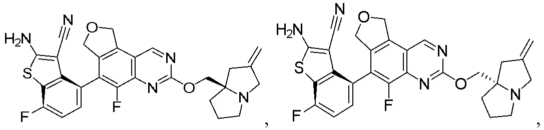 | | |
| 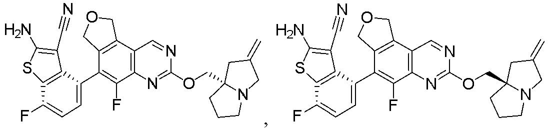 | | |
| **30** | 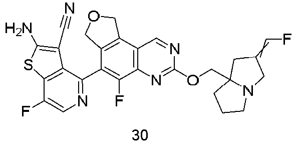 | 2-Amino-7-fluoro-4-(5-fluoro-3-((2-(fluoromethylene)tetrahydro-1*H*-pyrrolizin-7*a*(5*H*)-yl)methoxy)-7,9-dihydrofuro[3,4-*f*]quinazolin-6-yl)thieno[3,2-*c*]pyridine-3-carbonitrile **30** |
| 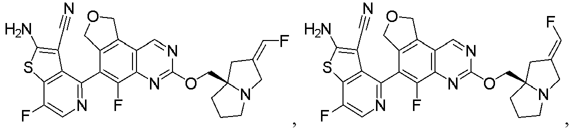 | | |
| 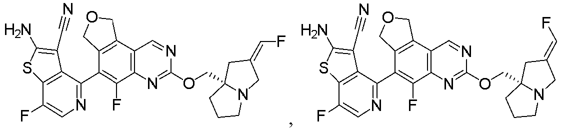 | | |
| **31** | 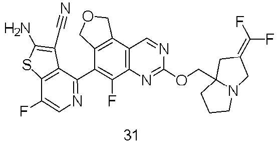 | 2-Amino-4-(3-((2-(difluoromethylene)tetrahydro-1*H*-pyrrolizin-7*a*(5*H*)-yl)methoxy)-5-fluoro-7,9-dihydrofuro[3,4-*f*]quinazolin-6-yl)-7-fluorothieno[3,2-*c*]pyridine-3-carbonitrile **31** |
| 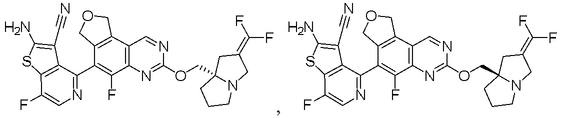 | | |
| **32** | 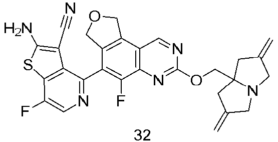 | 2-Amino-4-(3-((2,6-dimethylenetetrahydro-1*H-*pyrrolizin-7*a*(5*H*)-yl)methoxy)-5-fluoro-7,9-dihydrofuro[3,4-*f*]quinazolin-6-yl)-7-fluorothieno[3,2-*c*]pyridine-3-carbonitrile **32** |
| **33** | 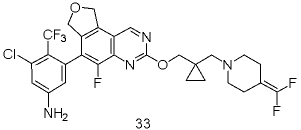 | 3-Chloro-5-(3-((1-((4-(difluoromethylene)piperidin-1-yl)methyl)cyclopropyl)methoxy)-5-fluoro-7,9-dihydrofuro[3,4-*f*]quinazolin-6-yl)-4-(trifluoromethyl)aniline **33** |
| 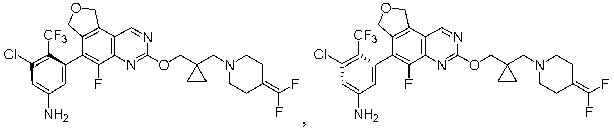 | | |
| **34** | 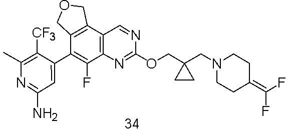 | 4-(3-((1-((4-(Difluoromethylene)piperidin-1-yl)methyl)cyclopropyl)methoxy)-5-fluoro-7,9-dihydrofuro[3,4-f]quinazolin-6-yl)-6-methyl-5-(trifluoromethyl)pyridin-2-amine **34** |
| 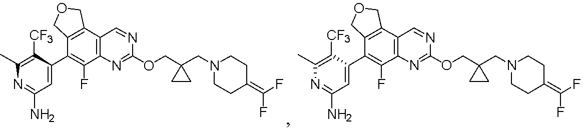 | | |
| **35** | 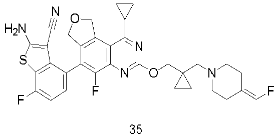 | 2-Amino-4-(1-cyclopropyl-5-fluoro-3-((1-((4-(fluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **35** |
| 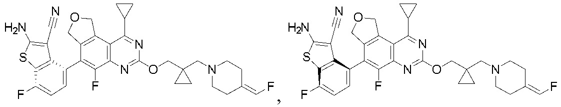 | | |
| **36** | 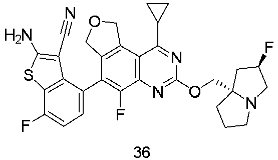 | 2-Amino-4-(1-cyclopropyl-5-fluoro-3-(((2*R*,7*aS*)-2-fluorotetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **36** |
| **36-p1** | 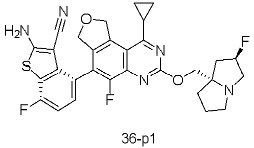 | 2-Amino-4-((*R*)-1-cyclopropyl-5-fluoro-3-(((2*R*,7a*S*)-2-fluorotetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **36-p1** |
| **36-p2** | 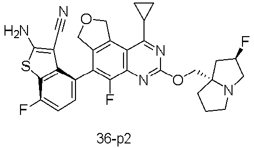 | 2-Amino-4-((*S*)-1-cyclopropyl-5-fluoro-3-(((2*R*,7*aS*)-2-fluorotetrahydro-1*H*-pyrrolizin-7*a*(5*H*)-yl)methoxy)-7,9-dihydrofaro[3,4-*f*]quinazolin-6-yl)-7-fluorobenzo[*b*]thiophene-3-carbonitrile **36-p2** |
| 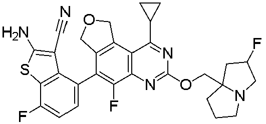 | | |
| **37** | 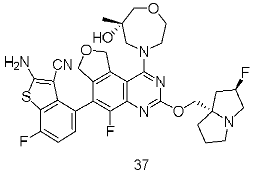 | 2-Amino-7-fluoro-4-(5-fluoro-3-(((2*R*,7*aS*)-2-fluorotetrahydro-1*H*-pyrrolizin-7a(5*H*)-yl)methoxy)-1-((*S*)-6-hydroxyl-6-methyl-1,4-oxazepan-4-yl)-7,9-dihydrofaro[3,4-*f*]quinazolin-6-yl)benzo[*b*]thiophene-3-carbonitrile **37** |
| 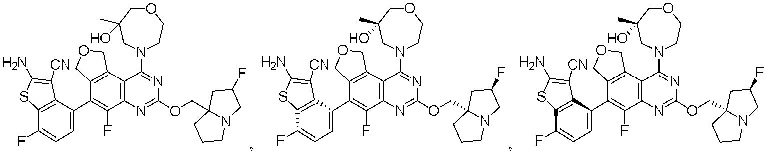 | | |
| **38** | 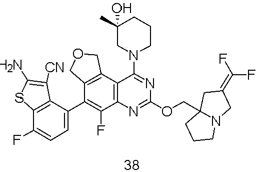 | 2-Amino-4-(3-((2-(difluoromethylene)tetrahydro-**1*H***-pyrrolizin-7*a*(5*H*)-yl)methoxy)-5-fluoro-1-((*R*)-3-hydroxyl-3-methylpiperidin-1-yl)-7,9-dihydrofuro[3,4-*f*]quinazolin-6-yl)-7-fluorobenzo[*b*]thiophene-3-carbonitrile **38** |
| 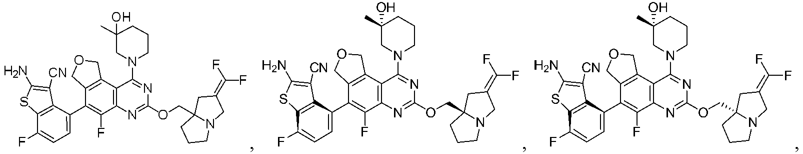 | | |
| 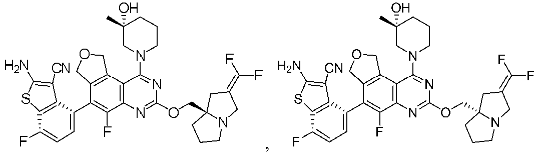 | | |
| **39** | 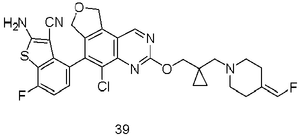 | 2-Amino-4-(5-chloro-3-((1-((4-(fluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **39** |
| **39-p1** | 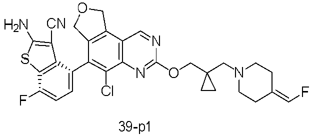 | (*R*)-2-Amino-4-(5-chloro-3-((1-((4-(fluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **39-p1** |
| **39-p2** | 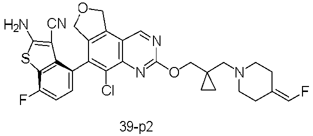 | (*S*)-2-Amino-4-(5-chloro-3-((1-((4-(fluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **39-p2** |
| **40** | 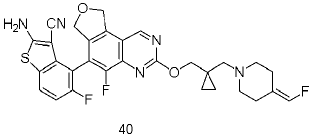 | 2-Amino-5-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)benzo[*b*]thiophene-3-carbonitrile **40** |
| 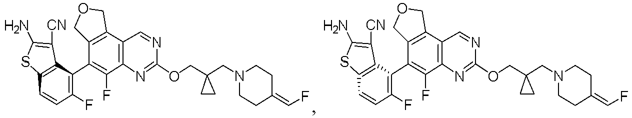 | | |
| **41** | 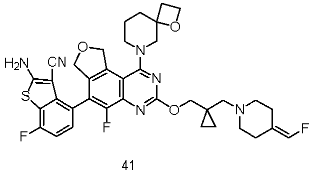 | 2-Amino-7-fluoro-4-(5-fluoro-3-((1-((4-(fluoromethylene)piperidin-1-yl)methyl)cyclopropyl)methoxy)-1-(1-oxa-6-azaspiro[3.5]non-6-yl)-7,9-dihydrofuro[3,4-*f*]quinazolin-6-yl)benzo[*b*]thiophene-3-carbonitrile **41** |
| 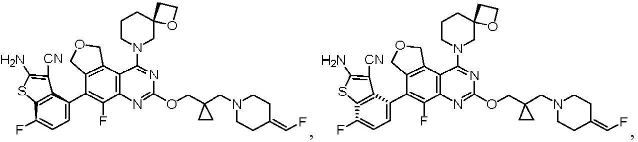 | | |
| 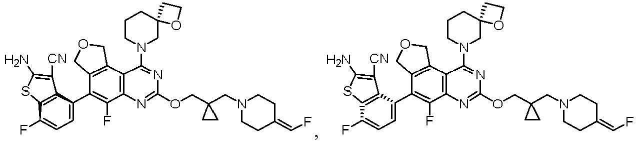 | | |
| **42** | 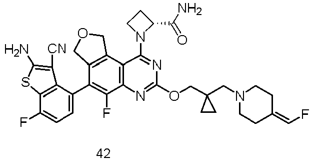 | (2*R*)-1-(6-(2-Amino-3-cyano-7-fluorobenzo[*b*]thiophen-4-yl)-5-fluoro-3-((1-((4-(fluoromethylene)piperidin-1-yl)methyl)cyclopropyl)methoxy)-7,9-dihydrofuro[3,4-*f*]quinazolin-1-yl)azetidine-2-carboxamide **42** |
| **42-p1** | 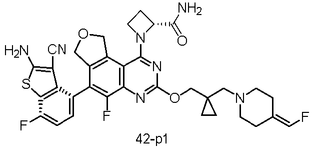 | (*R*)-1-((*R*)-6-(2-Amino-3-cyano-7-fluorobenzo[*b*]thiophen-4-yl)-5-fluoro-3-((1-((4-(fluoromethylene)piperidin-1-yl)methyl)cyclopropyl)methoxy)-7,9-dihydrofuro[3,4-*f*]quinazolin-1-yl)azetidine-2-carboxamide **42-p1** |
| 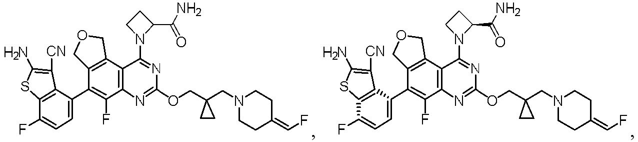 | | |
| 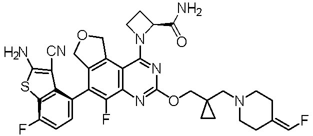 | | |
| **42-p2** | 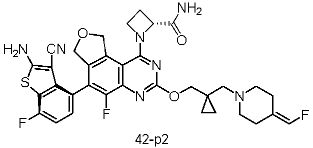 | (*R*)-1-((*S*)-6-(2-Amino-3-cyano-7-fluorobenzo[*b*]thiophen-4-yl)-5-fluoro-3-((1-((4-(fluoromethylene)piperidin-1-yl)methyl)cyclopropyl)methoxy)-7,9-dihydrofuro[3,4-*f*]quinazolin-1-yl)azetidine-2-carboxamide **42-p2** |
| **43-1** | 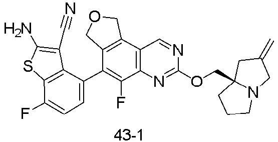 | 2-Amino-7-fluoro-4-(5-fluoro-3-(((*R*)-2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **43-1** |
| 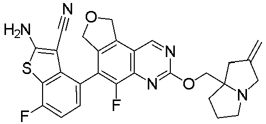 | | |
| **43-2** | 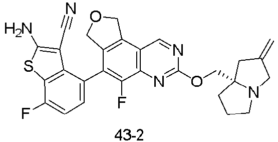 | 2-Amino-7-fluoro-4-(5-fluoro-3-(((*S*)-2-methylenetetrahydro-1*H*-pyrrolizin-7a(5*H*)-yl)methoxy)-7,9-dihydrofuro[3,4-*f*]quinazolin-6-yl)benzo[*b*]thiophene-3-carbonitrile **43-2** |
| **43-1-p1** | 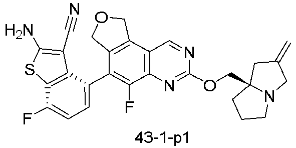 | 2-Amino-7-fluoro-4-((*R*)-5-fluoro-3-(((*R*)-2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **43-1-p1** |
| **43-1-p2** | 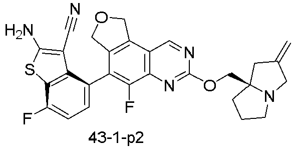 | 2-Amino-7-fluoro-4-((*S*)-5-fluoro-3-(((*R*)-2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **43-1-p2** |
| **43-2-p1** | 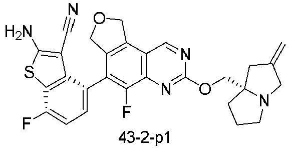 | 2-Amino-7-fluoro-4-((*R*)-5-fluoro-3-(((*S*)-2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **43-2-p1** |
| **43-2-p2** | 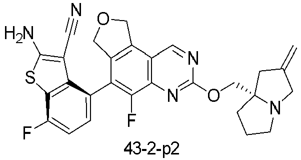 | 2-Amino-7-fluoro-4-((*S*)-5-fluoro-3-(((*S*)-2-methylenetetrahydro-1*H*-pyrrolizin-7*a*(5*H*)-yl)methoxy)-7,9-dihydrofuro[3,4-*f*]quinazolin-6-yl)benzo[*b*]thiophene-3-carbonitrile **43-2-p2** |
| **44-1-p1** | 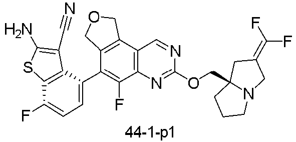 | 2-Amino-4-((*R*)-3-(((*R*)-2-(difluoromethylene)tetrahydro-1*H-*pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **44-1-p1** |
| 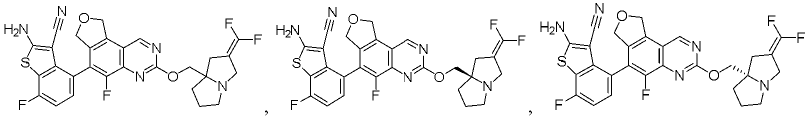 | | |
| **44-1-p2** | 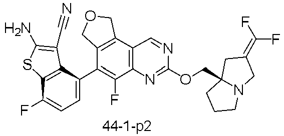 | 2-Amino-4-((*S*)-3-(((*R*)-2-(difluoromethylene)tetrahydro-1*H-*pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **44-1-p2** |
| **44-2-p1** | 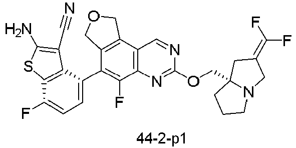 | 2-Amino-4-((*R*)-3-(((*S*)-2-(difluoromethylene)tetrahydro-1*H-*pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **44-2-p1** |
| **44-2-p2** | 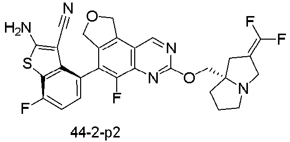 | 2-Amino-4-((*S*)-3-(((*S*)-2-(difluoromethylene)tetrahydro-1*H*-pyrrolizin-7*a*(5*H*)-yl)methoxy)-5-fluoro-7,9-dihydrofuro[3,4-*f*]quinazolin-6-yl)-7-fluorobenzo[*b*]thiophene-3-carbonitrile **44-2-p2** |
| **45** | 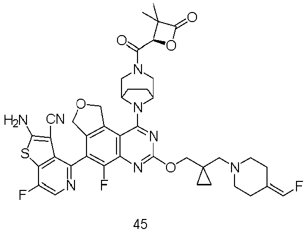 | 2-Amino-4-(1-((1*R*,5*S*)-3-((*R*)-3,3-dimethyl-4-oxooxetane-2-carbonyl)-3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-*f*]quinazolin-6-yl)-7-fluorothieno[3,2-*c*]pyridine-3-carbonitrile **45** |
| 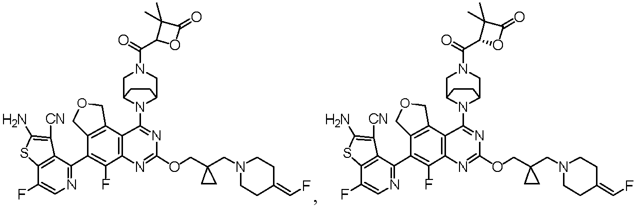 | | |
| **46** | 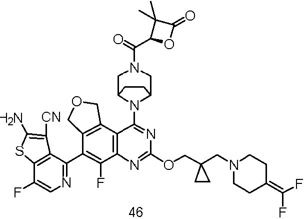 | 2-Amino-4-(3-((1-((4-(difluoromethylene)piperidin-1-yl)methyl)cyclopropyl)methoxy)-1-((1*R*,5*S*)-3-((*R*)-3,3-dimethyl-4-oxooxetane-2-carbonyl)-3,8-diazabicyclo[3.2.1]oct-8-yl)-5-fluoro-7,9-dihydrofuro[3,4-*f*]quinazolin-6-yl)-7-fluorothieno[3,2-*c*]pyridine-3-carbonitrile **46** |
| 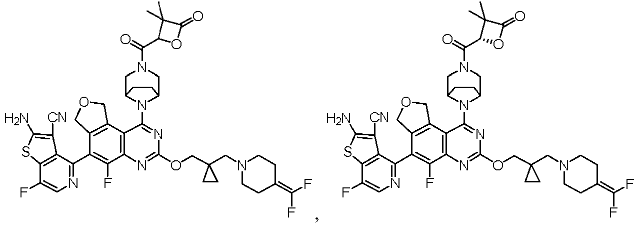 | | |
| **47** | 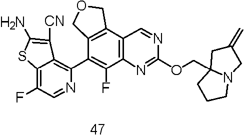 | 2-Amino-7-fluoro-4-(5-fluoro-3-((2-methylenetetrahydro-1*H*-pyrrolizin-7*a*(5*H*)-yl)methoxy)-7,9-dihydrofuro[3,4-*f*]quinazolin-6-yl)thieno[3,2-*c*]pyridine-3-carbonitrile **47** |
| 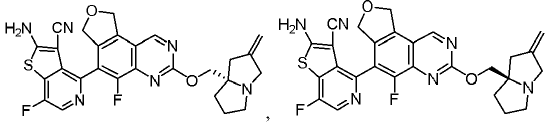 | | |
| **48** | 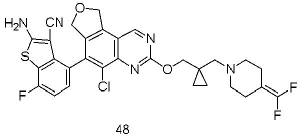 | 2-Amino-4-(5-chloro-3-((1-((4-(difluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **48** |
| **48-p1** | 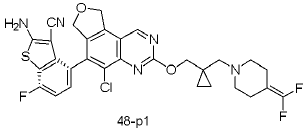 | (*R*)-2-Amino-4-(5-chloro-3-((1-((4-(difluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **48-p1** |
| **48-p2** | 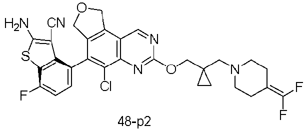 | (*S*)-2-Amino-4-(5-chloro-3-((1-((4-(difluoromethylene)piperidin-1-yl)methyl)cyclopropyl)methoxy)-7,9-dihydrofuro[3,4-*f*]quinazolin-6-yl)-7-fluorobenzo[*b*]thiophene-3-carbonitrile **48-p2** |
| **49** | 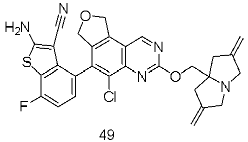 | 2-Amino-4-(5-chloro-3-((2,6-dimethylenetetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **49** |
| **49-p1** | 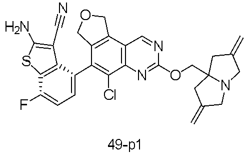 | (*R*)-2-Amino-4-(5-chloro-3-((2,6-dimethylenetetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile 49-**p1** |
| **49-p2** | 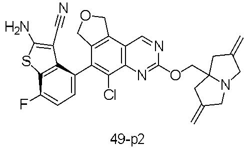 | (*S*)-2-Amino-4-(5-chloro-3-((2,6-dimethylenetetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **49-p2** |
| **50-1** | 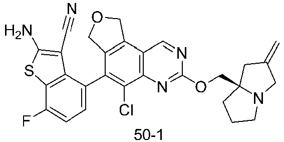 | 2-Amino-4-(5-chloro-3-(((*R*)-2-methylenetetrahydro-1*H*-pyrrolizin-7*a*(5*H*)-yl)methoxy)- 7,9-dihydrofuro[3,4-*f*]quinazolin-6-yl)-7-fluorobenzo[*b*]thiophene-3-carbonitrile **50-1** |
| **50-2** | 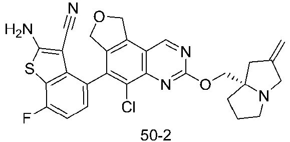 | 2-Amino-4-(5-chloro-3-(((*S*)-2-methylenetetrahydro-1*H*-pyrrolizin-7a(5*H*)-yl)methoxy)-7,9-dihydrofuro[3,4-*f*]quinazolin-6-yl)-7-fluorobenzo[*b*]thiophene-3-carbonitrile **50-2** |
| **50-1-p1** | 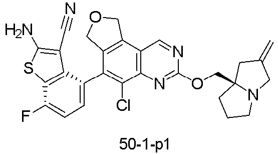 | 2-Amino-4-((*R*)-5-chloro-3-(((*R*)-2-methylenetetrahydro-1*H*-pyrrolizin-7a(5*H*)-yl)methoxy)-7,9-dihydrofuro[3,4-*f*]quinazolin-6-yl)-7-fluorobenzo[*b*]thiophene-3-carbonitrile **50-1-p1** |
| **50-1-p2** | 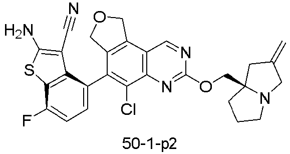 | 2-Amino-4-((*S*)-5-chloro-3-(((*R*)-2-methylenetetrahydro-1*H*-pyrrolizin-7a(5*H*)-yl)methoxy)-7,9-dihydrofuro[3,4-*f*]quinazolin-6-yl)-7-fluorobenzo[*b*]thiophene-3-carbonitrile 50-**1-p2** |
| **50-2-p1** | 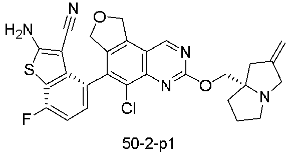 | 2-Amino-4-((*R*)-5-chloro-3-(((*S*)-2-methylenetetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **50-2-p1** |
| **50-2-p2** | 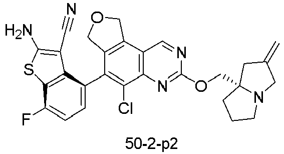 | 2-Amino-4-((*S*)-5-chloro-3-(((*S*)-2-methylenetetrahydro-1*H*-pyrrolizin-7a(5*H*)-yl)methoxy)-7,9-dihydrofuro[3,4-*f*]quinazolin-6-yl)-7-fluorobenzo[*b*]thiophene-3-carbonitrile **50-2-p2** |
| 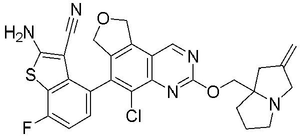 | | |
| **51-1** | 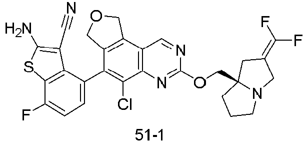 | 2-Amino-4-(5-chloro-3-(((*R*)-2-(difluoromethylene)tetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **51-1** |
| **51-2** | 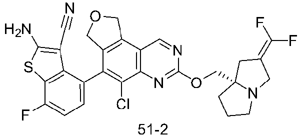 | 2-Amino-4-(5-chloro-3-(((*S*)-2-(difluoromethylene)tetrahydro- 1*H-*pyrrolizin-7*a*(5*H*)-yl)methoxy)- 7,9-dihydrofuro[3,4-*f*]quinazolin-6-yl)-7-fluorobenzo[*b*]thiophene-3-carbonitrile **51-2** |
| **51-1-p1** | 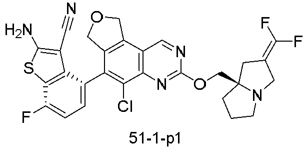 | 2-Amino-4-((R)-5-chloro-3-(((R)-2-(difluoromethylene)tetrahydro- 1*H-*pyrrolizin-7*a*(5*H*)-yl)methoxy)-7,9-dihydrofuro[3,4-*f*]quinazolin-6-yl)-7-fluorobenzo[*b*]thiophene-3-carbonitrile **51-1-p1** |
| **51-1-p2** | 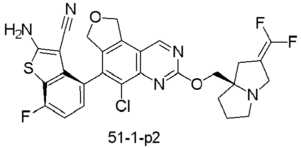 | 2-Amino-4-((*S*)-5-chloro-3-(((*R*)-2-(difluoromethylene)tetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **51-1-p2** |
| **51-2-p1** | 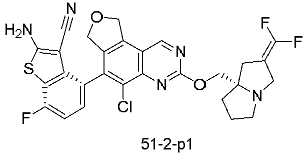 | 2-Amino-4-((*R*)-5-chloro-3-(((*S*)-2-(difluoromethylene)tetrahydro-1*H*-pyrrolizin-7*a*(5*H*)-yl)methoxy)-7,9-dihydrofuro[3,4-*f*]quinazolin-6-yl)-7-fluorobenzo[*b*]thiophene-3-carbonitrile **51-2-p1** |
| **51-2-p2** | 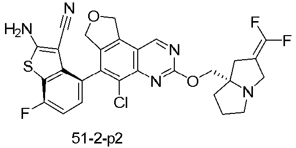 | 2-Amino-4-((*S*)-5-chloro-3-(((*S*)-2-(difluoromethylene)tetrahydro-1*H-*pyrrolizin-7*a*(5*H*)-yl)methoxy)-7,9-dihydrofuro[3,4-*f*]quinazolin-6-yl)-7-fluorobenzo[*b*]thiophene-3-carbonitrile **51-2-p2** |
| 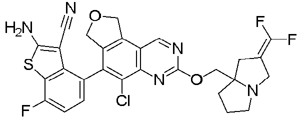 | | |
| **52** | 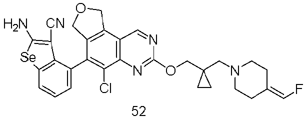 | 2-Amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[*b*]selenophen-3-carbonitrile **52** |
| **52-p1** | 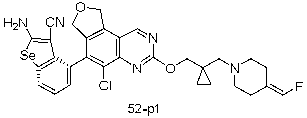 | (*R*)-2-Amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[*b*]selenophen-3-carbonitrile **52-p1** |
| **52-p2** | 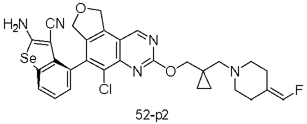 | (*S*)-2-Amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[*b*]selenophen-3-carbonitrile **52-p2** |
| **53-1-p1** | 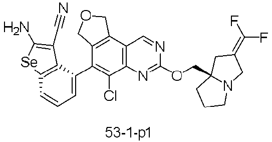 | 2-Amino-4-((*R*)-5-chloro-3-(((*R*)-2-(difluoromethylene)tetrahydro-1*H*-pyrrolizin-7*a*(5*H*)-yl)methoxy)-7,9-dihydrofuro[3,4-*f*]quinazolin-6-yl)benzo[*b*]selenophene-3-carbonitrile **53-1-p1** |
| **53-1-p2** | 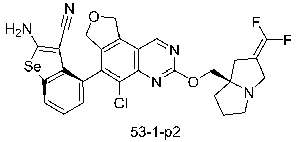 | 2-Amino-4-((*S*)-5-chloro-3-(((*R*)-2-(difluoromethylene)tetrahydro- 1*H*-pyrrolizin-7*a*(5*H*)-yl)methoxy)-7,9-dihydrofuro[3,4-*f*]quinazolin-6-yl)benzo[*b*]selenophene-3-carbonitrile **53-1-p2** |
| **53-2-p1** | 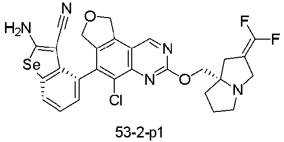 | 2-Amino-4-((*R*)-5-chloro-3-(((*S*)-2-(difluoromethylene)tetrahydro-1*H*-pyrrolizin-7*a*(5*H*)-yl)methoxy)-7,9-dihydrofuro[3,4-*f*]quinazolin-6-yl)benzo[*b*]selenophene-3-carbonitrile **53-2-p1** |
| **53-2-p2** | 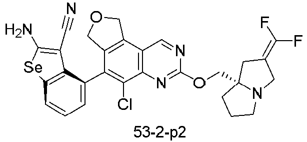 | 2-Amino-4-((*S*)-5-chloro-3-(((*S*)-2-(difluoromethylene)tetrahydro- 1*H*-pyrrolizin-7*a*(5*H*)-yl)methoxy)-7,9-dihydrofuro[3,4-*f*]quinazolin-6-yl)benzo[*b*]selenophene-3-carbonitrile **53-2-p2** |
| | 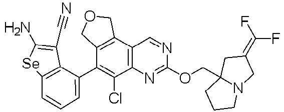 | |
| **54-1** | 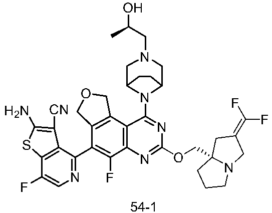 | 2-Amino-4-(3-(((*S*)-2-(difluoromethylene)tetrahydro-1*H*-pyrrolizin-7*a*(5*H*)-yl)methoxy)-5-fluoro-1-(3-((*R*)-2-hydroxypropyl)-3,8-diazabicyclo[3.2.1]oct-8-yl)-7,9-dihydrofuro[3,4-*f*]quinazolin-6-yl)-7-fluorothieno[3,2-*c*]pyridine-3-carbonitrile **54-1** |
| 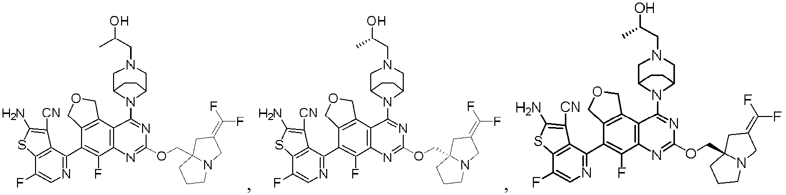 | | |
| **54-2** | 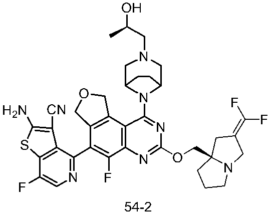 | 2-Amino-4-(3-(((*R*)-2-(difluoromethylene)tetrahydro-1*H*-pyrrolizin-7*a*(5*H*)-yl)methoxy)-5-fluoro-1-(3-((*R*)-2-hydroxypropyl)-3,8-diazabicyclo[3.2.1]oct-8-yl)-7,9-dihydrofuro[3,4-*f*]quinazolin-6-yl)-7-fluorothieno[3,2-*c*]pyridine-3-carbonitrile **54-2** |
| **55** | 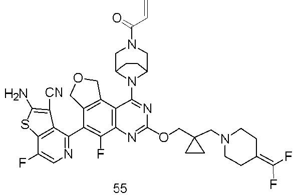 | 4-(1-(3-Acryloyl-3,8-diazabicyclo[3.2.1]oct-8-yl)-3-((1-(4-(difluoromethylene)piperidin-1-yl)methyl)cyclopropyl)methoxy)-5-fluoro-7,9-dihydrofuro[3,4-*f*]quinazolin-6-yl)-2-amino-7-fluorothieno[3,2-*c*]pyridine-3-carbonitrile **55** |
| **56** | 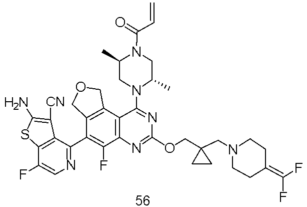 | 4-(1-((2*S*,5*R*)-4-Acryloyl-2,5-dimethylpiperazin-1-yl)-3-((1-(4-(difluoromethylene)piperidin-1-yl)methyl)cyclopropyl)methoxy)-5-fluoro-7,9-dihydrofuro[3,4-*f*]quinazolin-6-yl)-2-amino-7-fluorothieno[3,2-*c*]pyridine-3-carbonitrile **56** |
| 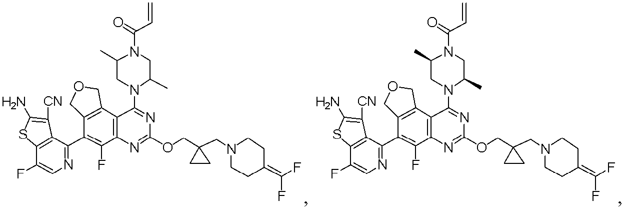 | | |
| 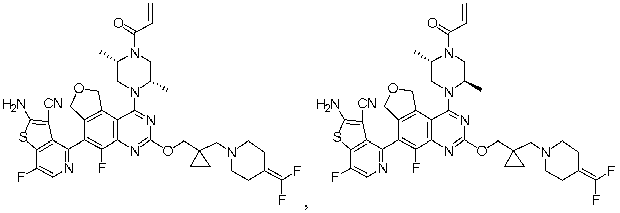 | | |
| 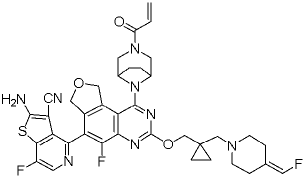 | | |

Another aspect of the present disclosure relates to a compound represented by general formula (IIa) or a salt thereof, 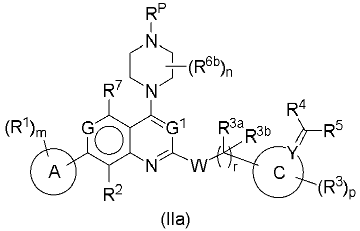
wherein, R^{P} is an amino protecting group, and in some embodiments, R^{P} is Boc;
ring A, ring C, R¹, m, G, G¹, R², R⁷, R^{6b}, n, W, R^{3a}, R^{3b}, r, Y, R³, R⁴, R⁵ and p are as defined in general formula (II).

Another aspect of the present disclosure relates to a compound represented by general formula (IIIa) or a salt thereof, 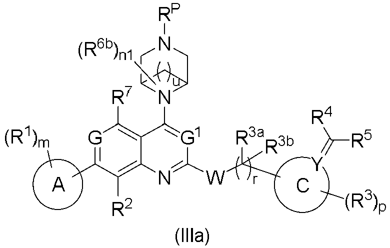
wherein, R^{P} is an amino protecting group, and in some embodiments, R^{P} is Boc;
ring A, ring C, R¹, m, G, G¹, R², R⁷, R^{6b}, n1, u, W, R^{3a}, R^{3b}, r, Y, R³, R⁴, R⁵ and p are as defined in general formula (III).

Another aspect of the present disclosure relates to a compound represented by general formula (IVa) or a salt thereof, 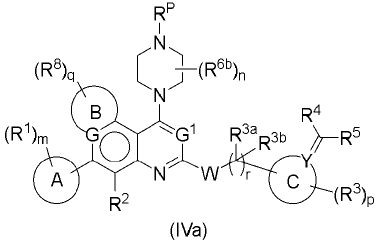
wherein, R^{P} is an amino protecting group, and in some embodiments, R^{P} is Boc;
ring A, ring B, ring C, R¹, m, G, G¹, R², R⁸, q, R^{6b}, n, W, R^{3a}, R^{3b}, r, Y, R³, R⁴, R⁵ and p are as defined in general formula (IV).

Another aspect of the present disclosure relates to a compound represented by general formula (Va) or a salt thereof, 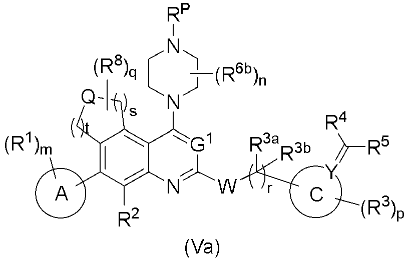
wherein, R^{P} is an amino protecting group, and in some embodiments, R^{P} is Boc;
ring A, ring C, R¹, m, Q, s, t, R⁸, q, G¹, R², R^{6b}, n, W, R^{3a}, R^{3b}, r, Y, R³, R⁴, R⁵ and p are as defined in general formula (V).

Another aspect of the present disclosure relates to a compound represented by general formula (VIA) or a salt thereof, 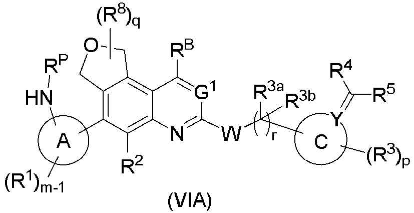
wherein, R^{P} is an amino protecting group, and in some embodiments, R^{P} is Boc;
ring A, ring C, R¹, m, R⁸, q, R², G¹, R^{B}, W, R^{3a}, R^{3b}, r, Y, R³, R⁴, R⁵ and p are as defined in general formula (VI).

**Table B. Typical intermediate compounds of the present disclosure, including but not limited to:**

| Compound No. | Compound structure | Name |
|---|---|---|
| **1h** | 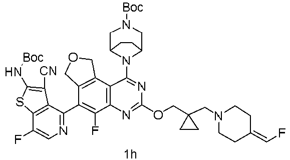 | tert-Butyl 8-(6-(2-((tert-butoxycarbonyl)amino)-3-cyano-7-fluorothieno[3,2-*c*]pyridin-4-yl)-5-fluoro-3-((1-(4-(fluoromethylene)piperidin-1-yl)methyl)cyclopropyl)methoxy)-7,9-dihydrofuro[3,4-*f*]quinazolin-1-yl)-3,8-diazabicyclo[3.2.1]octane-3-carboxylate **1h** |
| 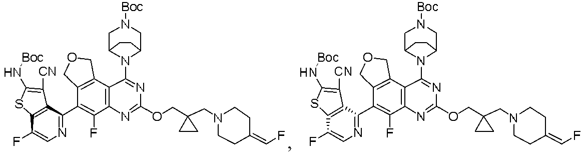 | | |
| **2e** | 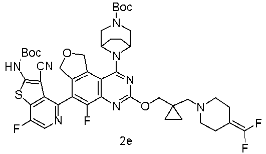 | tert-Butyl 8-(6-(2-((tert-butoxycarbonyl)amino)-3-cyano-7-fluorothieno[3,2-*c*]pyridin-4-yl)-3-((1-((4-(difluoromethylene)piperidin-1-yl)methyl)cyclopropyl)methoxy)-5-fluoro-7,9-dihydrofuro[3,4-*f*]quinazolin-1-yl)-3,8-diazabicyclo[3.2.1]octane-3-carboxylate **2e** |
| | 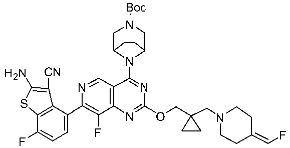 | 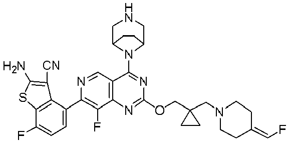 |
| **6d** | 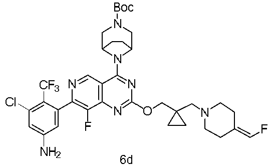 | tert-Butyl 8-(7-(5-amino-3-chloro-2-(trifluoromethyl)phenyl)-8-fluoro-2-((1-((4-(fluoromethylene)piperidin-1-yl)methyl)cyclopropyl)methoxy)pyrido[4,3-*d*]pyrimidin-4-yl)-3,8-diazabicyclo[3.2.1]octane-3-carboxylate **6d** |
| **6e** | 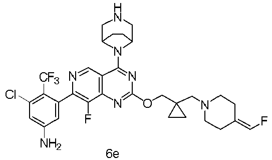 | 3-(4-(3,8-Diazabicyclo[3.2.1]oct-8-yl)-8-fluoro-2-((1-((4-(fluoromethylene)piperidin-1-yl)methyl)cyclopropyl)methoxy)pyrido[4,3-*d*]pyrimidin-7-yl)-5-chloro-4-(trifluoromethyl)aniline **6e** |
| | 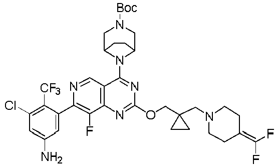 | 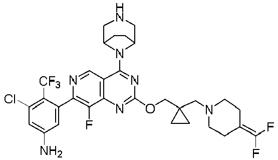 |
| | 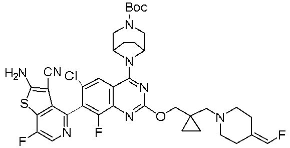 | 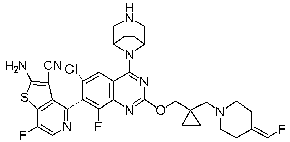 |
| **9a** | 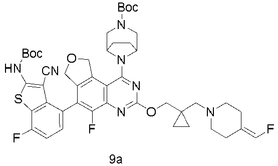 | tert-Butyl 8-(6-(2-((tertbutoxycarbonyl)amino)-3-cyano-7-fluorobenzo[*b*]thiophen-4-yl)-5-fluoro-3-((1-((4-(fluoromethylene)piperidin-1-yl)methyl)cyclopropyl)methoxy)-7,9-dihydrofuro[3,4-*f*]quinazolin-1-yl)-3,8-diazabicyclo[3.2.1]octane-3-carboxylate **9a** |
| 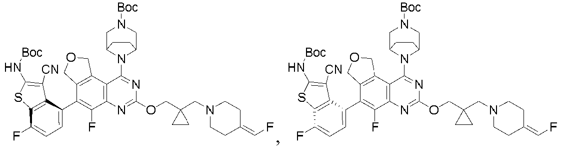 | | |
| **10a** | 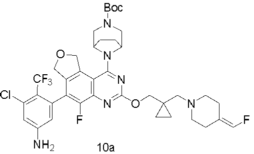 | tert-Butyl 8-(6-(5-amino-3-chloro-2-(trifluoromethyl)phenyl)-5-fluoro-3-((1-((4-(fluoromethylene)piperidin-1-yl)methyl)cyclopropyl)methoxy)-7,9-dihydrofuro[3,4-*f*]quinazolin-1-yl)-3,8-diazabicyclo[3.2.1]octane-3-carboxylate **10a** |
| 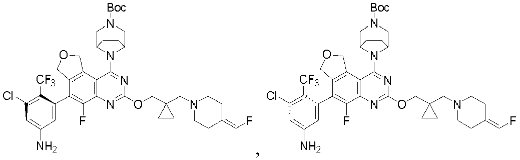 | | |
| | 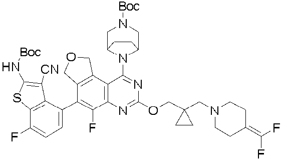 | |
| 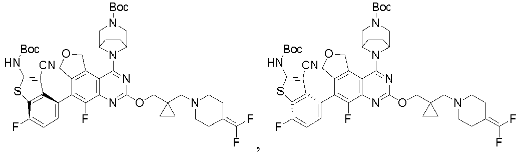 | | |
| | 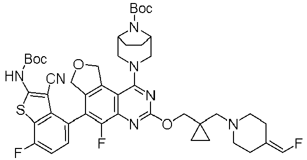 | |
| 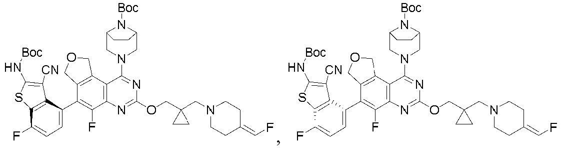 | | |
| | 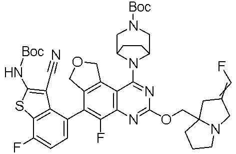 | |
| 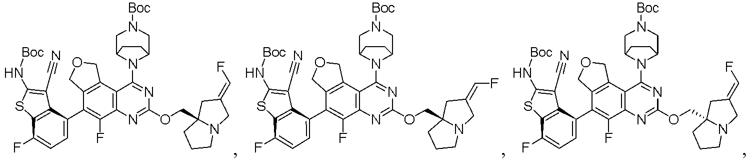 | | |
| 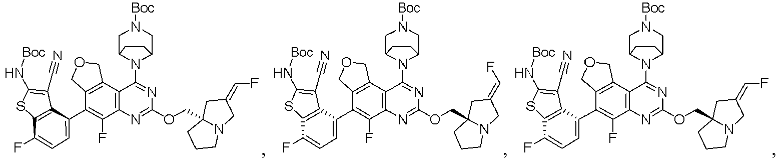 | | |
| 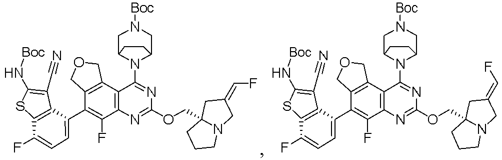 | | |
| **14d** | 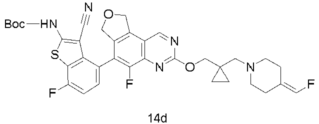 | tert-Butyl (3-cyano-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[*b*]thiophen-2-yl)carbamate **14d** |
| 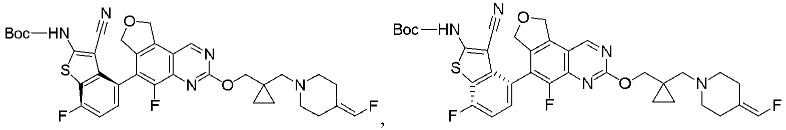 | | |
| | 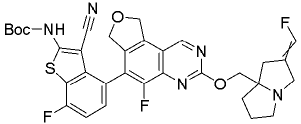 | |
| 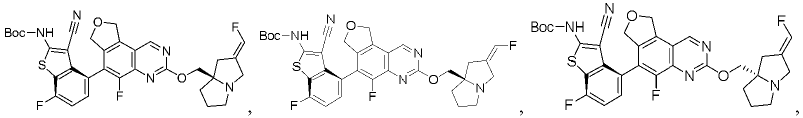 | | |
| 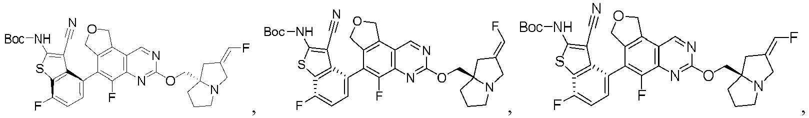 | | |
| 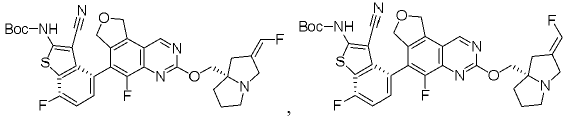 | | |
| 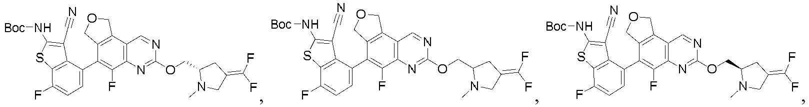 | | |
| 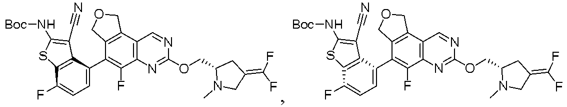 | | |
| | 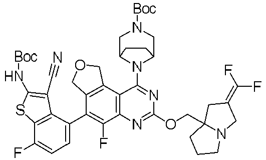 | |
| 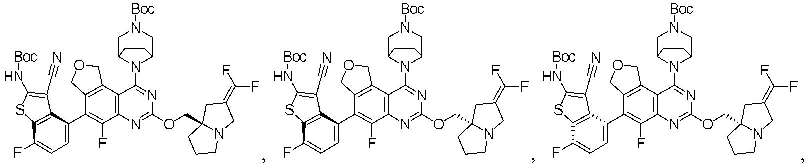 | | |
| 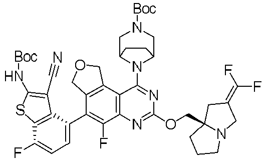 | | |
| | 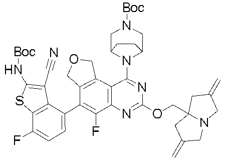 | |
| 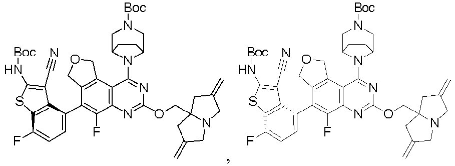 | | |
| | 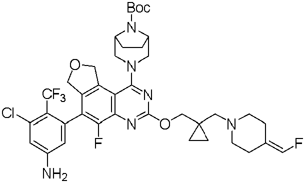 | |
| 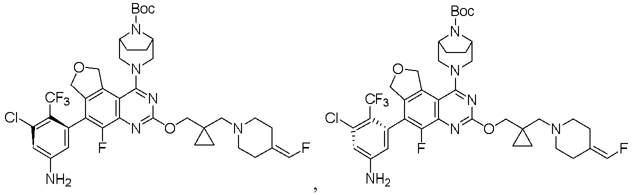 | | |
| **21a** | 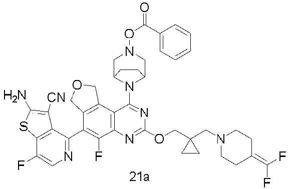 | 8-(6-(2-Amino-3-cyano-7-fluorothieno[3,2-*c*]pyridin-4-yl)-3-((1-((4-(difluoromethylene))piperidin-1-yl)methyl)cyclopropyl)methoxy)-5-fluoro-7,9-dihydrofuro[3,4-*f*]quinazolin-1-yl)-3,8-diazabicyclo[3.2.1]oct-3-yl benzoate **21a** |
| | 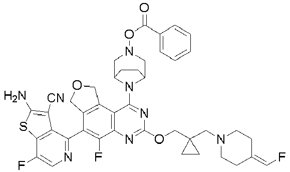 | |
| **23a** | 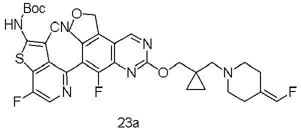 | tert-Butyl (3-cyano-7-fluoro-4-(5-fluoro-3-((1-((4-(fluoromethylene)piperidin-1-yl)methyl)cyclopropyl)methoxy)-7,9-dihydrofuro[3,4-*f*]quinazolin-6-yl)thieno[3,2-*c*]pyridin-2-yl)carbamate **23a** |
| | 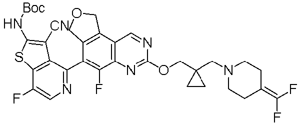 | |
| | 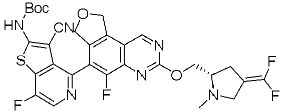 | |
| 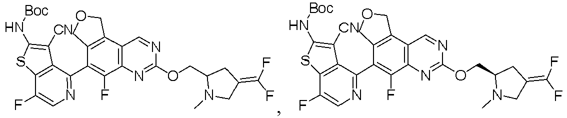 | | |
| | 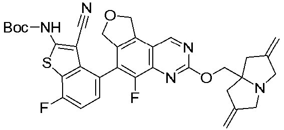 | |
| 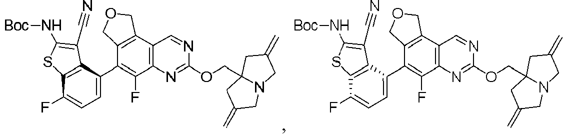 | | |
| | 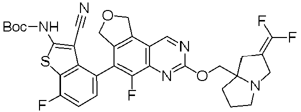 | |
| 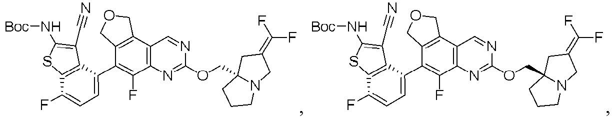 | | |
| 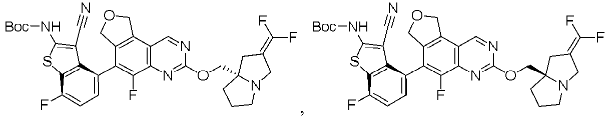 | | |
| | 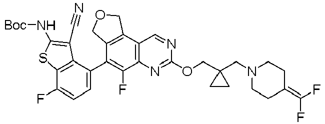 | |
| 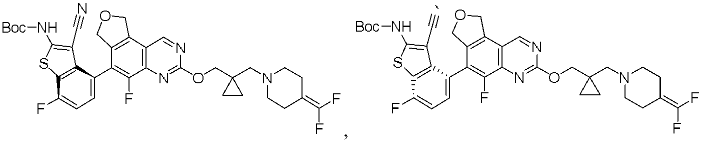 | | |
| | 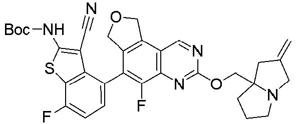 | |
| 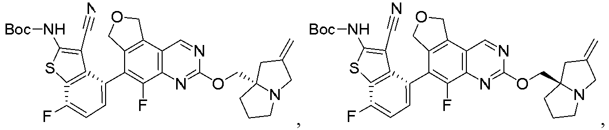 | | |
| 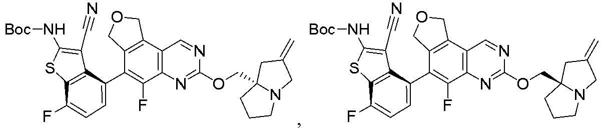 | | |
| | 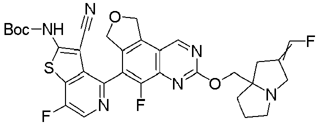 | |
| 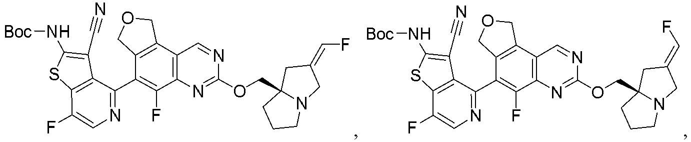 | | |
| 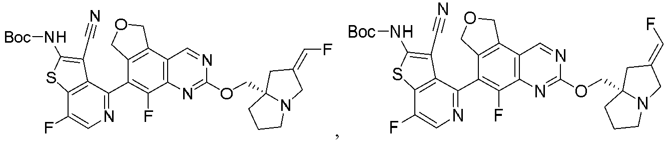 | | |
| | 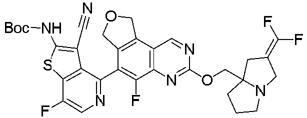 | |
| 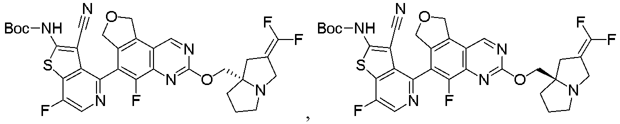 | | |
| | 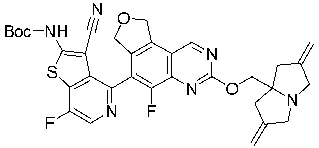 | |
| | 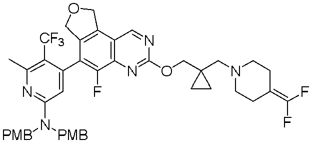 | |
| 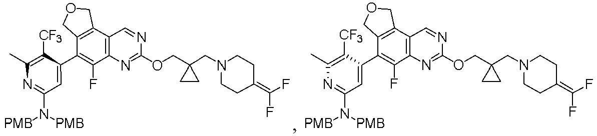 | | |
| | 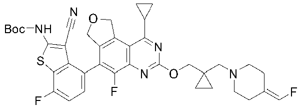 | |
| 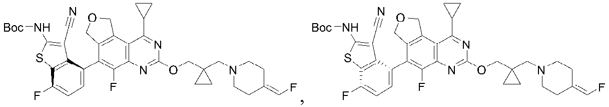 | | |
| | 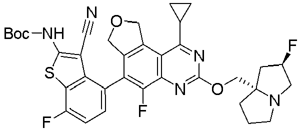 | |
| 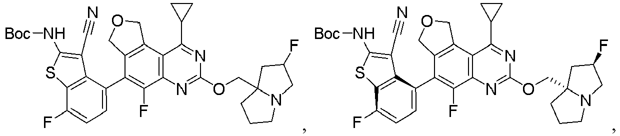 | | |
| 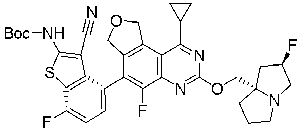 | | |
| **37e** | 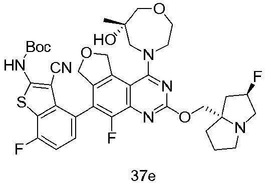 | tert-Butyl (3-cyano-7-fluoro-4-(5-fluoro-3-(((2*R*,7*aS*)-2-fluorotetrahydro-1*H*-pyrrolizin-7*a*(5*H*)-yl)methoxy)-1-((*S*)-6-hydroxyl-6-methyl-1,4-oxazepan-4-yl)-7,9-dihydrofuro[3,4-*f*]quinazolin-6-yl)benzothiophen-2-yl)carbamate **37e** |
| 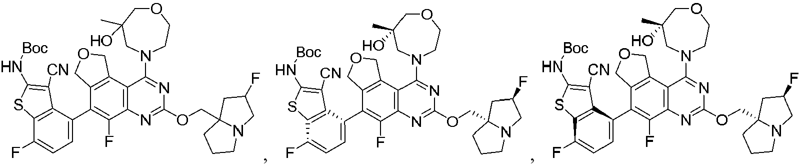 | | |
| | 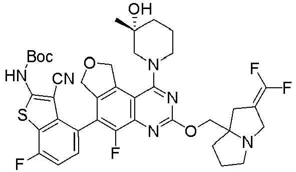 | |
| 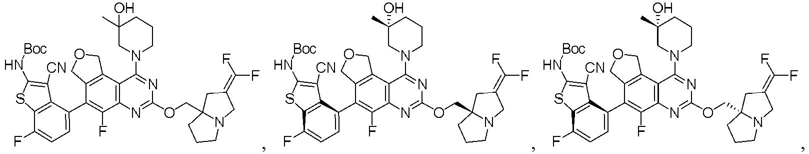 | | |
| 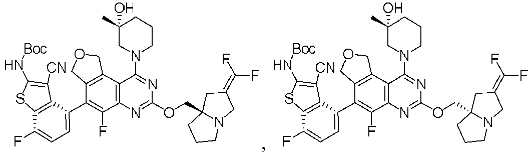 | | |
| **39d** | 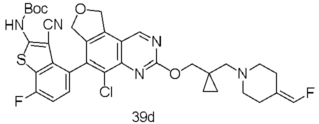 | tert-Butyl (4-(5-chloro-3-((1-((4-(fluoromethylene)piperidin-1-yl)methyl)cyclopropyl)methoxy)-7,9-dihydrofuro[3,4-*f*]quinazolin-6-yl)-3-cyano-7-fluorobenzo[*b*]thiophen-2-yl)carbamate **39d** |
| 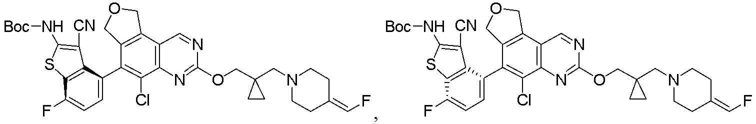 | | |
| **40d** | 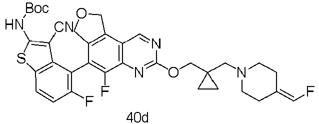 | tert-Butyl (3-cyano-5-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[*b*]thiophen-2-yl)carbamate **40d** |
| 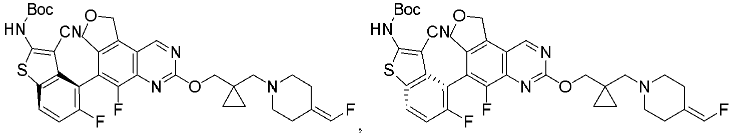 | | |
| | 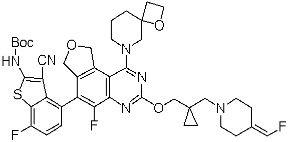 | |
| 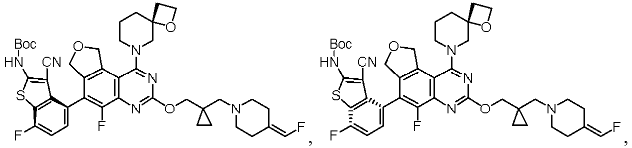 | | |
| 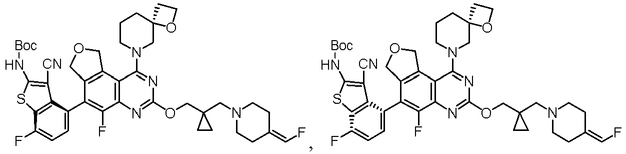 | | |
| | 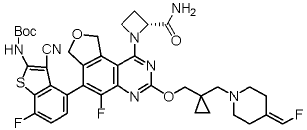 | |
| 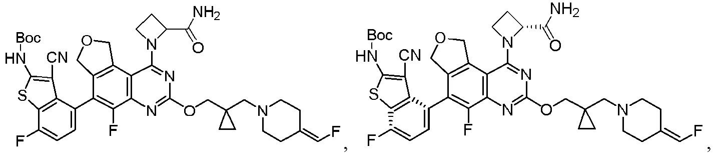 | | |
| 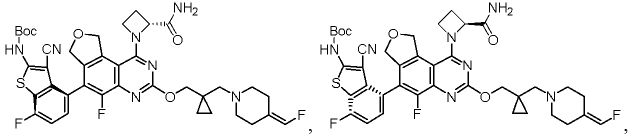 | | |
| 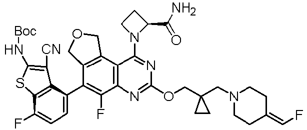 | | |
| | 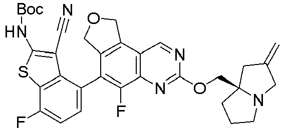 | |
| | 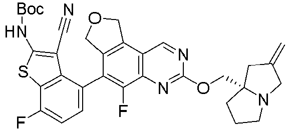 | |
| 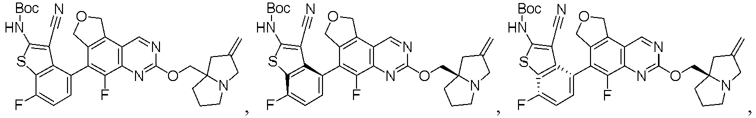 | | |
| 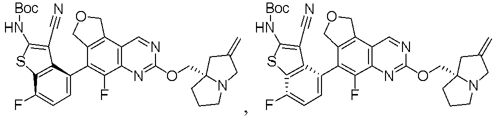 | | |
| | 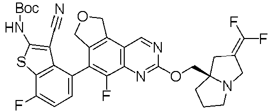 | |
| | 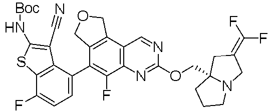 | |
| 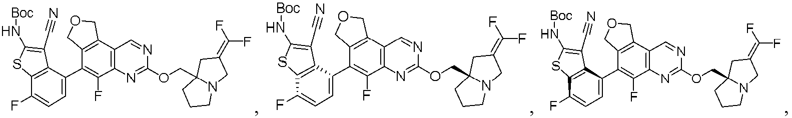 | | |
| 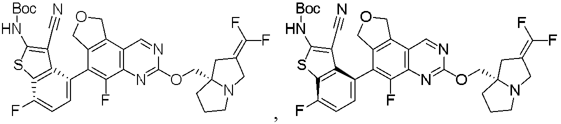 | | |
| 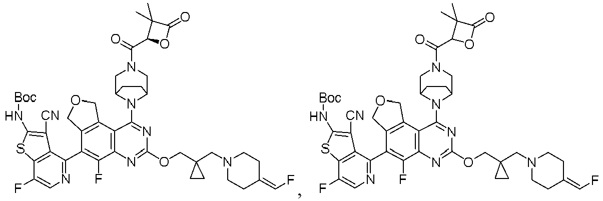 | | |
| 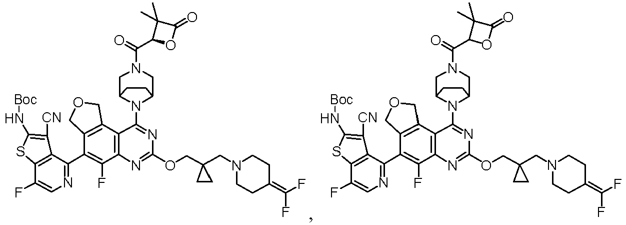 | | |
| 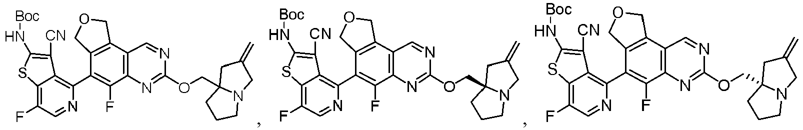 | | |
| | 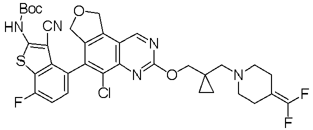 | |
| 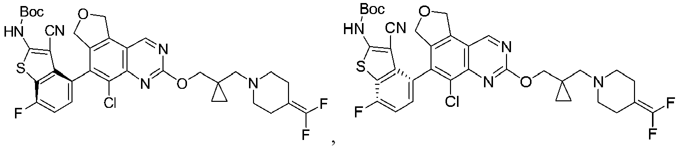 | | |
| | 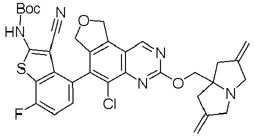 | |
| 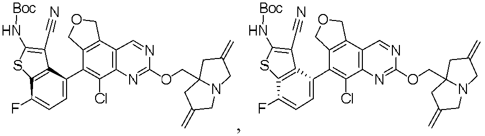 | | |
| **50b-1** | 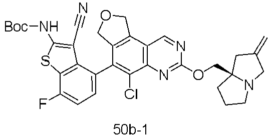 | tert-Butyl (4-(5-chloro-3-(((*R*)-2-methylenetetrahydro-1*H*-pyrrolizin-7*a*(5*H*)-yl)methoxy)-7,9-dihydrofuro[3,4-*f*]quinazolin-6-yl)-3-cyano-7-fluorobenzo[*b*]thiophen-2-yl)carbamate **50b-1** |
| **50b-2** | 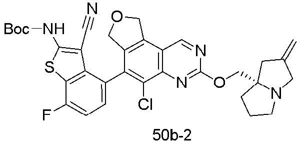 | tert-Butyl (4-(5-chloro-3-(((*S*)-2-methylenetetrahydro-1*H*-pyrrolizin-7*a*(5*H*)-yl)methoxy)-7,9-dihydrofuro[3,4-*f*]quinazolin-6-yl)-3-cyano-7-fluorobenzo[*b*]thiophen-2-yl)carbamate **50b-2** |
| 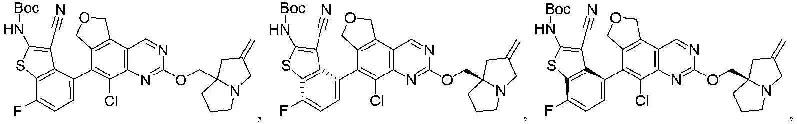 | | |
| 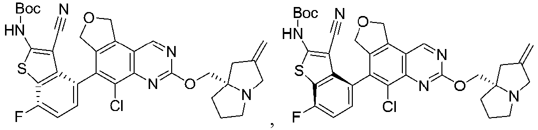 | | |
| **51b-1** | 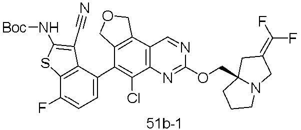 | tert-Butyl (4-(5-chloro-3-(((*R*)-2-(difluoromethylene)tetrahydro-1*H-*pyrrolizin-7*a*(5*H*)-yl)methoxy)-7,9-dihydrofuro[3,4-*f*]quinazolin-6-yl)-3-cyano-7-fluorobenzo[*b*]thiophen-2-yl)carbamate **51b-1** |
| **51b-2** | 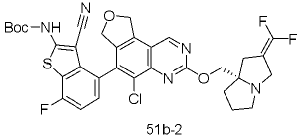 | tert-Butyl (4-(5-chloro-3-(((S)-2-(difluoromethylene)tetrahydro-1*H-*pyrrolizin-7*a*(5*H*)-yl)methoxy)-7,9-dihydrofuro[3,4-*f*]quinazolin-6-yl)-3-cyano-7-fluorobenzo[*b*]thiophen-2-yl)carbamate **51b-2** |
| 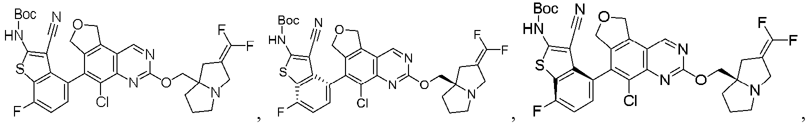 | | |
| 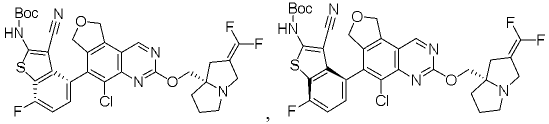 | | |
| 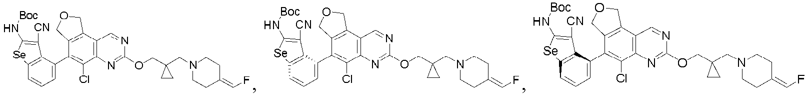 | | |
| 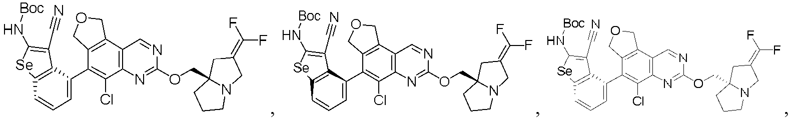 | | |
| 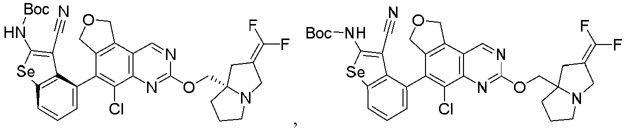 | | |
| 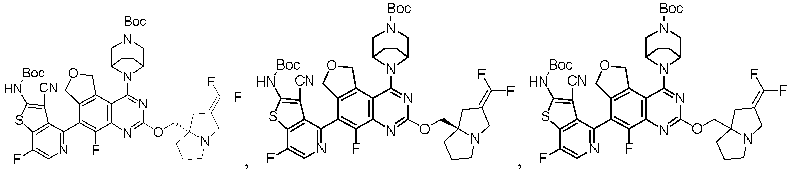 | | |
| **56d** | 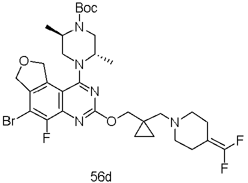 | tert-Butyl (2*R*,5*S*)-4-(6-bromo-3-(1-((4-(difluoromethylene)piperidin-1-yl)methyl)cyclopropyl)methoxy)-5-fluoro-7,9-dihydrofuro[3,4-*f*]quinazolin-1-yl)-2,5-dimethylpiperazine-1-carboxylate **56d** |
| 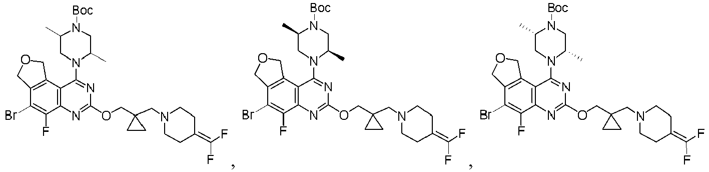 | | |
| 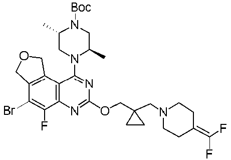 | | |
| **56e** | 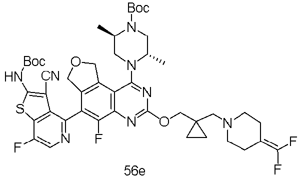 | tert-Butyl (2*R*,5*S*)-4-(6-(2-((tert-butoxycarbonyl)amino)-3-cyano-7-fluorothieno[3,2-*c*]pyridin-4-yl)-3-((1-((4-(difluoromethylene)piperidin-1-yl)methyl)cyclopropyl)methoxy)-5-fluoro-7,9-dihydrofuro[3,4-*f*]quinazolin-1-yl)-2,5-dimethylpiperazine-1-carboxylate **56e** |
| 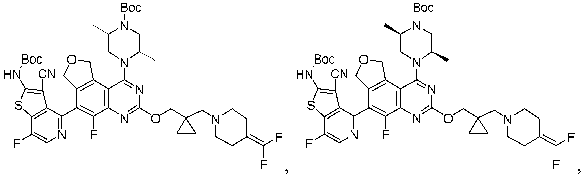 | | |
| 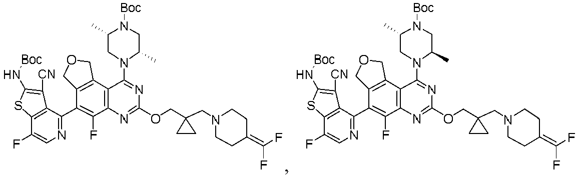 | | |
| **56f** | 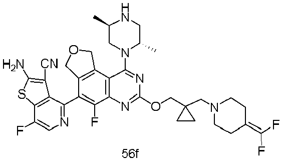 | 2-Amino-4-(3-((1-((4-(difluoromethylene)piperidin-1-yl)methyl)cyclopropyl)methoxy)-1-((2*S*,5*R*)-2,5-dimethylpiperazin-1-yl)-5-fluoro-7,9-dihydrofuro[3,4-*f*]quinazolin-6-yl)-7-fluorothieno[3,2-*c*]pyridine-3-carbonitrile **56f** |
| 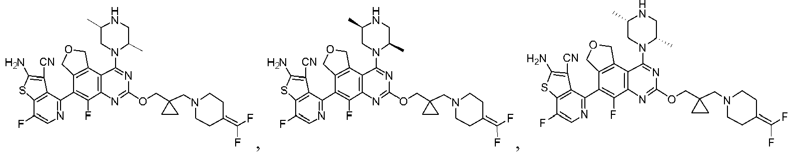 | | |
| 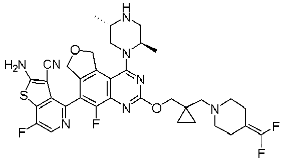 | | |

Another aspect of the present disclosure relates to a method for preparing the compound represented by general formula (II) or the pharmaceutically acceptable salt thereof, wherein the method comprises: 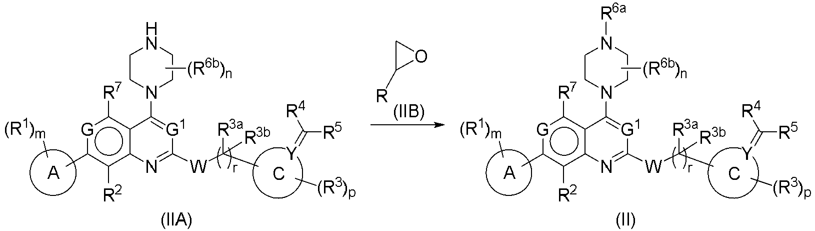
subjecting a compound represented by general formula (IIA) or a salt thereof, and a compound represented by general formula (IIB) or a salt thereof to a ring-opening reaction to obtain the compound represented by general formula (II) or the pharmaceutically acceptable salt thereof,
wherein, R^{6a} is 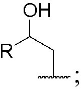 R is hydrogen atom or alkyl;
ring A, ring C, R¹, m, G, G¹, R², R⁷, R^{6b}, n, W, R^{3a}, R^{3b}, r, Y, R³, R⁴, R⁵ and p are as defined in general formula (II).

Another aspect of the present disclosure relates to a method for preparing the compound represented by general formula (III) or the pharmaceutically acceptable salt thereof, wherein the method comprises: 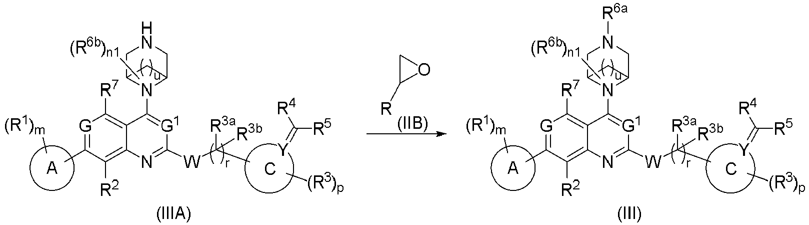
subjecting a compound represented by general formula (IIIA) or a salt thereof, and a compound represented by general formula (IIB) or a salt thereof to a ring-opening reaction to obtain the compound represented by general formula (III) or the pharmaceutically acceptable salt thereof,
wherein, R^{6a} is 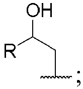 R is hydrogen atom or alkyl;
ring A, ring C, R¹, m, G, G¹, R², R⁷, R^{6b}, n1, u, W, R^{3a}, R^{3b}, r, Y, R³, R⁴, R⁵ and p are as defined in general formula (III).

Another aspect of the present disclosure relates to a method for preparing the compound represented by general formula (IV) or the pharmaceutically acceptable salt thereof, wherein the method comprises: 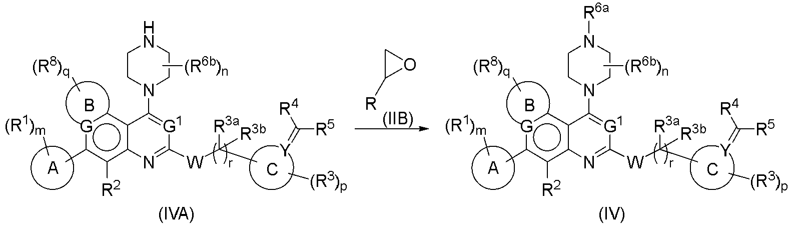
subjecting a compound represented by general formula (IVA) or a salt thereof, and a compound represented by general formula (IIB) or a salt thereof to a ring-opening reaction to obtain the compound represented by general formula (IV) or the pharmaceutically acceptable salt thereof,
wherein, R^{6a} is 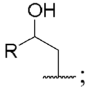 R is hydrogen atom or alkyl;
ring A, ring B, ring C, R¹, m, G, G¹, R², R⁸, q, R^{6b}, n, W, R^{3a}, R^{3b}, r, Y, R³, R⁴, R⁵ and p are as defined in general formula (IV).

Another aspect of the present disclosure relates to a method for preparing the compound as represented by general formula (V) or the pharmaceutically acceptable salt thereof, wherein the method comprises: 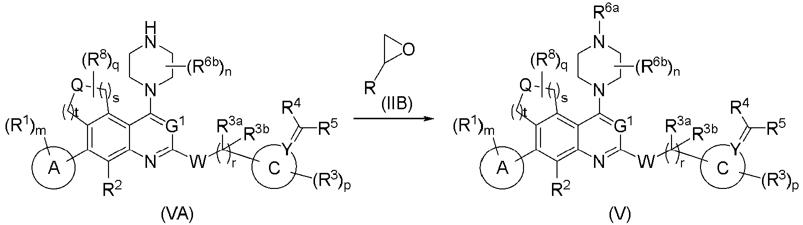
subjecting a compound represented by general formula (VA) or a salt thereof, and a compound represented by general formula (IIB) or a salt thereof to a ring-opening reaction to obtain the compound represented by general formula (V) or the pharmaceutically acceptable salt thereof,
wherein, R^{6a} is 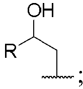 R is hydrogen atom or alkyl;
ring A, ring C, R¹, m, Q, s, t, R⁸, q, R², G¹, R^{6b}, n, W, R^{3a}, R^{3b}, r, Y, R³, R⁴, R⁵ and p are as defined in general formula (V).

In some embodiments of the present disclosure, provided is a method for preparing the compound represented by any of general formulae (II) to (V) or the pharmaceutically acceptable salt thereof, wherein R^{6a} is 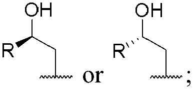 in some embodiments, R^{6a} is 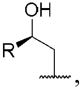 wherein R is defined as above.

In some embodiments of the present disclosure, provided is a method for preparing the compound represented by any of general formulae (II) to (V) or the pharmaceutically acceptable salt thereof, wherein R is hydrogen atom or C₁₋₆ alkyl; in some embodiments, R is C₁₋₆ alkyl; in some embodiments, R is methyl.

In some embodiments of the present disclosure, provided is a method for preparing the compound represented by any of general formulae (II) to (V) or the pharmaceutically acceptable salt thereof, wherein 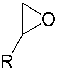 is 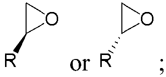 in some embodiments, R is 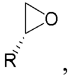 wherein R is defined as above.

Another aspect of the present disclosure relates to a method for preparing the compound as represented by general formula (IM) or the pharmaceutically acceptable salt thereof, wherein the method comprises: 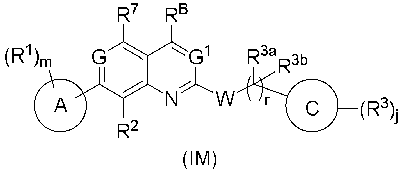
subjecting a compound represented by general formula (IM) in which at least one R¹ is -amino-amino protecting group or -O-hydroxyl protecting group, or a pharmaceutically acceptable salt thereof to a deprotection reaction, to obtain a compound represented by general formula (IM) in which at least one R¹ is amino or hydroxyl, or a pharmaceutically acceptable salt thereof,
wherein, in some embodiments, the amino protecting group is Boc;
ring A, ring C, the remaining R¹, m, G, G¹, R², R⁷, R^{B}, W, R^{3a}, R^{3b}, r, R³ and j are as defined in general formula (IM).

Another aspect of the present disclosure relates to a method for preparing the compound represented by general formula (IN) or the pharmaceutically acceptable salt thereof, wherein the method comprises: 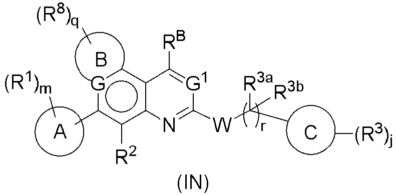
subjecting a compound represented by general formula (IN) in which at least one R¹ is -amino-amino protecting group or -O-hydroxyl protecting group, or a pharmaceutically acceptable salt thereof to a deprotection reaction, to obtain a compound represented by general formula (IN) in which at least one R¹ is amino or hydroxyl, or a pharmaceutically acceptable salt thereof,
wherein, in some embodiments, the amino protecting group is Boc;
ring A, ring B, ring C, the remaining R¹, m, G, R⁸, q, R², G¹, R^{B}, W, R^{3a}, R^{3b}, r, R³ and j are as defined in general formula (IN).

Another aspect of the present disclosure relates to a method for preparing the compound represented by general formula (IIA) or the pharmaceutically acceptable salt thereof, wherein the method comprises: 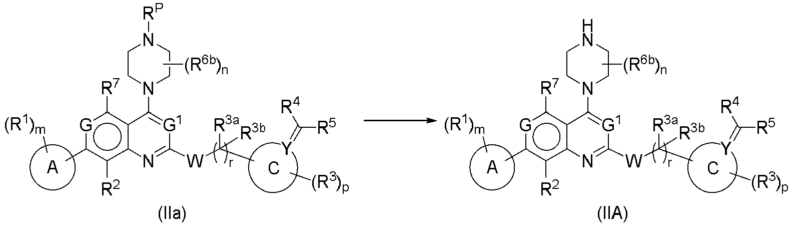
subjecting a compound represented by general formula (IIa) or a salt thereof to a deprotection reaction, to obtain a compound represented by general formula (IIA) or a pharmaceutically acceptable salt thereof,
wherein, R^{P} is an amino protecting group, and in some embodiments, R^{P} is Boc;
ring A, ring C, R¹, m, G, G¹, R², R⁷, R^{6b}, n, W, R^{3a}, R^{3b}, r, Y, R³, R⁴, R⁵ and p are as defined in general formula (IIA).

Another aspect of the present disclosure relates to a method for preparing the compound represented by general formula (IIIA) or the pharmaceutically acceptable salt thereof, wherein the method comprises: 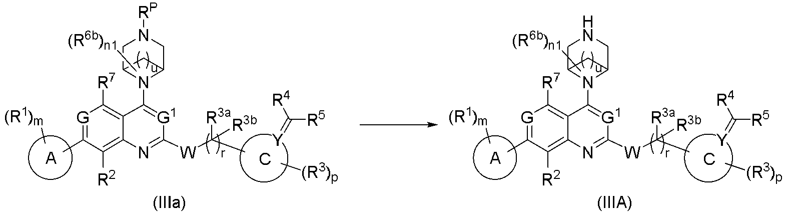
subjecting a compound represented by general formula (IIIa) or a salt thereof to a deprotection reaction, to obtain a compound represented by general formula (IIIA) or a pharmaceutically acceptable salt thereof,
wherein, R^{P} is an amino protecting group, and in some embodiments, R^{P} is Boc;
ring A, ring C, R¹, m, G, G¹, R², R⁷, R^{6b}, n1, u, W, R^{3a}, R^{3b}, r, Y, R³, R⁴, R⁵ and p are as defined in general formula (IIIA).

Another aspect of the present disclosure relates to a method for preparing the compound represented by general formula (IVA) or the pharmaceutically acceptable salt thereof, wherein the method comprises: 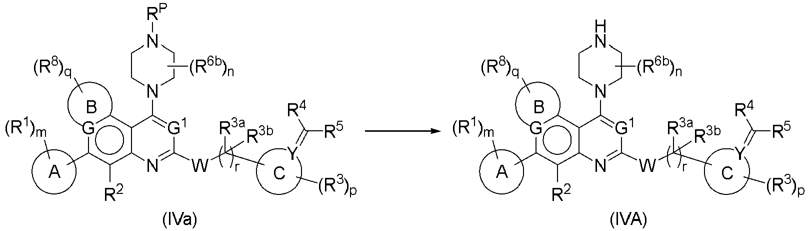
subjecting a compound represented by general formula (IVa) or a salt thereof to a deprotection reaction, to obtain a compound represented by general formula (IVA) or a pharmaceutically acceptable salt thereof,
wherein, R^{P} is an amino protecting group, and in some embodiments, R^{P} is Boc;
ring A, ring B, ring C, R¹, m, G, G¹, R², R⁸, q, R^{6b}, n, W, R^{3a}, R^{3b}, r, Y, R³, R⁴, R⁵ and p are as defined in general formula (IVA).

Another aspect of the present disclosure relates to a method for preparing the compound represented by general formula (VA) or the pharmaceutically acceptable salt thereof, wherein the method comprises: 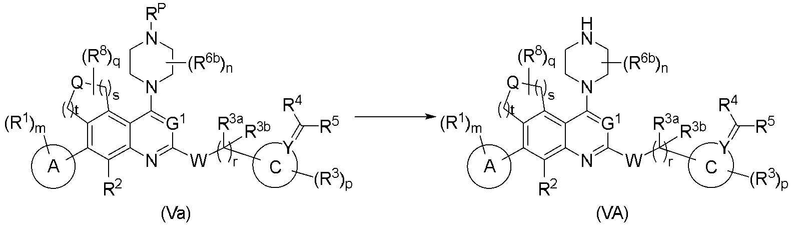
subjecting a compound represented by general formula (Va) or a salt thereof to a deprotection reaction, to obtain a compound represented by general formula (VA) or a pharmaceutically acceptable salt thereof,
wherein, R^{P} is an amino protecting group, and in some embodiments, R^{P} is Boc;
ring A, ring C, R¹, m, Q, s, t, R⁸, q, R², G¹, R^{6b}, n, W, R^{3a}, R^{3b}, r, Y, R³, R⁴, R⁵ and p are as defined in general formula (VA).

Another aspect of the present disclosure relates to a method for preparing the compound represented by general formula (II) or the pharmaceutically acceptable salt thereof, wherein the method comprises: 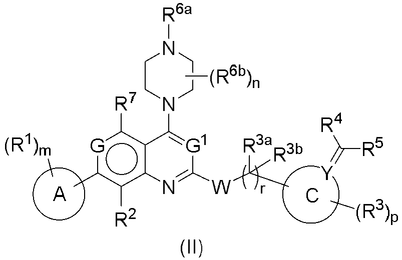
subjecting a compound represented by general formula (II) in which R^{6a} is OR¹⁴, or a pharmaceutically acceptable salt thereof to a deprotection reaction, to obtain a compound represented by general formula (II) in which R^{6a} is OH, or a pharmaceutically acceptable salt thereof,
wherein R¹⁴ is hydroxyl protecting group, and in some embodiments, benzoyl;
wherein, ring A, ring C, R¹, m, G, G¹, R², R⁷, R^{6a}, R^{6b}, n, W, R^{3a}, R^{3b}, r, Y, R³, R⁴, R⁵ and p are as defined in general formula (II).

Another aspect of the present disclosure relates to a method for preparing the compound represented by general formula (III) or the pharmaceutically acceptable salt thereof, wherein the method comprises: 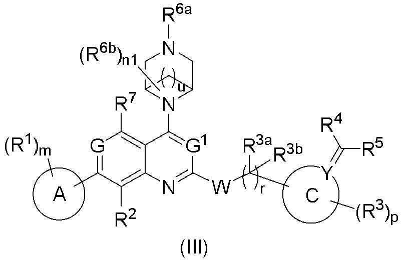
subjecting a compound represented by general formula (III) in which R^{6a} is OR¹⁴, or a pharmaceutically acceptable salt thereof to a deprotection reaction, to obtain a compound represented by general formula (III) in which R^{6a} is OH, or a pharmaceutically acceptable salt thereof;
wherein R¹⁴ is hydroxyl protecting group, and in some embodiments, benzoyl;
wherein, ring A, ring C, R¹, m, G, G¹, R², R⁷, R^{6a}, R^{6b}, n1, u, W, R^{3a}, R^{3b}, r, Y, R³, R⁴, R⁵ and p are as defined in general formula (III).

Another aspect of the present disclosure relates to a method for preparing the compound represented by general formula (IV) or the pharmaceutically acceptable salt thereof, wherein the method comprises: 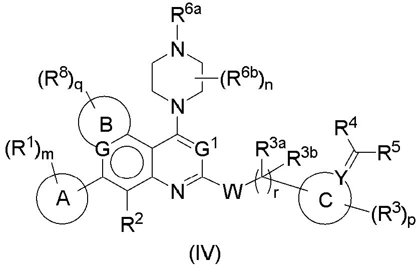
subjecting a compound represented by general formula (IV) in which R^{6a} is OR¹⁴, or a salt thereof to a deprotection reaction, to obtain a compound represented by general formula (IV) in which R^{6a} is OH, or a pharmaceutically acceptable salt thereof;
wherein, ring A, ring B, ring C, R¹, m, G, G¹, R², R⁸, q, R^{6a}, R^{6b}, n, W, R^{3a}, R^{3b}, r, Y, R³, R⁴, R⁵ and p are as defined in general formula (IV).

Another aspect of the present disclosure relates to a method for preparing the compound as represented by general formula (V) or the pharmaceutically acceptable salt thereof, wherein the method comprises: 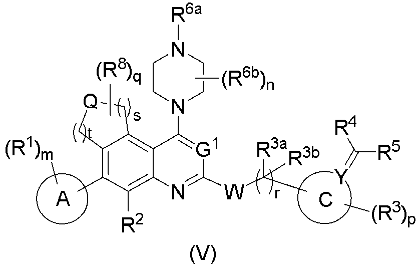
subjecting a compound represented by general formula (V) in which R^{6a} is OR¹⁴, or a pharmaceutically acceptable salt thereof to a deprotection reaction, to obtain a compound represented by general formula (V) in which R^{6a} is OH, or a pharmaceutically acceptable salt thereof,
wherein R¹⁴ is hydroxyl protecting group, and in some embodiments, benzoyl;
wherein, ring A, ring C, R¹, m, R², Q, R⁸, q, s, t, G¹, R^{6a}, R^{6b}, n, W, R^{3a}, R^{3b}, r, Y, R³, R⁴, R⁵ and p are as defined in general formula (V).

Another aspect of the present disclosure relates to a method for preparing the compound as represented by general formula (VI) or the pharmaceutically acceptable salt thereof, wherein the method comprises: 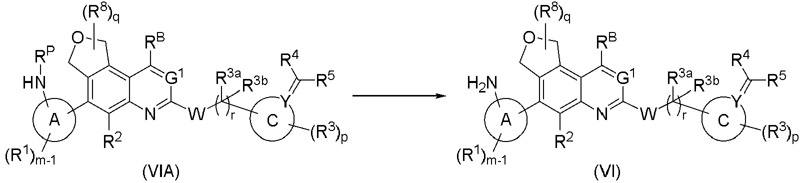
subjecting a compound represented by general formula (VIA) or a salt thereof to a deprotection reaction to obtain a compound represented by general formula (VI) or a pharmaceutically acceptable salt thereof,
wherein, R^{P} is an amino protecting group, and in some embodiments, R^{P} is Boc;
ring A, ring C, R¹, m, R⁸, q, R², G¹, R^{B}, W, R^{3a}, R^{3b}, r, Y, R³, R⁴, R⁵ and p are as defined in general formula (VI).

Another aspect of the present disclosure relates to a method for preparing the compound represented by general formula (VII-1) or (VII-2) or the pharmaceutically acceptable salt thereof, wherein the method comprises: 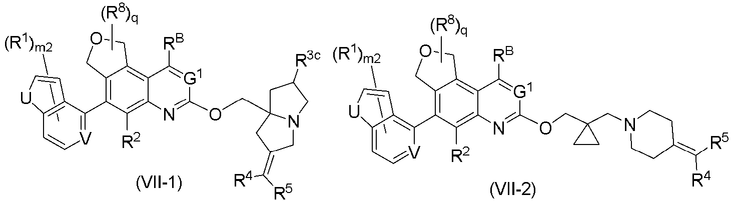
subjecting a compound represented by general formula (VII-1) in which at least one R¹ is -NH-amino protecting group, or a pharmaceutically acceptable salt thereof to a deprotection reaction, to obtain a compound represented by general formula (VII-1) in which at least one R¹ is amino, or a pharmaceutically acceptable salt thereof,
subjecting a compound represented by general formula (VII-2) in which at least one R¹ is -NH-amino protecting group, or a pharmaceutically acceptable salt thereof to a deprotection reaction, to obtain a compound represented by general formula (VII-2) in which at least one R¹ is amino, or a pharmaceutically acceptable salt thereof,
wherein, m2 is 1, 2, 3 or 4; in some embodiments, the amino protecting group is Boc;
U, V, the remaining R¹, R⁸, q, R², G¹, R^{B}, R^{3c}, R⁴ and R⁵ are as defined in general formula (VII-1) or (VII-2).

Another aspect of the present disclosure relates to a method for preparing the compound as represented by general formula (V) or the pharmaceutically acceptable salt thereof, wherein the method comprises: 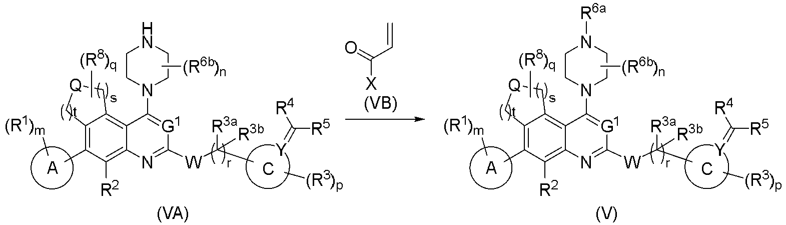
reacting a compound represented by general formula (VA) or a salt thereof, with a compound represented by general formula (VB) or a salt thereof to obtain the compound represented by general formula (V) or the pharmaceutically acceptable salt thereof,
wherein, R^{6a} is 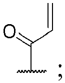 X is OH or halogen, in some embodiments, X is halogen, and in some embodiments, X is Cl;
ring A, ring C, R¹, m, Q, s, t, R⁸, q, R², G¹, R^{6b}, n, W, R^{3a}, R^{3b}, r, Y, R³, R⁴, R⁵ and p are as defined in general formula (V).

Another aspect of the present disclosure relates to a pharmaceutical composition, wherein the pharmaceutical composition comprises the compound represented by any of general formulae (I) to (VII-2), (I'), (I'A), (IM), (IN), (IIA), (IIIA), (IVA) and (VA) or shown in Table A or the pharmaceutically acceptable salt thereof according to the present disclosure, and one or more pharmaceutically acceptable carriers, diluents or excipients.

The present disclosure further relates to the use of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same in the preparation of a medicament for inhibiting KRAS amplification and/or KRAS mutant activity; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to the use of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same in the preparation of a medicament for treating and/or preventing a disease or condition mediated by KRAS amplification and/or KRAS mutant; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to the use of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same in the preparation of a medicament for treating and/or preventing cancer; in some embodiments, the cancer is selected from the group consisting of brain cancer, thyroid cancer, head and neck cancer, nasopharyngeal cancer, pharyngolaryngeal cancer, oral cancer, salivary gland cancer, oesophageal cancer, gastric cancer, lung cancer, liver cancer, kidney cancer, pleural carcinoma, peritoneal carcinoma, pancreatic cancer, gallbladder cancer, bile duct cancer, colourectal cancer, small intestine cancer, gastrointestinal stromal tumour, urothelial cancer, urethral cancer, bladder cancer, anal cancer, joint cancer, breast cancer, vaginal cancer, ovarian cancer, endometrial cancer, cervical cancer, fallopian tube cancer, testicular cancer, prostate cancer, haemangioma, leukaemia, lymphoma, myeloma, skin cancer, melanoma, lipoma, bone cancer, soft tissue sarcoma, neurofibroma, glioma, neuroblastoma, and glioblastoma; in some embodiments, the cancer is selected from the group consisting of pancreatic cancer, colourectal cancer and non-small-cell lung cancer.

The present disclosure further relates to a method for inhibiting KRAS amplification and/or KRAS mutant activity, comprising administering to a patient in need thereof a therapeutically effective amount of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to a method for treating and/or preventing a disease or condition mediated by KRAS amplification and/or KRAS mutant, comprising administering to a patient in need thereof a therapeutically effective amount of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to a method for treating and/or preventing cancer, comprising administering to a patient in need thereof a therapeutically effective amount of the compound represented by any of general formulae (I) to (VII-2), (I'), (I'A), (IM), (IN), (IIA), (IIIA), (IVA) and (VA) or shown in table A or the pharmaceutically acceptable salt thereof, or the pharmaceutical composition comprising same; in some embodiments, the cancer is selected from the group consisting of brain cancer, thyroid cancer, head and neck cancer, nasopharyngeal cancer, pharyngolaryngeal cancer, oral cancer, salivary gland cancer, oesophageal cancer, gastric cancer, lung cancer, liver cancer, kidney cancer, pleural carcinoma, peritoneal carcinoma, pancreatic cancer, gallbladder cancer, bile duct cancer, colourectal cancer, small intestine cancer, gastrointestinal stromal tumour, urothelial cancer, urethral cancer, bladder cancer, anal cancer, joint cancer, breast cancer, vaginal cancer, ovarian cancer, endometrial cancer, cervical cancer, fallopian tube cancer, testicular cancer, prostate cancer, haemangioma, leukaemia, lymphoma, myeloma, skin cancer, melanoma, lipoma, bone cancer, soft tissue sarcoma, neurofibroma, glioma, neuroblastoma, and glioblastoma; in some embodiments, the cancer is selected from the group consisting of pancreatic cancer, colourectal cancer and non-small-cell lung cancer.

The present disclosure further relates to a compound represented by any of general formulae (I) to (VII-2), (I'), (I'A), (IM), (IN), (IIA), (IIIA), (IVA) and (VA) or shown in table A or a pharmaceutically acceptable salt thereof, or a pharmaceutical composition comprising same, for use as a medicament.

The present disclosure further relates to a compound represented by any of general formulae (I) to (VII-2), (I'), (I'A), (IM), (IN), (IIA), (IIIA), (IVA) and (VA) or shown in table A or a pharmaceutically acceptable salt thereof, or a pharmaceutical composition comprising same for inhibiting KRAS amplification and/or KRAS mutant activity; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to a compound represented by any of general formulae (I) to (VII-2), (I'), (I'A), (IM), (IN), (IIA), (IIIA), (IVA) and (VA) or shown in table A or a pharmaceutically acceptable salt thereof, or a pharmaceutical composition comprising same for treating and/or preventing a disease or condition mediated by KRAS amplification and/or KRAS mutant; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The present disclosure further relates to a compound represented by any of general formulae (I) to (VII-2), (I'), (I'A), (IM), (IN), (IIA), (IIIA), (IVA) and (VA) or shown in table A or a pharmaceutically acceptable salt thereof, or a pharmaceutical composition comprising same for treating and/or preventing cancer; in some embodiments, the cancer is selected from the group consisting of brain cancer, thyroid cancer, head and neck cancer, nasopharyngeal cancer, pharyngolaryngeal cancer, oral cancer, salivary gland cancer, oesophageal cancer, gastric cancer, lung cancer, liver cancer, kidney cancer, pleural carcinoma, peritoneal carcinoma, pancreatic cancer, gallbladder cancer, bile duct cancer, colourectal cancer, small intestine cancer, gastrointestinal stromal tumour, urothelial cancer, urethral cancer, bladder cancer, anal cancer, joint cancer, breast cancer, vaginal cancer, ovarian cancer, endometrial cancer, cervical cancer, fallopian tube cancer, testicular cancer, prostate cancer, haemangioma, leukaemia, lymphoma, myeloma, skin cancer, melanoma, lipoma, bone cancer, soft tissue sarcoma, neurofibroma, glioma, neuroblastoma, and glioblastoma; in some embodiments, the cancer is selected from the group consisting of pancreatic cancer, colourectal cancer and non-small-cell lung cancer.

The disease or condition of the present disclosure is one that is treated and/or prevented by inhibiting KRAS amplification and/or KRAS mutant activity; in some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s).

The disease or condition mediated by KRAS amplification and/or KRAS mutant of the present disclosure is cancer. In some embodiments, the KRAS mutant is selected from the group consisting of one or more of KRAS G12A, G12C, G12D, G12V, G12R, G12S, G13A, G13C, G13D, G13R, G13S, G13V, Q61E, Q61H, Q61K, Q61L, Q61P, Q61R, A146T, A146P, A146V and A146T mutations; in some embodiments, the KRAS mutant is KRAS G12D and/or KRAS G12V mutation(s); in some embodiments, the cancer is selected from the group consisting of brain cancer, thyroid cancer, head and neck cancer, nasopharyngeal cancer, pharyngolaryngeal cancer, oral cancer, salivary gland cancer, oesophageal cancer, gastric cancer, lung cancer, liver cancer, kidney cancer, pleural carcinoma, peritoneal carcinoma, pancreatic cancer, gallbladder cancer, bile duct cancer, colourectal cancer, small intestine cancer, gastrointestinal stromal tumour, urothelial cancer, urethral cancer, bladder cancer, anal cancer, joint cancer, breast cancer, vaginal cancer, ovarian cancer, endometrial cancer, cervical cancer, fallopian tube cancer, testicular cancer, prostate cancer, haemangioma, leukaemia, lymphoma, myeloma, skin cancer, melanoma, lipoma, bone cancer, soft tissue sarcoma, neurofibroma, glioma, neuroblastoma, and glioblastoma; in some embodiments, the cancer is selected from the group consisting of pancreatic cancer, colourectal cancer and non-small-cell lung cancer.

The colourectal cancer of the present disclosure is either colon cancer or rectal cancer.

The brain cancer of the present disclosure is selected from the group consisting of polymorphic glioblastoma or neuroblastoma; the soft tissue carcinoma is selected from the group consisting of fibrosarcoma, gastrointestinal sarcoma, rhabdomyoma, leiomyosarcoma, dedifferentiated liposarcoma, pleomorphic liposarcoma, malignant fibrous histiocytoma, round cell sarcoma, and synovial sarcoma; the lymphoma is selected from the group consisting of Hodgkin's disease and non-Hodgkin lymphomas (e.g., mantle cell lymphoma, diffuse large B-cell lymphoma, follicular centre lymphoma, marginal zone B-cell lymphoma, lymphoplasmacytic lymphoma, and peripheral T-cell lymphoma); in some embodiments, the liver cancer is hepatocellular carcinoma; the lung cancer (also known as bronchogenic carcinoma) is selected from the group consisting of non-small cell lung cancer (NSCLC), small cell lung cancer (SCLC), and squamous cell carcinoma; the kidney cancer is selected from the group consisting of renal cell carcinoma, clear cell carcinoma, and renal oncocytoma; the leukaemia is selected from the group consisting of chronic lymphocytic leukaemia (CLL), chronic granulocytic leukaemia, acute lymphoblastic leukaemia (ALL), T-cell acute lymphoblastic leukaemia (T-ALL), chronic myeloid leukaemia (CML), and acute myeloid leukaemia (AML); the skin cancer is selected from the group consisting of malignant melanoma, squamous cell carcinoma, basal cell carcinoma, and angiosarcoma; in some embodiments, the myeloma is multiple myeloma.

The active compound can be formulated into a form suitable for administration via any appropriate route, and the compositions of the present disclosure can be formulated using conventional methods with one or more pharmaceutically acceptable carriers. Therefore, the active compounds of the present disclosure can be formulated into various dosage forms for oral administration, injection (e.g., intravenous injection, intramuscular injection, or subcutaneous injection), inhalation, or insufflation. The compounds of the present disclosure can also be formulated into, for example, dosage forms such as tablets, hard or soft capsules, aqueous or oily suspensions, emulsions, injectable solutions, dispersible powders or granules, suppositories, lozenges and syrups.

As a general guide, the active compound of the present disclosure is presented in a unit dosage form, or in a manner that allows the patient to self-administer a single dose. The compounds or compositions of the present disclosure can be presented in unit dosage forms such as tablets, capsules, cachets, bottled oral solutions, powders, granules, lozenges, suppositories, reconstitutable powders, or liquid preparations. An appropriate unit dose can be 0.1-1000 mg.

In addition to the active compound, the pharmaceutical composition of the present disclosure may contain one or more auxiliary materials selected from the group consisting of: fillers (diluent), binders, wetting agents, disintegrants, excipients, etc. Depending on the administration method, the composition can contain 0.1% to 99% by weight of the active compound.

In some embodiments, the unit dose of the pharmaceutical composition is 0.001 mg-1000 mg.

In certain embodiments, the pharmaceutical composition comprises 0.01%-99.99% of the aforementioned compound or a pharmaceutically acceptable salt thereof, or an isotopic substitute thereof, on the basis of the total weight of the composition. In certain embodiments, the pharmaceutical composition comprises 0.1%-99.9% of the aforementioned compound or a pharmaceutically acceptable salt thereof, or an isotopic substitute thereof. In certain embodiments, the pharmaceutical composition comprises 0.5%-99.5% of the aforementioned compound or a pharmaceutically acceptable salt thereof, or an isotopic substitute thereof. In certain embodiments, the pharmaceutical composition comprises 1%-99% of the aforementioned compound or a pharmaceutically acceptable salt thereof, or an isotopic substitute thereof. In certain embodiments, the pharmaceutical composition comprises 2%-98% of the aforementioned compound or a pharmaceutically acceptable salt thereof, or an isotopic substitute thereof.

In certain embodiments, the pharmaceutical composition comprises 0.01%-99.99% of the pharmaceutically acceptable excipient, on the basis of the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

The tablet contains an active ingredient, and a non-toxic, pharmaceutically acceptable excipient suitable for compounding into a tablet. These excipients can be inert excipients, granulators, disintegrants, binders, and lubricants. These tablets can be uncoated, or coated using known techniques to mask the taste of the drug or provide a sustained-release effect over an extended period by delaying disintegration and absorption in the gastrointestinal tract.

Oral preparations can also be provided as soft gelatine capsules in which the active ingredient is mixed with an inert solid diluent or in which the active ingredient is mixed with a water-soluble carrier or an oil-based vehicle.

The aqueous suspension contains an active substance, and an excipient suitable for compounding into an aqueous suspension. Such excipients are suspending agents, dispersants, or wetting agents. The aqueous suspension can also contain one or more preservatives, one or more colourants, one or more flavouring agents, and one or more sweeteners.

The oil suspension can be prepared by suspending active ingredients in vegetable oil or mineral oil. The oil suspension can contain thickeners. The above-described sweeteners and flavouring agents can be added to provide a palatable preparation. These compositions can be preserved by adding antioxidants.

The pharmaceutical composition of the present disclosure can also be in the form of an oil-in-water emulsion. The oil phase can be vegetable oil, mineral oil, or a mixture thereof. Suitable emulsifiers can be naturally occurring phospholipids, and emulsions can also contain sweeteners, flavouring agents, preservatives, and antioxidants. Such preparations can also contain demulcents, preservatives, colourants, and antioxidants.

The pharmaceutical composition of the present disclosure can be in the form of a sterile injectable aqueous solution. Acceptable vehicles or solvents that can be used include water, Ringer's solution, and isotonic sodium chloride solution. Sterile injectable preparations can be sterile injectable oil-in-water microemulsions in which the active ingredient is dissolved in the oil phase. The injectable solutions or microemulsions can be injected into the patient's bloodstream through local high-volume injection. Alternatively, the solutions and microemulsions are preferably administered in a manner that maintains a constant circulating concentration of the compounds of the present disclosure. To maintain such a constant concentration, a continuous intravenous delivery device can be used. An example of such a device is the Deltec CADD-PLUS. TM. 5400 intravenous injection pump.

The pharmaceutical composition of the present disclosure can be in the form of a sterile injectable water or an oil suspension for intramuscular and subcutaneous administration. The suspension can be prepared using the above-described suitable dispersants or wetting agents and suspending agents according to known techniques. Sterile injectable preparations can also be sterile injectable solutions or suspensions prepared in non-toxic diluents or solvents that are acceptable for parenteral administration. Furthermore, sterile fixed oils can conveniently be used as solvents or suspension media. For this purpose, any blended fixed oil can be used. In addition, fatty acids can also be used in the preparation of injections.

The compounds of the present disclosure can be administered in suppository form for rectal administration. These pharmaceutical compositions can be prepared by mixing the drug with a suitable, non-irritating excipient that is solid at normal temperatures but liquid in the rectum, and thus dissolves in the rectum to release the drug.

The compounds of the present disclosure can be prepared by adding water to form water-dispersible powders and granules. These pharmaceutical compositions can be prepared by mixing the active ingredient with a dispersant or a wetting agent, a suspending agent, or one or more preservatives.

As will be appreciated by a person skilled in the art, the administration dosage of the drug depends on a variety of factors, including, but not limited to: the activity of the specific compound used, severity of disease, the patient's age, weight, health status, behaviour and diet, administration time, administration method, excretion rate, and combination of drugs. Furthermore, the optimal treatment method, such as the mode of treatment, the daily dosage of the compound, or the type of pharmaceutically acceptable salts, can be validated by conventional therapeutic protocols.

### Description of Terminology

Unless stated to the contrary, the terms used in the description and claims have the following meanings.

The term "alkyl" refers to a saturated aliphatic hydrocarbon group, which is a straight- or branched-chain group containing 1 to 20 carbon atoms. In some embodiments, it refers to an alkyl group having 1 to 12 carbon atoms (e.g., 1, 2, 3, 4, 5, 6, 7, 8, 9, 10, 11, and 12 carbon atoms) (i.e., C₁₋₁₂ alkyl), and in some embodiments, it refers to an alkyl group having 1 to 6 carbon atoms (i.e., C₁₋₆ alkyl). Non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When substituted, the alkyl can be substituted at any available point of attachment, and the substituents are one or more selected from the group consisiting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkylene" refers to a divalent alkyl group, wherein the alkyl, as defined above, has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). In some embodiments, the alkylene has 1 to 12 carbon atoms (i.e., C₁₋₁₂ alkylene). In some embodiments, the alkylene has 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene can be substituted or unsubstituted. When substituted, the alkylene can be substituted at any available point of attachment, and the substituents are one or more selected from the group consisiting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkenyl" refers to an alkyl group containing at least one carbon-carbon double bond in the molecule, wherein the definition of alkyl is as described above. In some embodiments, it has 2 to 12 carbon atoms (e.g., 2, 3, 4, 5, 6, 7, 8, 9, 10, 11, and 12 carbon atoms) (i.e., C₂₋₁₂ alkenyl), and in some embodiments, it has 2 to 6 carbon atoms (i.e., C₂₋₆ alkenyl). Non-limiting examples include: ethenyl, propenyl, isopropenyl, butenyl, etc. The alkenyl can be substituted or unsubstituted. When substituted, the substituents are one or more selected from the group consisiting of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkynyl" refers to an alkyl group containing at least one carbon-carbon triple bond in the molecule, wherein the definition of alkyl is as described above. In some embodiments, it has 2 to 12 carbon atoms (e.g., 2, 3, 4, 5, 6, 7, 8, 9, 10, 11, and 12 carbon atoms) (i.e., C₂₋₁₂ alkynyl), and in some embodiments, it has 2 to 6 carbon atoms (i.e., C₂₋₆ alkynyl). Non-limiting examples include: ethynyl, propynyl, butynyl, pentynyl, hexynyl, etc. The alkynyl can be substituted or unsubstituted. When substituted, the substituents are one or more selected from the group consisiting of alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyl" refers to a saturated or partially unsaturated monocyclic or polycyclic cyclic hydrocarbon substituent. The cycloalkyl ring contains 3 to 20 carbon atoms. In some embodiments, it contains 3 to 14 carbon atoms (e.g., 3, 4, 5, 6, 7, 8, 9, 10, 11, 12, 13, and 14 carbon atoms) (i.e., 3- to 14-membered cycloalkyl). In some embodiments, it contains 3 to 8 carbon atoms (e.g., 3, 4, 5, 6, 7, and 8 carbon atoms) (i.e., 3- to 8-membered cycloalkyl). In some embodiments, it contains 3 to 6 carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; polycyclic cycloalkyl includes spirocycloalkyl, fused cycloalkyl and bridged cycloalkyl.

The term "spirocycloalkyl" refers to a 5- to 20-membered polycyclic group in which monocycles share one carbon atom (referred to as the spiro atom), which may contain one or more double bonds. In some embodiments, the spirocycloalkyl is 6-to 14-membered. In some embodiments, the spirocycloalkyl is 7- to 10-membered (such as 7-, 8-, 9- or 10-membered). The spirocycloalkyl can be classified into monospirocycloalkyl and polyspirocycloalkyl (e.g., dispirocycloalkyl) based on the number of spiro atoms shared between rings. In some embodiments, the spirocycloalkyl can be classified into monospirocycloalkyl and dispirocycloalkyl. In some embodiments, the spirocycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl. Non-limiting examples of spirocycloalkyl include: 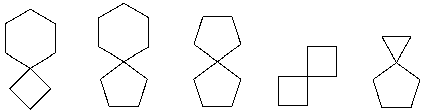 (the point of attachment can be at any position); 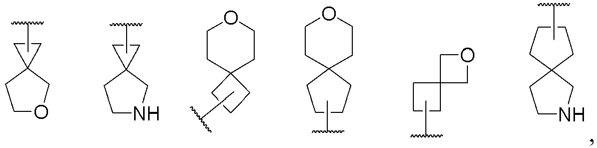 etc.

The term "fused cycloalkyl" refers to a 5- to 20-membered all-carbon polycyclic group in which each ring in the system shares an adjacent pair of carbon atoms with other rings in the system, and one or more rings may contain one or more double bonds. In some embodiments, the fused cycloalkyl is 6- to 14-membered. In some embodiments, the fused cycloalkyl is 7- to 10-membered (such as 7-, 8-, 9- or 10-membered). Based on the number of constituent rings, the fused cycloalkyl can be classified into bicyclic or polycyclic fused cycloalkyl (e.g., tricyclic or tetracyclic). In some embodiments, the fused cycloalkyl is bicyclic or tricyclic. In some embodiments, the fused cycloalkyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicycloalkyl. Non-limiting examples of fused cycloalkyl include: 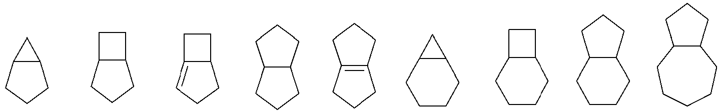 (the point of attachment can be at any position); 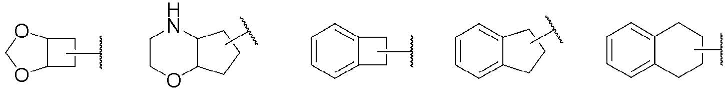 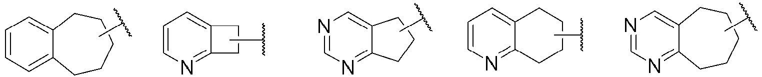 etc.

The term "bridged cycloalkyl" refers to a 5- to 20-membered all-carbon polycyclic group in which any two rings share two carbon atoms that are not directly connected, which may contain one or more double bonds. In some embodiments, the fused cycloalkyl is 6- to 14-membered. In some embodiments, the fused cycloalkyl is 7- to 10-membered (such as 7-, 8-, 9- or 10-membered). According to the number of constituent rings, it can be classified into bicyclic or polycyclic (e.g., tricyclic or tetracyclic) bridged cycloalkyl. In some embodiments, it is bicyclic, tricyclic, or tetracyclic; in some embodiments, it is bicyclic or tricyclic. Non-limiting examples of bridged cycloalkyl include: 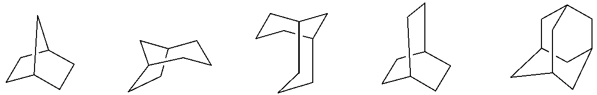 (the point of attachment can be at any position).

The cycloalkyl ring includes the cycloalkyl as described above (including monocyclic, spiro, fused, and bridged rings) fused to an aryl, heteroaryl, or heterocycloalkyl ring, wherein the ring connected to the parent structure is cycloalkyl. Non-limiting examples thereof include 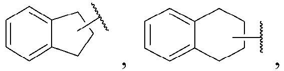 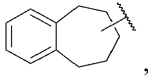 etc.; in some embodiments, the cycloalkyl ring is 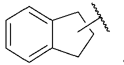 or

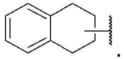

The cycloalkyl can be substituted or unsubstituted. When substituted, the cycloalkyl can be substituted at any available point of attachment, and the substituents are one or more selected from the group consisiting of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include: methoxy, ethoxy, propoxy, and butoxy. The alkoxy can be optionally substituted or unsubstituted. When substituted, the substituents are selected from the group consisiting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "heterocyclyl" refers to a saturated or partially unsaturated monocyclic or polycyclic cyclic substituent, containing 3 to 20 ring atoms (e.g., 3, 4, 5, 6, 7, 8, 9, 10, 11, 12, 13, 14, 15, 16, 17, 18, 19, or 20 ring atoms) (i.e., 3- to 20-membered heterocyclyl), wherein one or more ring atoms are heteroatoms selected from nitrogen, oxygen, and sulfur, and the sulfur may be optionally oxidised (i.e., forming a sulfoxide or sulfone), but excluding ring moieties of -O-O-, -O-S-, or -S-S-, with the remaining ring atoms being carbon. In some embodiments, it contains 3 to 14 ring atoms (e.g., 3, 4, 5, 6, 7, 8, 9, 10, 11, 12, 13, and 14 ring atoms) (i.e., 3- to 14-membered heterocyclyl), 1 to 4 (e.g., 1, 2, 3, and 4) of which are heteroatoms; in some embodiments, it contains 3 to 8 ring atoms (e.g., 3, 4, 5, 6, 7, and 8) (i.e., 3- to 8-membered heterocyclyl) or 6 to 14 ring atoms (e.g., 6, 7, 8, 9, 10, 11, 12, 13, and 14), 1 to 3 (e.g., 1, 2, and 3) of which are heteroatoms; in some embodiments, it contains 3 to 8 ring atoms, 1 to 3 (e.g., 1, 2, and 3) of which are heteroatoms; in some embodiments, it contains 5 or 6 ring atoms (i.e., 5-membered or 6-membered heterocyclyl), 1 to 3 of which are heteroatoms. Non-limiting examples of monocyclic heterocyclyl include pyrrolidinyl, tetrahydropyranyl, 1,2,3,6-tetrahydropyridyl, piperidyl, piperazinyl, morpholinyl, thiomorpholinyl, homopiperazinyl, etc. Polycyclic heterocyclyl includes spiroheterocyclyl, fused heterocyclyl, and bridged heterocyclyl.

The term "spiroheterocyclyl" refers to a 5- to 20-membered polycyclic heterocyclic group in which monocyclic rings share one atom (referred to as the spiro atom), wherein one or more ring atoms are heteroatoms selected from nitrogen, oxygen, and sulfur, and the sulfur may be optionally oxidised (i.e., forming a sulfoxide or sulfone), with the remaining ring atoms being carbon. It may contain one or more double bonds. In some embodiments, it is 6- to 14-membered (e.g., 6, 7, 8, 9, 10, 11, 12, 13, and 14-membered) (i.e., 6- to 14-membered spiroheterocyclyl), and in some embodiments, it is 7- to 10-membered (e.g., 7, 8, 9, or 10-membered) (i.e., 7- to 10-membered spiroheterocyclyl). The spiroheterocyclyl can be classified into monospiroheterocyclyl or polyspiroheterocyclyl (e.g., dispiroheterocyclyl) based on the number of spiro atoms shared between rings. In some embodiments, the spiro heterocyclyl is monospiroheterocyclyl or dispiroheterocyclyl. In some embodiments, the spiroheterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocyclyl. Non-limiting examples of spiroheterocyclyl include:

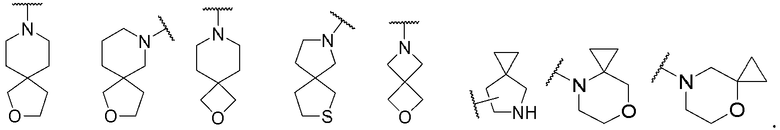

The term "fused heterocyclyl" refers to a 5- to 20-membered polycyclic heterocyclic group in which each ring in the system shares an adjacent pair of atoms with other rings in the system. One or more rings may contain one or more double bonds, wherein one or more ring atoms are heteroatoms selected from nitrogen, oxygen, and sulfur, and the sulfur may be optionally oxidised (i.e., forming a sulfoxide or sulfone), with the remaining ring atoms being carbon. In some embodiments, it is 6- to 14-membered (e.g., 6, 7, 8, 9, 10, 11, 12, 13, and 14-membered) (i.e., 6- to 14-membered fused heterocyclyl), and in some embodiments, it is 7- to 10-membered (e.g., 7, 8, 9, or 10-membered) (i.e., 7- to 10-membered fused heterocyclyl). Based on the number of constituent rings, the fused heterocyclyl can be classified into bicyclic or polycyclic (e.g., tricyclic or tetracyclic) fused heterocyclyl. In some embodiments, it is bicyclic or tricyclic. In some embodiments, the fused heterocyclyl is 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples of fused heterocyclyl include:

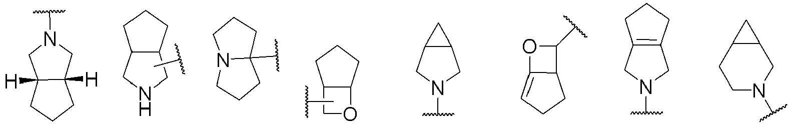

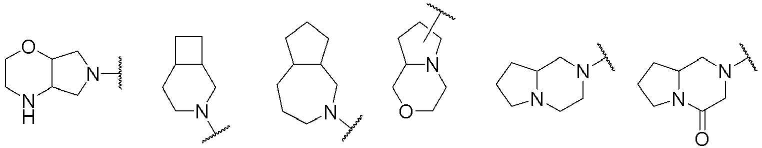

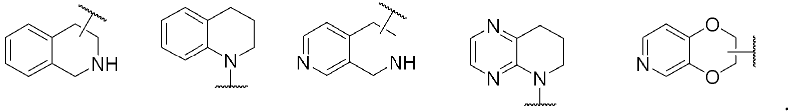

The term "bridged heterocyclyl" refers to a 5- to 20-membered polycyclic heterocyclic group in which any two rings share two atoms that are not directly connected, which may contain one or more double bonds, wherein one or more ring atoms are heteroatoms selected from nitrogen, oxygen, and sulfur, and the sulfur may be optionally oxidised (i.e., forming a sulfoxide or sulfone), with the remaining ring atoms being carbon. In some embodiments, it is 6- to 14-membered (e.g., 6, 7, 8, 9, 10, 11, 12, 13, and 14-membered) (i.e., 6- to 14-membered bridged heterocyclyl), and in some embodiments, it is 7- to 10-membered (e.g., 7, 8, 9, or 10-membered) (i.e., 7- to 10-membered bridged heterocyclyl). According to the number of constituent rings, it can be classified into bicyclic or polycyclic (e.g., tricyclic or tetracyclic) bridged heterocyclyl. In some embodiments, it is bicyclic, tricyclic, or tetracyclic; in some embodiments, it is bicyclic or tricyclic. Non-limiting examples of bridged heterocyclyl include:

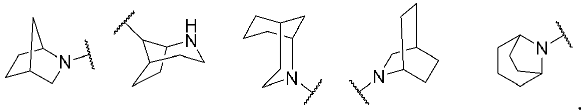

The heterocyclyl ring includes the heterocyclyl as described above (including monocyclic, spiroheterocyclic, fused heterocyclic, and bridged heterocyclic rings) fused to an aryl, heteroaryl, or cycloalkyl ring, wherein the ring connected to the parent structure is heterocyclyl. Non-limiting examples thereof include:

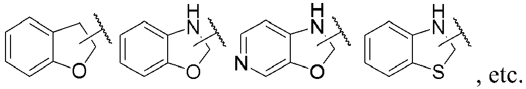

The heterocyclyl can be substituted or unsubstituted. When substituted, the heterocyclyl can be substituted at any available point of attachment, and the substituents are one or more selected from the group consisiting of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "aryl" refers to a 6- to 14-membered all-carbon monocyclic or fused polycyclic (fused polycyclic rings are rings that share a pair of adjacent carbon atoms) group with a conjugated π-electron system. In some embodiments, it is 6- to 10-membered, for example, phenyl and naphthyl. The aryl ring includes the aryl ring as described above fused to a heteroaryl, heterocyclyl, or cycloalkyl ring, wherein the ring connected to the parent structure is an aryl ring. Non-limiting examples thereof include:

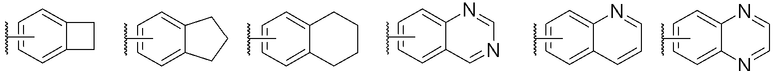

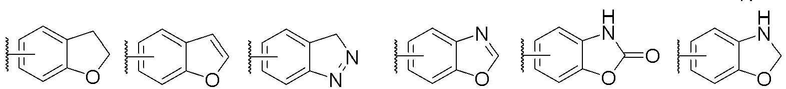

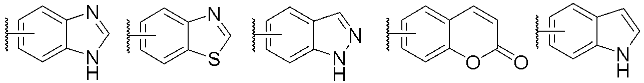

The aryl can be substituted or unsubstituted. When substituted, the aryl can be substituted at any available point of attachment, and the substituents are one or more selected from the group consisiting of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3, and 4) heteroatoms and 5 to 14 ring atoms, wherein the heteroatoms are selected from the group consisiting of oxygen, sulfur, nitrogen, and selenium (in some embodiments, the heteroatoms are selected from the group consisiting of oxygen, sulfur, and nitrogen). In some embodiments, the heteroaryl is 5- to 10-membered (e.g., 5-, 6-, 7-, 8-, 9-, or 10-membered) (i.e., 5- to 10-membered heteroaryl); in some embodiments, it is 8- to 10-membered (e.g., 8-, 9-, or 10-membered); in some embodiments, it is 5-membered or 6-membered (i.e., 5-membered or 6-membered heteroaryl), such as furyl, thienyl, pyridyl, pyrrolyl, N-alkylpyrrolyl, pyrimidinyl, pyrazinyl, pyridazinyl, imidazolyl, pyrazolyl, triazolyl, tetrazolyl, and selenophenyl. The heteroaryl ring includes the heteroaryl as described above fused to an aryl, heterocyclyl, or cycloalkyl ring, wherein the ring connected to the parent structure is a heteroaryl ring. Non-limiting examples thereof include: 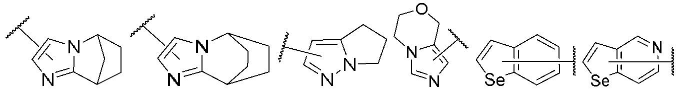 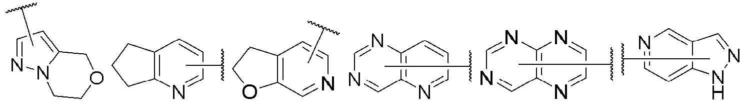 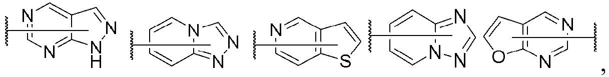 etc.

The heteroaryl can be substituted or unsubstituted. When substituted, the heteroaryl can be substituted at any available point of attachment, and the substituents are one or more selected from the group consisiting of halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The aforementioned cycloalkyl, heterocyclyl, aryl, and heteroaryl include residues derived from the removal of one hydrogen atom from a parent ring atom, or residues derived from the removal of two hydrogen atoms from the same ring atom or two different ring atoms of the parent, i.e., "divalent cycloalkyl", "divalent heterocyclyl", "arylene" and "heteroarylene".

The term "amino protecting group" refers to a group that is easily removable and used to protect an amino group so that the amino group remains unchanged while reactions are carried out on other parts of the molecule. Non-limiting examples include (trimethylsilyl)ethoxymethyl (SEM), tetrahydropyranyl, tert-butoxycarbonyl (Boc), acetyl, benzyl, allyl, p-toluenesulfonyl (Ts), p-methoxybenzyl (PMB), etc. These groups may be optionally substituted with 1 to 3 substituents selected from halogen, alkoxy, and nitro; in some embodiments, the amino protecting group is Boc or PMB.

The term "hydroxy protecting group" refers to a derivative of a hydroxy group that is commonly used to block or protect the hydroxy group while reactions are carried out on other functional groups of the compound. By way of example, the hydroxy protecting groups include, but are not limited to: triethylsilyl, triisopropylsilyl, tert-butyldimethylsilyl (TBS), tert-butyldiphenylsilyl, methyl, tert-butyl, allyl, benzyl, methoxymethyl (MOM), tert-butyldiphenylsilyl (TBDPS), ethoxyethyl, 2-tetrahydropyranyl (THP), formyl, acetyl, benzoyl, and p-nitrobenzoyl; in some embodiments, the hydroxy protecting group is benzoyl.

The term "alkynyl protecting group" refers to a group that is easily removable and introduced onto an alkynyl group to protect the active hydrogen in acetylene or terminal alkynes, so that it remains unchanged while reactions are carried out on other parts of the molecule. Non-limiting examples include: trimethylsilyl (TMS), triethylsilyl (TES), tert-butyldimethylsilyl (TBS), triisopropylsilyl (TIPS), tert-butyldimethylsilyl (TBDMS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, etc.; in some embodiments, the alkynyl protecting group is TIPS.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "cycloalkylalkyl" refers to an alkyl group substituted with one or more cycloalkyl groups, wherein the cycloalkyl and alkyl are as defined above.

The term "heterocyclylalkyl" refers to an alkyl group substituted with one or more heterocyclyl groups, wherein the heterocyclyl and alkyl are as defined above.

The term "arylalkyl" refers to an alkyl group substituted with one or more aryl groups, wherein the aryl and alkyl are as defined above.

The term "heteroarylalkyl" refers to an alkyl group substituted with one or more heteroaryl groups, wherein the heteroaryl and alkyl are as defined above.

The term "alkylthio" refers to alkyl-S-, wherein the alkyl is as defined above.

The term "haloalkyl" refers to an alkyl group substituted with one or more halogen, wherein the alkyl is as defined above.

The term "haloalkoxy" refers to an alkoxy group substituted with one or more halogen, wherein the alkoxy is as defined above.

The term "deuterioalkyl" refers to an alkyl group substituted with one or more deuterium atoms, wherein the alkyl is as defined above.

The term "hydroxyalkyl" refers to alkyl substituted with one or more hydroxyl groups, wherein the alkyl is as defined above.

The term "alkoxyalkyl" refers to an alkyl group substituted with one or more alkoxy groups, wherein the alkyl and alkoxy are as defined above; in some embodiments, the alkoxyalkyl is -alkyl-alkoxy, including, but not limited to, methoxyethyl and methoxypropyl.

The term "halogen" refers to fluorine, chlorine, bromine or iodine.

The term "hydroxyl" refers to -OH.

The term "mercapto" refers to -SH.

The term "amino" refers to -NH₂.

The term "amino-amino protecting group" refers to -NH-amino protecting group.

The term "cyano" refers to -CN.

The term "nitro" refers to -NO₂.

The term "oxo group" or "oxo" refers to "=O".

The term "carbonyl" refers to C=O.

The term "carboxyl" refers to -C(O)OH.

The term "carboxylate ester group" refers to -C(O)O(alkyl),-C(O)O(cycloalkyl), (alkyl)C(O)O- or (cycloalkyl)C(O)O-, wherein the alkyl and cycloalkyl are as defined above.

MOM refers to methoxymethyl.

Boc refers to tert-butoxycarbonyl.

TIPS refers to triisopropylsilyl.

TBS refers to tert-butyldimethylsilyl.

The compounds of the present disclosure may include all forms of their rotamers and conformationally restricted states. Also included are atropisomers. The term "atropisomer" refers to stereoisomers resulting from hindered rotation around a single bond, where the energy difference due to steric strain or other contributing factors creates a sufficiently high rotational barrier to allow for the separation of individual conformers. For example, certain compounds of the present disclosure may exist as a mixture of atropisomers (such as an equal proportion mixture and a mixture enriched in one atropisomer) or as a purified single atropisomer. Non-limiting examples include:

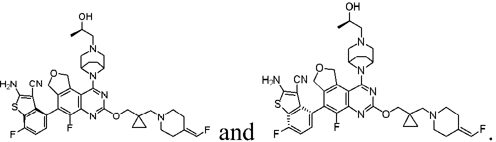

The compounds and intermediates of the present disclosure may also exist in different tautomeric forms, and all such forms are encompassed within the scope of the present disclosure. The term "tautomer" or "tautomeric form" refers to structural isomers of different energies which are interconvertible via a low energy barrier. For example, proton tautomers (also known as proton transfer tautomers) include interconversion via proton migration, such as keto-enol, imine-enamine and lactam-lactim isomerization. An example of keto-enol equilibrium is as shown below: 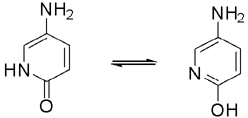 all tautomeric forms are included within the scope of the present disclosure. The names of the compounds do not exclude any tautomers.

The compounds of the present disclosure can exist in specific stereoisomeric forms. The term "stereoisomer" refers to isomers with the same structure but different spatial arrangements of atoms. It includes cis- and trans- (or *Z*- and *E*-) isomers, (-)- and (+)-isomers, (*R*)- and (*S*)-enantiomers, diastereomers, *(D*)- and (*L*)-isomers, tautomers, atropisomer, conformational isomers and mixtures thereof (such as mixtures of racemates and diastereomers). The substituents in the compounds of the present disclosure can contain additional asymmetric atoms. All these stereoisomer and mixtures thereof are encompassed within the scope of the present disclosure. For all carbon-carbon double bonds, both the *Z*- and *E*-forms are included, even if only one configuration is named. Optically active (-)- and (+)-isomers, (*R*)- and (*S*)-enantiomers, and *(D*)- and (*L*)-isomers can be prepared by using chiral synthesis, chiral reagents or other conventional techniques. An isomer of a compound of the present disclosure can be prepared by asymmetric synthesis or derivatization with a chiral auxiliary. Alternatively, where the molecule contains a basic functional group (such as amino) or an acidic functional group (such as carboxyl), diastereomeric salts can be formed with an appropriate optically active acid or base, followed by resolution of the diastereomers using conventional methods well known in the art to obtain the pure isomers. In addition, the separation of enantiomers and diastereomers is usually performed by chromatography.

In the chemical structure of the compound of the present disclosure, the bond " 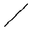 " represents an unspecified configuration; that is, if the chemical structure exists as stereoisomers, the bond " 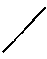 " can be " 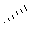 " or " 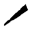 ", or both the configurations of " 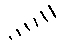 " and " 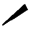 " are involved; the bond " 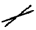 " does not specify configuration, that is, it can be in the Z configuration or the E configuration, or include both configurations simultaneously.

The compounds of the present disclosure include all suitable isotopic derivatives thereof. The term "isotopic derivative" refers to a compound in which at least one atom is replaced by an atom having the same atomic number but a different atomic mass. Examples of isotopes that can be introduced into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D),³H (tritium, T), **^{2c}**, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I. In some embodiments, the isotope is deuterium.

Deuterated drugs offer advantages over their non-deuterated counterparts, such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom can be independently replaced by a deuterium atom, where such deuterium replacement can be partial or complete, and partial deuterium replacement means that at least one hydrogen atom is replaced by at least one deuterium atom.

"Optionally" or "optional" means that the event or circumstance subsequently described may but need not occur, and the description includes the case where the event or circumstance occurs or does not occur. For example, "C₁₋₆ alkyl optionally substituted with halogen or cyano" means that halogen or cyano may but need not be present. The description includes the case where the alkyl is substituted with halogen or cyano and the case where the alkyl is not substituted with halogen or cyano.

The term "substituted" means that one or more hydrogen atoms in a group, such as 1 to 6 hydrogen atoms, and in some embodiments 1 to 3 hydrogen atoms, are each independently substituted with a corresponding number of substituents. A person skilled in the art would have been able to determine the possible or impossible substitutions (by experiment or theory) without undue effort. For example, an amino group or hydroxyl group having free hydrogen may be unstable when it binds to a carbon atom bearing an unsaturated (e.g., olefinic) bond.

The term "pharmaceutical composition" refers to a mixture of one or more of the compounds described herein, or pharmaceutically acceptable salts or prodrugs thereof, and other chemical components, as well as other ingredients such as pharmaceutically acceptable carriers and excipients. The purpose of the pharmaceutical compositions is to facilitate administration to living organisms and facilitate the absorption of active ingredients to exert biological activity.

The term "pharmaceutically acceptable salt" refers to a salt of the compound of the present disclosure, which can be selected from an inorganic salt or an organic salt. Such salts are safe and effective when used in mammals, and possess the expected biological activity. The salts can be prepared during the final isolation and purification of the compound, or separately by reacting the appropriate group with a suitable base or acid. The bases commonly used to form pharmaceutically acceptable salts include inorganic bases such as sodium hydroxide and potassium hydroxide, and organic bases such as ammonia. The acids commonly used to form pharmaceutically acceptable salts include inorganic acids and organic acids.

As used herein, the term "pharmaceutically acceptable" refers to those compounds, materials, compositions and/or dosage forms that are, within the scope of sound medical judgment, suitable for use in contact with patient tissues without excessive toxicity, irritation, allergic response, or other problems or complications, commensurate with a reasonable benefit/risk ratio, and effective for the intended use.

As used herein, the singular forms "a," "an," and "the" include plural referents and vice versa, unless otherwise clearly indicated in the context.

When the term "about" is applied to parameters such as pH, concentration, and temperature, it indicates that the parameters can vary by ±10%, and sometimes within ±5%. As will be understood by a person skilled in the art, numerical values for non-critical parameters are generally provided for illustrative purposes only and not as limitations.

### Methods for synthesising compounds of the present disclosure

In order to achieve the objectives of the present disclosure, the following technical solutions are adopted:

### Scheme I

Provided in the present disclosure is a method for preparing the compound represented by general formula (II) or the pharmaceutically acceptable salt thereof, wherein the method comprises: 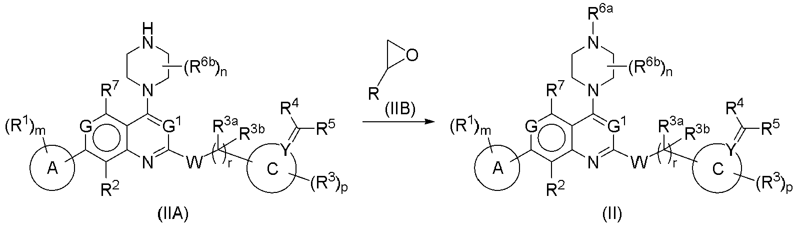
subjecting a compound represented by general formula (IIA) or a salt thereof, and a compound represented by general formula (IIB) or a salt thereof to a ring-opening reaction to obtain the compound represented by general formula (II) or the pharmaceutically acceptable salt thereof,
wherein, R^{6a} is 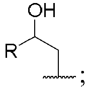 R is hydrogen atom or alkyl;
ring A, ring C, R¹, m, G, G¹, R², R⁷, R^{6b}, n, W, R^{3a}, R^{3b}, r, Y, R³, R⁴, R⁵ and p are as defined in general formula (II).

### Scheme I-2

Provided in the present disclosure is a method for preparing the compound represented by general formula (II) or the pharmaceutically acceptable salt thereof, wherein the method comprises: 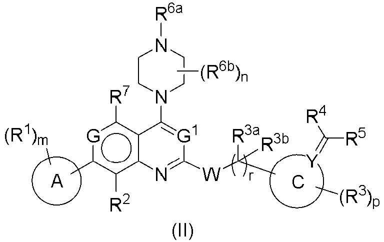
subjecting a compound represented by general formula (II) in which R^{6a} is OR¹⁴, or a pharmaceutically acceptable salt thereof to a deprotection reaction under alkaline conditions, to obtain a compound represented by general formula (II) in which R^{6a} is OH, or a pharmaceutically acceptable salt thereof,
wherein R¹⁴ is hydroxyl protecting group, and in some embodiments, benzoyl.
wherein, ring A, ring C, R¹, m, G, G¹, R² , R⁷, R^{6a}, R^{6b}, n, W, R^{3a}, R^{3b}, r, Y, R³, R⁴, R⁵ and p are as defined in general formula (II).

### Scheme II

Provided in the present disclosure is a method for preparing the compound represented by general formula (III) or the pharmaceutically acceptable salt thereof, wherein the method comprises: 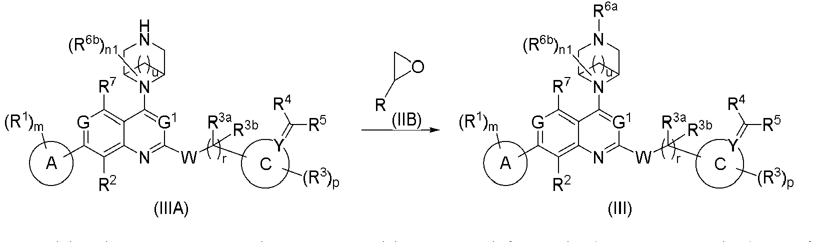
subjecting a compound represented by general formula (IIIA) or a salt thereof, and a compound represented by general formula (IIB) or a salt thereof to a ring-opening reaction to obtain the compound represented by general formula (III) or the pharmaceutically acceptable salt thereof,
wherein, R^{6a} is 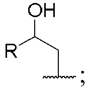 R is hydrogen atom or alkyl;
ring A, ring C, R¹, m, G, G¹, R², R⁷, R^{6b}, n1, u, W, R^{3a}, R^{3b}, r, Y, R³, R⁴, R⁵ and p are as defined in general formula (III).

### Scheme II-2

Provided in the present disclosure is a method for preparing the compound represented by general formula (III) or the pharmaceutically acceptable salt thereof, wherein the method comprises: 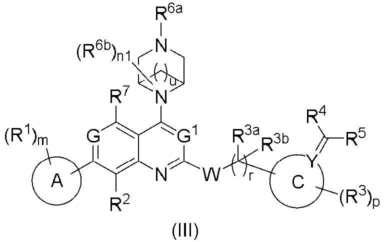
subjecting a compound represented by general formula (III) in which R^{6a} is OR¹⁴, or a pharmaceutically acceptable salt thereof to a deprotection reaction under alkaline conditions, to obtain a compound represented by general formula (III) in which R^{6a} is OH, or a pharmaceutically acceptable salt thereof,
wherein R¹⁴ is hydroxyl protecting group, and in some embodiments, benzoyl;
wherein, ring A, ring C, R¹, m, G, G¹, R², R⁷, R^{6a}, R^{6b}, n1, u, W, R^{3a}, R^{3b}, r, Y, R³, R⁴, R⁵ and p are as defined in general formula (III).

### Scheme III

Provided in the present disclosure is a method for preparing the compound represented by general formula (IV) or the pharmaceutically acceptable salt thereof, wherein the method comprises: 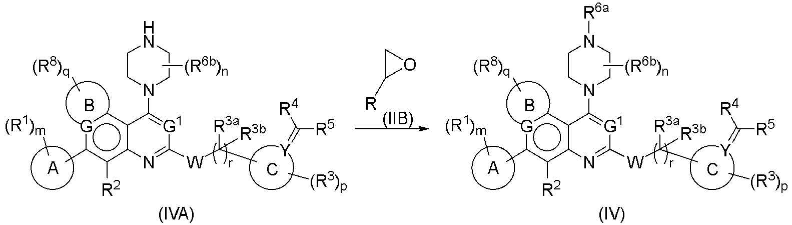
subjecting a compound represented by general formula (IVA) or a salt thereof, and a compound represented by general formula (IIB) or a salt thereof to a ring-opening reaction to obtain the compound represented by general formula (IV) or the pharmaceutically acceptable salt thereof,
wherein, R^{6a} is 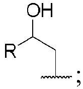 R is hydrogen atom or alkyl;
ring A, ring B, ring C, R¹, m, G, G¹, R², R⁸, q, R^{6b}, n, W, R^{3a}, R^{3b}, r, Y, R³, R⁴, R⁵ and p are as defined in general formula (IV).

### Scheme III-2

Provided in the present disclosure is a method for preparing the compound represented by general formula (IV) or the pharmaceutically acceptable salt thereof, wherein the method comprises: 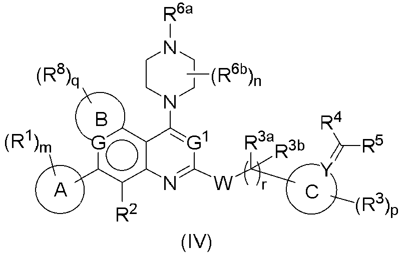
subjecting a compound represented by general formula (IV) in which R^{6a} is OR¹⁴, or a pharmaceutically acceptable salt thereof to a deprotection reaction under alkaline conditions, to obtain a compound represented by general formula (IV) in which R^{6a} is OH, or a pharmaceutically acceptable salt thereof,
wherein R¹⁴ is hydroxyl protecting group, and in some embodiments, benzoyl.
wherein, ring A, ring B, ring C, R¹, m, G, G¹, R², R⁸, q, R^{6a}, R^{6b}, n, W, R^{3a}, R^{3b}, r, Y, R³, R⁴, R⁵ and p are as defined in general formula (IV).

### Scheme IV

Provided in the present disclosure is a method for preparing the compound represented by general formula (V) or the pharmaceutically acceptable salt thereof, wherein the method comprises: 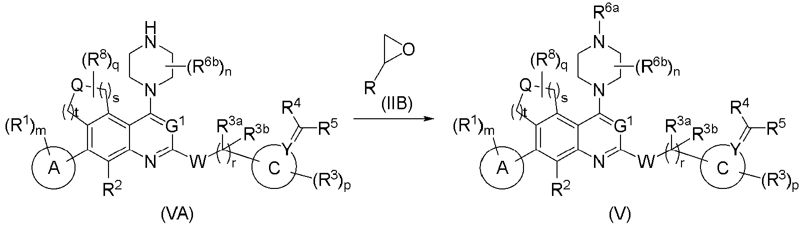
subjecting a compound represented by general formula (VA) or a salt thereof, and a compound represented by general formula (IIB) or a salt thereof to a ring-opening reaction to obtain the compound represented by general formula (V) or the pharmaceutically acceptable salt thereof,
wherein, R^{6a} is 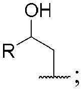 R is hydrogen atom or alkyl;
ring A, ring C, R¹, m, Q, s, t, R⁸, q, R², G¹, R^{6b}, n, W, R^{3a}, R^{3b}, r, Y, R³, R⁴, R⁵ and p are as defined in general formula (V).

### Scheme IV-2

Provided in the present disclosure is a method for preparing the compound represented by general formula (V) or the pharmaceutically acceptable salt thereof, wherein the method comprises: 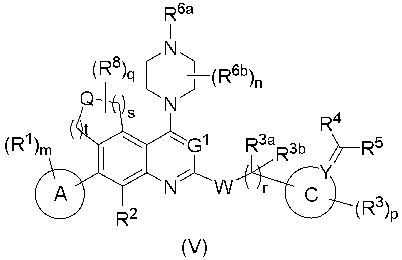
subjecting a compound represented by general formula (V) in which R^{6a} is OR¹⁴, or a pharmaceutically acceptable salt thereof to a deprotection reaction under alkaline conditions, to obtain a compound represented by general formula (V) in which R^{6a} is OH, or a pharmaceutically acceptable salt thereof,
wherein R¹⁴ is hydroxyl protecting group, and in some embodiments, benzoyl,
wherein, ring A, ring C, R¹, m, R², Q, R⁸, q, s, t, G¹, R^{6a}, R^{6b}, n, W, R^{3a}, R^{3b}, r, Y, R³, R⁴, R⁵ and p are as defined in general formula (V).

### Scheme V

Provided in the present disclosure is a method for preparing the compound represented by general formula (IM) or the pharmaceutically acceptable salt thereof, wherein the method comprises: 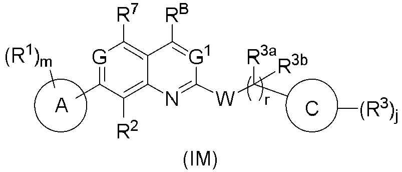
subjecting a compound represented by general formula (IM) in which at least one R¹ is -amino-amino protecting group or -O-hydroxyl protecting group under acidic conditions, or a pharmaceutically acceptable salt thereof to a deprotection reaction, to obtain a compound represented by general formula (IM) in which at least one R¹ is amino or hydroxyl, or a pharmaceutically acceptable salt thereof,
wherein, in some embodiments, the amino protecting group is Boc;
ring A, ring C, the remaining R¹, m, G, G¹, R², R⁷, R^{B}, W, R^{3a}, R^{3b}, r, R³ and j are as defined in general formula (IM).

### Scheme VI

Provided in the present disclosure is a method for preparing the compound represented by general formula (IN) or the pharmaceutically acceptable salt thereof, wherein the method comprises: 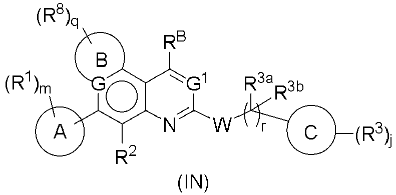
subjecting a compound represented by general formula (IN) in which at least one R¹ is -amino-amino protecting group or -O-hydroxyl protecting group under acidic conditions, or a pharmaceutically acceptable salt thereof to a deprotection reaction, to obtain a compound represented by general formula (IN) in which at least one R¹ is amino or hydroxyl, or a pharmaceutically acceptable salt thereof,
wherein, in some embodiments, the amino protecting group is Boc;
ring A, ring B, ring C, the remaining R¹, m, G, R⁸, q, R², G¹, R^{B}, W, R^{3a}, R^{3b}, r, R³ and j are as defined in general formula (IN).

### Scheme VII

Provided in the present disclosure is a method for preparing the compound represented by general formula (IIA) or the pharmaceutically acceptable salt thereof, wherein the method comprises: 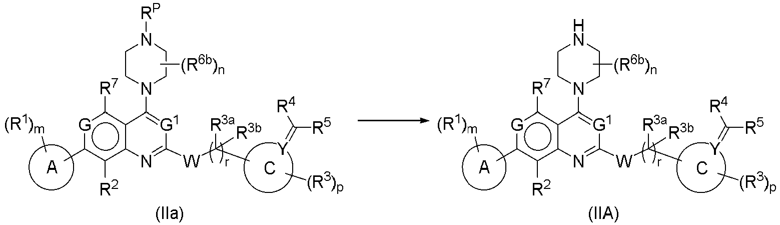
subjecting a compound represented by general formula (IIa) or a salt thereof to a deprotection reaction under acidic conditions, to obtain a compound represented by general formula (IIA) or a pharmaceutically acceptable salt thereof,
wherein, R^{P} is an amino protecting group, and in some embodiments, R^{P} is Boc;
ring A, ring C, R¹, m, G, G¹, R², R⁷, R^{6b}, n, W, R^{3a}, R^{3b}, r, Y, R³, R⁴, R⁵ and p are as defined in general formula (IIA).

### Scheme VIII

Provided in the present disclosure is a method for preparing the compound represented by general formula (IIIA) or the pharmaceutically acceptable salt thereof, wherein the method comprises: 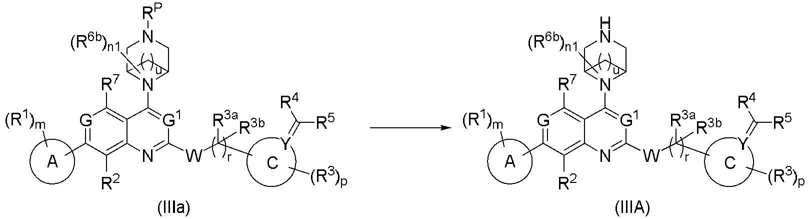
subjecting a compound represented by general formula (IIIa) or a salt thereof to a deprotection reaction under acidic conditions, to obtain a compound represented by general formula (IIIA) or a pharmaceutically acceptable salt thereof,
wherein, R^{P} is an amino protecting group, and in some embodiments, R^{P} is Boc;
ring A, ring C, R¹, m, G, G¹, R², R⁷, R^{6b}, n1, u, W, R^{3a}, R^{3b}, r, Y, R³, R⁴, R⁵ and p are as defined in general formula (IIIA).

### Scheme IX

Provided in the present disclosure is a method for preparing the compound represented by general formula (IVA) or the pharmaceutically acceptable salt thereof, wherein the method comprises: 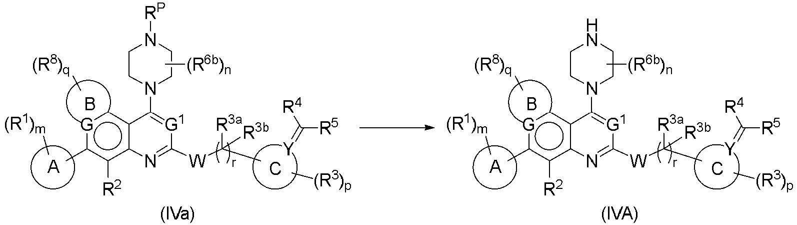
subjecting a compound represented by general formula (IVa) or a salt thereof to a deprotection reaction under acidic conditions, to obtain a compound represented by general formula (IVA) or a pharmaceutically acceptable salt thereof,
wherein, R^{P} is an amino protecting group, and in some embodiments, R^{P} is Boc;
ring A, ring B, ring C, R¹, m, G, G¹, R², R⁸, q, R^{6b}, n, W, R^{3a}, R^{3b}, r, Y, R³, R⁴, R⁵ and p are as defined in general formula (IVA).

### Scheme X

Provided in the present disclosure is a method for preparing the compound represented by general formula (VA) or the pharmaceutically acceptable salt thereof, wherein the method comprises: 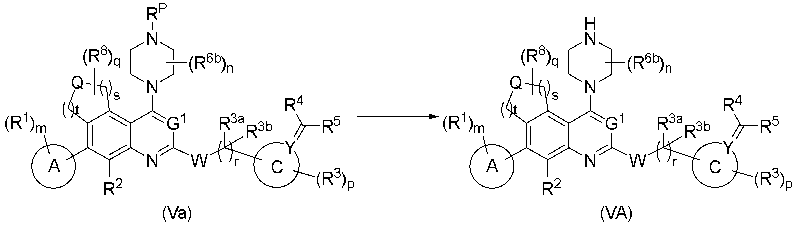
subjecting a compound represented by general formula (Va) or a salt thereof to a deprotection reaction under acidic conditions, to obtain a compound represented by general formula (VA) or a pharmaceutically acceptable salt thereof,
wherein, R^{P} is an amino protecting group, and in some embodiments, R^{P} is Boc;
ring A, ring C, R¹, m, Q, s, t, R⁸, q, R², G¹, R^{6b}, n, W, R^{3a}, R^{3b}, r, Y, R³, R⁴, R⁵ and p are as defined in general formula (VA).

### Scheme XI

Provided in the present disclosure is a method for preparing the compound represented by general formula (VI) or the pharmaceutically acceptable salt thereof, wherein the method comprises: 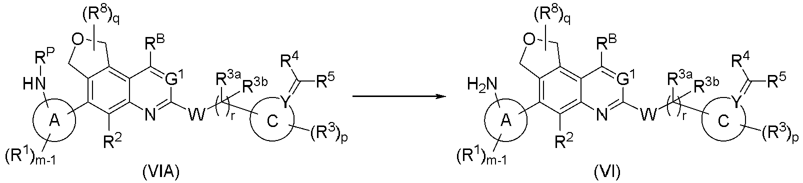
subjecting a compound represented by general formula (VIA) or a salt thereof to a deprotection reaction under acidic conditions, to obtain a compound represented by general formula (VI) or a pharmaceutically acceptable salt thereof,
wherein, R^{P} is an amino protecting group, and in some embodiments, R^{P} is Boc;
ring A, ring C, R¹, m, R⁸, q, R², G¹, R^{B}, W, R^{3a}, R^{3b}, r, Y, R³, R⁴, R⁵ and p are as defined in general formula (VI).

### Scheme XII

Provided in the present disclosure is a method for preparing the compound represented by general formula (VII-1) or (VII-2) or the pharmaceutically acceptable salt thereof, wherein the method comprises: 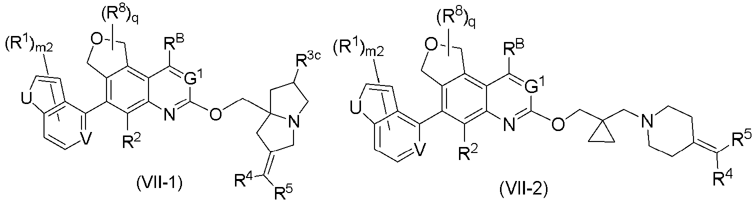
subjecting a compound represented by general formula (VII-1) in which at least one R¹ is -NH-amino protecting group, or a pharmaceutically acceptable salt thereof to a deprotection reaction under acidic conditions, to obtain a compound represented by general formula (VII-1) in which at least one R¹ is amino, or a pharmaceutically acceptable salt thereof,
subjecting a compound represented by general formula (VII-2) in which at least one R¹ is -NH-amino protecting group, or a pharmaceutically acceptable salt thereof to a deprotection reaction under acidic conditions, to obtain a compound represented by general formula (VII-2) in which at least one R¹ is amino, or a pharmaceutically acceptable salt thereof,
wherein, m2 is 1, 2, 3 or 4; in some embodiments, the amino protecting group is Boc;
U, V, the remaining R¹, R⁸, q, R², G¹, R^{B}, R^{3c}, R⁴ and R⁵ are as defined in general formula (VII-1) or (VII-2).

Optionally, in the above Schemes I to IV and VII to X, when the R¹ group contains a protecting group, the preparation method further includes a step of removing the protecting group on the R¹ group, such as the step of removing the amino protecting group and/or the hydroxy protecting group on R¹.

The reagents providing acidic conditions in the above synthetic schemes include organic acids and inorganic acids. The organic acids include, but are not limited to, trifluoroacetic acid, formic acid, acetic acid, methanesulfonic acid, trifluoromethanesulfonic acid, p-toluenesulfonic acid, Me₃SiCl, and TMSOTf. The inorganic acids include, but are not limited to, hydrogen chloride, a solution of hydrogen chloride in 1,4-dioxane, hydrochloric acid, sulfuric acid, nitric acid, and phosphoric acid. In some embodiments, the reagent is a solution of hydrogen chloride in 1,4-dioxane, trifluoroacetic acid, or trifluoromethanesulfonic acid; in some embodiments, the reagent is a solution of hydrogen chloride in 1,4-dioxane; in some embodiments, the reagent is trifluoroacetic acid.

In the above synthetic schemes, the bases providing basic conditions include organic bases and inorganic bases. The organic bases include, but are not limited to, tetrabutylammonium fluoride, triethylamine, N,N-diisopropylethylamine, n-butyllithium, lithium diisopropylamide, lithium bis(trimethylsilyl)amide, potassium acetate, sodium acetate, sodium ethoxide, sodium tert-butoxide, and potassium tert-butoxide. The inorganic bases include, but are not limited to, sodium hydride, potassium phosphate, sodium carbonate, potassium carbonate, anhydrous potassium carbonate, caesium carbonate, sodium hydroxide, lithium hydroxide monohydrate, lithium hydroxide, and potassium hydroxide. In some embodiments, the base providing basic conditions is anhydrous potassium carbonate.

In some embodiments, the reaction of the above steps is carried out in solvents, and the solvents used include, but are not limited to: pyridine, ethylene glycol dimethyl ether, acetic acid, trifluoroacetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, and mixtures thereof.

### Detailed Description of Embodiments

The present disclosure will be further described below in conjunction with examples, but these examples are not intended to limit the scope of the present disclosure.

### Examples

The structures of the compounds are determined by nuclear magnetic resonance (NMR) or/and mass spectrometry (MS). The NMR shift (δ) is given in the unit of 10⁻⁶ (ppm). NMR determination is performed by Bruker AVANCE NEO 500M nuclear magnetic resonance spectrometer; the solvents for determination are deuterated dimethyl sulfoxide (DMSO-*d₆*), deuterated chloroform (CDCl₃) and deuterated methanol (CD₃OD); and the internal standard is tetramethylsilane (TMS).

MS determination is carried out by Agilent 1200 /1290 DAD- 6110/6120 Quadrupole MS liquid chromatography-mass spectrometer (manufacturer: Agilent, MS model: 6110/6120 Quadrupole MS), waters ACQuity UPLC-QD/SQD (manufacturer: waters, MS model: waters ACQuity Qda Detector/waters SQ Detector), THERMO Ultimate 3000-Q Exactive (manufacturer: THERMO, MS model: THERMO Q Exactive).

High-performance liquid chromatography (HPLC) analysis is carried out by Agilent HPLC 1200DAD, Agilent HPLC 1200VWD and Waters HPLC e2695-2489 high-pressure liquid chromatographs.

Chiral HPLC analysis is determined by Agilent 1260 DAD high-performance liquid chromatograph.

Preparative high-performance liquid chromatography analysis is carried out using Waters 2767, Waters 2767-SQ Detecor2, Shimadzu LC-20AP and Gilson-281 preparative chromatographs.

Chiral preparative chromatography analysis is carried out using Shimadzu LC-20AP preparative chromatograph.

The CombiFlash flash preparative instrument used is Combiflash Rf200 (TELEDYNE ISCO).

Yantai Huanghai HSGF254 or Qingdao GF254 silica gel plate is used as a thin layer chromatography silica plate, and the silica gel plate for the thin layer chromatography (TLC) is of the specification of 0.15 mm - 0.2 mm, and the specification when separating and purifying a product by thin layer chromatography is 0.4 mm - 0.5 mm.

For silica gel column chromatography, Yantai Huanghai silica gel 200-300 mesh silica gel is generally used as a carrier.

The determination of the average kinase inhibition rate and IC₅₀ value is performed using NovoStar microplate reader (BMG Company, Germany).

Known starting materials of the present disclosure may be synthesised using or according to methods known in the art, or may be purchased from ABCR GmbH & Co. KG, Acros Organics, Aldrich Chemical Company, Accela ChemBio Inc., Chembee Chemicals, and other companies.

If there is no special instruction in the examples, reactions can all be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Pressurized hydrogenation reactions are performed using Parr 3916EKX hydrogenator, Qinglan QL-500 hydrogen generator or HC2-SS hydrogenator.

The hydrogenation reaction usually comprises the steps of vacuumizing and charging with hydrogen, and the operation is repeated 3 times.

CEM Discover-S 908860 microwave reactor is used for the microwave reaction.

Unless otherwise specified in the examples, the solution refers to an aqueous solution.

Unless otherwise specified in the examples, the reaction temperature refers to room temperature.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, B: petroleum ether/ethyl acetate, wherein the volume ratio of the solvent is adjusted according to the polarity of compounds and can also be adjusted by adding a small quantity of basic reagents such as triethylamine or acidic reagents such as acetic acid.

### Example 1

### 2-amino-7-fluoro-4-(5-fluoro-3-((1-((4-(fluoromethylene)piperidin-1-yl)methyl)cyclopropyl)methoxy)-1-(3-((R)-2-hydroxypropyl)-3,8-diazabicyclo[3.2.1]oct-8-yl)-7,9-dihydrofuro[3,4-f]quinazolin-6-yl)thieno[3,2-c]pyridine-3-carbonitrile 1

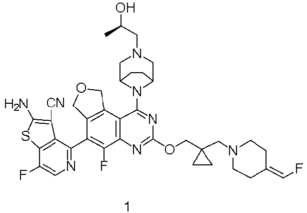

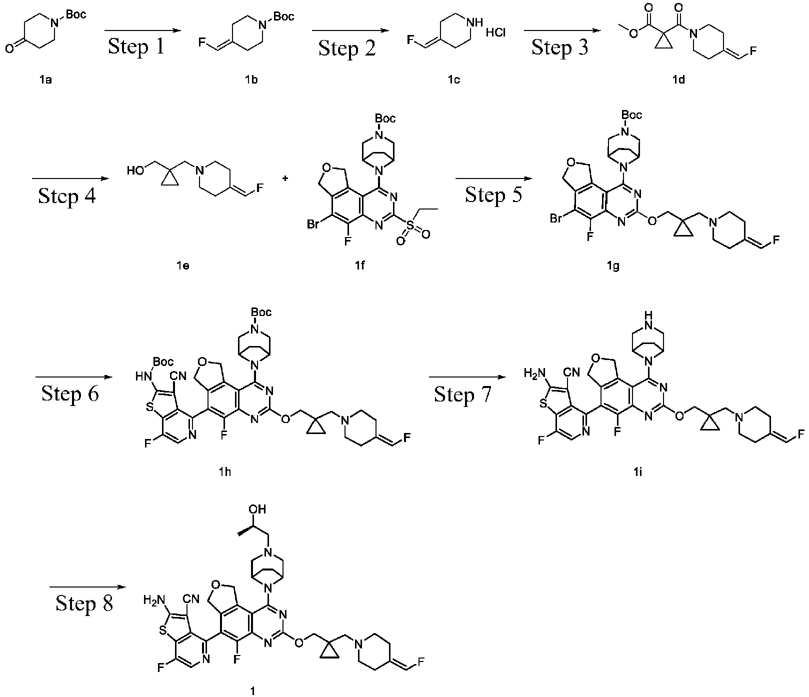

### Step 1

### tert-butyl 4-(fluoromethylene)piperidine-1-carboxylate 1b

(Fluoromethyl)triphenylphosphonium tetrafluoroborate (30.2 g, 79 mmol, from Bide Pharmatech Ltd.) was dissolved in tetrahydrofuran (200 mL). A 1 M solution of potassium bis(trimethylsilyl)amide in tetrahydrofuran (75.3 mL) was added at - 70°C, and the mixture was reacted with stirring at this temperature for 1 hour. Then, N-tert-butoxycarbonyl-4-piperidone **1a** (15 g, 75.3 mmol, from Bide Pharmatech Ltd.) was added, and the reaction mixture was reacted with stirring at -70°C for 1 hour, followed by stirring at room temperature for 16 hours. The reaction was quenched by adding saturated ammonium chloride solution, and the mixture was extracted with ethyl acetate (100 mL × 3). The organic phases were combined and dried over anhydrous sodium sulfate, filtered to remove the drying agent, and concentrated under reduced pressure. The residue was purified by silica gel column chromatography using eluent system B to afford the title compound **1b** (13.7 g, yield: 84.3%).

### Step 2

### 4-(fluoromethylene)piperidine hydrochloride 1c

In an ice bath, compound **1b** (13.7 g, 63.5 mmol) was dissolved in a 4.0 M solution of hydrogen chloride in 1,4-dioxane (80 mL). The mixture was allowed to warm to room temperature and reacted with stirring for 2 hours. The reaction mixture was concentrated under reduced pressure to afford the crude title compound **1c** (9.6 g). The product was used directly in the next step without purification.

MS m/z (ESI):116.1 [M+1].

### Step 3

### methyl 1-(4-(fluoromethylene)piperidine-1-carbonyl)cyclopropane-1-carboxylate 1d

1,1-cyclopropanedicarboxylic acid monomethyl ester (22 g, 152.6 mmol, from Leyan) was dissolved in dichloromethane (400 mL). The atmosphere was purged with nitrogen three times. Oxalyl chloride (77.5 g, 610.4 mmol) and *N,N-*dimethylformamide (DMF) (391 mg, 5.3 mmol) were added dropwise at 0°C. The mixture was allowed to warm to room temperature naturally and reacted with stirring for 3 hours. The reaction mixture was concentrated under reduced pressure, and the residue was dissolved in dichloromethane (80 mL) for later use.

Crude compound **1c** (23.1 g, 152.6 mmol) was dissolved in dichloromethane (400 mL). Triethylamine (61.8 g, 610.4 mmol) was added at 0°C, followed by the dropwise addition of the previously prepared reserve solution. The mixture was reacted with stirring for 1 hour. Saturated sodium bicarbonate solution was added to the reaction mixture, and the layers were separated. The organic phase was washed with saturated sodium chloride solution and concentrated under reduced pressure. The residue was purified by silica gel column chromatography using eluent system B to afford the title compound **1d** (27.3 g, yield: 74.2%).

MS m/z (ESI):242.1 [M+1].

### Step 4

### (1-((4-(fluoromethylene)piperidin-1-yl)methyl)cyclopropyl)methanol 1e

Compound **1d** (14.3 g, 59.34 mmol) was dissolved in tetrahydrofuran (300 mL). A 1.0 M solution of lithium aluminium hydride in tetrahydrofuran (118.8 mL) was added dropwise at 0°C. The mixture was allowed to warm to room temperature naturally and reacted with stirring for 2 hours. Water and saturated potassium sodium tartrate solution were added sequentially to the reaction mixture, followed by extraction with ethyl acetate (300 mL × 3). The organic phases were combined and dried over anhydrous sodium sulfate, filtered to remove the drying agent, and concentrated under reduced pressure. The residue was purified by silica gel column chromatography using eluent system B to afford the title compound **1e** (10.8 g, yield: 91.3%).

MS m/z (ESI):200.1 [M+1].

### Step 5

### tert-butyl 8-(6-bromo-5-fluoro-3-((1-((4-(fluoromethylene)piperidin-1-yl)methyl)cyclopropyl)methoxy)-7,9-dihydrofuro[3,4-f]quinazolin-1-yl)-3,8-diazabicyclo[3.2.1]octane-3-carboxylate 1g

Compound **1e** (500 mg, 2.5 mmol) was dissolved in tetrahydrofuran (30 mL). A 1 M solution of lithium bis(trimethylsilyl)amide in tetrahydrofuran (2.5 mL) was added in an ice bath, and the mixture was stirred at this temperature for 30 minutes. Then, a solution of tert-butyl 8-(6-bromo-3-(ethylsulfonyl)-5-fluoro-7,9-dihydrofuro[3,4-f]quinazolin-1-yl)-3,8-diazabicyclo[3.2.1]octane-3-carboxylate **1f** (1.2 g, 2 mmol, prepared according to the method disclosed in Preparation 114 on page 224 of the specification of patent application "WO 2023183585 A1") in tetrahydrofuran (20 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered to remove the drying agent, and concentrated under reduced pressure. The residue was purified by silica gel column chromatography using eluent system B to afford the title compound **1g** (820 mg, yield: 61%).

MS m/z (ESI):676.2 [M+1].

### Step 6

### tert-butyl 8-(6-(2-((tert-butoxycarbonyl)amino)-3-cyano-7-fluorothieno[3,2-c]pyridin-4-yl)-5-fluoro-3-((1-(4-(fluoromethylene)piperidin-1-yl)methyl)cyclopropyl)methoxy)-7,9-dihydrofuro[3,4-f]quinazolin-1-yl)-3,8-diazabicyclo[3.2.1]octane-3-carboxylate 1h

Compound **1g** (500 mg, 740 µmol) and neopentyl glycol diboronate (250 mg, 1.11 mmol) were dissolved in 1,4-dioxane (10 mL). Potassium acetate (218 mg, 2.2 mmol) and [1,1'-bis(diphenylphosphino)ferrocene]dichloropalladium(II) (54 mg, 74 µmol) were added. The mixture was reacted at 95°C for 2 hours under nitrogen atmosphere. The reaction mixture was cooled to room temperature, and water (3 mL), tert-butyl (4-chloro-3-cyano-7-fluorothieno[3,2-c]pyridin-2-yl)carbamate (242 mg, 740 µmol, prepared according to the method disclosed in Preparation 231 on page 202 of the specification of patent application "WO 2023183585 A1"), methanesulfonato(2-dicyclohexylphosphino-2',4',6'-triisopropyl-1,1'-biphenyl)(2'-methylamino-1,1'-biphen-2-yl)palladium(II) (64 mg, 74 µmol), and potassium phosphate (233 mg, 1.1 mmol) were added. The mixture was reacted at 85°C for 1 hour under nitrogen atmosphere. The reaction mixture was concentrated under reduced pressure. The residue was dissolved in dichloromethane, and water was added. The layers were separated, and the aqueous phase was extracted with dichloromethane (30 mL × 2). The organic phases were combined and dried over anhydrous sodium sulfate, filtered to remove the drying agent, and concentrated under reduced pressure. The residue was purified by silica gel column chromatography using eluent system B to afford the title compound **1h** (360 mg, yield: 55%).

MS m/z (ESI):889.2 [M+1].

### Step 7

### 4-(1-(3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorothieno[3,2-c]pyridine-3-carbonitrile 1i

Compound **1h** (360 mg, 405 µmol) was dissolved in dichloromethane (3 mL). Trifluoroacetic acid (2 mL) was added, and the mixture was reacted with stirring for 0.5 hours. The reaction mixture was concentrated under reduced pressure. The residue was dissolved in dichloromethane, and the pH was adjusted to > 7 with saturated sodium bicarbonate solution. The layers were separated, and the aqueous phase was extracted with dichloromethane (10 mL × 2). The organic phases were combined and dried over anhydrous sodium sulfate, filtered to remove the drying agent, and concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-42%, flow rate: 30 mL/min) to afford the title compound **1i** (71.4 mg, yield: 24.8%).

MS m/z (ESI):689.2 [M+1].

¹H NMR (500 MHz, DMSO-*d₆*): δ 8.42 (s, 2H), 7.20 (s, 1H), 6.72-6.54 (m, 2H), 5.47-5.41 (m, 2H), 5.03-5.00 (m, 1H), 4.69-4.67 (m, 1H), 4.35-4.07 (m, 4H), 3.09-2.99 (m, 2H), 2.70-2.64 (m, 2H), 2.41-2.38 (m, 4H), 2.36-2.27 (m, 2H), 2.20-2.18 (m, 2H), 2.02-1.98 (m, 2H), 1.88-1.71 (m, 4H), 0.66-0.64 (m, 2H), 0.40-0.39 (m, 2H).

### Step 8

### 2-amino-7-fluoro-4-(5-fluoro-3-((1-((4-(fluoromethylene)piperidin-1-yl)methyl)cyclopropyl)methoxy)-1-(3-((R)-2-hydroxypropyl)-3,8-diazabicyclo[3.2.1]oct-8-yl)-7,9-dihydrofuro[3,4-f]quinazolin-6-yl)thieno[3,2-c]pyridine-3-carbonitrile 1

Compound **1i** (100 mg, 145 µmol) was dissolved in methanol (5 mL). (R)-Propylene oxide (42 mg, 725 µmol, from Titan) was added, and the mixture was reacted at 70°C for 2 hours. The reaction mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-42%, flow rate: 30 mL/min), to afford the title compound **1** (21.6 mg, yield: 22%).

MS m/z (ESI):747.2 [M+1].

¹H NMR (500 MHz, CD₃OD): δ 8.31 (d, 1H), 6.51 (d, 1H), 5.56 (s, 2H), 5.26-5.09 (m, 1H), 4.78 (dt, 1H), 4.46-4.43 (m, 5H), 4.11-3.86 (m, 1H), 3.03-2.85 (m, 2H), 2.78-2.60 (m, 3H), 2.58-2.29 (m, 8H), 2.18-2.02 (m, 4H), 2.00-1.86 (m, 2H), 1.20 (d, 3H), 0.74 (s, 2H), 0.51 (s, 2H).

### Example 2

### 2-amino-4-(3-((1-((4-(difluoromethylene)piperidin-1-yl)methyl)cyclopropyl)methoxy)-5-fluoro-1-(3-((R)-2-hydroxypropyl)-3,8-diazabicyclo[3.2.1]oct-8-yl)-7,9-dihydrofuro[3,4-f]quinazolin-6-yl)-7-fluorothieno[3,2-c]pyridine-3-carbonitrile 2

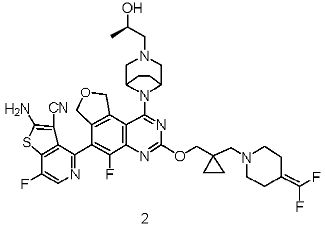

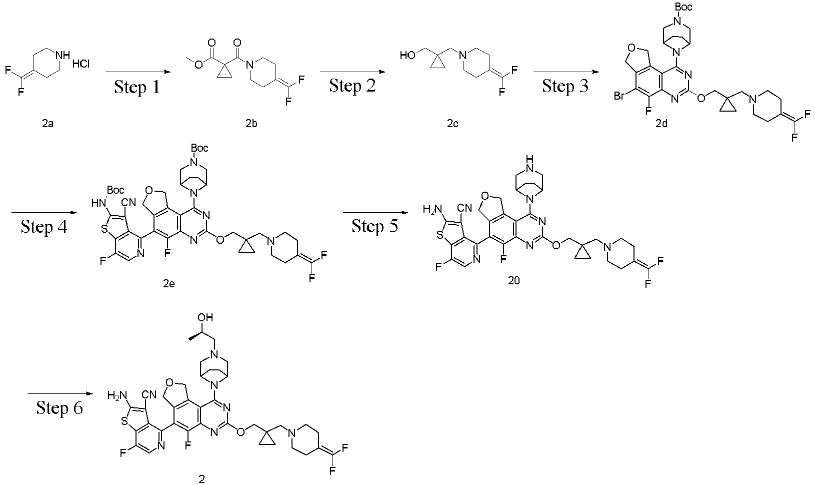

### Step 1

### methyl 1-(4-(difluoromethylene)piperidine-1-carbonyl)cyclopropane-1-carboxylate 2b

1,1-cyclopropanedicarboxylic acid monomethyl ester (3.16 g, 21.9 mmol) was dissolved in dichloromethane (50 mL). The atmosphere was purged with nitrogen three times. Oxalyl chloride (9.28 g, 73.1 mmol) and *N,N*-dimethylformamide (DMF) (1.34 g, 18.3 mmol) were added dropwise at 0°C. The mixture was allowed to warm to room temperature naturally and reacted with stirring for 2 hours. The reaction mixture was concentrated under reduced pressure the residue was dissolved in dichloromethane (80 mL) for later use.

4-(Difluoromethylene)piperidine hydrochloride **2a** (3.1 g, 18.3 mmol, prepared according to the method disclosed in the literature "Bioorganic and Medicinal Chemistry, 2004, vol. 12, # 7, p. 1713 - 1730") was dissolved in dichloromethane (50 mL). Triethylamine (7.39 g, 73 mmol) was added at 0°C, followed by the dropwise addition of the previously prepared reserve solution. The mixture was reacted with stirring for 1 hour. Saturated sodium bicarbonate solution was added to the reaction mixture, and the layers were separated. The organic phase was washed with saturated sodium chloride solution and concentrated under reduced pressure. The residue was purified by silica gel column chromatography using eluent system B to afford the title compound **2b** (4.4 g, yield: 92.8%).

MS m/z (ESI):260.3 [M+1].

### Step 2

### (1-((4-(difluoromethylene)piperidin-1-yl)methyl)cyclopropyl)methanol 2c

Compound **2b** (3 g, 11.57 mmol) was dissolved in tetrahydrofuran (80 mL). A 1.0 M solution of lithium aluminium hydride in tetrahydrofuran (9.3 mL) was added dropwise at 0°C. The mixture was allowed to warm to room temperature naturally and reacted with stirring for 2 hours. Sodium sulfate decahydrate was added to the reaction mixture. After stirring for 0.5 hours, the mixture was filtered. The filter cake was washed with ethyl acetate, and the filtrate was concentrated under reduced pressure to afford the crude title compound **2c** (2 g). The product was used directly in the next step without purification.

MS m/z (ESI):218.2 [M+1].

### Step 3

### tert-butyl 8-(6-bromo-3-((1-((4-(difluoromethylene)piperidin-1-yl)methyl)cyclopropyl)methoxy)-5-fluoro-7,9-dihydrofuro[3,4-f]quinazolin-1-yl)-3,8-diazabicyclo[3.2.1]octane-3-carboxylate 2d

Compound **2c** (1.37 g, 6.3 mmol) was dissolved in tetrahydrofuran (50 mL). A 1 M solution of sodium bis(trimethylsilyl)amide in tetrahydrofuran (3.7 mL) was added in an ice bath, and the mixture was stirred at this temperature for 10 minutes. Then, a solution of compound **1f** (2.4 g, 4.2 mmol) in tetrahydrofuran (15 mL) was added in an ice bath. The mixture was reacted with stirring at this temperature for 2 hours. The reaction was quenched by adding saturated ammonium chloride solution, and the mixture was extracted with ethyl acetate (30 mL × 2). The organic phases were combined and dried over anhydrous sodium sulfate, filtered to remove the drying agent, and concentrated under reduced pressure. The residue was purified by silica gel column chromatography using eluent system B to afford the title compound **2d** (1.4 g, yield: 48%).

MS m/z (ESI):694.3 [M+1].

### Step 4

### tert-butyl 8-(6-(2-((tert-butoxycarbonyl)amino)-3-cyano-7-fluorothieno[3,2-c]pyridin-4-yl)-3-((1-((4-(difluoromethylene)piperidin-1-yl)methyl)cyclopropyl)methoxy)-5-fluoro-7,9-dihydrofuro[3,4-f]quinazolin-1-yl)-3,8-diazabicyclo[3.2.1]octane-3-carboxylate 2e

Compound **2d** (1.4 g, 2.01 mmol) and neopentyl glycol diboronate (683 mg, 302 mmol) were dissolved in 1,4-dioxane (35 mL). Potassium acetate (692 mg, 7.05 mmol) and [1,1'-bis(diphenylphosphino)ferrocene]dichloropalladium(II) (144 mg, 201 µmol) were added. The mixture was stirred at 95°C for 2 hours under nitrogen atmosphere. The reaction mixture was cooled to room temperature, and water (9 mL), tert-butyl (4-chloro-3-cyano-7-fluorothieno[3,2-c]pyridin-2-yl)carbamate (727 mg, 2.21 mmol), methanesulfonato(2-dicyclohexylphosphino-2',4',6'-triisopropyl-1,1'-biphenyl)(2'-methylamino-1,1'-biphen-2-yl)palladium(II) (173 mg, 201 µmol), and potassium phosphate (642 mg, 3.02 mmol) were added. The mixture was stirred at 85°C for 1 hour under nitrogen atmosphere. The reaction mixture was concentrated under reduced pressure. The residue was dissolved in dichloromethane, and water was added. The layers were separated, and the aqueous phase was extracted with dichloromethane (50 mL × 2). The organic phases were combined and dried over anhydrous sodium sulfate, filtered to remove the drying agent, and concentrated under reduced pressure. The residue was purified by silica gel column chromatography using eluent system B to afford the title compound **2e** (753 mg, yield: 41.1%).

MS m/z (ESI):907.8 [M+1].

### Step 5

### 4-(1-(3,8-diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-c]pyridine-3-carbonitrile 20

Compound **2e** (753 mg, 830 µmol) was dissolved in dichloromethane (10 mL). Trifluoroacetic acid (1.5 mL) was added, and the mixture was reacted with stirring for 2 hours. The reaction mixture was concentrated under reduced pressure. The residue was dissolved in dichloromethane, and the pH was adjusted to > 7 with saturated sodium bicarbonate solution. The layers were separated, and the aqueous phase was extracted with dichloromethane (15 mL × 2). The organic phases were combined and dried over anhydrous sodium sulfate, filtered to remove the drying agent, and concentrated under reduced pressure to afford the crude title compound **20** (563 mg). The product was used directly in the next step without purification.

MS m/z (ESI):707.5 [M+1].

### Step 6

### 2-amino-4-(3-((1-((4-(difluoromethylene)piperidin-1-yl)methyl)cyclopropyl)methoxy)-5-fluoro-1-(3-((R)-2-hydroxypropyl)-3,8-diazabicyclo[3.2.1]oct-8-yl)-7,9-dihydrofuro[3,4-f]quinazolin-6-yl)-7-fluorothieno[3,2-c]pyridine-3-carbonitrile 2

Compound **20** (563 mg, 796.6 µmol) was dissolved in ethanol (25 mL). (*R*)-Propylene oxide (232 mg, 4 mmol) was added, and the mixture was reacted at 70°C for 2 hours. The reaction mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-42%, flow rate: 30 mL/min) to afford the title compound **2** (154 mg, yield: 25.2%).

MS m/z (ESI): 765.2[M+1].

¹H NMR (500 MHz, DMSO-*d₆*): *δ* 8.42 (s, 2H), 7.19 (s, 1H), 6.65 (s, 1H), 5.44-5.33 (m, 2H), 5.04-5.02 (m, 1H), 4.70-4.67 (m, 1H), 4.34-4.26 (m, 4H), 3.80-3.74 (m, 1H), 3.45-3.41 (m, 1H), 3.24-3.12 (m, 1H), 2.86-2.79 (m, 2H), 2.42-2.21 (m, 7H), 2.09-1.97 (m, 4H), 1.88-1.69 (m, 3H), 1.49-1.32 (m, 2H), 1.11-0.99 (m, 3H), 0.65-0.40 (m, 4H).

### Example 3

### 2-amino-7-fluoro-4-(5-fluoro-3-((1-((4-(fluoromethylene)piperidin-1-yl)methyl)cyclopropyl)methoxy)-1-(3-((R)-2-hydroxypropyl)-3,8-diazabicyclo[3.2.1]oct-8-yl)-7,9-dihydrofuro[3,4-f]quinazolin-6-yl)benzo[b]thiophene-3-carbonitrile 3

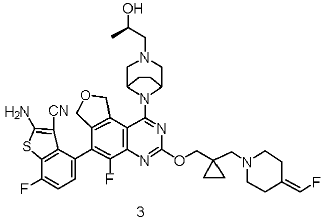

Following the synthetic route described in Example 1, the title compound 3 was obtained by replacing the compound **1i** in step 8 with compound **12**.

MS m/z (ESI): 746.2[M+1].

### Example 4

### (2R)-1-(8-(6-(5-amino-3-chloro-2-(trifluoromethyl)phenyl)-5-fluoro-3-((1-((4-(fluoromethylene)piperidin-1-yl)methyl)cyclopropyl)methoxy)-7,9-dihydrofuro[3,4-f]quinazolin-1-yl)-3,8-diazabicyclo[3.2.1]oct-3-yl)propan-2-ol 4

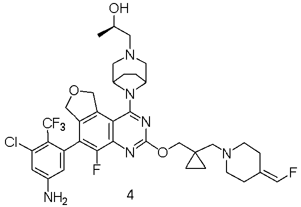

Following the synthetic route described in Example 1, the title compound 4 was obtained by replacing the compound **1i** in step 8 with compound **19**.

MS m/z (ESI): 749.2[M+1].

### Example 5

### 2-amino-7-fluoro-4-(8-fluoro-2-((1-((4-(fluoromethylene)piperidin-1-yl)methyl)cyclopropyl)methoxy)-4-(3-((R)-2-hydroxypropyl)-3,8-diazabicyclo[3.2.1]oct-8-yl)pyrido[4,3-d]pyrimidin-7-yl)benzo[b]thiophene-3-carbonitrile 5

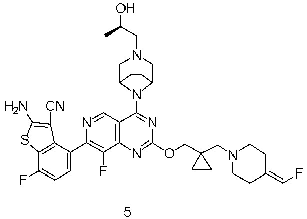

Following the synthetic route described in Example 6, the title compound **5** (33 mg, yield: 30%) was obtained by replacing the 3-chloro-5-(4,4,5,5-tetramethyl-1,3,2-dioxaborolan-2-yl)-4-(trifluoromethyl)aniline in step 3 with tert-butyl (3-cyano-4-(5,5-dimethyl-1,3,2-dioxaborinan-2-yl)-7-fluorobenzo[*b*]thiophen-2-yl)carbamate (prepared according to the method disclosed in Preparation 15 on page 50 of the specification of patent application "WO 2021118877").

MS m/z (ESI): 705.2[M+1].

¹H NMR (500 MHz, CDCl₃): δ 9.09 (s, 1H), 7.51-7.48 (m, 1H), 7.07-7.03 (m, 1H), 6.50-6.32 (m, 1H), 5.43 (s, 2H), 5.06 (s, 2H), 4.44 (s, 2H), 3.90-3.88 (m, 1H), 3.31-3.28 (m, 1H), 3.03-3.02 (m, 1H), 2.91-2.85 (m, 2H), 2.57-2.30 (m, 10H), 2.03-2.02 (m, 6H), 1.19-1.17 (m, 3H), 0.72-0.69 (m, 2H), 0.48-0.46 (m, 2H).

### Example 6

### (R)-1-(8-(7-(5-amino-3-chloro-2-(trifluoromethyl)phenyl)-8-fluoro-2-((1-((4-(fluoromethylene)piperidin-1-yl)methyl)cyclopropyl)methoxy)pyrido[4,3-d]pyrimidin-4-yl)-3,8-diazabicyclo[3.2.1]oct-3-yl)propan-2-ol 6

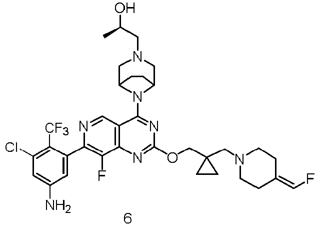

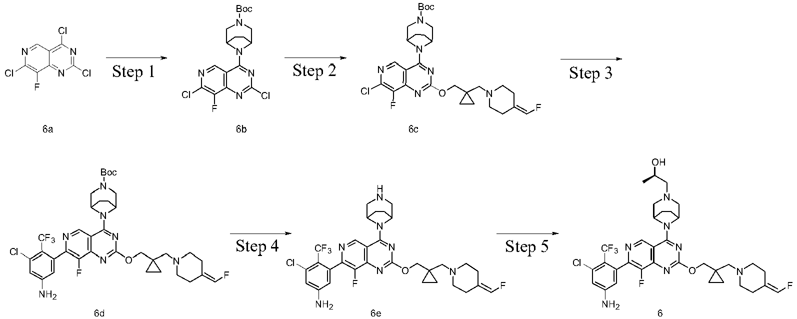

### Step 1

### tert-butyl 8-(2,7-dichloro-8-fluoropyrido[4,3-d]pyrimidin-4-yl)-3,8-diazabicyclo[3.2.1]octane-3-carboxylate 6b

Compound 2,4,7-trichloro-8-fluoropyrido[4,3-d]pyrimidine **6a** (550 mg, 2.17 mmol, prepared according to the method disclosed in Intermediate 4 on page 80 of the specification of patent application "WO2021/041671") and N,N-diisopropylethylamine (2.25 g, 17.4 mmol) were dissolved in 10 mL of dichloromethane. tert-Butyl 3,8-diazabicyclo[3.2.1]octane-3-carboxylate (226.3 mg, 2.17 mmol, from Bide Pharmatech Ltd.) was added at -40°C. The mixture was reacted with stirring at this temperature for 1 hour. Then, 10 mL of water was added to the reaction mixture, followed by extraction with dichloromethane (10 mL × 3). The organic phases were combined and dried over anhydrous sodium sulfate, filtered to remove the drying agent, and concentrated under reduced pressure. The residue was purified by silica gel column chromatography using eluent system B to afford the title compound **6b** (400 mg, yield: 41%).

MS m/z (ESI): 428.2[M+1].

### Step 2

### tert-butyl 8-(7-chloro-8-fluoro-2-((1-((4-(fluoromethylene)piperidin-1-yl)methyl)cyclopropyl)methoxy)pyrido[4,3-d]pyrimidin-4-yl)-3,8-diazabicyclo[3.2.1]octane-3-carboxylate 6c

Compound **6b** (160 mg, 361.7 µmol) was dissolved in 1,4-dioxane (2 mL). Compound **1e** (108 mg, 542.6 µmol), *N,N*-diisopropylethylamine (140.2 mg, 1.08 mmol), and 4A molecular sieves (160 mg) were added. The mixture was reacted with stirring at 90°C for 14 hours. The reaction mixture was cooled to room temperature and concentrated under reduced pressure. The residue was purified by silica gel column chromatography using eluent system A to afford the title compound **6c** (96 mg, yield: 44.9%).

MS m/z (ESI): 591.2[M+1].

### Step 3

### tert-butyl 8-(7-(5-amino-3-chloro-2-(trifluoromethyl)phenyl)-8-fluoro-2-((1-((4-(fluoromethylene)piperidin-1-yl)methyl)cyclopropyl)methoxy)pyrido[4,3-d]pyrimidin-4-yl)-3,8-diazabicyclo[3.2.1]octane-3-carboxylate 6d

Compound **6c** (96 mg, 162.2 µmol), 3-chloro-5-(4,4,5,5-tetramethyl-1,3,2-dioxaborolan-2-yl)-4-(trifluoromethyl)aniline (62 mg, 194.6 µmol, prepared according to the method disclosed in Example 80 on page 257 of the specification of patent application "WO 2022148422"), tetrakis(triphenylphosphine)palladium (38 mg, 33 µmol, from Titan), and anhydrous caesium carbonate (158 mg, 486 µmol) were dissolved in 3 mL of a mixed solvent of 1,4-dioxane and water (V : V = 5 : 1). Under nitrogen atmosphere, the mixture was reacted at 100°C for 14 hours. The reaction mixture was concentrated under reduced pressure. The residue was purified by silica gel column chromatography using eluent system A to afford the title compound **6d** (74 mg, yield: 61%).

MS m/z (ESI): 750.2[M+1].

### Step 4

### 3-(4-(3,8-diazabicyclo[3.2.1]oct-8-yl)-8-fluoro-2-((1-((4-(fluoromethylene)piperidin-1-yl)methyl)cyclopropyl)methoxy)pyrido[4,3-d]pyrimidin-7-yl)-5-chloro-4-(trifluoromethyl)aniline 6e

Compound **6d** (74 mg, 99 µmol) was dissolved in dichloromethane (1 mL). Trifluoroacetic acid (1 mL) was added, and the mixture was reacted with stirring for 0.5 hours. The reaction mixture was concentrated under reduced pressure. The residue was dissolved in dichloromethane, and the pH was adjusted to > 7 with saturated sodium bicarbonate solution. The layers were separated, and the aqueous phase was extracted with dichloromethane (10 mL × 2). The organic phases were combined and dried over anhydrous sodium sulfate, filtered to remove the drying agent, and concentrated under reduced pressure to afford the crude title compound **6e** (64 mg). The product was used directly in the next step without purification.

MS m/z (ESI): 650.2[M+1].

### Step 5

### (R)-1-(8-(7-(5-amino-3-chloro-2-(trifluoromethyl)phenyl)-8-fluoro-2-((1-((4-(fluoromethylene)piperidin-1-yl)methyl)cyclopropyl)methoxy)pyrido[4,3-d]pyrimidin-4-yl)-3,8-diazabicyclo[3.2.1]oct-3-yl)propan-2-ol 6

Compound **6e** (64 mg, 99 µmol) was dissolved in methanol (1 mL). (*R*)-Propylene oxide (23 mg, 400 µmol) was added, and the mixture was reacted at 70°C for 2 hours. The reaction mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-42%, flow rate: 30 mL/min) to afford the title compound **6** (13 mg, yield: 18.9%).

MS m/z (ESI): 708.2[M+1].

¹H NMR (500 MHz, CDCl₃): δ 8.98 (s, 1H), 6.87 (s, 1H), 6.49 (s, 1H), 5.07-4.99 (m, 2H), 4.43 (s, 2H), 4.18 (s, 2H), 3.91-3.89 (m, 1H), 3.25-3.23 (m, 1H), 3.04-3.02 (m, 1H), 2.87-2.85 (m, 2H), 2.54-2.29 (m, 10H), 2.12-2.02 (m, 6H), 1.80-1.78 (m, 2H), 1.30-1.28 (m, 1H), 1.27-1.26 (m, 2H), 0.70-0.68 (m, 2H), 0.47-0.45 (m, 2H).

### Example 7

### (R)-1-(8-(7-(5-amino-3-chloro-2-(trifluoromethyl)phenyl)-2-((1-((4-(difluoromethylene)piperidin-1-yl)methyl)cyclopropyl)methoxy)-8-fluoropyrido[4,3-d]pyrimidin-4-yl)-3,8-diazabicyclo[3.2.1]oct-3-yl)propan-2-ol 7

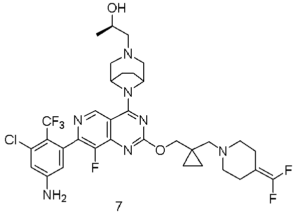

MS m/z (ESI): 726.2[M+1].

### Example 8

### 2-amino-4-(6-chloro-8-fluoro-2-((1-((4-(fluoromethylene)piperidin-1-yl)methyl)cyclopropyl)methoxy)-4-(3-((R)-2-hydroxypropyl)-3,8-diazabicyclo[3.2.1]oct-8-yl)quinazolin-7-yl)-7-fluorothieno[3,2-c]pyridine-3-carbonitrile 8

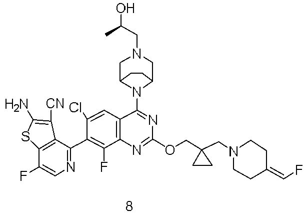

MS m/z (ESI): 739.2[M+1].

### Example 9

### 4-(1-(3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9

### 4-((R)-1-(-3,8-diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9-p1

### 4-((S)-1-(3,8-diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9-p2

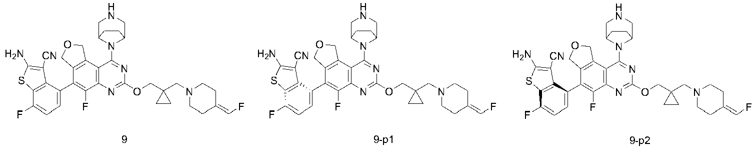

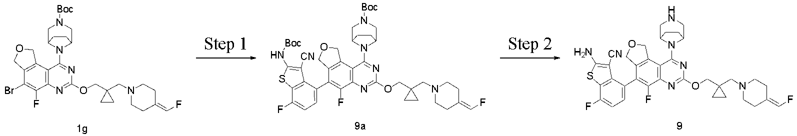

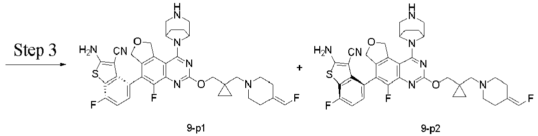

### Step 1

### tert-butyl 8-(6-(2-((tertbutoxycarbonyl)amino)-3-cyano-7-fluorobenzo[b]thiophen-4-yl)-5-fluoro-3-((1-((4-(fluoromethylene)piperidin-1-yl)methyl)cyclopropyl)methoxy)-7,9-dihydrofuro[3,4-f]quinazolin-1-yl)-3,8-diazabicyclo[3.2.1]octane-3-carboxylate 9a

Compound **1g** (100 mg, 147.8 µmol), tert-butyl (3-cyano-4-(5,5-dimethyl-1,3,2-dioxaborinan-2-yl)-7-fluorobenzo[*b*]thiophen-2-yl)carbamate (120 mg, 296.8 µmol), [1,1'-bis(diphenylphosphino)ferrocene]dichloropalladium(II) (22 mg, 30 µmol), and anhydrous caesium carbonate (97 mg, 297.7 µmol) were dissolved in 1,4-dioxane (15 mL). Under nitrogen atmosphere, the mixture was reacted at 100°C for 3 hours. After cooling to room temperature, the reaction mixture was filtered. The filtrate was concentrated under reduced pressure to afford the crude title compound **9a** (131 mg). The product was used directly in the next step without purification.

MS m/z (ESI): 888.4[M+1].

### Step 2

### 4-(1-(3,8-diazabicyclo[3.2.1]octan-8-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9

Crude compound **9a** (131 mg, 147.5 µmol) was dissolved in dichloromethane (10 mL). Trifluoroacetic acid (5 mL) was added. After reacting with stirring for 1 hour, the mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-45%, flow rate: 30 mL/min), to afford the title compound **9** (39.8 mg, yield: 39.3%).

MS m/z (ESI): 688.3[M+1].

¹H NMR (500 MHz, DMSO-*d₆*): *δ* 8.09 (s, 2H), 7.32-7.29 (m, 1H), 7.15-7.12 (m, 1H), 6.72-6.55 (m, 2H), 5.48-5.45 (m, 2H), 4.88-4.84 (m, 1H), 4.69-4.66 (m, 1H), 4.34-4.28 (m, 2H), 4.23-4.19 (m, 2H), 3.10-3.03 (m, 2H), 2.75-2.72 (m, 2H), 2.41-2.38 (m, 4H), 2.36-2.27 (m, 2H), 2.21-2.19 (m, 2H), 2.02-1.98 (m, 2H), 1.87-1.75 (m, 4H), 0.66-0.64 (m, 2H), 0.41-0.40 (m, 2H).

### Step 3

### 4-((R)-1-(3,8-diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9-p1

### 4-((S)-1-(3,8-diazabicyclo[3.2.1]oct-8-yl)-5-fluoro-3-((1-((4-(fluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 9-p2

Compound **9** (606 mg, 0.68 mmol) was separated by chiral column chromatography (chromatographic column: Daicel CHIRAL PAK IC, 20*250 mm, 10 µm; mobile phase A: n-hexane, mobile phase B: ethanol (0.5% ammonia-methanol solution), gradient ratio: A : B = 70 : 30, flow rate: 20 mL/min) to afford the title compounds (92 mg, 19.6%), (95 mg, 20.2%).

Single-configuration compound (shorter retention time, designated as 9A, i.e., **9-pl or 9-p2**): (92 mg, 19.6%).

Chiral HPLC analysis: retention time: 4.973 minutes, purity: 99% (chromatographic column: CHIRAL PAK IC 150*4.6 mm, 5 µm; mobile phase: n-hexane and ethanol (containing 0.1% diethylamine), gradient ratio: A : B = 70 : 30, flow rate: 1.0 mL/min).

MS m/z (ESI): 688.3[M+1].

¹H NMR (500 MHz, DMSO-*d₆*): δ 8.09 (s, 2H), 7.32-7.26 (m, 1H), 7.15-7.11 (m, 1H), 6.72-6.54 (m, 2H), 5.44-5.36 (m, 2H), 4.86-4.84 (m, 1H), 4.68-4.65 (m, 1H), 4.34-4.28 (m, 2H), 4.20-4.17 (m, 2H), 3.07-3.00 (m, 2H), 2.70-2.64 (m, 2H), 2.40-2.35 (m, 4H), 2.29-2.27 (m, 1H), 2.19-2.08 (m, 3H), 1.98-1.78 (m, 6H), 0.64-0.40 (m, 4H).

Single-configuration compound (longer retention time, designated as 9B, i.e., **9-p2 or 9-p1**): (95 mg, 20.2%).

Chiral HPLC analysis: retention time: 6.586 minutes, purity: 99% (chromatographic column: CHIRAL PAK IC 150*4.6 mm, 5 µm; mobile phase: n-hexane and ethanol (containing 0.1% diethylamine), gradient ratio: A : B = 70 : 30, flow rate: 1.0 mL/min).

MS m/z (ESI): 688.3[M+1].

¹H NMR (500 MHz, DMSO-d6): δ 8.19 (s, 2H), 7.32-7.29 (m, 1H), 7.15-7.11 (m, 1H), 6.72-6.55 (m, 2H), 5.51-5.38 (m, 2H), 4.86-4.84 (m, 1H), 4.68-4.65 (m, 1H), 4.34-4.17 (m, 4H), 3.13-3.00 (m, 2H), 2.70-2.63 (m, 2H), 2.64-2.35 (m, 4H), 2.29-2.19 (m, 2H), 2.04-1.98 (m, 3H), 1.86-1.72 (m, 5H), 0.65-0.40 (m, 4H).

### Example 10

### 3-(1-(3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-5-chloro-4-(trifluoromethyl)aniline 10

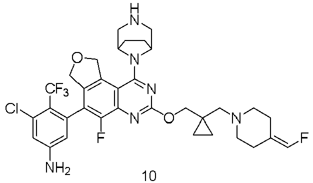

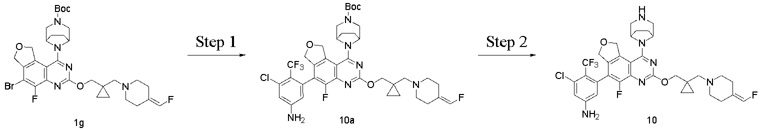

### Step 1

### tert-butyl 8-(6-(5-amino-3-chloro-2-(trifluoromethyl)phenyl)-5-fluoro-3-((1-((4-(fluoromethylene)piperidin-1-yl)methyl)cyclopropyl)methoxy)-7,9-dihydrofuro[3,4-f]quinazolin-1-yl)-3,8-diazabicyclo[3.2.1]octane-3-carboxylate 10a

Compound **1g** (100 mg, 147.8 µmol), 3-chloro-5-(4,4,5,5-tetramethyl-1,3,2-dioxaborolan-2-yl)-4-(trifluoromethyl)aniline (72 mg, 223.9 µmol), tetrakis(triphenylphosphine)palladium (35 mg, 30 µmol), and anhydrous caesium carbonate (97 mg, 297.7 µmol) were dissolved in 1,4-dioxane (15 mL) and water (2 mL). Under nitrogen atmosphere, the mixture was reacted at 100°C for 3 hours. After cooling to room temperature, the reaction mixture was filtered. The filtrate was concentrated under reduced pressure to afford the crude title compound **10a** (116 mg). The product was used directly in the next step without purification.

MS m/z (ESI): 791.3[M+1].

### Step 2

### 3-(1-(3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-5-chloro-4-(trifluoromethyl)aniline 10

Crude compound **10a** (116 mg, 146.6 µmol) was dissolved in dichloromethane (10 mL). Trifluoroacetic acid (5 mL) was added. After reacting with stirring for 1 hour, the mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-45%, flow rate: 30 mL/min), to afford the title compound 10 (23 mg, yield: 22.7%).

MS m/z (ESI): 691.3[M+1].

¹H NMR (500 MHz, DMSO-*d₆*): δ 6.88-6.87 (m, 1H), 6.72-6.54 (m, 2H), 6.39-6.38 (m, 1H), 6.32 (s, 2H), 5.47-5.44 (m, 2H), 4.86-4.84 (m, 1H), 4.72-4.70 (m, 1H), 4.33-4.27 (m, 2H), 4.21-4.17 (m, 2H), 3.06-2.99 (m, 2H), 2.69-2.67 (m, 2H), 2.39-2.36 (m, 4H), 2.31 (s, 2H), 1.99-1.96 (m, 2H), 1.85-1.77 (m, 4H), 0.65-0.63 (m, 2H), 0.41-0.39 (m, 2H).

### Example 11

### 4-(1-(3,8-diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorobenzo[b]thiophene-3-carbonitrile 11 4-((R)-1-(3,8-diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 11-p1

### 4-((S)-1-(3,8-diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 11-p2

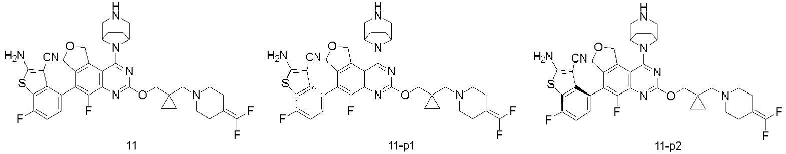

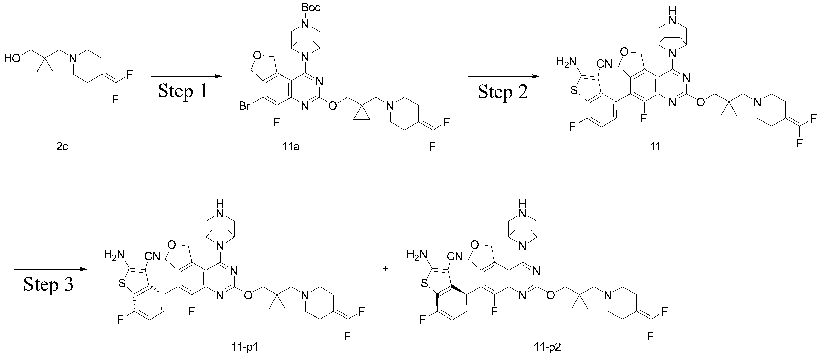

### Step 1

### tert-butyl 8-(6-bromo-3-((1-((4-(difluoromethylene)piperidin-1-yl)methyl)cyclopropyl)methoxy)-5-fluoro-7,9-dihydrofuro[3,4-f]quinazolin-1-yl)-3,8-diazabicyclo[3.2.1]octane-3-carboxylate 11a

Compound **2c** (100 mg, 0.17 mmol) was dissolved in tetrahydrofuran (15 mL). A 2 M solution of lithium bis(trimethylsilyl)amide in tetrahydrofuran (0.14 mL) was added in an ice bath, and the mixture was stirred at this temperature for 30 minutes. Then, a solution of compound **1f** (1.2 g, 2 mmol) in tetrahydrofuran (5 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **11a** (100 mg, yield: 84.7%).

MS m/z (ESI):650.3 [M+1].

### Step 2

### 4-(1-(3,8-diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorobenzo[b]thiophene-3-carbonitrile 11

Following the synthetic route described in Example 9, the title compound **11** (7 mg, yield: 8.8%) was obtained by replacing the starting material compound **1g** in step 1 with compound **11a.**

MS m/z (ESI):706.3 [M+1].

¹H NMR (500 MHz, DMSO-*d₆*): *δ* 8.09 (s, 2H), 7.67-7.13 (m, 3H), 5.61-5.44 (m, 2H), 4.85-4.67 (m, 2H), 4.30-4.20 (m, 4H), 3.17-3.06 (m, 2H), 2.71-2.50 (m, 5H), 2.28-2.09 (m, 5H), 1.85-1.78 (m, 4H), 1.46-1.23 (m, 2H), 0.84-0.39 (m, 4H).

### Step 3

### 4-((R)-1-(3,8-diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 11-p1

### 4-((S)-1-(3,8-diazabicyclo[3.2.1]oct-8-yl)-5-fluoro-3-((1-((4-(difluoromethylene))piperidin-1-yl)methyl)cyclopropyl)methoxy)-7,9-dihydrofuro[3,4-f]quinazolin-6-yl)-2-amino-7-fluorobenzo[b]thiophene-3-carbonitrile 11-p2

Compound **11** (652 mg, 0.719 mmol) was separated by chiral column chromatography (chromatographic column: Daicel CHIRAL PAK IC, 20*250 mm, 10 µm; mobile phase A: n-hexane, mobile phase B: ethanol (0.5% ammonia-methanol solution), gradient ratio: A : B = 75 : 25, flow rate: 20 mL/min) to afford the title compounds (98.5 mg, 19.4%), (97.6 mg, 19.2%).

Single-configuration compound (shorter retention time, designated as 11A, i.e., **11-p1 or 11-p2):** (98.5 mg, 19.4%).

Chiral HPLC analysis: retention time: 4.389 minutes, purity: 99% (chromatographic column: CHIRAL PAK IC 150*4.6 mm, 5 µm; mobile phase: n-hexane and ethanol (containing 0.1% diethylamine), gradient ratio: A : B = 75 : 25, flow rate: 1.0 mL/min).

MS m/z (ESI):706.3 [M+1].

¹H NMR (500 MHz, DMSO-d6): δ 8.09 (s, 2H), 7.32-7.29 (m, 1H), 7.15-7.11 (m, 1H), 6.69-6.64 (m, 1H), 5.47-5.41 (m, 2H), 4.87-4.84 (m, 1H), 4.68-4.66 (m, 1H), 4.33-4.08 (m, 4H), 3.07-3.00 (m, 2H), 2.70-2.64 (m, 2H), 2.46-2.28 (m, 6H), 2.11-1.98 (m, 4H), 1.86-1.72 (m, 4H), 0.66-0.40 (m, 4H).

Single-configuration compound (longer retention time, designated as 11B, i.e., **11-p2 or 11-p1):** (97.6 mg, 19.2%).

Chiral HPLC analysis: retention time: 5.804 minutes, purity: 99% (chromatographic column: CHIRAL PAK IC 150*4.6 mm, 5 µm; mobile phase: n-hexane and ethanol (containing 0.1% diethylamine), gradient ratio: A : B = 75 : 25, flow rate: 1.0 mL/min).

MS m/z (ESI):706.3 [M+1].

¹H NMR (500 MHz, DMSO-d6): δ 8.09 (s, 2H), 7.33-7.29 (m, 1H), 7.15-7.11 (m, 1H), 6.70-6.64 (m, 1H), 5.48-5.45 (m, 2H), 4.87-4.85 (m, 1H), 4.68-4.66 (m, 1H), 4.33-4.20 (m, 4H), 3.12-3.05 (m, 2H), 2.77-2.73 (m, 2H), 2.46-2.28 (m, 6H), 2.11-1.98 (m, 4H), 1.91-1.78 (m, 4H), 0.66-0.41 (m, 4H).

### Example 12

### 4-(1-(3,8-diazabicyclo[3.2.1]oct-3-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorobenzo[b]thiophene-3-carbonitrile 12

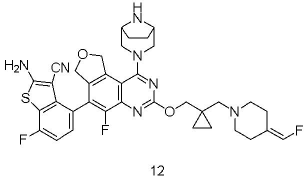

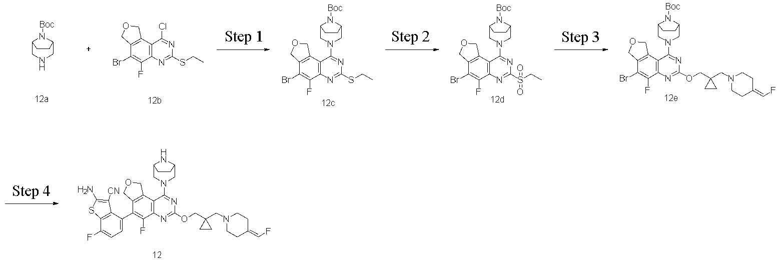

### Step 1

### tert-butyl 3-(6-bromo-3-(ethylthio)-5-fluoro-7,9-dihydrofuro[3,4-f]quinazolin-1-yl)-3,8-diazabicyclo[3.2.1]octane-8-carboxylate 12c

6-Bromo-1-chloro-3-(ethylthio)-5-fluoro-7,9-dihydrofuro[3,4-*f*]quinazoline **12b** (500 mg, 1.37 mmol, prepared according to the method disclosed in Preparation 60 on page 189 of the specification of patent application "WO 2023183585A1") was dissolved in 1,2-dichloroethane (15 mL). N,N-Diisopropylethylamine (355 mg, 2.75 mmol) and compound tert-butyl 3,8-diazabicyclo[3.2.1]octane-8-carboxylate **12a** (292 mg, 1.37 mmol, from Accela ChemBio Co., Ltd.) were added sequentially. The mixture was reacted at room temperature overnight. The mixture was filtered, and the filtrate was concentrated under reduced pressure. The residue was purified by silica gel column chromatography using eluent system A to afford the title compound **12c** (715 mg, yield: 96.5%).

MS m/z (ESI):539.1 [M+1].

### Step 2

### tert-butyl 3-(6-bromo-3-(ethylsulfonyl)-5-fluoro-7,9-dihydrofuro[3,4-f]quinazolin-1-yl)-3,8-diazabicyclo[3.2.1]octane-8-carboxylate 12d

Compound **12c** (715 mg, 1.32 mmol) was dissolved in dichloromethane (25 mL), meta-chloroperoxybenzoic acid (457 mg, 2.65 mmol) was added, and the mixture was reacted at room temperature for 2 hours. Saturated sodium bicarbonate solution was added to the reaction mixture, followed by extraction with dichloromethane (30 mL × 2). The organic phases were combined and dried over anhydrous sodium sulfate, filtered, and the filtrate was concentrated under reduced pressure. The obtained crude solid compound **12d** was used directly in the next step without purification.

MS m/z (ESI):571.1 [M+1].

### Step 3

### tert-butyl 3-(6-bromo-5-fluoro-3-((1-((4-(fluoromethylene)piperidin-1-yl)methyl)cyclopropyl)methoxy)-7,9-dihydrofuro[3,4-f]quinazolin-1-yl)-3,8-diazabicyclo[3.2.1]octane-8-carboxylate 12e

Crude compound **1e** (84 mg, 0.42 mmol) was dissolved in tetrahydrofuran (10 mL). A 2 M solution of lithium bis(trimethylsilyl)amide in tetrahydrofuran (0.30 mL) was added in an ice bath, and the mixture was stirred at this temperature for 30 minutes. Then, a solution of compound **12d** (200 mg, 0.35 mmol) in tetrahydrofuran (5 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **12e** (100 mg, yield: 42.3%).

MS m/z (ESI):676.2 [M+1].

### Step 4

### 4-(1-(3,8-diazabicyclo[3.2.1]oct-3-yl)-5-fluoro-3-((1-((4-(fluoromethylene)piperidin-1-yl)methyl)cyclopropyl)methoxy)-7,9-dihydrofuro[3,4-f]quinazolin-6-yl)-2-amino-7-fluorobenzo[b]thiophene-3-carbonitrile 12

Following steps 1 and 2 of the synthetic route described in Example 9, the title compound **12** (2 mg, yield: 5.8%) was obtained by replacing the starting material compound **1g** in step 1 with compound **12e.**

MS m/z (ESI):688.3 [M+1].

¹H NMR (500 MHz, CD₃OD): δ 7.27 (dd, 1H), 7.04 (t, 1H), 6.51 (d, 1H), 5.41 (q, 2H), 4.86-4.73 (m, 3H), 4.44 (s, 2H), 4.27 (d, 1H), 4.00 (d, 1H), 3.66-3.44 (m, 4H), 2.59- 2.42 (m, 6H), 2.36-2.30 (m, 2H), 2.11-2.06 (m, 2H), 1.78 (s, 3H), 0.73 (s, 2H), 0.50 (s, 2H).

### Example 13

### 4-(1-(3,8-diazabicyclo[3.2.1]oct-8-yl)-5-fluoro-3-((2-(fluoromethylene)tetrahydro-1H-pyrrolizin-7a(5H)-yl)methoxy)-7,9-dihydrofuro[3,4-f]quinazolin-6-yl)-2-amino-7-fluorobenzo[b]thiophene-3-carbonitrile 13

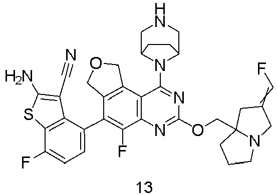

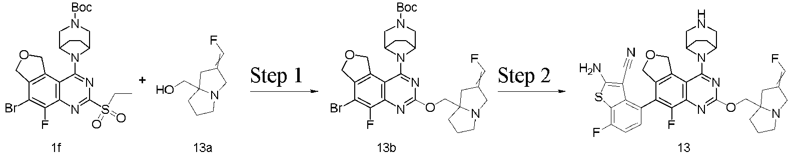

### Step 1

### tert-butyl 8-(6-bromo-5-fluoro-3-((2-(fluoromethylene)tetrahydro-1H-pyrrolizin-7a(5H)-yl)methoxy)-7,9-dihydrofuro[3,4-f]quinazolin-1-yl)-3,8-diazabicyclo[3.2.1]octane-3-carboxylate 13b

(2-(Fluoromethylene)tetrahydro-1*H*-pyrrolizin-7a(5*H*)-yl)methanol **13a** (128 mg, 0.128 mmol, prepared according to the method disclosed in Example 5 on page 32 of the specification of patent application "WO 2022247757") was dissolved in tetrahydrofuran (10 mL). A 2 M solution of lithium bis(trimethylsilyl)amide in tetrahydrofuran (0.53 mL) was added in an ice bath, and the mixture was reacted with stirring at this temperature for 30 minutes. Then, a solution of compound **1f** (357 mg, 0.624 mmol) in tetrahydrofuran (5 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **13b** (243 mg, yield: 60%).

MS m/z (ESI):604.2 [M+1].

### Step 2

### 4-(1-(3,8-diazabicyclo[3.2.1]oct-8-yl)-5-fluoro-3-((2-(fluoromethylene)tetrahydro-1H-pyrrolizin-7a(5H)-yl)methoxy)-7,9-dihydrofuro[3,4-f]quinazolin-6-yl)-2-amino-7-fluorobenzo[b]thiophene-3-carbonitrile 13

Following the synthetic route described in Example **9,** the title compound **13** (4 mg, yield: 20%) was obtained by replacing the starting material compound **1g** in step 2 with compound **13b.**

MS m/z (ESI): 660.2 [M+1].

¹H NMR (500 MHz, CD₃OD): δ 7.28 (dd, 1H), 7.05 (t, 1H), 6.68 (d, 1H), 4.98 - 4.78 (m, 3H), 4.55 - 4.29 (m, 4H), 3.95 (d, 1H), 3.56 (d, 1H), 2.94 - 2.75 (m, 4H), 2.50 (d, 1H), 2.24 - 2.14 (m, 2H), 2.13 - 1.86 (m, 8H), 1.67 - 1.57 (s, 1H), 0.92 (t, 1H).

### Example 14

### 2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 14

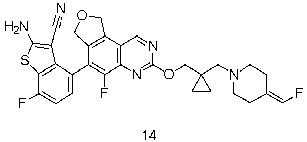

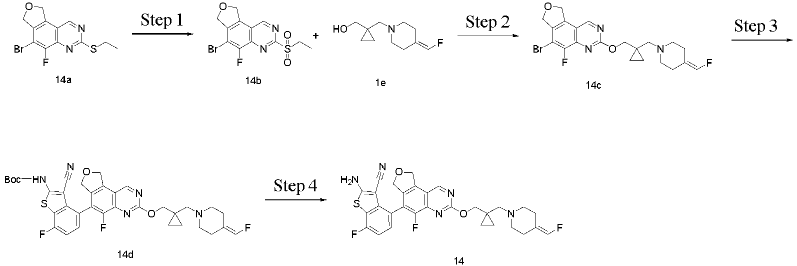

### Step 1

### 6-bromo-3-(ethylsulfonyl)-5-fluoro-7,9-dihydrofuro[3,4-f]quinazoline 14b

6-Bromo-3-(ethylthio)-5-fluoro-7,9-dihydrofuro[3,4-f]quinazoline **14a** (100 mg, 0.30 mmol, prepared according to the method disclosed in Preparation 301 on page 193 of the specification of patent application "WO 2023183585 A1") was dissolved in dichloromethane (10 mL). meta-Chloroperoxybenzoic acid (124 mg, 0.61 mmol) was added, and the mixture was reacted at room temperature for 2 hours. Saturated sodium bicarbonate solution was added to the reaction mixture, followed by extraction with dichloromethane (30 mL × 2). The organic phases were combined and dried over anhydrous sodium sulfate, filtered, and the filtrate was concentrated under reduced pressure. The obtained compound **14b** was used directly in the next step.

MS m/z (ESI):361.0 [M+1].

### Step 2

### 6-bromo-5-fluoro-3-((1-((4-(fluoromethylene)piperidin-1-yl)methyl)cyclopropyl)methoxy)-7,9-dihydrofuro[3,4-f]quinazoline 14c

Compound **1e** (77 mg, 0.38 mmol) was dissolved in tetrahydrofuran (5 mL). A 2 M solution of lithium bis(trimethylsilyl)amide in tetrahydrofuran (0.19 mL) was added in an ice bath, and the mixture was reacted with stirring at this temperature for 30 minutes. Then, a solution of compound **14b** (70 mg, 0.19 mmol) in tetrahydrofuran (5 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **14c** (30 mg, yield: 33.3%).

MS m/z (ESI):466.1 [M+1].

### Step 3

### tert-butyl (3-cyano-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophen-2-yl)carbamate 14d

Compound **14c** (30 mg, 164 µmol), tert-butyl (3-cyano-4-(5,5-dimethyl-1,3,2-dioxaborinan-2-yl)-7-fluorobenzo[b]thiophen-2-yl)carbamate (52 mg, 128.6 µmol), [1,1'-bis(diphenylphosphino)ferrocene]dichloropalladium(II) (10 mg, 13 µmol), and anhydrous caesium carbonate (52 mg, 159 µmol) were dissolved in 1,4-dioxane (5 mL). Under nitrogen atmosphere, the mixture was reacted at 100°C for 3 hours. After cooling to room temperature, the reaction mixture was filtered. The filtrate was concentrated under reduced pressure to afford the crude title compound **14d** (43 mg). The product was used directly in the next step without purification.

MS m/z (ESI): 678.2[M+1].

### Step 4

### 2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 14

Crude compound **14d** (43 mg, 64 µmol) was dissolved in dichloromethane (5 mL). Trifluoroacetic acid (2.5 mL) was added. After reacting with stirring for 1 hour, the mixture was concentrated under reduced pressure. The residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient ratio: acetonitrile 30%-45%, flow rate: 30 mL/min) to afford the title compound **14** (5 mg, yield: 19.5%).

MS m/z (ESI): 578.2 [M+1].

¹H NMR (500 MHz, DMSO-*d₆*): δ 9.47 (s, 1H), 8.13 (s, 2H), 7.36-7.33 (m, 1H), 7.16-7.13 (m, 1H), 6.72-6.54 (m, 1H), 5.53 (s, 2H), 5.01-4.98 (m, 1H), 4.81-4.77 (m, 1H), 4.40 (s, 2H), 2.43-2.38 (m, 6H), 2.20-2.18 (m, 2H), 2.01-1.98 (m, 2H), 0.72-0.69 (m, 2H), 0.45-0.43 (m, 2H).

### Examples 14-p1, 14-p2

### (R)-2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 14-p1

### (S)-2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 14-p2

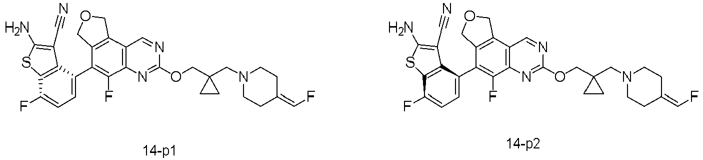

Isomer mixture **14** (450 mg) was separated by chiral column chromatography (chromatographic column: CHIRALPAK IE, 20×250 mm, 5 µm; mobile phase A: n-hexane, mobile phase B: ethanol (0.5% 7 M ammonia-methanol solution), gradient ratio: A:B=65:35, flow rate: 20 mL/min) to afford the title compounds (194 mg, yield: 43.1%) and (191 mg, yield: 42.4%).

Single-configuration compound (shorter retention time, designated as 14A, i.e., **14-p1 or 14-p2):** (194 mg, yield: 43.1%).

MS m/z (ESI): 578.2 [M+1].

Chiral HPLC analysis: retention time: 4.132 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 60 : 40, flow rate: 1 mL/min).

¹H NMR (500 MHz, DMSO-*d₆*): *δ* 9.47 (s, 1H), 8.13 (s, 2H), 7.36-7.33 (m, 1H), 7.16-7.13 (m, 1H), 6.72-6.55 (m, 1H), 5.53 (s, 2H), 5.01-4.98 (m, 1H), 4.80-4.77 (m, 1H), 4.44-4.38 (m, 2H), 2.43-2.33 (m, 6H), 2.20-2.09 (m, 2H), 2.02-1.97 (m, 2H), 0.72-0.43 (m, 4H).

Single-configuration compound (longer retention time, designated as 14B, i.e., **14-p2 or 14-p1):** (191 mg, yield: 42.4%).

MS m/z (ESI): 578.2 [M+1].

Chiral HPLC analysis: retention time: 6.778 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 60 : 40, flow rate: 1 mL/min).

¹H NMR (500 MHz, DMSO-*d₆*): *δ* 9.47 (s, 1H), 8.15 (s, 2H), 7.36-7.33 (m, 1H), 7.16-7.13 (m, 1H), 6.72-6.55 (m, 1H), 5.53 (s, 2H), 5.01-4.98 (m, 1H), 4.80-4.77 (m, 1H), 4.42-4.36 (m, 2H), 2.43-2.33 (m, 6H), 2.20-2.08 (m, 2H), 2.02-1.97 (m, 2H), 0.72-0.43 (m, 4H).

### Example 15

### 2-amino-7-fluoro-4-(5-fluoro-3-((2-(fluoromethylene)tetrahydro-1H-pyrrolizin-7a(5H)-yl)methoxy)-7,9-dihydrofuro[3,4-f]quinazolin-6-yl)benzo[b]thiophene-3-carbonitrile 15

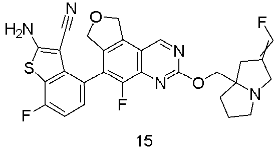

Following the synthetic route described in Example **14,** the title compound **15** (3 mg, yield: 4.4%) was obtained by replacing the starting material compound **1e** in step 2 with (2-(fluoromethylene)tetrahydro-1*H*-pyrrolizin-7*a*(5*H*)-yl)methanol (prepared according to the method disclosed in Example 5 on page 32 of the specification of patent application "WO 2022247757").

MS m/z (ESI): 550.2 [M+1].

¹H NMR (500 MHz, CD₃OD): δ 9.37 (s, 1H), 7.30 (dd, 1H), 7.06 (t, 1H), 6.67 (dd, 1H), 5.60 (s, 2H), 5.06 (dt, 1H), 4.90 (d, 1H), 4.62 - 4.15 (m, 2H), 4.01 - 3.77 (m, 1H), 3.51 (d, 1H), 3.21 (dt, 1H), 2.91 -2.64 (m, 2H), 2.50 (d, 1H), 2.20 (qd, 1H), 2.09 - 1.76 (m, 4H), 1.33 (d, 1H).

### Example 16

### 2-amino-4-(3-(((S)-4-(difluoromethylene)-1-methylpyrrolidin-2-yl)methoxy)-5-fluoro-7,9-dihydrofuro[3,4-f]quinazolin-6-yl)-7-fluorobenzo[b]thiophene-3-carbonitrile 16

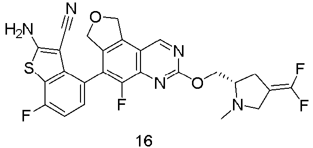

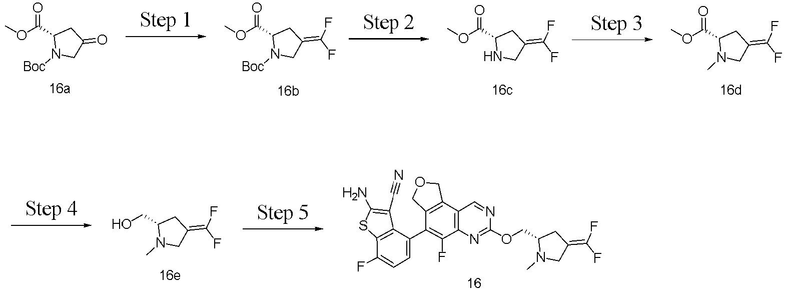

### Step 1

### 1-(tert-butyl) 2-methyl (S)-4-(difluoromethylene)pyrrolidine-1,2-dicarboxylate 16b

1-(tert-Butyl) 2-methyl (S)-4-oxopyrrolidine-1,2-dicarboxylate 16a (500 mg, 2.1 mmol, from Bide Pharmatech Ltd.) was dissolved in N,N-dimethylformamide (6.0 mL). (Triphenylphosphonio)difluoroacetate (1.8 g, 5.1 mmol) was added. The mixture was reacted at 80°C for 17 hours, then cooled to room temperature. Water was added, and the mixture was extracted with ethyl acetate three times. The organic phases were combined and dried over anhydrous sodium sulfate, filtered, and the filtrate was concentrated. The residue was purified by silica gel column chromatography using eluent system A to afford the title compound **16b** (500 mg, yield: 87.7%).

MS m/z (ESI): 278.1 [M+1].

### Step 2

### methyl (S)-4-(difluoromethylene)pyrrolidine-2-carboxylate 16c

Compound **16b** (800 mg, 2.9 mmol) was dissolved in dichloromethane (10 mL). Trifluoroacetic acid (5 mL) was added. The mixture was reacted at room temperature for 1 hour. The reaction mixture was concentrated. Saturated sodium bicarbonate solution was added, and the mixture was extracted with ethyl acetate three times. The organic phases were combined and dried over anhydrous sodium sulfate, and the filtrate was concentrated under reduced pressure to afford the crude title compound 16c (420 mg, yield: 82%).

MS m/z (ESI): 178.1 [M+1].

### Step 3

### methyl (S)-4-(difluoromethylene)-1-methylpyrrolidine-2-carboxylate 16d

Compound 16c (420 mg, 2.4 mmol) was dissolved in acetonitrile (10 mL). Iodomethane (404 mg, 2.8 mmol) and sodium bicarbonate (400 mg, 4.7 mmol) were added. The mixture was reacted at room temperature for 2 hours. The reaction mixture was concentrated. Water was added, and the mixture was extracted with ethyl acetate three times. The organic phases were combined and dried over anhydrous sodium sulfate, and the filtrate was concentrated. The residue was purified by silica gel column chromatography using eluent system A to afford the title compound **16d** (150 mg, yield: 33%).

MS m/z (ESI): 192.1 [M+1].

### Step 4

### (S)-(4-(difluoromethylene)-1-methylpyrrolidin-2-yl)methanol 16e

Compound **16d** (150 mg, 0.78 mmol) was dissolved in tetrahydrofuran (10 mL). A 1 M solution of lithium aluminium hydride in tetrahydrofuran (0.87 mL) was added in an ice-water bath. After stirring for 1 hour, the reaction was quenched by adding sodium sulfate decahydrate. The mixture was filtered, and the filtrate was concentrated to afford the title compound **16e** (90 mg, yield: 70%).

MS m/z (ESI): 164.1 [M+1].

### Step 5

### 2-amino-4-(3-(((S)-4-(difluoromethylene)-1-methylpyrrolidin-2-yl)methoxy)-5-fluoro-7,9-dihydrofuro[3,4-f]quinazolin-6-yl)-7-fluorobenzo[b]thiophene-3-carbonitrile 16

Following the synthetic route described in Example 14, the title compound 16 (30 mg, yield: 50%) was obtained by replacing the starting material compound **1e** in step 2 with compound **16e.**

MS m/z (ESI): 542.1 [M+1].

¹H NMR (500 MHz, CDCl₃): δ 9.17 (s, 1H), 7.28-7.23 (m, 1H), 7.07-7.03 (m, 1H), 5.65-5.60 (m, 2H), 5.54-5.52 (m, 2H), 5.06-4.96 (m, 2H), 4.75-4.63 (m, 2H), 3.85-3.80 (m, 1H), 3.06-3.01 (m, 2H), 2.85-2.81 (m, 1H), 2.62-2.55 (m, 4H).

### Example 17

4-(1-(3,8-diazabicyclo[3.2.1]oct-8-yl)-3-((2-(difluoromethylene)tetrahydro-1*H-*pyrrolizin-7*a*(5*H*)-yl)methoxy)-5-fluoro-7,9-dihydrofuro[3,4-*f*]quinazolin-6-yl)-2-amino-7-fluorobenzo[b]thiophene-3-carbonitrile **17**

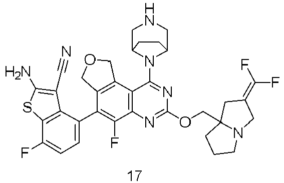

Following the synthetic route described in Example 13, the title compound **17** (5 mg, yield: 10%) was obtained by replacing the starting material compound **13a** in step 1 with (2-(difluoromethylene)tetrahydro-1*H*-pyrrolizin-7*a*(5*H*)-yl)methanol (prepared according to the method disclosed in Example 11 on page 54 of the specification of patent application "WO 2022247757").

MS m/z (ESI): 678.2 [M+1].

¹H NMR (500 MHz, CD₃OD): δ 7.28 (dd, 1H), 7.05 (t, 1H), 5.57 (s, 2H), 4.94 - 4.76 (m, 3H), 4.57 - 4.23 (m, 5H), 3.83 (d, 1H), 3.46 (d, 1H), 3.34 - 3.17 (m, 3H), 2.86 (dd, 3H), 2.73 (q, 1H), 2.53 (d, 1H), 2.34 - 1.76 (m, 6H).

### Example 18

### 4-(1-(3,8-diazabicyclo[3.2.1]oct-8-yl)-3-((2,6-dimethylenetetrahydro-1H-pyrrolizin-7a(5H)-yl)methoxy)-5-fluoro-7,9-dihydrofuro[3,4-f]quinazolin-6-yl)-2-amino-7-fluorobenzo[b]thiophene-3-carbonitrile 18

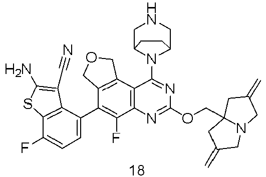

Following the synthetic route described in Example 13, the title compound **18** (3 mg, yield: 19.6%) was obtained by replacing the starting material compound **13a** in step 1 with (2,6-dimethylenetetrahydro-1*H*-pyrrolizin-7a(5*H*)-yl)methanol (prepared according to the method disclosed in Example 4 on page 29 of the specification of patent application "WO 2022247757").

MS m/z (ESI): 654.2 [M+1].

¹H NMR (500 MHz, CD₃OD): δ 7.28 (dd, 1H), 7.05 (t, 1H), 5.57 (s, 2H), 5.04 (d, 4H), 4.46 - 4.29 (m, 4H), 3.81 (d, 2H), 3.38 - 3.25 (m, 6H), 2.95 - 2.77 (m, 4H), 2.62 (d, 2H), 2.22 - 1.90 (m, 4H).

### Example 19

### 3-(1-(3,8-diazabicyclo[3.2.1]oct-3-yl)-5-fluoro-3-((1-((4-(fluoromethylene)piperidin-1-yl)methyl)cyclopropyl)methoxy)-7,9-dihydrofuro[3,4-f]quinazolin-6-yl)-5-chloro-4-(trifluoromethyl)aniline 19

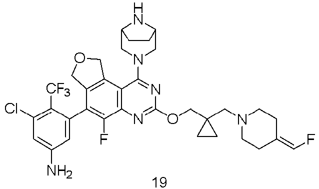

Following the synthetic route described in Example **12,** the title compound **19** (3 mg, yield: 11.4%) was obtained by replacing the starting material tert-butyl (3-cyano-4-(5,5-dimethyl-1,3,2-dioxaborinan-2-yl)-7-fluorobenzo[b]thiophen-2-yl)carbamate in step 4 with 3-chloro-5-(4,4,5,5-tetramethyl-1,3,2-dioxaborolan-2-yl)-4-(trifluoromethyl)aniline.

MS m/z (ESI): 691.3 [M+1].

¹H NMR (500 MHz, CD₃OD): δ 6.90 (d, 1H), 6.61 - 6.36 (m, 2H), 5.43 - 5.35 (m, 1H), 4.84 - 4.76 (m, 2H), 4.44 (d, 2H), 4.09 (t, 2H), 3.62 - 3.52 (m, 4H), 2.58 - 2.45 (m, 4H), 2.34 - 2.28 (m, 1H), 2.21 (t, 1H), 2.10 - 2.03 (m, 3H), 1.92 - 1.79 (m, 3H), 1.66 - 1.58 (m, 1H), 0.92 (t, 2H), 0.73 (s, 2H), 0.51 (s, 2H).

### Example 20

### 4-(1-(3,8-diazabicyclo[3.2.1]oct-8-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-c]pyridine-3-carbonitrile 20

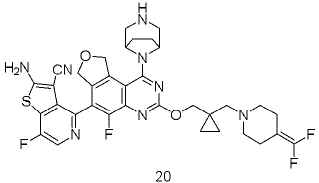

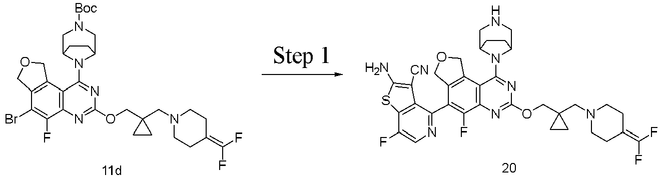

Following steps 6 and 7 of the synthetic route described in Example 1, the title compound **20** (71 mg, yield: 23.8%) was obtained by replacing the starting material compound **1g** in step 6 with compound **11d.**

MS m/z (ESI): 707.5 [M+1].

¹H NMR (500 MHz, DMSO-*d₆*): δ 8.42 (s, 2H), 7.49 (s, 1H), 7.19-6.65 (m, 1H), 5.44-5.32 (m, 2H), 5.02-5.01 (m, 1H), 4.68-4.65 (m, 1H), 4.31-4.18 (m, 4H), 3.11-3.01 (m, 2H), 2.43-2.29 (m, 6H), 2.09-1.99 (m, 4H), 1.86-1.74 (m, 4H), 1.46-1.24 (m, 2H), 0.65-0.40 (m, 4H).

### Example 21

### 2-amino-4-(3-((1-((4-(difluoromethylene)piperidin-1-yl)methyl)cyclopropyl)methoxy)-5-fluoro-1-(3-hydroxyl-3,8-diazabicyclo[3.2.1]oct-8-yl)-7,9-dihydrofuro[3,4-f]quinazolin-6-yl)-7-fluorothieno[3,2-c]pyridine-3-carbonitrile 21

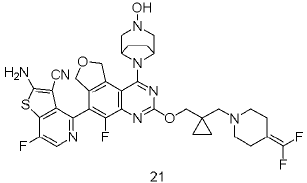

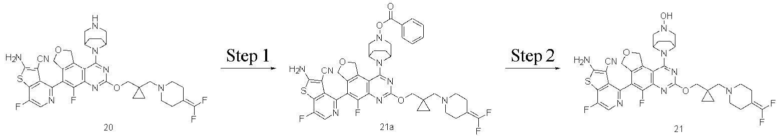

### Step 1

### 8-(6-(2-amino-3-cyano-7-fluorothieno[3,2-c]pyridin-4-yl)-3-((1-((4-(difluoromethylene)piperidin-1-yl)methyl)cyclopropyl)methoxy)-5-fluoro-7,9-dihydrofuro[3,4-f]quinazolin-1-yl)-3,8-diazabicyclo[3.2.1]oct-3-yl benzoate 21a

Under nitrogen atmosphere, dipotassium hydrogen phosphate trihydrate was suspended in 2 mL of N,N-dimethylformamide, followed by the addition of dibenzoyl peroxide (24 mg, 0.099 mmol). A solution of compound **20** (44 mg, 0.062 mmol) in N,N-dimethylformamide (2 mL) was added in an ice-water bath. The mixture was reacted at room temperature overnight. Water was added, and the mixture was extracted with ethyl acetate. The organic phases were combined and dried over anhydrous sodium sulfate, filtered, and the filtrate was concentrated under reduced pressure to afford the crude title compound **21a** (46 mg), which was used directly in the next step without purification.

MS m/z (ESI): 827.3 [M+1].

### Step 2

### 2-amino-4-(3-((1-((4-(difluoromethylene)piperidin-1-yl)methyl)cyclopropyl)methoxy)-5-fluoro-1-(3-hydroxyl-3,8-diazabicyclo[3.2.1]oct-8-yl)-7,9-dihydrofuro[3,4-f]quinazolin-6-yl)-7-fluorothieno[3,2-c]pyridine-3-carbonitrile 21

Compound **21a** was dissolved in 5 mL of methanol, followed by the addition of anhydrous potassium carbonate (15 mg, 0.11 mmol). The mixture was reacted at room temperature for 1 hour, filtered, and the filtrate was concentrated under reduced pressure. The residue was purified by preparative high-performance liquid chromatography (Waters-2545, chromatographic column: YMC Triart-Exrs C18, 30*150 mm, 5 um; mobile phase: aqueous phase (10 mmol/L ammonium bicarbonate) and acetonitrile, gradient ratio: acetonitrile 30%-45%, flow rate: 30 mL/min) to afford the title compound **21.**

MS m/z (ESI): 723.2 [M+1].

¹H NMR (500 MHz, DMSO-*d₆*): *δ* 8.42 (s, 2H), 8.12 (s, 1H), 7.19-6.65 (m, 1H), 5.45-5.33 (m, 2H), 5.05-5.03 (m, 1H), 4.70-4.68 (m, 1H), 4.44-4.28 (m, 4H), 3.15-3.13 (m, 2H), 2.88-2.78 (m, 2H), 2.42-2.27 (m, 5H), 2.10-1.98 (m, 4H), 1.88-1.76 (m, 3H), 1.46-1.35 (m, 2H), 0.66-0.41 (m, 4H).

### Example 22

### 2-amino-7-fluoro-4-(5-fluoro-3-((1-((4-(fluoromethylene)piperidin-1-yl)methyl)cyclopropyl)methoxy)-1-(3-hydroxyl-3,8-diazabicyclo[3.2.1]oct-8-yl)-7,9-dihydrofuro[3,4-f]quinazolin-6-yl)thieno[3,2-c]pyridine-3-carbonitrile 22

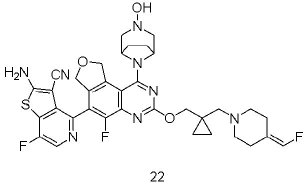

Following the synthetic route for compound 21, the title compound 22 (2 mg, yield: 4%) was obtained by replacing the starting material compound **20** in step 1 with compound **1i**.

MS m/z (ESI): 705.3 [M+1].

¹H NMR (500 MHz, DMSO-*d₆*): δ 8.43 (s, 2H), 8.12 (s, 1H), 6.63 (d, 1H), 5.85-5.37 (m, 2H), 5.18-4.86 (m, 1H), 4.70 (d, 1H), 4.69-4.02 (m, 3H), 3.14 (dt, 2H), 2.83 (dd, 2H), 2.46-2.34 (m, 5H), 2.28 (d, 1H), 2.20 (d, 2H), 2.15-1.96 (m, 3H), 1.92-1.85 (m, 3H), 1.84-1.74 (m, 1H), 1.57-1.41 (m, 1H), 0.66 (s, 2H), 0.40 (d, 2H).

### Example 23

### 2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)thieno[3,2-c]pyridine-3-carbonitrile 23

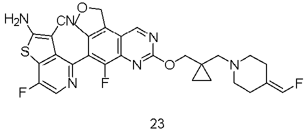

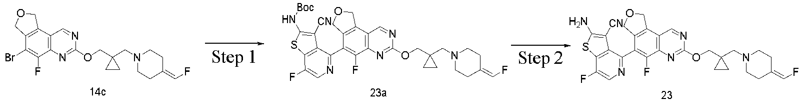

### Step 1

### tert-butyl (3-cyano-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)thieno[3,2-c]pyridin-2-yl)carbamate 23a

Compound **14c** (50 mg, 107 µmol) and neopentyl glycol diboronate (38 mg, 0.17 mmol) were dissolved in 1,4-dioxane (5 mL). Potassium acetate (33 mg, 0.33 mmol) and [1,1'-bis(diphenylphosphino)ferrocene]dichloropalladium(II) (7.65 mg, 11 µmol) were added. The mixture was reacted at 95°C for 2 hours under nitrogen atmosphere. The reaction mixture was cooled to room temperature, and water (1 mL), tert-butyl (4-chloro-3-cyano-7-fluorothieno[3,2-c]pyridin-2-yl)carbamate (36 mg, 110 µmol, prepared according to the method disclosed in Preparation 231 on page 202 of the specification of patent application "WO 2023183585 A1"), methanesulfonato(2-dicyclohexylphosphino-2',4',6'-triisopropyl-1,1'-biphenyl)(2'-methylamino-1,1'-biphen-2-yl)palladium(II) (9.6 mg, 11 µmol), and potassium phosphate (35 mg, 0.17 mmol) were added. The mixture was reacted at 85°C for 1 hour under nitrogen atmosphere. The reaction mixture was concentrated under reduced pressure. The residue was dissolved in dichloromethane, and water was added. The layers were separated, and the aqueous phase was extracted with dichloromethane (10 mL × 2). The organic phases were combined and dried over anhydrous sodium sulfate, filtered, and the filtrate was concentrated under reduced pressure. The residue was purified by silica gel column chromatography using eluent system B to afford the title compound **23a** (30 mg, yield: 41%).

MS m/z (ESI):679.2 [M+1].

### Step 2

### 2-amino-7-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)thieno[3,2-c]pyridine-3-carbonitrile 23

Compound **23a** (30 mg, 44 µmol) was dissolved in dichloromethane (2 mL). Trifluoroacetic acid (1 mL) was added. After reacting with stirring for 1 hour, the mixture was concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 30%-45%, flow rate: 30 mL/min) to afford the title compound **23** (9 mg, yield: 37%).

MS m/z (ESI): 579.2 [M+1].

¹H NMR (500 MHz, CD₃OD) δ 9.39 (s, 1H), 8.33 (s, 1H), 6.52 (d, 1H), 5.60 (s, 2H), 5.25 (d, 1H), 4.55 (s, 2H), 2.71 - 2.46 (m, 5H), 2.36 (s, 2H), 2.21 (t, 1H), 2.16 - 2.02 (m, 3H), 0.80 (s, 2H), 0.56 (s, 2H).

### Example 24

### 2-amino-4-(3-((1-((4-(difluoromethylene)piperidin-1-yl)methyl)cyclopropyl)methoxy)-5-fluoro-7,9-dihydrofuro[3,4-f]quinazolin-6-yl)-7-fluorothieno[3,2-c]pyridine-3-carbonitrile 24

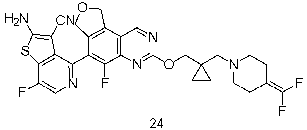

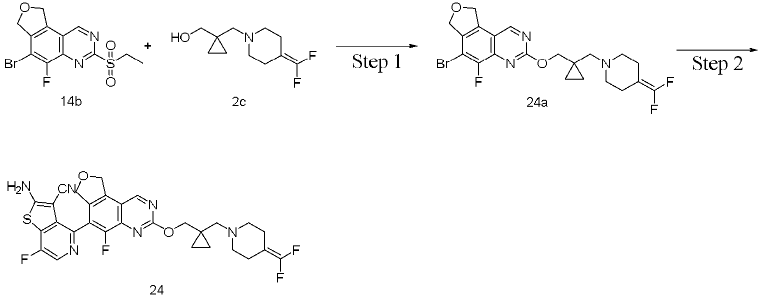

### Step 1

### 6-bromo-3-((1-((4-(difluoromethylene)piperidin-1-yl)methyl)cyclopropyl)methoxy)-5-fluoro-7,9-dihydrofuro[3,4-f]quinazoline 24a

Compound **2c** (108 mg, 0.50 mmol) was dissolved in tetrahydrofuran (3 mL). A 2 M solution of lithium bis(trimethylsilyl)amide in tetrahydrofuran (0.25 mL) was added in an ice bath, and the mixture was reacted with stirring at this temperature for 30 minutes. Then, a solution of compound **14b** (100 mg, 0.27 mmol) in tetrahydrofuran (3 mL) was added in an ice bath. The mixture was reacted with stirring at this temperature for 1 hour. The reaction was quenched by adding saturated ammonium chloride solution, and the mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **24a** (20 mg, yield: 15.0%).

MS m/z (ESI):484.1 [M+1].

### Step 2

### 2-amino-4-(3-((1-((4-(difluoromethylene)piperidin-1-yl)methyl)cyclopropyl)methoxy)-5-fluoro-7,9-dihydrofuro[3,4-f]quinazolin-6-yl)-7-fluorothieno[3,2-c]pyridine-3-carbonitrile 24

Following the synthetic route described in Example 23, the title compound 24 (1 mg, yield: 11.6%) was obtained by replacing the starting material compound **14c** in step 1 with compound **24a.**

MS m/z (ESI): 597.2 [M+1].

¹H NMR (500 MHz, CD₃OD) 9.38 (s, 1H), 8.33 (s, 1H), 5.59 (d, 2H), 5.25 (dt, 1H), 4.85 - 4.78 (m, 1H), 4.54 (d, 2H), 2.63 - 2.39 (m, 6H), 2.20 (t, 4H), 0.78 (d, 2H), 0.53 (d, 2H).

### Example 25

### 2-amino-4-(3-(((S)-4-(difluoromethylene)-1-methylpyrrolidin-2-yl)methoxy)-5-fluoro-7,9-dihydrofuro[3,4-f]quinazolin-6-yl)-7-fluorothieno[3,2-c]pyridine-3-carbonitrile 25

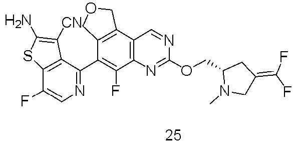

Following the synthetic route described in Example 24, the title compound 25 (28 mg, yield: 47.3%) was obtained by replacing the starting material compound **2c** in step 1 with compound **16e.**

MS m/z (ESI): 543.1 [M+1].

¹H NMR (500 MHz, DMSO-*d*₆) δ 9.50 (s, 1H), 8.48-8.47 (m, 2H), 8.45-8.44 (m, 1H), 5.54-5.53 (m, 2H), 5.17-5.15 (m, 1H), 4.83-4.81 (m, 1H), 4.56-4.55 (m, 2H), 3.67-3.64 (m, 1H), 2.94-2.87 (m, 2H), 2.71-2.68 (m, 1H), 2.43-2.41 (m, 1H), 2.40 (s, 3H).

### Example 26

### 2-amino-4-(3-((2,6-dimethylenetetrahydro-1H-pyrrolizin-7a(5H)-yl)methoxy)-5-fluoro-7,9-dihydrofuro[3,4-f]quinazolin-6-yl)-7-fluorobenzo[b]thiophene-3-carbonitrile 26

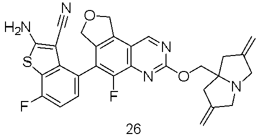

Following the synthetic route described in Example **14,** the title compound **26** (50 mg, yield: 53.8%) was obtained by replacing the starting material compound **1e** in step 2 with (2,6-dimethylenetetrahydro-1H-pyrrolizin-7a(5H)-yl)methanol.

¹H NMR (500 MHz, DMSO*-d₆*) δ 9.47 (s, 1H), 8.14-8.12 (m, 2H), 7.36-7.33 (m, 1H), 7.16-7.14 (m, 1H), 5.54-5.52 (m 2H), 5.02-4.93 (m, 5H), 4.80-4.78 (m, 1H), 4.21 (s, 2H), 3.67-3.64 (m, 2H), 3.23-3.21 (m, 2H), 2.70-2.66 (m, 2H), 2.52-2.48 (m, 2H).

MS m/z (ESI): 544.2 [M+1].

### Examples 26-p1, 26-p2

### (R)-2-amino-4-(3-((2,6-dimethylenetetrahydro-1H-pyrrolizin-7a(5H)-yl)methoxy)-5-fluoro-7,9-dihydrofuro[3,4-f]quinazolin-6-yl)-7-fluorobenzo[b]thiophene-3-carbonitrile 26-pl

### (S)-2-amino-4-(3-((2,6-dimethylenetetrahydro-1H-pyrrolizin-7a(5H)-yl)methoxy)-5-fluoro-7,9-dihydrofuro[3,4-f]quinazolin-6-yl)-7-fluorobenzo[b]thiophene-3-carbonitrile 26-p2

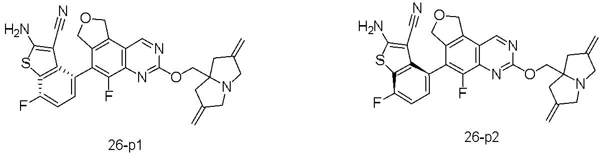

Isomer mixture 26 (50 mg) was separated by chiral column chromatography (chromatographic column: CHIRALPAK IE, 20×250 mm, 5 µm; mobile phase A: n-hexane, mobile phase B: ethanol (0.5% 7 M ammonia-methanol solution), gradient ratio: A : B = 50 : 50, flow rate: 15 mL/min), to afford the title compounds (16 mg, yield: 32%) and (15 mg, yield: 30%).

Single-configuration compound (shorter retention time, designated as 26A, i.e., 26-p1 or 26-p2): (16 mg, yield: 32%).

MS m/z (ESI): 544.2 [M+1].

Chiral HPLC analysis: retention time: 8.894 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, CDCl₃): *δ* 9.15 (s, 1H), 7.24-7.21 (m, 1H), 7.06-7.02 (m, 1H), 5.60-5.57 (m, 3H), 5.01-4.97 (m, 5H), 4.38-4.37 (m, 2H), 3.84-3.81 (m, 2H), 3.34-3.31 (m, 2H), 2.85-2.82 (m, 2H), 2.60-2.56 (m, 2H).

Single-configuration compound (longer retention time, designated as 26B, i.e., 26-p2 or 26-p1): (15 mg, yield: 30%).

MS m/z (ESI): 544.2 [M+1].

Chiral HPLC analysis: retention time: 20.218 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, CDCl₃): *δ* 9.15 (s, 1H), 7.24-7.22 (m, 1H), 7.06-7.02 (m, 1H), 5.63-5.53 (m, 3H), 5.04-4.95 (m, 5H), 4.38-4.37 (m, 2H), 3.83-3.80 (m, 2H), 3.34-3.31 (m, 2H), 2.84-2.82 (m, 2H), 2.60-2.56 (m, 2H).

### Example 27

### 2-amino-4-(3-((2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 27

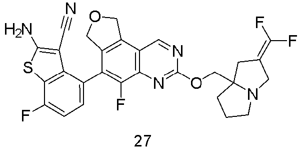

Following the synthetic route described in Example **14,** the title compound **27** (33.6 mg, yield: 13.7%) was obtained by replacing the starting material compound **1e** in step 2 with (2-(difluoromethylene)tetrahydro-1*H-*pyrrolizin-7*a*(5*H*)-yl)methanol.

¹H NMR (500 MHz, DMSO-*d₆*): δ 9.48 (s, 1H), 8.13 (s, 2H), 7.36-7.33 (m, 1H), 7.16-7.13 (m, 1H), 5.53 (s, 2H), 5.01-4.98 (m, 1H), 4.81-4.77 (m, 1H), 4.30-4.18 (m, 2H), 3.71-3.67 (m, 1H), 3.04-3.01 (m, 1H), 2.70-2.57 (m, 2H), 2.44-2.36 (m, 1H), 2.08-1.98 (m, 2H), 1.91-1.80 (m, 3H).

MS m/z (ESI): 568.1 [M+1].

### Example 28

### 2-amino-7-fluoro-4-(5-fluoro-3-((1-((4-(difluoromethylene)piperidin-1-yl)methyl)cyclopropyl)methoxy)-7,9-dihydrofuro[3,4-f]quinazolin-6-yl)benzo[b]thiophene-3-carbonitrile 28

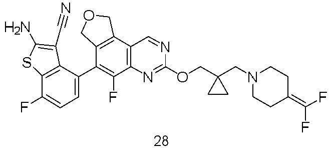

Following the synthetic route described in Example **14,** the title compound **28** (75 mg, yield: 17.5%) was obtained by replacing the starting material compound **1e** in step 2 with compound **2c.**

¹H NMR (500 MHz, CD₃OD) δ 9.35 (s, 1H), 7.30 (dd, 1H), 7.06 (t, 1H), 5.59 (s, 2H), 5.05 (d, 1H), 4.90 (s, 1H), 4.53 (s, 2H), 2.51 (d, 5H), 2.20 (s, 4H), 2.05 (s, 1H), 0.77 (d, 2H), 0.53 (s, 2H).

MS m/z (ESI): 596.2 [M+1].

### Examples 28-p1, 28-p2

### (R)-2-amino-4-(3-((1-((4-(difluoromethylene)piperidin-1-yl)methyl)cyclopropyl)methoxy)-5-fluoro-7,9-dihydrofuro[3,4-f]quinazolin-6-yl)-7-fluoro-benzo[b]thiophene-3-carbonitrile 28-p1

### (S)-2-amino-4-(3-((1-((4-(difluoromethylene)piperidin-1-yl)methyl)cyclopropyl)methoxy)-5-fluoro-7,9-dihydrofuro[3,4-f]quinazolin-6-yl)-7-fluoro-benzo[b]thiophene-3-carbonitrile 28-p2

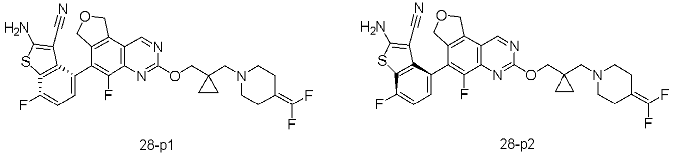

Isomer mixture **28** (75 mg) was separated by chiral column chromatography (chromatographic column: CHIRALPAK IE, 20×250 mm, 5 µm; mobile phase A: n-hexane, mobile phase B: ethanol (0.5% 7 M ammonia-methanol solution), gradient ratio: A : B = 75 : 25, flow rate: 20 mL/min), to afford the title compounds (25 mg, yield: 33.3%) and (25 mg, yield: 33.3%).

Single-configuration compound (shorter retention time, designated as 28A, i.e., 28-p1 or 28-p2): (25 mg, yield: 33.3%).

MS m/z (ESI): 596.2 [M+1].

Chiral HPLC analysis: retention time: 4.744 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, CD₃OD): δ 9.34 (s, 1H), 7.30 (dd, 1H), 7.06 (t, 1H), 5.59 (s, 2H), 5.05 (d, 2H), 4.52 (s, 2H), 2.67-2.41 (m, 6H), 2.21 (q, 4H), 0.78 (s, 2H), 0.53 (d, 2H).

Single-configuration compound (longer retention time, designated as 28B, i.e., 28-p2 or 28-p1): (25 mg, yield: 33.3%).

MS m/z (ESI): 596.2 [M+1].

Chiral HPLC analysis: retention time: 6.073 minutes, purity: 99% (chromatographic column: CHIRALPAK IE 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, CD₃OD): δ 9.34 (s, 1H), 7.30 (dd, 1H), 7.06 (t, 1H), 5.59 (s, 2H), 5.05 (d, 2H), 4.52 (s, 2H), 2.67-2.41 (m, 6H), 2.21 (q, 4H), 0.78 (s, 2H), 0.53 (d, 2H).

### Example 29

### 2-amino-7-fluoro-4-(5-fluoro-3-((2-methylenetetrahydro-1H-pyrrolizin-7a(5H)-yl)methoxy)-7,9-dihydrofuro[3,4-f]quinazolin-6-yl)benzo[b]thiophene-3-carbonitrile 29

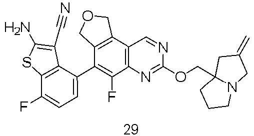

Following the synthetic route described in Example **14,** the title compound **29** was obtained by replacing the starting material compound **1e** in step 2 with (2-methylenehexahydrogen-1H-pyrrolizin-7a-yl)methanol (prepared according to the method disclosed in the literature: Tetrahedron, 2007, vol. 63, # 22, p. 4712 - 4724).

MS m/z (ESI): 532.2 [M+1].

### Example 30

### 2-amino-7-fluoro-4-(5-fluoro-3-((2-(fluoromethylene)tetrahydro-1H-pyrrolizin-7a(5H)-yl)methoxy)-7,9-dihydrofuro[3,4-f]quinazolin-6-yl)thieno[3,2-c]pyridine-3-carbonitrile 30

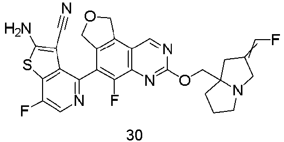

Following the synthetic route described in Example **24,** the title compound **30** was obtained by replacing the starting material compound **2c** in step 1 with (2-(fluoromethylene)tetrahydro-1*H*-pyrrolizin-7*a*(5*H*)-yl)methanol.

MS m/z (ESI): 551.1 [M+1].

### Example 31

### 2-amino-4-(3-((2-(difluoromethylene)tetrahydro-1H-pyrrolizin-7a(5R)-yl)methoxy)-5-fluoro-7,9-dihydrofuro[3,4-f]quinazolin-6-yl)-7-fluorothieno[3,2-c]pyridine-3-carbonitrile 31

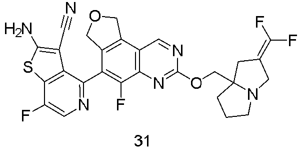

Following the synthetic route described in Example **24,** the title compound **31** was obtained by replacing the starting material compound **2c** in step 1 with (2-(difluoromethylene)tetrahydro-1*H*-pyrrolizin-7*a*(5*H*)-yl)methanol.

MS m/z (ESI): 569.1 [M+1].

### Example 32

### 2-amino-4-(3-((2,6-dimethylenetetrahydro-1H-pyrrolizin-7a(5H)-yl)methoxy)-5-fluoro-7,9-dihydrofuro[3,4-f]quinazolin-6-yl)-7-fluorothieno[3,2-c]pyridine-3-carbonitrile 32

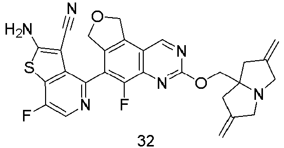

Following the synthetic route described in Example **24,** the title compound **32** was obtained by replacing the starting material compound **2c** in step 1 with (2-(difluoromethylene)tetrahydro-1*H*-pyrrolizin-7*a*(5*H*)-yl)methanol.

MS m/z (ESI): 545.2 [M+1].

### Example 33

### 3-chloro-5-(3-((1-((4-(difluoromethylene)piperidin-1-yl)methyl)cyclopropyl)methoxy)-5-fluoro-7,9-dihydrofuro[3,4-f]quinazolin-6-yl)-4-(trifluoromethyl)aniline 33

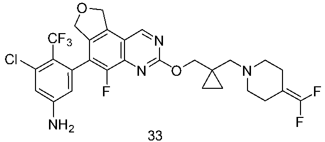

Following the synthetic route described in Example **14,** the title compound **33** (3 mg, yield: 8%) was obtained by replacing the starting material compound **1e** in step 2 with compound **2c,** and replacing tert-butyl (3-cyano-4-(4,4,5,5-tetramethyl-1,3,2-dioxaborolan-2-yl)-7-fluorobenzo[b]thiophen-2-yl)carbamate in step 3 with 3-chloro-5-(4,4,5,5-tetramethyl-1,3,2-dioxaborolan-2-yl)-4-(trifluoromethyl)aniline.

¹H NMR (500 MHz, CD₃OD) δ 9.31 (s, 1H), 6.89 (d, 1H), 6.43 - 6.38 (m, 1H), 5.55 (s, 2H), 5.00 (d, 1H), 4.89 (s, 2H), 4.54 (dd, 1H), 4.47 (dt, 1H), 2.48 (s, 5H), 2.16 (s, 4H), 0.75 (d, 2H), 0.51 (d, 2H).

MS m/z (ESI): 599.2 [M+1].

### Example 34

### 4-(3-((1-((4-(difluoromethylene)piperidin-1-yl)methyl)cyclopropyl)methoxy)-5-fluoro-7,9-dihydrofuro[3,4-f]quinazolin-6-yl)-6-methyl-5-(trifluoromethyl)pyridin-2-amine 34

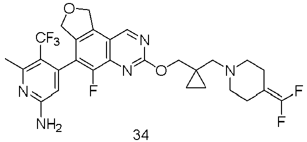

Following the synthetic route described in Example **14,** the title compound **34** (2.5 mg, yield: 14.1%) was obtained by replacing the starting material compound **1e** in step 2 with compound **2c,** and replacing the starting material tert-butyl (3-cyano-4-(4,4,5,5-tetramethyl-1,3,2-dioxaborolan-2-yl)-7-fluorobenzo[b]thiophen-2-yl)carbamate in step 3 with N,N-bis(4-methoxybenzyl)-6-methyl-4-(4,4,5,5-tetramethyl-1,3,2-dioxaborolan-2-yl)-5-(trifluoromethyl)pyridin-2-amine (prepared according to the method disclosed in Example 60 on page 244 of the specification of patent application "WO 2022042630").

¹H NMR (500 MHz, CD3OD) δ 9.35 (s, 1H), 6.29 (s, 1H), 5.57 (s, 2H), 5.04 (d, 1H), 4.92 (d, 1H), 4.57 (d, 1H), 4.49 (d, 1H), 2.62 - 2.51 (m, 8H), 2.24 - 2.15 (m, 5H), 0.80 (s, 2H), 0.56 (s, 2H).

MS m/z (ESI): 580.2 [M+1].

### Example 35

### 2-amino-4-(1-cyclopropyl-5-fluoro-3-((1-((4-(fluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 35

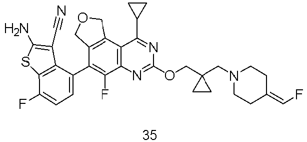

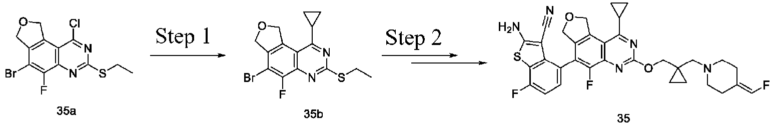

### Step 1

### 6-bromo-1-cyclopropyl-3-(ethylthio)-5-fluoro-7,9-dihydrofuro[3,4-f]quinazoline 35b

6-Bromo-1-chloro-3-(ethylthio)-5-fluoro-7,9-dihydrofuro[3,4-f]quinazoline **35a** (1.0 g, 2.75 mmol, prepared according to the method disclosed in Preparation 60 on page 189 of the specification of patent application "WO 2023183585 A1") was dissolved in tetrahydrofuran (30 mL). Iron(III) acetylacetonate (485.6 mg, 1.37 mmol) and a 1 M solution of cyclopropylmagnesium bromide in tetrahydrofuran (5.5 mL, 5.5 mmol) were added sequentially. Under nitrogen atmosphere, the mixture was reacted at 0°C for 2 hours. The reaction was quenched with saturated aqueous ammonium chloride solution and extracted with ethyl acetate. The organic phases were combined and dried over anhydrous sodium sulfate, filtered, and concentrated under reduced pressure. The residue was purified by silica gel column chromatography using eluent system A to afford the title compound **35b** (500 mg, yield: 50%).

### Step 2

### 2-amino-7-fluoro-4-(5-fluoro-3-((1-((4-(fluoromethylene)piperidin-1-yl)methyl)cyclopropyl)methoxy)-7,9-dihydrofuro[3,4 -f]quinazolin-6-yl)benzo[b]thiophene-3-carbonitrile 35

Following the synthetic route described in Example **12,** the title compound **35** was obtained by replacing the starting material **12c** in step 2 with compound **35b.**

MS m/z (ESI): 618.2 [M+1].

### Example 36

### 2-amino-4-(1-cyclopropyl-5-fluoro-3-(((2R,7aS)-2-fluorotetrahydro-1H-pyrrolizin-7a(5H)-yl)methoxy)-7,9-dihydrofuro[3,4-f]quinazolin-6-yl)-7-fluorobenzo[b]thiophene-3-carbonitrile 36

### 2-amino-4-((R)-1-cyclopropyl-5-fluoro-3-(((2R,7aS)-2-fluorotetrahydro-1H-pyrrolizin-7a(5H)-yl)methoxy)-7,9-dihydrofuro[3,4-f]quinazolin-6-yl)-7-fluorobenzo[b]thiophene-3-carbonitrile 36-p1 or

### 2-amino-4-((S)-1-cyclopropyl-5-fluoro-3-(((2R,7aS)-2-fluorotetrahydro-1H-pyrrolizin-7a(5H)-yl)methoxy)-7,9-dihydrofuro[3,4-f]quinazolin-6-yl)-7-fluorobenzo[b]thiophene-3-carbonitrile 36-p2

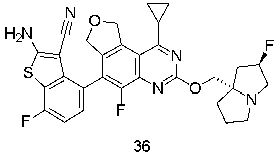

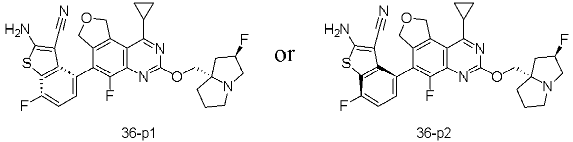

Following the synthetic route described in Example **35,** the crude title compound **36** (17 mg) was obtained by replacing the starting material compound **1e** with ((2*R*,7*aS*)-2-fluorotetrahydro-1*H*-pyrrolizin-7*a*-yl)methanol (from Bide Pharmatech Ltd.). The crude product was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 32%-45%, flow rate: 30 mL/min). The fraction with the longer retention time was collected to afford the title compound **36-p1 or 36-p2** (3 mg, yield: 17.6%).

¹H NMR (500 MHz, CD₃OD) δ 7.29 (dd, 1H), 7.06 (t, 1H), 5.79 (s, 2H), 5.41 - 5.25 (m, 1H), 5.05 (dt, 1H), 4.36 (d, 1H), 4.27 (d, 1H), 3.26 - 3.18 (m, 2H), 3.09 - 2.99 (m, 1H), 2.49 - 2.14 (m, 5H), 2.10 - 1.98 (m, 3H), 1.97 - 1.87 (m, 1H), 1.69 - 1.58 (m, 1H), 1.49 - 1.40 (m, 2H), 0.92 (t, 1H).

MS m/z (ESI): 578.2 [M+1].

### Example 37

### 2-amino-7-fluoro-4-(5-fluoro-3-(((2R,7aS)-2-fluorotetrahydro-1H-pyrrolizin-7a(5H)-yl)methoxy)-1-((S)-6-hydroxyl-6-methyl-1,4-oxazepan-4-yl)-7,9-dihydrofuro[3,4-f]quinazolin-6-yl)benzo[b]thiophene-3-carbonitrile 37

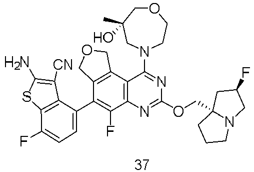

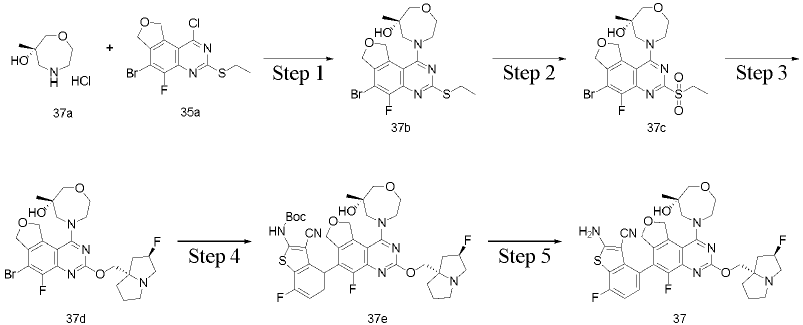

### Step 1

### (S)-4-(6-bromo-3-(ethylthio)-5-fluoro-7,9-dihydrofuro[3,4-f]quinazolin-1-yl)-6-methyl-1,4-oxazepan-6-ol 37b

Compound **35a** (500 mg, 1.37 mmol) and N,N-diisopropylethylamine (445 mg, 3.44 mmol) were dissolved in dichloromethane (20 mL). (S)-6-Methyl-1,4-oxazepan-6-ol hydrochloride **37a** (235 mg, 1.4 mmol, from Bide Pharmatech Ltd.) was added at -78°C. The mixture was reacted with stirring for 17 hours. Saturated sodium bicarbonate solution was added to the reaction mixture, followed by extraction with dichloromethane (15 mL × 3). The organic phases were combined and concentrated under reduced pressure. The residue was purified by silica gel column chromatography using eluent system B to afford the title compound **37b** (565 mg, yield: 89.6%).

MS m/z (ESI): 458.2 [M+1].

### Step 2

### (S)-4-(6-bromo-3-(ethylsulfonyl)-5-fluoro-7,9-dihydrofuro[3,4-f]quinazolin-1-yl)-6-methyl-1,4-oxazepan-6-ol 37c

Compound **37b** (565 mg, 1.23 mmol) was dissolved in dichloromethane (25 mL). meta-Chloroperoxybenzoic acid (503 mg, 2.47 mmol, 85% purity) was added, and the mixture was stirred at room temperature for 2 hours. The reaction was quenched by the addition of aqueous saturated sodium bicarbonate solution and aqueous saturated sodium sulfite solution. The layers was separated, and the aqueous phase was extracted with dichloromethane (10 mL × 3). The organic phases were combined and dried over anhydrous sodium sulfate, filtered, and the filtrate was concentrated under reduced pressure to afford the crude title compound **37c** (601 mg), which was used directly in the next step without purification.

MS m/z (ESI): 490.2 [M+1].

### Step 3

### (S)-4-(6-bromo-5-fluoro-3-(((2R,7aS)-2-fluorotetrahydro-1H-pyrrolizin-7a(5R)-yl)methoxy)-7,9-dihydrofuro[3,4-f]quinazolin-1-yl)-6-methyl-1,4-oxazepan-6-ol 37d

((2*R*,7*aS*)-2-Fluorotetrahydro-1*H*-pyrrolizin-7*a*(5*H*)-yl)methanol (246 mg, 1.54 mmol) was dissolved in tetrahydrofuran (25 mL). Sodium bis(trimethylsilyl)amide (2M, 823.4 µL) was added in an ice bath. After stirring for 10 minutes, a solution of crude compound **37c** (504 mg, 1.02 mmol) in tetrahydrofuran (5 mL) was added. The mixture was reacted with stirring at 0°C for 2 hours. The reaction was quenched by the addition of saturated aqueous ammonium chloride solution. The layers was separated, and the aqueous phase was extracted with ethyl acetate (10 mL × 3). The organic phases were combined and concentrated under reduced pressure. The residue was purified by silica gel column chromatography using eluent system B to afford the title compound **37d** (363 mg, yield: 63.5%).

MS m/z (ESI): 555.1 [M+1].

### Step 4

### tert-butyl (3-cyano-7-fluoro-4-(5-fluoro-3-(((2R,7aS)-2-fluorotetrahydro-1H-pyrrolizin-7a(5H)-yl)methoxy)-1-((S)-6-hydroxyl-6-methyl-1,4-oxazepan-4-yl)-7,9-dihydrofuro[3,4-f]quinazolin-6-yl)benzothiophen-2-yl)carbamate 37e

Compound **37d** (130 mg, 234.06 µmol), tert-butyl (3-cyano-4-(4,4,5,5-tetramethyl-1,3,2-dioxaborolan-2-yl)-7-fluorobenzo[b]thiophen-2-yl)carbamate (190 mg, 470 µmol), [1,1'-bis(diphenylphosphino)ferrocene]dichloropalladium(II) (35 mg, 47.8 µmol), and caesium carbonate (153 mg, 469.6 µmol) were suspended in 1,4-dioxane (10 mL). The mixture was reacted with stirring at 100°C for 3 hours under nitrogen atmosphere. The reaction mixture was concentrated under reduced pressure, and the residue was purified by silica gel column chromatography using eluent system B to afford the title compound **37e** (163 mg, yield: 90.8%). MS m/z (ESI): 767.2 [M+1].

### Step 5

### 2-amino-7-fluoro-4-(5-fluoro-3-(((2R,7aS)-2-fluorotetrahydro-1H-pyrrolizin-7a(5H)-yl)methoxy)-1-((S)-6-hydroxyl-6-methyl-1,4-oxazepan-4-yl)-7,9-dihydrofuro[3,4-f]quinazolin-6-yl)benzo[b]thiophene-3-carbonitrile 37

Compound **37e** (160 mg, 208.6 µmol) was dissolved in dichloromethane (10 mL). Trifluoroacetic acid (3 mL) was added, and the mixture was reacted with stirring for 0.5 hour. The reaction mixture was concentrated under reduced pressure. The residue was adjusted to pH > 7 with saturated sodium bicarbonate solution and extracted with dichloromethane (10 mL × 2). The organic phases were combined and dried over anhydrous sodium sulfate, filtered, and the filtrate was concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 30%-45%, flow rate: 30 mL/min), to afford the title compound **37** (52 mg, yield: 37.3%).

¹H NMR (500 MHz, DMSO-*d₆*): δ 8.13-8.10 (m, 2H), 7.34-7.26 (m, 1H), 7.15-7.12 (m, 1H), 5.76 (s, 1H), 5.48-5.42 (m, 1H), 5.33-5.23 (m, 1H), 4.94-4.83 (m, 1H), 4.75-4.65 (m, 1H), 4.29-4.02 (m, 3H), 3.96-3.88 (m, 1H), 3.74-3.63 (m, 4H), 3.53-3.49 (m, 1H), 3.41-3.38 (m, 1H), 3.10-3.03 (m, 3H), 2.86-2.82 (m, 2H), 2.17-1.99 (m, 3H), 1.86-1.75 (m, 3H), 1.03-1.00 (m, 3H).

MS m/z (ESI): 667.2 [M+1].

### Example 38

### 2-amino-4-(3-((2-(difluoromethylene)tetrahydro-1H-pyrrolizin-7a(5H)-yl)methoxy)-5-fluoro-1-((R)-3-hydroxyl-3-methylpiperidin-1-yl)-7,9-dihydrofuro[3,4-f]quinazolin-6-yl)-7-fluorobenzo[b]thiophene-3-carbonitrile 38

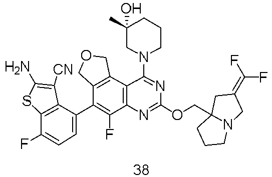

Following the synthetic route described in Example **37,** the title compound **38** (20 mg, yield: 22.9%) was obtained by replacing the starting material compound **37a** in step 1 with (3R)-3-methylpiperidin-3-ol hydrochloride, and replacing ((2*R*,7*aS*)-2-fluorotetrahydro-1H-pyrrolizin-7a(5H)-yl)methanol in step 3 with (2-(difluoromethylene)tetrahydro-1*H*-pyrrolizin-7*a*(5*H*)-yl)methanol.

¹H NMR (500 MHz, CD₃OD) δ 7.28 (ddd, 1H), 7.05 (td, 1H), 5.56 - 5.46 (m, 2H), 4.94 (d, 1H), 4.85 - 4.75 (m, 1H), 4.44 - 4.27 (m, 2H), 3.88 - 3.63 (m, 2H), 3.48 (tt, 3H), 3.18 (s, 2H), 2.85 (d, 1H), 2.74 (d, 1H), 2.54 (d, 1H), 2.27 - 2.11 (m, 2H), 2.10 - 1.86 (m, 3H), 1.69 (dd, 3H), 1.21 (dd, 3H).

MS m/z (ESI): 681.2 [M+1].

### Example 39

### 2-amino-4-(5-chloro-3-((1-((4-(fluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 39

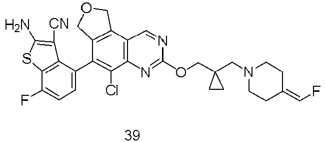

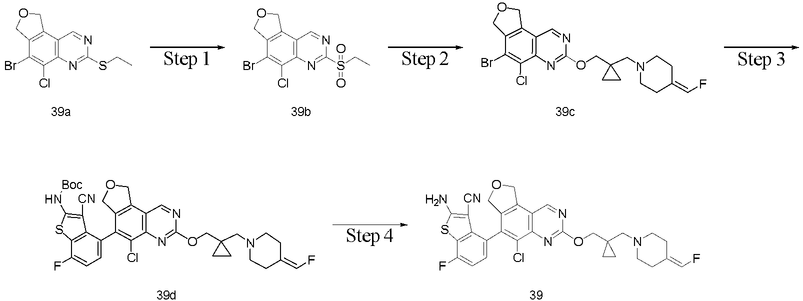

### Step 1

### 6-bromo-5-chloro-3-(ethylsulfonyl)-7,9-dihydrofuro[3,4-f]quinazoline 39b

6-Bromo-3-(ethylthio)-5-chloro-7,9-dihydrofuro[3,4-f]quinazoline **39a** (1.94 g, 5.61 mmol, prepared according to the method disclosed in Preparation 300 on page 192 of the specification of patent application "WO 2023183585 A1") was dissolved in dichloromethane (50 mL). meta-Chloroperoxybenzoic acid (3.42 g, 16.8 mmol, 85% purity) was added, and the mixture was reacted with stirring for 2 hours. Saturated sodium bicarbonate solution was added to the reaction mixture, followed by extraction with dichloromethane (30 mL × 2). The organic phases were combined and dried over anhydrous sodium sulfate, filtered, and the filtrate was concentrated under reduced pressure. The residue was slurried with ethyl acetate, filtered, and the filter cake was dried to afford the crude title compound **39b** (1.76 g), which was used directly in the next step without purification.

MS m/z (ESI):376.9 [M+1].

### Step 2

### 6-bromo-5-chloro-3-((1-((4-(fluoromethylene)piperidin-1-yl)methyl)cyclopropyl)methoxy)-7,9-dihydrofuro[3,4-f]quinazoline 39c

Compound **1e** (222 mg, 1.11 mmol) was dissolved in tetrahydrofuran (20 mL). A 1 M solution of sodium bis(trimethylsilyl)amide in tetrahydrofuran (0.6 mL) was added in an ice bath, and the mixture was reacted with stirring at this temperature for 30 minutes. Then, a solution of crude compound **39b** (300 mg, 794.4 µmol) in tetrahydrofuran (5 mL) was added in an ice bath. The mixture was stirred at this temperature for 1 hour. The reaction was quenched by adding saturated ammonium chloride solution, and the mixture was extracted with ethyl acetate (15 mL × 2). The organic phases were combined and dried over anhydrous sodium sulfate, filtered to remove the drying agent, and concentrated under reduced pressure. The residue was purified by silica gel column chromatography using eluent system B to afford the title compound **39c** (400 mg, yield: 104.2%).

MS m/z (ESI):482.2 [M+1].

### Step 3

### tert-butyl (4-(5-chloro-3-((1-((4-(fluoromethylene)piperidin-1-yl)methyl)cyclopropyl)methoxy)-7,9-dihydrofuro[3,4-f]quinazolin-6-yl)-3-cyano-7-fluorobenzo[b]thiophen-2-yl)carbamate 39d

Compound **39c** (400 mg, 828.5 µmol) and tert-butyl (3-cyano-4-(5,5-dimethyl-1,3,2-dioxaborinan-2-yl)-7-fluorobenzo[b]thiophen-2-yl)carbamate (670 mg, 1.65 mmol, prepared according to the method disclosed in Preparation 15 on page 50 of the specification of patent application "WO 2021118877") were dissolved in 1,4-dioxane (20 mL). Caesium carbonate (540 mg, 1.65 mmol) and [1,1'-bis(diphenylphosphino)ferrocene]dichloropalladium(II) (121 mg, 165.3 µmol) were added. The mixture was reacted at 100°C for 2 hours under nitrogen atmosphere. The reaction mixture was concentrated under reduced pressure. The residue was dissolved in dichloromethane, and water was added. The aqueous phase was extracted with dichloromethane (15 mL × 2). The organic phases were combined and dried over anhydrous sodium sulfate, filtered to remove the drying agent, and concentrated under reduced pressure. The residue was purified by silica gel column chromatography using eluent system B to afford the title compound **39d** (370 mg, yield: 64.3%).

MS m/z (ESI):694.2 [M+1].

### Step 4

### 2-amino-4-(5-chloro-3-((1-((4-(fluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 39

Compound **39d** (370 mg, 533 µmol) was dissolved in dichloromethane (10 mL). Trifluoroacetic acid (2 mL) was added, and the mixture was reacted with stirring for 1 hour. The reaction mixture was concentrated under reduced pressure. The residue was dissolved in dichloromethane, and the pH was adjusted to > 7 with saturated sodium bicarbonate solution. The aqueous phase was extracted with dichloromethane (10 mL × 2). The organic phases were combined and dried over anhydrous sodium sulfate, filtered to remove the drying agent, and concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 30%-45%, flow rate: 30 mL/min), to afford the title compound **39** (100 mg, yield: 37%).

MS m/z (ESI):594.1 [M+1].

¹H NMR (500 MHz, DMSO-*d₆*): *δ* 9.48 (s, 1H), 8.09 (s, 2H), 7.26-7.23 (m, 1H), 7.15 (t, 1H), 6.63 (d, 1H), 5.56-5.55 (m, 2H), 4.90-4.86 (m, 1H), 4.74-4.70 (m, 1H), 4.48-4.43 (m, 2H), 2.43-2.33 (m, 6H), 2.20-2.18 (m, 2H), 2.00-1.98 (m, 2H), 0.74-0.72 (m, 2H), 0.46-0.44 (m, 2H).

### Examples 39-p1, 39-p2

### (R)-2-amino-4-(5-chloro-3-((1-((4-(fluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 39-p1

### (S)-2-amino-4-(5-chloro-3-((1-((4-(fluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 39-p2

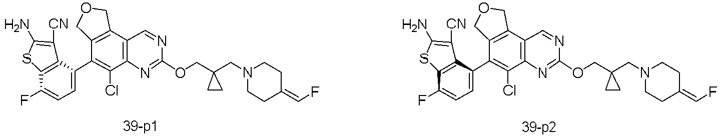

The isomer mixture **39** (100 mg) was separated by chiral column chromatography (Shimadzu LC-20A, chromatographic column: Enantiocel C9 (ColumnTek), in-house packed, SN:AS23122001, 20×250 mm, 10 µm; mobile phase A: n-hexane, mobile phase B: ethanol (0.5% 7 M ammonia-methanol solution), gradient ratio: A : B = 80 : 20, flow rate: 20 mL/min), to afford the title compounds (39 mg, yield: 39%) and (49 mg, yield: 49%).

Single-configuration compound (shorter retention time, designated as 39A, i.e., 39-pl or 39-p2): (39 mg, yield: 39%).

MS m/z (ESI):594.1 [M+1].

Chiral HPLC analysis: retention time: 7.263 minutes, purity: 99% (chromatographic column: CHIRALPAK IK 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, DMSO-*d₆*): *δ* 9.48 (s, 1H), 8.09 (s, 2H), 7.26-7.23 (m, 1H), 7.16-7.13 (m, 1H), 6.63 (d, 1H), 5.56-5.55 (m, 2H), 4.90-4.86 (m, 1H), 4.74-4.70 (m, 1H), 4.49-4.44 (m, 2H), 2.44-2.33 (m, 6H), 2.20-2.18 (m, 2H), 1.99-1.98 (m, 2H), 0.74-0.72 (m, 2H), 0.46-0.44 (m, 2H).

Single-configuration compound (longer retention time, designated as 39B, i.e., 39-p2 or 39-p1): (49 mg, yield: 49%).

MS m/z (ESI):594.1 [M+1].

Chiral HPLC analysis: retention time: 8.896 minutes, purity: 97.9% (chromatographic column: CHIRALPAK IK 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, DMSO-*d₆*): *δ* 9.48 (s, 1H), 8.09 (s, 2H), 7.25-7.23 (m, 1H), 7.16-7.12 (m, 1H), 6.63 (d, 1H), 5.56-5.55 (m, 2H), 4.90-4.86 (m, 1H), 4.73-4.69 (m, 1H), 4.49-4.44 (m, 2H), 2.44-2.33 (m, 6H), 2.20-2.18 (m, 2H), 1.99-1.97 (m, 2H), 0.74-0.72 (m, 2H), 0.46-0.44 (m, 2H).

### Example 40 2-amino-5-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 40

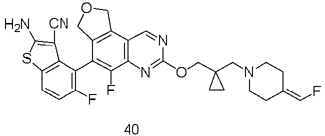

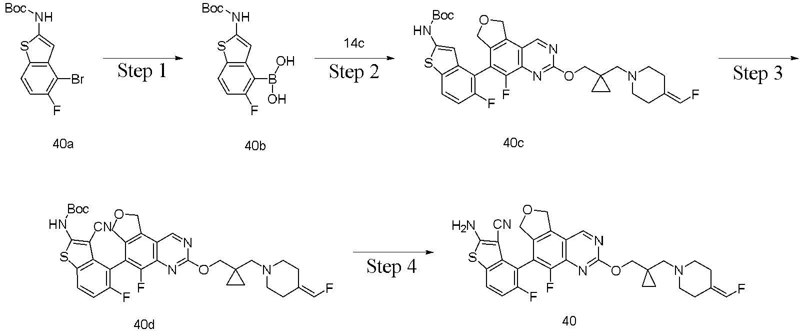

### Step 1

### (2-((tert-butoxycarbonyl)amino)-5-fluorobenzo[b]thiophen-4-yl)boronic acid 40b

tert-Butyl (4-bromo-5-fluorobenzo[b]thiophen-2-yl)carbamate **40a** (530 mg, 1.53 mmol, prepared according to the method disclosed in Preparation 71 on page 189 of the specification of patent application "US 2024043451 "), neopentyl glycol diboronate (691.6 mg, 3.06 mmol), [1,1'-bis(diphenylphosphino)ferrocene]dichloropalladium(II) (112 mg, 153 µmol), and potassium acetate (450.7 mg, 4.6 mmol) were dissolved in 1,4-dioxane (15 mL). Under nitrogen atmosphere, the mixture was reacted at 100°C for 3 hours. After the reaction mixture was cooled to room temperature, it was filtered, and the filtrate was concentrated under reduced pressure. The residue was purified by silica gel column chromatography using eluent system B to afford the title compound **40b** (110 mg, yield: 23.1%).

MS m/z (ESI): 256.0[M-55].

### Step 2

### tert-butyl (5-fluoro-4-(5-fluoro-3-((1-((4-(fluoromethylene)piperidin-1-yl)methyl)cyclopropyl)methoxy)- 7,9-dihydrofuro[3,4-f]quinazolin-6-yl)benzo[b]thiophen-2-yl)carbamate 40c

Compound **14c** (110 mg, 235.9 µmol), compound **40b** (110 mg, 353.8 µmol), [1,1'-bis(di-tert-butylphosphino)ferrocene]dichloropalladium(II) (16 mg, 23.6 µmol), and potassium phosphate (75 mg, 353.8 µmol) were dissolved in 1,4-dioxane (4 mL) and water (1 mL). Under nitrogen atmosphere, the mixture was reacted at 70°C for 1.5 hours. After the reaction mixture was cooled to room temperature, it was filtered, and the filtrate was concentrated under reduced pressure. The residue was purified by silica gel column chromatography using eluent system B to afford the title compound **40c** (45 mg, yield: 29.2%).

MS m/z (ESI): 653.5[M+1].

### Step 3

### tert-butyl (3-cyano-5-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophen-2-yl)carbamate 40d

Compound **40c** (45 mg, 68.9 µmol) was dissolved in tetrahydrofuran (1 mL). Chlorosulfonyl isocyanate (48.8 mg, 344.6 µmol) was added at -45°C. After reacting with stirring at this temperature for 1.5 hours, the mixture was poured into *N,N-*dimethylformamide (1.5 mL) and stirred at room temperature for 1 hour. The reaction mixture was diluted with ethyl acetate, washed with 2M lithium chloride solution and saturated sodium chloride solution. The organic phase was collected and concentrated under reduced pressure to afford the crude title compound **40d** (40 mg), which was used directly in the next step without purification.

MS m/z (ESI): 678.2 [M+1].

### Step 4

### 2-amino-5-fluoro-4-(5-fluoro-3-((1-((4-(fluoromethylene)piperidin-1-yl)methyl)cyclopropyl)methoxy)-7,9-dihydrofuro[3,4-f]quinazolin-6-yl)benzo[b]thiophene-3-carbonitrile 40

Crude compound **40d** (40 mg, 59 µmol) was dissolved in dichloromethane (1 mL). Trifluoroacetic acid (1 mL) was added. After reacting with stirring for 1 hour, the mixture was concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 30%-45%, flow rate: 30 mL/min), to afford the title compound **40** (3 mg, yield: 19.5%).

MS m/z (ESI): 578.3 [M+1].

¹H NMR (500 MHz, CD₃OD): δ 9.38 (d, 1H), 7.71 (dd, 1H), 7.08 (t, 1H), 6.52 (d, 1H), 5.61 (d, 2H), 4.96 (dd, 2H), 4.51 (s, 2H), 2.62 (s, 4H), 2.38 (s, 2H), 2.13 (s, 1H), 1.31 (d, 3H), 0.81 (s, 2H), 0.57 (s, 2H).

### Example 41

### 2-amino-7-fluoro-4-(5-fluoro-3-((1-((4-(fluoromethylene)piperidin-1-yl)methyl)cyclopropyl)methoxy)-1-(1-oxa-6-azaspiro[3.5]non-6-yl)-7,9-dihydrofuro[3,4-f]quinazolin-6-yl)benzo[b]thiophene-3-carbonitrile 41

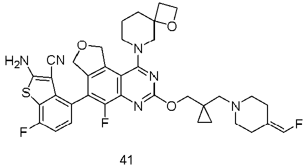

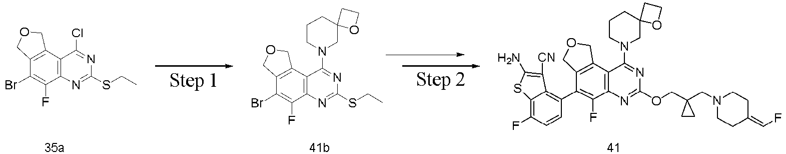

### Step 1

### 6-bromo-3-(ethylthio)-5-fluoro-1-(1-oxa-6-azaspiro[3.5]non-6-yl)- 7,9-dihydrofuro[3,4-f]quinazoline 41b

Compound **35a** (200 mg, 550 µmol) was dissolved in dichloromethane (5 mL). N,N-Diisopropylethylamine (177.7 mg, 1.37 mmol) and 1-oxa-6-azaspiro[3.5]nonane (84 mg, 660 µmol, from Accela ChemBio Co., Ltd.) were added sequentially. The mixture was reacted with stirring for 16 hours. The reaction mixture was concentrated under reduced pressure, and the residue was purified by silica gel column chromatography using eluent system A to afford the title compound **41b** (240 mg, yield: 96.3%).

MS m/z (ESI):454.2 [M+1].

### Step 2

### 2-amino-7-fluoro-4-(5-fluoro-3-((1-((4-(fluoromethylene)piperidin-1-yl)methyl)cyclopropyl)methoxy)-1-(1-oxa-6-azaspiro[3.5]non-6-yl)- 7,9-dihydrofuro[3,4-f]quinazolin-6-yl)benzo[b]thiophene-3-carbonitrile 41

Following the synthetic route described in Example 12, the title compound **41** (3 mg, yield: 2%) was obtained by replacing the starting material **12c** in step 2 with compound **41b.**

MS m/z (ESI):703.3 [M+1].

¹H NMR (500 MHz, CD₃OD): δ 7.29 (dd, 1H), 7.05 (t, 1H), 6.52 (d, 1H), 5.72-5.43 (m, 2H), 4.64-4.32 (m, 4H), 4.02-3.42 (m, 3H), 2.72-2.30 (m, 5H), 2.26-1.96 (m, 5H), 1.95 -1.55 (m, 2H), 1.49-1.29 (m, 5H), 0.94 (dt, 2H), 0.76 (s, 2H), 0.53 (s, 2H).

### Example 42

### (R)-1-((R)-6-(2-amino-3-cyano-7-fluorobenzo[b]thiophen-4-yl)-5-fluoro-3-((1-((4-(fluoromethylene)piperidin-1-yl)methyl)cyclopropyl)methoxy)-7,9-dihydrofuro[3,4-f]quinazolin-1-yl)azetidine-2-carboxamide 42-p1

### (R)-1-((S)-6-(2-amino-3-cyano-7-fluorobenzo[b]thiophen-4-yl)-5-fluoro-3-((1-((4-(fluoromethylene)piperidin-1-yl)methyl)cyclopropyl)methoxy)-7,9-dihydrofuro[3,4-f]quinazolin-1-yl)azetidine-2-carboxamide 42-p2

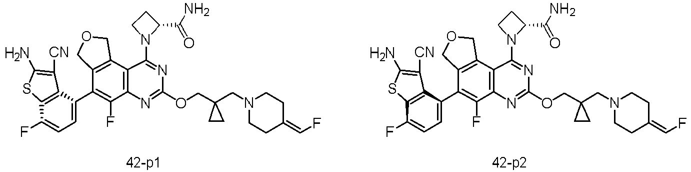

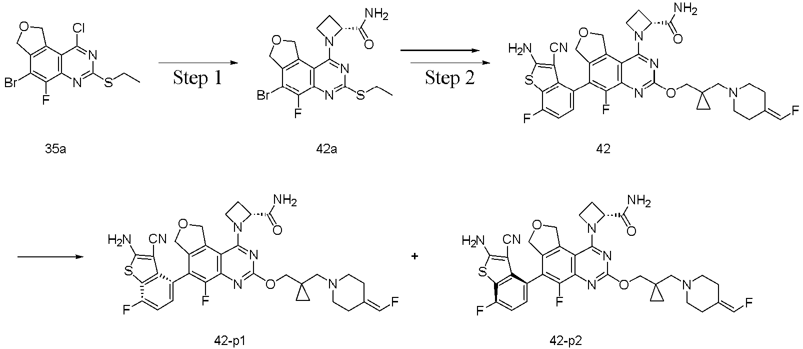

### Step 1

### (R)-1-(6-bromo-3-(ethylthio)-5-fluoro-7,9-dihydrofuro[3,4-f]quinazolin-1-yl)azetidine-2-carboxamide 42a

Compound **35a** (500 mg, 1.37 mmol) was dissolved in dichloromethane (12 mL). *N*,*N*-Diisopropylethylamine (444 mg, 3.44 mmol) and (R)-azetidine-2-carboxamide hydrochloride (188 mg, 1.37 mmol, prepared according to the method disclosed in Example 184 on page 237 of the specification of patent application "WO 2022156792") were added sequentially. The mixture was reacted with stirring for 2 hours. The reaction mixture was concentrated under reduced pressure, and the residue was purified by silica gel column chromatography using eluent system A to afford the title compound **42a** (500 mg, yield: 85.1%).

MS m/z (ESI):427.1 [M+1].

### Step 2

### (R)-1-((R)-6-(2-amino-3-cyano-7-fluorobenzo[b]thiophen-4-yl)-5-fluoro-3-((1-((4-(fluoromethylene)piperidin-1-yl)methyl)cyclopropyl)methoxy)-7,9-dihydrofuro[3,4-f]quinazolin-1-yl)azetidine-2-carboxamide 42-p1

### (R)-1-((S)-6-(2-amino-3-cyano-7-fluorobenzo[b]thiophen-4-yl)-5-fluoro-3-((1-((4-(fluoromethylene)piperidin-1-yl)methyl)cyclopropyl)methoxy)-7,9-dihydrofuro[3,4-f]quinazolin-1-yl)azetidine-2-carboxamide 42-p2

Following the synthetic route described in Example 12, the crude compound **42** (17 mg) was obtained by replacing the starting material **12c** in step 2 with compound **42a.** The crude product was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 30%-42%, flow rate: 30 mL/min), to afford the title compounds (5 mg, yield: 29.4%), (5 mg, yield: 29.4%).

Single-configuration compound (shorter retention time, designated as 42A, i.e., 42-p1 or 42-p2): (5 mg, yield: 29.4%).

MS m/z (ESI): 676.4 [M+1].

HPLC analysis: retention time: 1.45 minutes, purity: 99% (chromatographic column: ACQUITY UPLC^{®}BEH, C18, 1.7 µm, 2.1*50 mm; mobile phase: water (10 mM ammonium bicarbonate) and acetonitrile, gradient ratio: acetonitrile 10%-95%).

¹H NMR (500 MHz, CD₃OD): δ 7.29 (dd, 1H), 7.05 (dd, 1H), 6.51 (d, 1H), 5.56 (d, 1H), 5.47 (d, 1H), 5.29 (dd, 1H), 4.85 (d, 2H), 4.70 (q, 1H), 4.45 (d, 1H), 4.38 (d, 1H), 4.25 (dt, 1H), 2.71 (qd, 1H), 2.64-2.46 (m, 5H), 2.37 (d, 3H), 2.07 (d, 3H), 0.71 (s, 2H), 0.48 (d, 2H).

Single-configuration compound (longer retention time, designated as 42B, i.e., 42-p2 or 42-p1): (5 mg, yield: 29.4%).

MS m/z (ESI): 676.4 [M+1].

HPLC analysis: retention time: 1.49 minutes, purity: 99% (chromatographic column: ACQUITY UPLC^{®}BEH, C18, 1.7 µm, 2.1*50 mm; mobile phase: water (10 mM ammonium bicarbonate) and acetonitrile, gradient ratio: acetonitrile 10%-95%).

¹H NMR (500 MHz, CD₃OD): δ 7.24 (dd, 1H), 7.04 (dd, 1H), 6.51 (d, 1H), 5.57 (d, 1H), 5.50 (d, 1H), 5.39-5.28 (m, 2H), 5.02-4.96 (m, 2H), 4.76-4.67 (m, 2H), 4.49 (d, 1H), 4.34 (d, 1H), 4.20 (q, 1H), 2.73 (qd, 2H), 2.57-2.47 (m, 4H), 2.35 (s, 1H), 2.21 (t, 1H), 2.11 (s, 1H), 2.05 (d, 1H), 0.72 (s, 2H), 0.49 (s, 2H).

### Example 43

### 2-amino-7-fluoro-4-((R)-5-fluoro-3-(((R)-2-methylenetetrahydro-1H-pyrrolizin-7a(5H)-yl)methoxy)-7,9-dihydrofuro[3,4-f]quinazolin-6-yl)benzo[b]thiophene-3-carbonitrile 43-1-pl or

### 2-amino-7-fluoro-4-((S)-5-fluoro-3-(((R)-2-methylenetetrahydro-1H-pyrrolizin-7a(5H)-yl)methoxy)-7,9-dihydrofuro[3,4-f]quinazolin-6-yl)benzo[b]thiophene-3-carbonitrile 43-1-p2 or

### 2-amino-7-fluoro-4-((R)-5-fluoro-3-(((S)-2-methylenetetrahydro-1H-pyrrolizin-7a(5H)-yl)methoxy)-7,9-dihydrofuro[3,4-f]quinazolin-6-yl)benzo[b]thiophene-3-carbonitrile 43-2-p1 or

### 2-amino-7-fluoro-4-((S)-5-fluoro-3-(((S)-2-methylenetetrahydro-1H-pyrrolizin-7a(5H)-yl)methoxy)-7,9-dihydrofuro[3,4-f]quinazolin-6-yl)benzo[b]thiophene-3-carbonitrile 43-2-p2

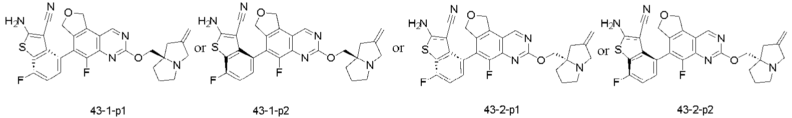

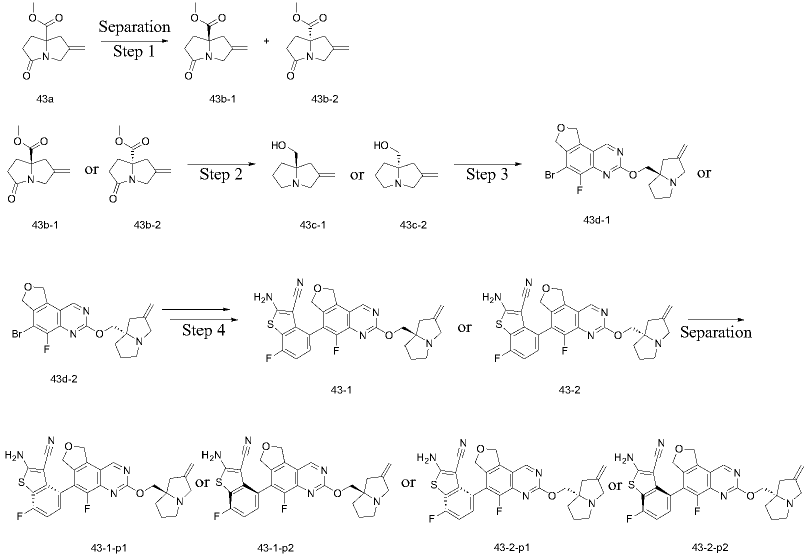

### Step 1

### methyl (R)-2-methylene-5-oxotetrahydro-1H-pyrrolizine-7a(5H)-carboxylate 43b-1

### methyl (S)-2-methylene-5-oxotetrahydro-1H-pyrrolizine-7a(5H)-carboxylate 43b-2

The isomer mixture of methyl 2-methylene-5-oxotetrahydro-1H-pyrrolizine-7*a*(5*H*)-carboxylate **43a** (5 g, prepared according to the method disclosed in Intermediate A-12 on page 111 of the specification of patent application "WO 2020146613") was separated by chiral column chromatography (Waters SFC 150, chromatographic column: DAICELCHIRALPAK^{®}AS, 30×250 mm, 10 µm; mobile phase A: Supercritical CO₂, mobile phase B: ethanol (0.1% 7M NH₃ in MeOH), gradient ratio: A : B: 85 : 15, flow rate: 120 mL/min), to afford the title compounds (1.85 g, yield: 37%), (2.22 g, yield: 44.4%).

Single-configuration compound (shorter retention time): (1.85 g, yield: 37%).

MS m/z (ESI): 196.2 [M+1].

Chiral HPLC analysis: retention time: 0.958 minutes, purity: 99% (Waters UPCC (CA-415), chromatographic column: DAICELCHIRALPAK^{®}AS, 3*100 mm, 3 µm; mobile phase A: Supercritical CO₂, mobile phase B: ethanol (0.1% diethylamine), gradient ratio: A : B: 90 : 10, flow rate: 1.5 mL/min).

Single-configuration compound (longer retention time): (2.22 g, yield: 44.4%).

Chiral HPLC analysis: retention time: 1.432 minutes, purity: 99% (Waters UPCC (CA-415), chromatographic column: DAICELCHIRALPAK^{®}AS, 3×100 mm, 3 µm; mobile phase A: Supercritical CO₂, mobile phase B: ethanol (0.1% diethylamine), gradient ratio: A : B: 90 : 10, flow rate: 1.5 mL/min).

### Step 2

### (R)-(2-methylenetetrahydro-1H-pyrrolizin-7a(5H)-yl)methanol 43c-1 or

### (S)-(2-methylenetetrahydro-1H-pyrrolizin-7a(5H)-yl)methanol 43c-2

The compound with the longer retention time between compound **43b-1** and compound **43b-2** (1 g, 5.1 mmol) was dissolved in tetrahydrofuran (20 mL). A 1 M solution of lithium aluminium hydride in tetrahydrofuran (11.5 mL) was added in an ice bath. The mixture was reacted with stirring at 70°C for 1 hour. After the reaction mixture was cooled to room temperature, sodium sulfate decahydrate was added and stirring was continued for 0.5 hours. The mixture was filtered, and the filtrate was concentrated under reduced pressure to afford the crude title compound **43c-1 or 43c-2** (780 mg), which was used directly in the next step without further purification.

MS m/z (ESI):154.2[M+1].

### Step 3

### (R)-6-bromo-5-fluoro-3-((2-methylenetetrahydro-1H-pyrrolizin-7a(5H)-yl)methoxy)-7,9-dihydrofuro[3,4-f]quinazoline 43d-1 or

### (S)-6-bromo-5-fluoro-3-((2-methylenetetrahydro-1H-pyrrolizin-7a(5H)-yl)methoxy)- 7,9-dihydrofuro[3,4-f]quinazoline 43d-2

Compound **43c-1** or compound **43c-2** (630 mg, 4.11 mmol) was dissolved in tetrahydrofuran (20 mL). A 2 M solution of sodium bis(trimethylsilyl)amide in tetrahydrofuran (2.5 mL) was added in an ice bath. After stirring at this temperature for 30 minutes, a solution of compound **14b** (1 g, 2.77 mmol) in tetrahydrofuran (5 mL) was added in an ice bath. The mixture was reacted with stirring at this temperature for 1 hour. The reaction was quenched by the addition of saturated ammonium chloride solution. The mixture was extracted with ethyl acetate (30 mL × 2). The organic phases were combined and dried over anhydrous sodium sulfate, filtered, and the filtrate was concentrated under reduced pressure. The residue was purified by silica gel column chromatography using eluent system B to afford the title compound **43d-1** or **43d-2** (310 mg, yield: 26.6%).

MS m/z (ESI):420.2[M+1].

### Step 4

### 2-amino-7-fluoro-4-((R)-5-fluoro-3-(((R)-2-methylenetetrahydro-1H-pyrrolizin-7a(5H)-yl)methoxy)-7,9-dihydrofuro[3,4-f]quinazolin-6-yl)benzo[b]thiophene-3-carbonitrile 43-1-p1 or

### 2-amino-7-fluoro-4-((S)-5-fluoro-3-(((R)-2-methylenetetrahydro-1H-pyrrolizin-7a(5H)-yl)methoxy)- 7,9-dihydrofuro[3,4-f]quinazolin-6-yl)benzo[b]thiophene-3-carbonitrile 43-1-p2 or

### 2-amino-7-fluoro-4-((R)-5-fluoro-3-(((S)-2-methylenetetrahydro-1H-pyrrolizin-7a(5H)-yl)methoxy)- 7,9-dihydrofuro[3,4-f]quinazolin-6-yl)benzo[b]thiophene-3-carbonitrile 43-2-p1 or

### 2-amino-7-fluoro-4-((S)-5-fluoro-3-(((S)-2-methylenetetrahydro-1H-pyrrolizin-7a(5H)-yl)methoxy)- 7,9-dihydrofuro[3,4-f]quinazolin-6-yl)benzo[b]thiophene-3-carbonitrile 43-2-p2

Following the synthetic route described in Example 14, compound **43-1** or **43-**2 (150 mg, yield: 29.4%) was obtained by replacing the starting material **14c** in step 3 with compound **43d-1** or **43d-2.**

The isomer mixture **43-1** or **43-2** (100 mg) was separated by chiral column chromatography (Gilson-281, chromatographic column: CHIRALPAK IE, 20×250 mm, 5 µm; mobile phase A: n-hexane, mobile phase B: ethanol (0.1% 7M NH₃ in MeOH), gradient ratio: A : B: 55 : 45, flow rate: 15mL/min), and the compound with the shorter retention time was collected to afford **43-1-p1** or **43-1-p2** or **43-2-pl** or **43-2-p2** (50 mg, yield: 50%, also designated as **43A).**

MS m/z (ESI):532.2[M+1].

¹H NMR (500 MHz, CD₃OD): δ 9.37 (s, 1H), 7.30 (dd, 1H), 7.06 (t, 1H), 5.60 (d, 2H), 5.08-5.03 (m, 1H), 5.01 (q, 1H), 4.90 (d, 1H), 4.48-4.35 (m, 2H), 3.79 (d, 1H), 3.20 (dt, 1H), 2.86 (d, 1H), 2.81-2.70 (m, 1H), 2.54 (d, 1H), 2.20 (ddt, 2H), 2.11-1.85 (m, 4H).

### Example 44

### 2-amino-4-((R)-3-(((R)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 44-1-pl or

### 2-amino-4-((S)-3-(((R)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 44-1-p2 or

### 2-amino-4-((R)-3-(((S)-2-(difluoromethylene)tetrahydro-1H-pyrrolizin-7a(5H)-yl)methoxy)-5-fluoro-7,9-dihydrofuro[3,4-f]quinazolin-6-yl)-7-fluorobenzo[b]thiophene-3-carbonitrile 44-2-p1 or

### 2-amino-4-((S)-3-(((S)-2-(difluoromethylene)tetrahydro-1H-pyrrolizin-7a(5H)-yl)methoxy)-5-fluoro-7,9-dihydrofuro[3,4-f]quinazolin-6-yl)-7-fluorobenzo[b]thiophene-3-carbonitrile 44-2-p2

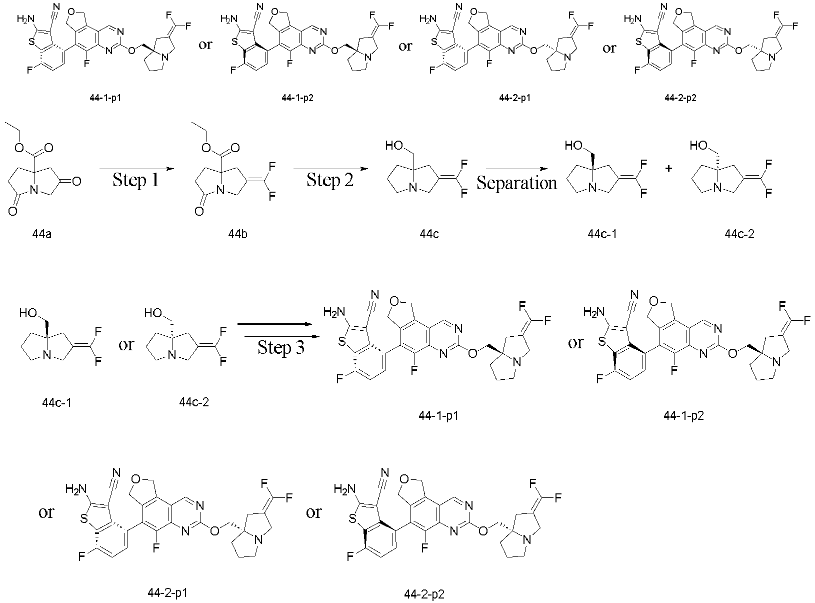

### Step 1

### ethyl 2-(difluoromethylene)-5-oxotetrahydro-1H-pyrrolizine-7a(5H)-carboxylate 44b

Ethyl 2,5-dioxotetrahydro-1*H*-pyrrolizine-7*a*(5*H*)-carboxylate **44a** (18.08 g, 85.60 mmol, from Jiangsu Aikang Biopharmaceutical) and (triphenylphosphonio)difluoroacetate (67.1 g, 188.3 mmol, from Jiangsu Aikang Biopharmaceutical) were dissolved in N,N-dimethylformamide (350 mL). The mixture was reacted with stirring at 85°C for 17 hours under nitrogen atmosphere. After the reaction mixture was cooled to room temperature, saturated sodium bicarbonate solution was added, followed by extraction with ethyl acetate (100 mL × 3). The organic phases were combined and concentrated under reduced pressure. The residue was purified by silica gel column chromatography using eluent system B to afford the title compound **44b** (4.83 g, yield: 23%).

MS m/z (ESI):246.1 [M+1].

### Step 2

### (2-(difluoromethylene)tetrahydro-1H-pyrrolizin-7a(5H)-yl)methanol 44c

### (R)-(2-(difluoromethylene)tetrahydro-1H-pyrrolizin-7a(5H)-yl)methanol 44c-1

### (S)-(2-(difluoromethylene)tetrahydro-1H-pyrrolizin-7a(5H)-yl)methanol 44c-2

Compound **44b** (4.83 g, 19.7 mmol) was dissolved in tetrahydrofuran (250 mL). A 1 M solution of diisobutylaluminium hydride in tetrahydrofuran (98.5 mL) was added in an ice bath. The mixture was allowed to warm to room temperature and stirred for 5 hours. Sodium sulfate decahydrate was then added, and stirring was continued for 0.5 hours. The mixture was filtered, and the filtrate was concentrated under reduced pressure to afford the crude title compound **44c** (2.1 g).

MS m/z (ESI):190.1[M+1].

The isomer mixture **44c** (1 g) was separated by chiral column chromatography (Shimadzu LC-20AP, chromatographic column: DAICELCHIRALPAK IG, 20×250 mm, 5 µm; mobile phase A: n-hexane, mobile phase B: ethanol (0.1% 7M NH₃ in MeOH), gradient ratio: A : B: 80 : 20, flow rate: 20 mL/min), to afford the title compounds (8.1 minutes, 450 mg, yield: 45%), (10.9 minutes, 450 mg, yield: 45%).

The isomer mixture **44c** was analysed by chiral HPLC: retention time: 3.224 minutes, 4.354 minutes (Agilent 1260 DAD, chromatographic column: CHIRALPAK IG 150*4.6 mm, 5 um; mobile phase A: ethanol (0.1% diethylamine, mobile phase B: n-hexane), gradient ratio: A : B: 20 : 80, flow rate: 1 mL/min).

### Step 3

### 2-amino-4-((R)-3-(((R)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile44-1-p1 or

### 2-amino-4-((S)-3-(((R)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 44-1-p2 or

### 2-amino-4-((R)-3-(((S)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 44-2-p1 or

### 2-amino-4-((S)-3-(((S)-2-(difluoromethylene)tetrahydro-1H-pyrrolizin-7a(5R)-yl)methoxy)-5-fluoro-7,9-dihydrofuro[3,4-f]quinazolin-6-yl)-7-fluorobenzo[b]thiophene-3-carbonitrile 44-2-p2

Following the synthetic route described in Example 43, the title compound **44-1-p1** or **44-1-p2** or **44-2-pl** or **44-2-p2** (50 mg, yield: 29.4%, also designated as **44A)** was obtained by replacing the starting material **43c-1 or 43c-2** in step 3 with the compound having the shorter retention time between compound **44c-1** and compound **44c-2.**

MS m/z (ESI):568.2[M+1].

¹H NMR (500 MHz, CD₃OD): δ 9.37 (d, 1H), 7.30 (dd, 1H), 7.06 (dd, 1H), 5.60 (d, 2H), 5.06 (dt, 1H), 4.49 (d, 1H), 4.39 (d, 1H), 3.88-3.80 (m, 1H), 3.49-3.42 (m, 1H), 3.18 (ddd, 1H), 2.89-2.81 (m, 1H), 2.77-2.70 (m, 1H), 2.61-2.53 (m, 1H), 2.23-2.15 (m, 1H), 2.07-1.91 (m, 4H).

### Example 45

### 2-amino-4-(1-((1R,5S)-3-((R)-3,3-dimethyl-4-oxooxetane-2-carbonyl)-3,8-diazabicyclo[3.2.1]oct-8-yl)-5-fluoro-3-((1-((4-(fluoromethylene)piperidin-1-yl)methyl)cyclopropyl)methoxy)-7,9-dihydrofuro[3,4-f]quinazolin-6-yl)-7-fluorothieno[3,2-c]pyridine-3-carbonitrile 45

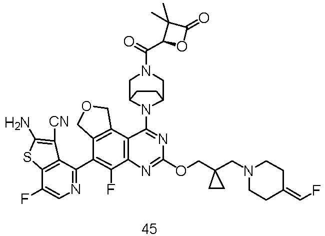

MS m/z (ESI):815.2[M+1].

### Example 46

### 2-amino-4-(3-((1-((4-(difluoromethylene)piperidin-1-yl)methyl)cyclopropyl)methoxy)-1-((1R,5S)-3-((R)-3,3-dimethyl-4-oxooxetane-2-carbonyl)-3,8-diazabicyclo[3.2.1]oct-8-yl)-5-fluoro-7,9-dihydrofuro[3,4-f]quinazolin-6-yl)-7-fluorothieno[3,2-c]pyridine-3-carbonitrile 46

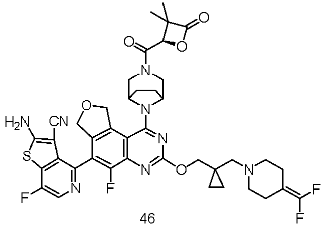

MS m/z (ESI):833.2[M+1].

### Example 47

### 2-amino-7-fluoro-4-(5-fluoro-3-((2-methylenetetrahydro-1H-pyrrolizin-7a(5H)-yl)methoxy)-7,9-dihydrofuro[3,4-f]quinazolin-6-yl)thieno[3,2-c]pyridine-3-carbonitrile 47

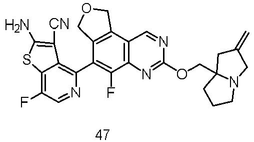

MS m/z (ESI):533.2[M+1].

### Example 48

### 2-amino-4-(5-chloro-3-((1-((4-(difluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 48

### (R)-2-amino-4-(5-chloro-3-((1-((4-(difluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 48-pl

### (S)-2-amino-4-(5-chloro-3-((1-((4-(difluoromethylene)piperidin-1-yl)methyl)cyclopropyl)methoxy)-7,9-dihydrofuro[3,4-f]quinazolin-6-yl)-7-fluorobenzo[b]thiophene-3-carbonitrile 48-p2

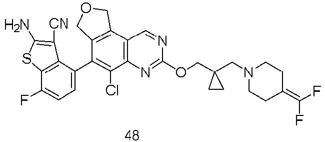

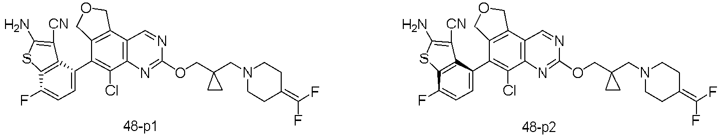

Following the synthetic route described in Example 14, the title compound **48** (60 mg, yield: 61.8%) was obtained by replacing the starting material compound **1e** in step 2 with compound 2c, and replacing compound **14b** with compound **39b.**

Isomer mixture **48** (60 mg) was separated by chiral column chromatography (chromatographic column: Enantiocel C9, 10 µm, 20 mm×250 mm (ColumnTek), in-house packed, SN: AS23122001; mobile phase A: n-hexane, mobile phase B: ethanol (0.5% 7 M ammonia-methanol solution), gradient ratio: A : B = 90 : 10, flow rate: 30 mL/min), to afford the title compounds (37 mg, yield: 45%) and (31 mg, yield: 51.6%).

Single-configuration compound (shorter retention time, designated as 48A, i.e., 48-p1 or 48-p2): (37 mg, yield: 45%).

MS m/z (ESI):612.2[M+1].

Chiral HPLC analysis: retention time: 6.537 minutes, purity: 99% (chromatographic column: CHIRALPAK IK 4.6×250 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, DMSO-*d₆*): δ 9.48 (s, 1H), 8.10-8.08 (m, 2H), 7.26-7.23 (m, 1H), 7.16-7.12 (m, 1H), 5.56-5.55 (m 2H), 4.97-4.86 (m, 5H), 4.73-4.70 (m, 1H), 4.26 (s, 2H), 3.67-3.64 (m, 2H), 3.23-3.20 (m, 2H), 2.72-2.68 (m, 2H), 2.52-2.51 (m, 2H).

Single-configuration compound (longer retention time, designated as 48B, i.e., 48-p2 or 48-p1): (31 mg, yield: 51.6%).

MS m/z (ESI):612.2[M+1].

Chiral HPLC analysis: retention time: 7.789 minutes, purity: 99% (chromatographic column: CHIRALPAK IK 4.6×250 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, DMSO-*d₆*): δ 9.48 (s, 1H), 8.10-8.08 (m, 2H), 7.26-7.23 (m, 1H), 7.16-7.12 (m, 1H), 5.56-5.55 (m, 2H), 4.97-4.87 (m, 5H), 4.73-4.70 (m, 1H), 4.26 (s, 2H), 3.67-3.64 (m, 2H), 3.23-3.20 (m, 2H), 2.72-2.68 (m, 2H), 2.52-2.51 (m, 2H).

MS m/z (ESI):612.2[M+1].

### Example 49

### 2-amino-4-(5-chloro-3-((2,6-dimethylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 49

### (R)-2-amino-4-(5-chloro-3-((2,6-dimethylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 49-pl

### (S)-2-amino-4-(5-chloro-3-((2,6-dimethylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 49-p2

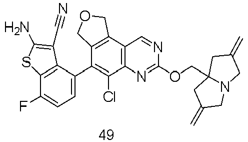

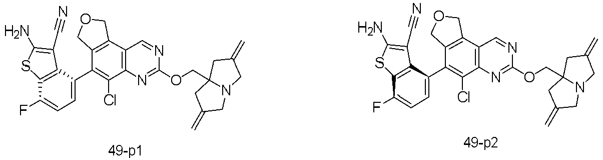

Following the synthetic route described in Example 14, the title compound **49** (40 mg, yield: 20.5%) was obtained by replacing the starting material compound **1e** in step 2 with (2,6-dimethylenetetrahydro-1*H*-pyrrolizin-7*a*(5*H*)-yl)methanol, and replacing compound **14b** with compound **39b,** followed by chiral column chromatography.

Isomer mixture **49** (40 mg) was separated by chiral column chromatography (chromatographic column: CHIRALPAK IE, 20×250 mm, 5 µm; mobile phase A: n-hexane, mobile phase B: ethanol (0.5% 7 M ammonia-methanol solution), gradient ratio: A : B = 75 : 25, flow rate: 20 mL/min), to afford the title compounds (16 mg, yield: 40%) and (18 mg, yield: 45%).

Single-configuration compound (shorter retention time, designated as 49A, i.e., 49-p1 or 49-p2): (16 mg, yield: 40%).

MS m/z (ESI):560.2[M+1].

Chiral HPLC analysis: retention time: 12.389 minutes, purity: 99% (chromatographic column: CHIRALPAK IE 4.6×150 mm, 5 µm; mobile phase A: n-hexane, mobile phase B: ethanol (0.1% diethylamine), gradient ratio: A : B = 80 : 20, flow rate: 1 mL/min).

¹H NMR (500 MHz, DMSO-*d₆*): δ 9.48 (s, 1H), 8.10-8.08 (m, 2H), 7.26-7.23 (m, 1H), 7.16-7.12 (m, 1H), 5.56-5.55 (m 2H), 4.97-4.86 (m, 5H), 4.73-4.70 (m, 1H), 4.26 (s, 2H), 3.67-3.64 (m, 2H), 3.23-3.20 (m, 2H), 2.72-2.68 (m, 2H), 2.52-2.51 (m, 2H).

Single-configuration compound (longer retention time, designated as 49B, i.e., 49-p2 or 49-p1): (18 mg, yield: 45%).

MS m/z (ESI): 596.2 [M+1].

Chiral HPLC analysis: retention time: 15.991 minutes, purity: 99% (chromatographic column: CHIRALPAK IE 4.6×150 mm, 5 µm; mobile phase A: n-hexane, mobile phase B: ethanol (0.1% diethylamine), gradient ratio: A : B = 80 : 20, flow rate: 1 mL/min).

¹H NMR (500 MHz, DMSO-*d₆*): δ 9.48 (s, 1H), 8.10-8.08 (m, 2H), 7.26-7.23 (m, 1H), 7.16-7.12 (m, 1H), 5.56-5.55 (m 2H), 4.97-4.87 (m, 5H), 4.73-4.70 (m, 1H), 4.26 (s, 2H), 3.67-3.64 (m, 2H), 3.23-3.20 (m, 2H), 2.72-2.68 (m, 2H), 2.52-2.51 (m, 2H).

### Example 50

### 2-amino-4-((R)-5-chloro-3-(((R)-2-methylenetetrahydro-1H-pyrrolizin-7a(5R)-yl)methoxy)-7,9-dihydrofuro[3,4-f]quinazolin-6-yl)-7-fluorobenzo[b]thiophene-3-carbonitrile 50-1-p1 or

### 2-amino-4-((S)-5-chloro-3-(((R)-2-methylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 50-1-p2 or

### 2-amino-4-((R)-5-chloro-3-(((S)-2-methylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 50-2-pl or

### 2-amino-4-((S)-5-chloro-3-(((S)-2-methylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 50-2-p2

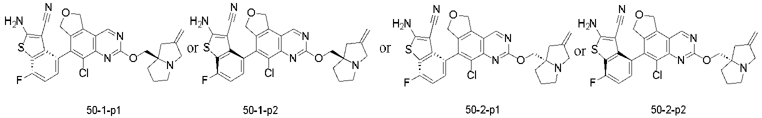

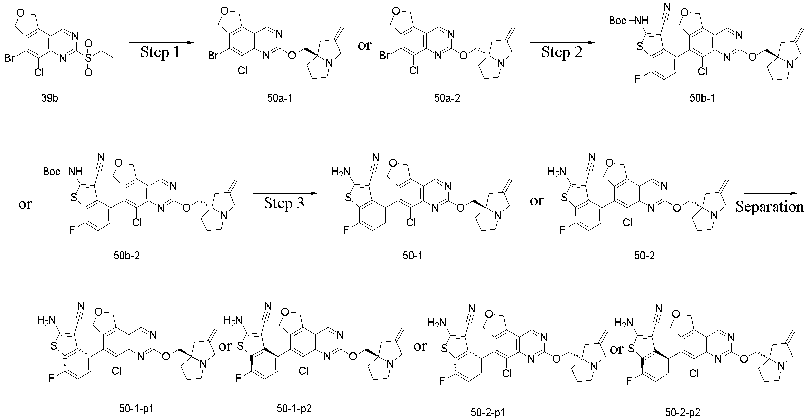

### Step 1

### (R)-6-bromo-5-chloro-3-((2-methylenetetrahydro-1H-pyrrolizin-7a(5H)-yl)methoxy)-7,9-dihydrofuro[3,4-f]quinazoline 50a-1 or

### (S)-6-bromo-5-chloro-3-((2-methylenetetrahydro-1H-pyrrolizin-7a(5R)-yl)methoxy)-7,9-dihydrofuro[3,4-f]quinazoline 50a-2

Compound **43c-1 or 43c-2** (365.15 mg, 2.38 mmol, prepared from the compound with the longer retention time between compound **43b-1** and compound **43b-2)** was dissolved in tetrahydrofuran (20 mL). A 2 M solution of sodium bis(trimethylsilyl)amide in tetrahydrofuran (1.43 mL) was added in an ice bath. After stirring at this temperature for 30 minutes, a solution of compound **39b** (600 mg, 1.58 mmol) in tetrahydrofuran (5 mL) was added in an ice bath. The mixture was reacted with stirring at this temperature for 1 hour. The reaction was quenched by the addition of saturated ammonium chloride solution. The mixture was extracted with ethyl acetate (15 mL × 2). The organic phases were combined and dried over anhydrous sodium sulfate, filtered, and the filtrate was concentrated under reduced pressure. The residue was purified by silica gel column chromatography using eluent system B to afford the title compound **50a-1 or 50a-2** (150 mg, yield: 21.6%).

MS m/z (ESI):436.2[M+1].

### Step 2

### tert-butyl (4-(5-chloro-3-(((R)-2-methylenetetrahydro-1H-pyrrolizin-7a(5H)-yl)methoxy)-7,9-dihydrofuro[3,4-f]quinazolin-6-yl)-3-cyano-7-fluorobenzo[b]thiophen-2-yl)carbamate 50b-1 or

### tert-butyl (4-(5-chloro-3-(((S)-2-methylenetetrahydro-1H-pyrrolizin-7a(5H)-yl)methoxy)-7,9-dihydrofuro[3,4-f] quinazolin-6-yl)-3-cyano-7-fluorobenzo[b]thiophen-2-yl)carbamate 50b-2

Compound **50a-1 or 50a-2** (150 mg, 343.4 µmol) and tert-butyl (3-cyano-4-(5,5-dimethyl-1,3,2-dioxaborinan-2-yl)-7-fluorobenzo[b]thiophen-2-yl)carbamate (277.6 mg, 686.7 µmol) were dissolved in 1,4-dioxane (20 mL). Caesium carbonate (335.7 mg, 1.03 mmol) and [1,1'-bis(diphenylphosphino)ferrocene] dichloropalladium(II) (50.2 mg, 68.6 µmol) were added. The mixture was reacted at 100°C for 2 hours under nitrogen atmosphere. The reaction mixture was concentrated under reduced pressure. The residue was dissolved in dichloromethane, and water was added. The aqueous phase was extracted with dichloromethane (15 mL × 2). The organic phases were combined and dried over anhydrous sodium sulfate, filtered to remove the drying agent, and concentrated under reduced pressure. The residue was purified by silica gel column chromatography using eluent system B to afford the title compound **50b-1** or **50b-2** (130 mg, yield: 57.4%).

MS m/z (ESI):648.5 [M+1].

### Step 3

### 2-amino-4-(5-chloro-3-(((R)-2-methylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 50-1 or

### 2-amino-4-(5-chloro-3-(((S)-2-methylenetetrahydro-1H-pyrrolizin-7a(5H)-yl)methoxy)-7,9-dihydrofuro[3,4-f]quinazolin-6-yl)-7-fluorobenzo[b]thiophene-3-carbonitrile 50-2

Compound **50b-1** or **50b-2** (230 mg, 354.8 µmol) was dissolved in dichloromethane (3 mL). Trifluoroacetic acid (3 mL) was added, and the mixture was reacted with stirring for 1 hour. The reaction mixture was concentrated under reduced pressure. The residue was dissolved in dichloromethane, and the pH was adjusted to > 7 with saturated sodium bicarbonate solution. The aqueous phase was extracted with dichloromethane (10 mL × 2). The organic phases were combined and dried over anhydrous sodium sulfate, filtered to remove the drying agent, and concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 32%-45%, flow rate: 30 mL/min), to afford the title compound **50-1 or 50-2** (70 mg, yield: 36%).

The isomer mixture **50-1 or 50-2** (70 mg) was separated by chiral column chromatography (Gilson-281, chromatographic column: CHIRALPAK IC, 20×250 mm, 5 µm; mobile phase A: n-hexane, mobile phase B: ethanol (0.5% 7 M ammonia-methanol solution), gradient ratio: A:B: 65:35, flow rate: 20 mL/min), and the compound with the shorter retention time was collected to afford **50-1-p1 or 50-1-p2 or 50-2-p1 or 50-2-p2** (23 mg, yield: 32.8%, also designated as 50A).

MS m/z (ESI):548.2[M+1].

Chiral HPLC analysis: retention time: 7.010 minutes, purity: 99% (chromatographic column: CHIRALPAK IE, 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, CD₃OD): δ 9.38 (s, 1H), 7.23 (dd, 1H), 7.06 (t, 1H), 5.62 (d, 2H), 5.01 - 4.82 (m, 5H), 4.55 - 4.35 (m, 2H), 3.85 (d, 1H), 3.04 - 2.78 (m, 2H), 2.57 (d, 1H), 2.23 (s, 1H), 2.12 - 1.86 (m, 4H).

### Example 51

### 2-amino-4-((R)-5-chloro-3-(((R)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-1-p1 or

### 2-amino-4-((S)-5-chloro-3-(((R)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-1-p2 or

### 2-amino-4-((R)-5-chloro-3-(((S)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-2-p1 or

### 2-amino-4-((S)-5-chloro-3-(((S)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-2-p2

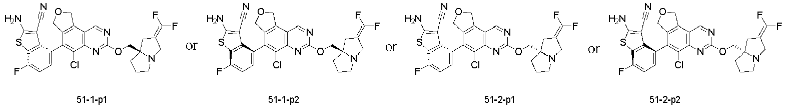

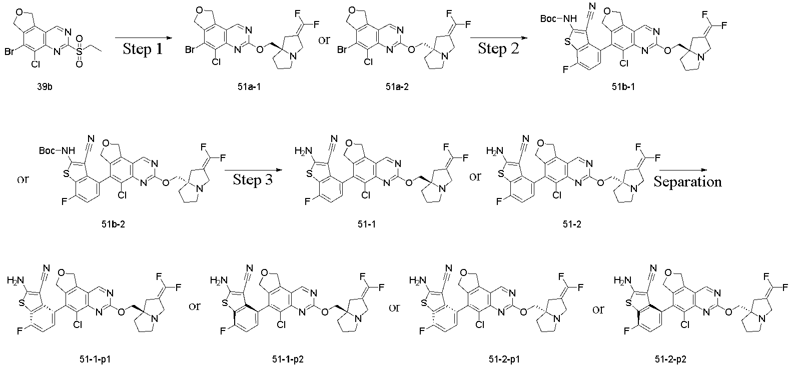

### Step 1

### (R)-6-bromo-5-chloro-3-((2-(difluoromethylene)tetrahydro-1H-pyrrolizin-7a(5H)-yl)methoxy)-7,9-dihydrofuro[3,4-f]quinazoline 51a-1 or

### (S)-6-bromo-5-chloro-3-((2-(difluoromethylene)tetrahydro-1H-pyrrolizin-7a(5H)-yl)methoxy)-7,9-dihydrofuro[3,4-f]quinazoline 51a-2

The compound with the shorter retention time between compound **44c-1** and compound **44c-2** (142 mg, 750.52 µmol) was dissolved in tetrahydrofuran (10 mL). A 2 M solution of sodium bis(trimethylsilyl)amide in tetrahydrofuran (373.5515 µL) was added in an ice bath. After stirring at this temperature for 30 minutes, a solution of compound **39b** (225 mg, 595.8 µL) in tetrahydrofuran (5 mL) was added in an ice bath. The mixture was warmed to 50°C and stirred for 1 hour. After the reaction mixture was cooled to room temperature, it was quenched by the addition of saturated ammonium chloride solution. The mixture was extracted with ethyl acetate (20 mL × 2). The organic phases were combined and dried over anhydrous sodium sulfate, filtered, and the filtrate was concentrated under reduced pressure. The residue was purified by silica gel column chromatography using eluent system B to afford the title compound **51a-1 or 51a-2** (176 mg, yield: 62.4%).

MS m/z (ESI):472.2[M+1].

### Step 2

### tert-butyl (4-(5-chloro-3-(((R)-2-(difluoromethylene)tetrahydro-1H-pyrrolizin-7a(5H)-yl)methoxy)-7,9-dihydrofuro[3,4-f]quinazolin-6-yl)-3-cyano-7-fluorobenzo[b]thiophen-2-yl)carbamate 51b-1 or

### tert-butyl (4-(5-chloro-3-(((S)-2-(difluoromethylene)tetrahydro-1H-pyrrolizin-7a(5H)-yl)methoxy)-7,9-dihydrofuro[3,4-f]quinazolin-6-yl)-3-cyano-7-fluorobenzo[b]thiophen-2-yl)carbamate 51b-2

Compound **51a-1** or **51a-2** (176 mg, 372.3 µmol) and tert-butyl (3-cyano-4-(5,5-dimethyl-1,3,2-dioxaborinan-2-yl)-7-fluorobenzo[b]thiophen-2-yl)carbamate (300 mg, 742.1 µmol) were dissolved in 1,4-dioxane (10 mL). Caesium carbonate (303 mg, 0.93 mmol) and [1,1'-bis(diphenylphosphino)ferrocene] dichloropalladium(II) (55 mg, 75.1 µmol) were added. The mixture was reacted at 100°C for 2 hours under nitrogen atmosphere. The reaction mixture was concentrated under reduced pressure. The residue was dissolved in dichloromethane, and water was added. The aqueous phase was extracted with dichloromethane (15 mL × 2). The organic phases were combined and dried over anhydrous sodium sulfate, filtered to remove the drying agent, and concentrated under reduced pressure. The residue was purified by silica gel column chromatography using eluent system B to afford the title compound **51b-1** or **51b-2** (211 mg, yield: 82.8%).

MS m/z (ESI):684.5 [M+1].

### Step 3

### 2-amino-4-(5-chloro-3-(((R)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-1 or

### 2-amino-4-(5-chloro-3-(((S)-2-(difluoromethylene)tetrahydro-1H-pyrrolizin-7a(5H)-yl)methoxy)- 7,9-dihydrofuro[3,4-f]quinazolin-6-yl)-7-fluorobenzo[b]thiophene-3-carbonitrile 51-2

### 2-amino-4-((R)-5-chloro-3-(((R)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-1-p1 or

### 2-amino-4-((S)-5-chloro-3-(((R)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-1-p2 or

### 2-amino-4-((R)-5-chloro-3-(((S)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-2-p1 or

### 2-amino-4-((S)-5-chloro-3-(((S)-2-(difluoromethylene)tetrahydro-1H-pyrrolizin-7a(5H)-yl)methoxy)-7,9-dihydrofuro[3,4-f]quinazolin-6-yl)-7-fluorobenzo[b]thiophene-3-carbonitrile 51-2-p2

Compound **51b-1** or **51b-2** (211 mg, 308.4 µmol) was dissolved in dichloromethane (5 mL). Trifluoroacetic acid (1 mL) was added, and the mixture was reacted with stirring for 1 hour. The reaction mixture was concentrated under reduced pressure. The residue was dissolved in dichloromethane, and the pH was adjusted to > 7 with saturated sodium bicarbonate solution. The aqueous phase was extracted with dichloromethane (10 mL × 2). The organic phases were combined and dried over anhydrous sodium sulfate, filtered to remove the drying agent, and concentrated under reduced pressure. The residue was purified by preparative high-performance liquid chromatography (Waters-2545, chromatographic column: YMC Triart-Exrs C18, 30×150 mm, 5 µm; mobile phase: aqueous phase (10 mmol/L ammonium bicarbonate) and acetonitrile, gradient ratio: acetonitrile 32%-45%, flow rate: 30 mL/min), to afford the title compound **51-1 or 51-2** (95 mg, yield: 52.7%).

The isomer mixture **51-1 or 51-2** (90 mg) was separated by chiral column chromatography (Gilson-281, chromatographic column: CHIRALPAK IG, 20×250 mm, 5 µm; mobile phase A: n-hexane, mobile phase B: ethanol (0.1% 7 M ammonia-methanol solution), gradient ratio: A : B: 70 : 30, flow rate: 20 mL/min), and the compound with the shorter retention time was collected to afford **51-1-p1** or **51-1-p2** or **51-2-p1** or **51-2-p2** (30 mg, yield: 33.3%, also designated as 51A).

MS m/z (ESI):584.2[M+1].

Chiral HPLC analysis: retention time: 6.434 minutes, purity: 99% (chromatographic column: CHIRALPAK IG 4.6*150 mm, 5 µm; mobile phase A: n-hexane, mobile phase B: ethanol (0.1% diethylamine), gradient ratio: A : B = 70 : 30, flow rate: 1 mL/min).

¹H NMR (500 MHz, DMSO-*d₆*): δ 9.49 (s, 1H), 8.10-8.08 (m, 2H), 7.26-7.24 (m, 1H), 7.17-7.13 (m, 1H), 5.56-5.55 (m, 2H), 4.90-4.87 (m, 1H), 4.74-4.71 (m, 1H), 4.38-4.36 (m, 1H), 4.24-4.22 (m, 1H), 3.71-3.68 (m, 1H), 3.32-3.31 (m, 1H), 3.04-3.00 (m, 1H), 2.72-2.69 (m, 1H), 2.59-2.55 (m, 1H), 2.48-2.45 (m, 1H), 2.06-2.00 (m, 1H), 1.92-1.78 (m, 3H).

### Example 52

### 2-amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[b]selenophen-3-carbonitrile 52

### (R)-2-amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[b]selenophen-3-carbonitrile 52-p1

### (S)-2-amino-4-(5-chloro-3-((1-((4-(fluoromethylene)piperidin-1-yl)methyl)cyclopropyl)methoxy)-7,9-dihydrofuro[3,4-f]quinazolin-6-yl)benzo[b]selenophen-3-carbonitrile 52-p2

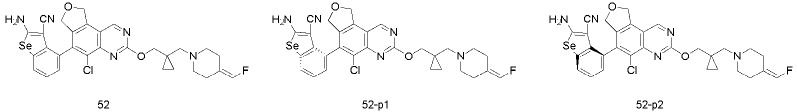

Following the synthetic route described in Example 14, the title compound 52 was obtained by replacing the starting material compound **14b** in step 2 with compound **39b,** and replacing tert-butyl (3-cyano-4-(5,5-dimethyl-1,3,2-dioxaborinan-2-yl)-7-fluorobenzo[*b*]thiophen-2-yl)carbamate in step 3 with tert-butyl (3-cyano-4-(5,5-dimethyl-1,3,2-dioxaborinan-2-yl)benzo[*b*]selenophen-2-yl)carbamate, followed by chiral column chromatography.

MS m/z (ESI): 624.4 [M+1].

### Example 53

### 2-amino-4-((R)-5-chloro-3-(((R)-2-(difluoromethylene)tetrahydro-1H-pyrrolizin-7a(5H)-yl)methoxy)-7,9-dihydrofuro[3,4-f]quinazolin-6-yl)benzo[b]selenophene-3-carbonitrile 53-1-p1

### 2-amino-4-((S)-5-chloro-3-(((R)-2-(difluoromethylene)tetrahydro-1H-pyrrolizin-7a(5H)-yl)methoxy)-7,9-dihydrofuro[3,4-f]quinazolin-6-yl)benzo[b]selenophene-3-carbonitrile 53-1-p2 or

### 2-amino-4-((R)-5-chloro-3-(((S)-2-(difluoromethylene)tetrahydro-1H-pyrrolizin-7a(5H)-yl)methoxy)-7,9-dihydrofuro[3,4-f]quinazolin-6-yl)benzo[b]selenophene-3-carbonitrile 53-2-pl

### 2-amino-4-((S)-5-chloro-3-(((S)-2-(difluoromethylene)tetrahydro-1H-pyrrolizin-7a(5H)-yl)methoxy)-7,9-dihydrofuro[3,4-f]quinazolin-6-yl)benzo[b]selenophene-3-carbonitrile 53-2-p2

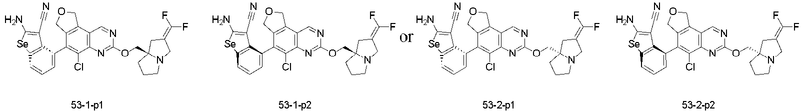

Following the synthetic route described in Example 14, the title compounds (60 mg, 23.3%), (30 mg, 11.6%) were obtained by replacing the starting material compound **1e** in step 2 with the compound having the shorter retention time between compound **44c-1** and compound **44c-2,** replacing compound **14b** with compound **39b,** and replacing tert-butyl (3-cyano-4-(5,5-dimethyl-1,3,2-dioxaborinan-2-yl)-7-fluorobenzo[*b*]thiophen-2-yl)carbamate in step 3 with tert-butyl (3-cyano-4-(5,5-dimethyl-1,3,2-dioxaborinan-2-yl)benzo[*b*]selenophen-2-yl)carbamate (prepared according to the method disclosed in Intermediate IN-1 on page 26 of the specification of patent application "WO 2023246903A1"), followed by chiral column chromatography (chromatographic column: Daicel CHIRALPAK IE, 20×250 mm, 5 µm; mobile phase A: n-hexane, mobile phase B: ethanol (0.5% ammonia-methanol solution), gradient ratio: A : B = 75 : 25, flow rate: 20 mL/min).

Single-configuration compound (shorter retention time, designated as 53A, i.e., 53-p1 or 53-p2): (60 mg, 23.3%).

Chiral HPLC analysis: retention time: 7.475 minutes, purity: 99% (chromatographic column: CHIRALPAK IE 150×4.6 mm, 5 µm; mobile phase: n-hexane and ethanol (containing 0.1% diethylamine), gradient ratio: A : B = 70 : 30, flow rate: 1.0 mL/min).

MS m/z (ESI): 614.4 [M+1].

¹H NMR (500 MHz, CD₃OD): δ 9.37 (s, 1H), 7.76 (dd, 1H), 7.41 - 6.78 (m, 2H), 5.62 (q, 2H), 4.96 - 4.93 (m, 1H), 4.91 - 4.89 (m, 1H), 4.58 (d, 1H), 4.42 (d, 1H), 3.86 (d, 1H), 3.68 - 3.40 (m, 1H), 3.19 (dt, 1H), 2.87 (d, 1H), 2.75 (q, 1H), 2.58 (d, 1H), 2.21 (dd, 1H), 2.14 - 1.79 (m, 3H).

Single-configuration compound (longer retention time, designated as 53B, i.e., 53-p2 or 53-p1): (30 mg, 11.6%).

Chiral HPLC analysis: retention time: 9.073 minutes, purity: 99% (chromatographic column: CHIRAL PAK IC 150×4.6 mm, 5 µm; mobile phase: n-hexane and ethanol (containing 0.1% diethylamine), gradient ratio: A:B=70:30, flow rate: 1.0 mL/min).

MS m/z (ESI): 614.4 [M+1].

¹H NMR (500 MHz, CD₃OD): δ 9.36 (s, 1H), 7.76 (dd, 1H), 7.56 - 6.86 (m, 2H), 5.62 (q, 2H), 5.04 - 4.91 (m, 1H), 4.89 - 4.80 (m, 2H), 4.54 (d, 1H), 4.45 (d, 1H), 3.86 (d, 1H), 3.58 - 3.43 (m, 1H), 3.19 (dt, 1H), 2.88 (d, 1H), 2.79 - 2.71 (m, 1H), 2.58 (d, 1H), 2.39 - 2.14 (m, 1H), 2.09 - 1.74 (m, 2H).

### Example 54

### 2-amino-4-(3-(((S)-2-(difluoromethylene)tetrahydro-1H-pyrrolizin-7a(5H)-yl)methoxy)-5-fluoro-1-(3-((R)-2-hydroxypropyl)-3,8-diazabicyclo[3.2.1]oct-8-yl)-7,9-dihydrofuro[3,4-f]quinazolin-6-yl)-7-fluorothieno[3,2-c]pyridine-3-carbonitrile 54-1 or

### 2-amino-4-(3-(((R)-2-(difluoromethylene)tetrahydro-1H-pyrrolizin-7a(5H)-yl)methoxy)-5-fluoro-1-(3-((R)-2-hydroxypropyl)-3,8-diazabicyclo[3.2.1]oct-8-yl)-7,9-dihydrofuro[3,4-f]quinazolin-6-yl)-7-fluorothieno[3,2-c]pyridine-3-carbonitrile 54-2

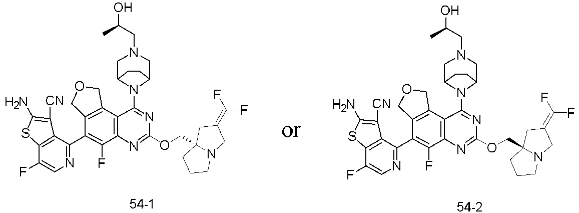

Following the synthetic route described in Example 2, the title compound **54-1 or 54-2** (100 mg, 23.3%) was obtained by replacing the starting material compound **2c** in step 3 with the compound having the shorter retention time between compound **44c-1** and compound **44c-2.**

MS m/z (ESI): 737.5 [M+1].

¹H NMR (500 MHz, CD₃OD): δ 8.31 (s, 1H), 5.56 (s, 2H), 5.14 (d, 1H), 4.79 (dt, 1H), 4.46 - 4.18 (m, 4H), 4.08 - 3.92 (m, 1H), 3.82 (d, 1H), 3.54 - 3.42 (m, 2H), 3.18 - 3.11 (m, 1H), 2.96 - 2.80 (m, 3H), 2.73 - 2.59 (m, 3H), 2.58 - 2.31 (m, 3H), 2.15 (t, J = 4.3 Hz, 1H), 2.05 (d, 3H), 2.00 - 1.88 (m, 3H), 1.21 (d, 3H).

### Example 55

### 4-(1-(3-acryloyl-3,8-diazabicyclo[3.2.1]oct-8-yl)-3-((1-(4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-c]pyridine-3-carbonitrile 55

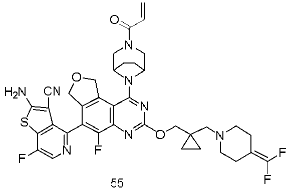

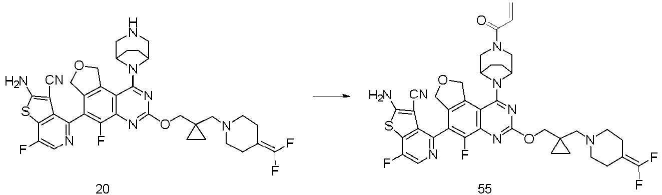

Compound **20** (2 g, 2.83 mmol) and potassium carbonate (1.2 g, 8.68 mmol) were suspended in tetrahydrofuran (10 mL), ethyl acetate (10 mL), and water (10 mL). Acryloyl chloride (310 mg, 3.42 mmol) was added in an ice bath. The mixture was reacted with stirring at this temperature for 30 minutes. Water was added to the reaction mixture, followed by extraction with ethyl acetate (30 mL × 2). The organic phases were combined and concentrated under reduced pressure. The residue was purified by preparative high-performance liquid chromatography (Waters-2545, chromatographic column: Boston phlex prep C18, 30×150 mm, 5 um; mobile phase: aqueous phase (10 mmol/L ammonium bicarbonate) and acetonitrile, gradient ratio: acetonitrile 30%-50%, flow rate: 30 mL/min), to afford the title compound 55 (500 mg, yield: 23.2%).

MS m/z (ESI): 761.7 [M+1].

¹H NMR (500 MHz, CD₃OD): δ 8.32 (s, 1H), 6.81 (dd, 1H), 6.29 (dd, 1H), 5.81 (dd, 1H), 5.60 (s, 2H), 5.21 - 5.08 (m, 1H), 4.82 - 4.73 (m, 1H), 4.62 - 4.34 (m, 6H), 4.06 (d, 1H), 3.78 (t, 1H), 2.51 (d, 5H), 2.20 (d, 3H), 2.07 (d, 2H), 1.89 - 1.71 (m, 2H), 1.33 (d, 2H), 0.74 (d, 2H), 0.51 (d, 2H).

### Example 56

### 4-(1-((2S,5R)-4-acryloyl-2,5-dimethylpiperazin-1-yl)-3-((1-(4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-c]pyridine-3-carbonitrile 56

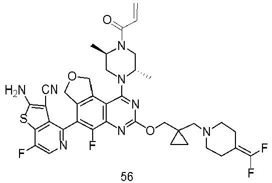

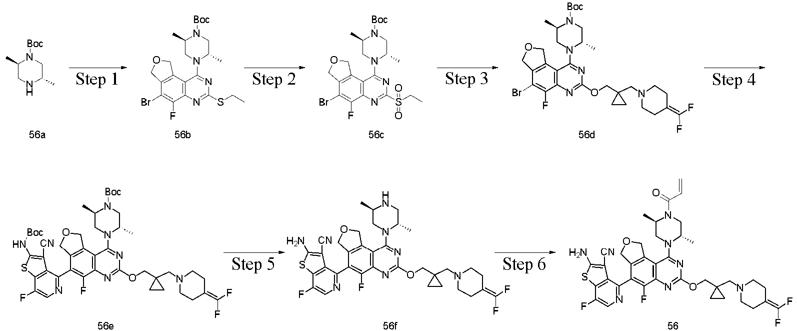

### Step 1

### tert-butyl (2R,5S)-4-(6-bromo-3-(ethylthio)-5-fluoro-7,9-dihydrofuro[3,4-f]quinazolin-1-yl)-2,5-dimethylpiperazine-1-carboxylate 56b

Compound **35a** (10 g, 37.5 mmol) and N,N-diisopropylethylamine (8.88 g, 68.75 mmol) were dissolved in dichloromethane (100 mL). tert-Butyl (2*R*,5*S*)-2,5-dimethylpiperazine-1-carboxylate **56a** (6.5 g, 30.33 mmol, from Bide Pharmatech Ltd.) was added in an ice bath. The mixture was allowed to warm to room temperature and reacted with stirring for 17 hours. Saturated ammonium chloride solution was added to the reaction mixture, followed by extraction with dichloromethane (100 mL × 3). The organic phases were combined and concentrated under reduced pressure. The residue was purified by silica gel column chromatography using eluent system B to afford the title compound **56b** (14.8 g, yield: 99%).

MS m/z (ESI): 541.2 [M+1].

### Step 2

### tert-butyl (2R,5S)-4-(6-bromo-3-(ethylsulfonyl)-5-fluoro-7,9-dihydrofuro[3,4-f]quinazolin-1-yl)-2,5-dimethylpiperazine-1-carboxylate 56c

Compound **56b** (14.8 g, 27.33 mmol) was dissolved in dichloromethane (100 mL). meta-Chloroperoxybenzoic acid (14 g, 68.96 mmol, 85% purity) was added in an ice bath, and the mixture was allowed to warm to room temperature and stirred for 1 hour. The reaction was quenched by the addition of aqueous saturated sodium bicarbonate solution and aqueous saturated sodium sulfite solution. The aqueous phase was extracted with dichloromethane (100 mL × 3). The organic phases were combined and dried over anhydrous sodium sulfate, filtered, and the filtrate was concentrated under reduced pressure to afford the crude title compound **56c** (15.6 g), which was used directly in the next step without purification.

MS m/z (ESI): 573.3 [M+1].

### Step 3

### tert-butyl (2R,5S)-4-(6-bromo-3-(1-((4-(difluoromethylene)piperidin-1-yl)methyl)cyclopropyl)methoxy)-5-fluoro-7,9-dihydrofuro[3,4-f]quinazolin-1-yl)-2,5-dimethylpiperazine-1-carboxylate 56d

Compound **2c** (1.3 g, 5.98 mmol) and compound **56c** (3 g, 5.23 mmol) were dissolved in tetrahydrofuran (50 mL). A 1 M solution of lithium bis(trimethylsilyl)amide in tetrahydrofuran (6.6 mL) was added in an ice bath. The mixture was allowed to warm to room temperature and reacted with stirring for 1 hour. The reaction was quenched by adding saturated ammonium chloride solution, and the mixture was extracted with ethyl acetate (50 mL × 2). The organic phases were combined and dried over anhydrous sodium sulfate, filtered to remove the drying agent, and concentrated under reduced pressure. The residue was purified by silica gel column chromatography using eluent system B to afford the title compound **56d** (2.98 g, yield: 81.7%).

MS m/z (ESI): 696.5 [M+1].

### Step 4

### tert-butyl (2R,5S)-4-(6-(2-((tert-butoxycarbonyl)amino)-3-cyano-7-fluorothieno[3,2-c]pyridin-4-yl)-3-((1-((4-(difluoromethylene)piperidin-1-yl)methyl)cyclopropyl)methoxy)-5-fluoro-7,9-dihydrofuro[3,4-f]quinazolin-1-yl)-2,5-dimethylpiperazine-1-carboxylate 56e

Compound **56d** (1 g, 1.43 mmol) and neopentyl glycol diboronate (487 mg, 2.15 mmol) were dissolved in 1,4-dioxane (20 mL). Potassium acetate (494 mg, 5.03 mmol) and [1,1'-bis(diphenylphosphino)ferrocene]dichloropalladium(II) (103 mg, 143.8 µmol) were added. The mixture was reacted at 95°C for 2 hours under nitrogen atmosphere. After the reaction mixture was cooled to room temperature, water (5 mL), tert-butyl (4-chloro-3-cyano-7-fluorothieno[3,2-*c*]pyridin-2-yl)carbamate (424 mg, 1.29 mmol), methanesulfonato(2-dicyclohexylphosphino-2',4',6'-triisopropyl-1,1'-biphenyl)(2'-methylamino-1,1'-biphen-2-yl)palladium(II) (124 mg, 144.2 µmol), and potassium phosphate (305 mg, 1.43 mmol) were added. The mixture was reacted at 85°C for 1 hour under nitrogen atmosphere. After the reaction mixture was cooled to room temperature, it was filtered, and the filtrate was concentrated under reduced pressure. The residue was purified by silica gel column chromatography using eluent system B to afford the title compound **56e** (1.09 g, yield: 83.5%).

MS m/z (ESI):909.7[M+1].

### Step 5

### 2-amino-4-(3-((1-((4-(difluoromethylene)piperidin-1-yl)methyl)cyclopropyl)methoxy)-1-((2S,5R)-2,5-dimethylpiperazin-1-yl)-5-fluoro-7,9-dihydrofuro[3,4-f]quinazolin-6-yl)-7-fluorothieno[3,2-c]pyridine-3-carbonitrile 56f

Compound **56e** (1.09 g, 1.2 mmol) was dissolved in dichloromethane (10 mL). Trifluoroacetic acid (10 mL) was added in an ice bath. The mixture was allowed to warm to room temperature and reacted with stirring for 2 hours. The reaction mixture was concentrated under reduced pressure. The residue was dissolved in dichloromethane, and the pH was adjusted to > 7 with saturated sodium bicarbonate solution. The aqueous phase was extracted with dichloromethane (15 mL × 2). The organic phases were combined and dried over anhydrous sodium sulfate, filtered to remove the drying agent, and concentrated under reduced pressure to afford the crude title compound **56f** (780 mg). The product was used directly in the next step without purification.

MS m/z (ESI):709.5 [M+1].

### Step 6

### 4-(1-((2S,5R)-4-acryloyl-2,5-dimethylpiperazin-1-yl)-3-((1-((4-(difluoromethylene)piperidin-1-yl)methyl)cyclopropyl)methoxy)-5-fluoro-7,9-dihydrofuro[3,4-f]quinazolin-6-yl)-2-amino-7-fluorothieno[3,2-c]pyridine-3-carbonitrile 56

Compound **56f** (204 mg, 287.82 µmol) and potassium carbonate (120 mg, 868.27 µmol) were suspended tetrahydrofuran (2 mL), ethyl acetate (2 mL), and water (2 mL). Acryloyl chloride (32 mg, 353.56 µmol) was added in an ice bath. The mixture was reacted with stirring at this temperature for 30 minutes. Saturated sodium bicarbonate solution was added to the reaction mixture, followed by extraction with ethyl acetate (10 mL × 2). The organic phases were combined and concentrated under reduced pressure. The residue was purified by preparative high-performance liquid chromatography (Waters-2545, chromatographic column: Boston phlex prep C18, 30×150 mm, 5 um; mobile phase: aqueous phase (10 mmol/L ammonium bicarbonate) and acetonitrile, gradient ratio: acetonitrile 30%-55%, flow rate: 30 mL/min) to afford the title compound **56** (50 mg, yield: 22.7%).

MS m/z (ESI): 763.6 [M+1].

¹H NMR (500 MHz, CD₃OD): δ 8.32 (s, 1H), 6.83-6.78(m, 1H), 6.28 (dd, 1H), 5.81 (ddd, 1H), 5.58 (dd, 2H), 5.45 (dd, 2H), 5.12 (t, 2H), 4.72 (d, 1H), 4.60 (s, 1H), 4.51 (dd, 2H), 4.44 (t, 2H), 3.43 (d, 2H), 2.44 (dd, 2H), 2.29-2.14 (m, 7H), 2.09-2.01 (m, 1H), 1.69-1.57 (m, 1H), 0.92 (t, 1H), 0.75-0.74 (m, 3H), 0.51-0.50 (m, 3H).

### Examples 57, 58 and 59 (reference)

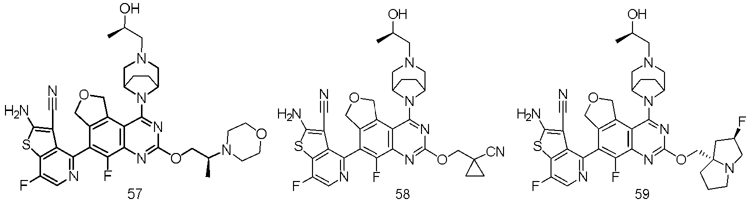

Examples 57, 58, and 59 were prepared as reference compounds with reference to the methods described on pages 446, 447, 435, and 429 of WO 2023183585 A1.

### Example 60 (reference)

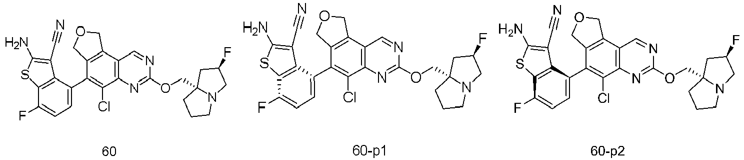

Following the synthetic route described in Example 51, the crude compound **60** (1.35 g) was obtained by replacing the compound with the shorter retention time between compound **44c-1** and compound **44c-2** in step 1 with ((2*R*,7*aS*)-2-fluorotetrahydro-1*H*-pyrrolizin-7*a*-yl)methanol. The crude product was purified by preparative high-performance liquid chromatography (Waters-2545, chromatographic column: Boston phlex prep C18, 30×150 mm, 5 um; mobile phase: aqueous phase (10 mmol/L ammonium bicarbonate) and acetonitrile, gradient ratio: acetonitrile 30%-55%, flow rate: 30 mL/min), to afford compounds (300 mg, yield: 22.2%), (300 mg, yield: 22.2%).

Single-configuration compound (shorter retention time, designated as 60A, i.e., **60-pl or 60-p2):** (300 mg, yield: 22.2%).

HPLC analysis: retention time: 2.72 minutes, purity: 99% (chromatographic column: Xtimate, C18, 1.8 µm, 2.1× 50 mm; mobile phase: water (10 mM ammonium bicarbonate) and acetonitrile, gradient ratio: acetonitrile 10%-95%).

MS m/z (ESI): 554.5[M+1].

¹H NMR (500 MHz, CD₃OD):δ 9.37 (s, 1H), 7.22 (dd, 1H), 7.06 (t, 1H), 5.62 (q, 2H), 5.44 - 5.20 (m, 1H), 4.98 (dt, 1H), 4.85 - 4.77 (m, 1H), 4.49 (d, 1H), 4.40 (d, 1H), 3.28 - 3.15 (m, 2H), 3.09 - 3.01 (m, 1H), 2.56 - 2.35 (m, 1H), 2.35 - 2.16 (m, 3H), 2.14 - 1.88 (m, 3H).

Single-configuration compound (longer retention time, designated as 60B, i.e., **60-p2 or 60-p1**): (300 mg, yield: 22.2%).

HPLC analysis: retention time: 2.78 minutes, purity: 96% (chromatographic column: Xtimate, C18, 1.8 µm, 2.1×50 mm; mobile phase: water (10 mM ammonium bicarbonate) and acetonitrile, gradient ratio: acetonitrile 10%-95%).

### MS m/z (ESI): 554.5[M+1].

¹H NMR (500 MHz, CD₃OD): δ9.37 (s, 1H), 7.22 (dd, 1H), 7.06 (dd, 1H), 5.62 (q, 2H), 5.46 - 5.22 (m, 1H), 4.97 (dt, 1H), 4.82 (dt, 1H), 4.47 (d, 1H), 4.41 (d, 1H), 3.29 - 3.19 (m, 2H), 3.05 (td, 1H), 2.46 - 2.16 (m, 4H), 2.08 - 1.89 (m, 3H).

### Biological evaluation

### Test example 1: 3D cell proliferation inhibition assay

This experiment uses the cells listed in the table below to evaluate the inhibitory effects of the compounds on the proliferation of KRAS mutant or KRAS amplified cells.

| Cells | KRAS mutation | Seeding density Number of cells/well | Culture medium | Culture conditions |
|---|---|---|---|---|
| GP2d | G12D | 1000 | DMEM/HIGH GLUCOSE, 10% FBS | 37°C, 5% CO₂ |
| AsPC-1 | G12D | 1500 | RPMI, 10% FBS | 37°C, 5% CO₂ |
| AGS | G12D | 1000 | RPMI, 10% FBS | 37°C, 5% CO₂ |
| MKN1 | Amp | 1000 | RPMI, 10% FBS | 37°C, 5% CO₂ |
| SW620 | G12V | 1000 | Leibovitz's L-15, 10% FBS | 37°C, CO₂-free |
| H358 | G12C | 1200 | RPMI, 10% FBS | 37°C, 5% CO₂ |
| MIA PaCa-2 | G12C | 1000 | DMEM/HIGH GLUCOSE, 10% FBS, 2.5% horse serum | 37°C, 5% CO₂ |

On the first day of the experiment, cells that were growing well and had reached 70%-80% confluence were digested, resuspended in culture medium, and the cell density was adjusted to the desired level. In a U-shaped low-attachment 96-well plate (Corning, 7007), 90 µL of cell suspension was added per well, with the cell density as shown in the table above. The cell plate was centrifuged at 2500 rpm for 5 minutes and then placed in an incubator for 24 hours of culture. On the second day, the 20 mM test compound dissolved in DMSO was diluted with DMSO to the first concentration of 2 or 0.2 mM, and then serially diluted in a 5-fold gradient to obtain 9 concentration points, with the control well containing DMSO. The serially diluted compound was then further diluted 20-fold with culture medium. 10 µL of the test compound diluted in culture medium was added to each well of the cell plate. The final concentrations of the compound were 9 points in a 5-fold serial dilution starting from 10 or 1 µM. Cell wells containing 0.5% DMSO were set as vehicle control wells, and wells containing only culture medium and 0.5% DMSO were set as blank control wells. The cell plate was centrifuged at 2000 rpm for 3 minutes and then placed in an incubator for 5 days of culture. On the seventh day, the 96-well cell culture plate was taken out, and 50 µL of the luminescent cell viability detection reagent, CellTiter-Glo^{®} 3D Cell Viability Assay (Promega, G9683), was added to each well. After shaking at room temperature for 25 minutes, the mixture was pipetted up and down to mix, and 100 µL was transferred from each well to a white, opaque-bottom OptiPlate^{™}-96 well plate (PerkinElmer, 6005290). The luminescence signal value was read using a multimode microplate reader (PerkinElmer, EnVision2105).

The inhibition rate was calculated using the following formula: Inhibition rate = (Luminescence value _{vehicle control well} - Luminescence value _{test compound})/(Luminescence value _{vehicle control well} - Luminescence value _{blank control well}) × 100%. The IC₅₀ values of the compound inhibitory activity were calculated using Graphpad Prism software. The results are shown in Table 1 below.

**Table 1. Data of inhibitory activity on 3D cell proliferation (unit: nM)**

| Example No. | GP2d/IC₅₀ | AGS/IC₅₀ | AsPC-1/IC₅₀ | SW620/IC₅₀ | MKN1/IC₅₀ | H358/IC₅₀ |
|---|---|---|---|---|---|---|
| 1 | 0.28 | 1.03 | 4.17 | - | - | - |
| 1i | 0.34 | 1.03 | 5.07 | - | - | - |
| 2 | 1.79 | 2.36 | 6.18 | - | - | - |
| 9 | 0.26 | 1.09 | 9.95 | - | - | - |
| 9A | 0.16 | 4.22 | - | - | - | - |
| 9B | 2.60 | - | - | - | - | - |
| 11 | 0.86 | 8.64 | - | - | - | - |
| 11A | 0.39 | 5.41 | 12.81 | - | - | - |
| 11B | 3.38 | - | - | - | - | - |
| 13 | 0.39 | 2.19 | - | - | - | - |
| 14 | 5.85 | 15.14 | - | - | 8.1 | - |
| 14A | 2.28 | 4.64 | 14.15 | 10.91 | 3.4 | 5.86 |
| 15 | 1.71 | 9.12 | 14.74 | 7.9 | 6.9 | - |
| 17 | 0.57 | 2.86 | - | - | - | - |
| 18 | 0.53 | 4.89 | - | - | - | - |
| 20 | 0.27 | 2.09 | 7.56 | - | - | - |
| 21 | 2.12 | - | - | - | - | - |
| 22 | 0.26 | 1.57 | 4.64 | - | - | - |
| 26 | 9.79 | - | - | - | 13.16 | - |
| 26A | 3.93 | 11.27 | - | 7.4 | 3.1 | 4.91 |
| 27 | 6.61 | - | - | 12.4 | 8.2 | 8.85 |
| 28 | - | - | - | - | - | - |
| 28A | 5.24 | 11.46 | - | - | 4.6 | 5.16 |
| 36 | - | - | - | 10.12 | - | 8.04 |
| 39 | 2.95 | 4.07 | 7.65 | 12.70 | 4.0 | 4.76 |
| 39A | 2.33 | 4.10 | 7.59 | 7.7 | 2.7 | 4.46 |
| 40 | 8.4 | - | - | - | 11.08 | - |
| 43A | 0.36 | 0.7 | 1.68 | 0.1-3 | 0.1-3 | 0.1-3 |
| 44A | 2.59 | 5.03 | 8.36 | 3.4 | 1.3 | 2.78 |
| 48A | 5.87 | 13.41 | - | - | 6.01 | 8.63 |
| 49A | 1.80 | 3.58 | 10.86 | 9.07 | 4.47 | 3.32 |
| 50A | 0.41 | 0.55 | 0.1-2 | 0.1-3 | 0.1-3 | 1.35 |
| 51A | 4.57 | 7.78 | 15.57 | 6.4 | 3.2 | 5.16 |
| 55 | - | - | - | - | - | 0.63 |
| 56 | - | - | - | - | - | 6.76 |
| 57 (reference) | 282.6 | 160.4 | 373 | - | - | - |
| 58 (reference) | >1000 | 1995 | 3333 | - | - | - |

Conclusion: The compounds of the present disclosure exhibit good inhibitory effects on the 3D proliferation of the aforementioned cells.

### Test example 2: Pharmacokinetic evaluation

### I. Mouse experiment

### 1. Abstract

Using balb/c nude mice as the test animals, the plasma concentrations of the example compounds at different time points were determined by LC-MS/MS following oral gavage (i.g.) administration. The pharmacokinetic behaviours of the compounds of the present disclosure in mice were investigated, and their pharmacokinetic profiles were evaluated.

### 2. Experimental Protocol

### 2.1 Experimental compounds

### Compound 2.

### 2.2 Experimental animals

Nine balb/c nude mice, female, were provided by Beijing Vital River Laboratory Animal Technology Co., Ltd. (Production License Number: SCXK(Zhejiang) 2024-0001).

### 2.3 Drug preparation

A given amount of the test compound was weighed and formulated with 5% DMF + 45% PG + 50% (10% HS15-pH7.4 buffer) to prepare a 2.0 mg/mL clear and colourless solution.

### 2.4. Administration

The administration dose was 20.0 mg/kg, and the administration volume was 10 mL/kg.

### 3. Procedures

Blood samples (0.1 ml) were collected from the orbit before administration and at 0.25, 0.5, 1.0, 2.0, 4.0, 6.0, 8.0, 11.0, and 24.0 hours after administration. The samples were placed in EDTA-K2 anticoagulant tubes, centrifuged at 10,000 rpm for 1 minute (4°C), and the plasma was separated within 1 hour and stored at -20°C or -80°C until analysis. The entire process from blood collection to centrifugation was performed in ice bath conditions. Food was provided 2 hours after administration.

The concentration of the test compound in mouse plasma after administration was determined as follows: 20 µL of the plasma sample collected at each time point after administration was taken, and 200 µL of internal standard acetonitrile solution (containing 20 ng/mL verapamil) was added. The mixture was vortexed and then centrifuged at 4000 rpm for 15 minutes; the supernatant was taken for LC-MS/MS analysis.

### 4. Pharmacokinetic parameter results

**Table 2. Pharmacokinetic parameters of compounds of the present disclosure in balb/c nude mice**

| Compound No. | Plasma concentration Cmax (ng/mL) | Area under the curve AUC₀₋ₜ (h*ng/mL) | Half life T1/2 (h) | Clearance CL/F (mL/min/kg) | Apparent volume of distribution Vz/F (mL/kg) |
|---|---|---|---|---|---|
| 2 | 1061 | 9099 | 2.87 | 36.5 | 9073 |

Conclusion: The compounds of the present disclosure exhibit higher plasma concentrations and greater exposure in balb/c nude mice, demonstrating pharmacokinetic advantages.

### II. Rat experiment

### 1. Abstract

Using SD rats as the test animals, the plasma concentrations of the example compounds at different time points were determined by LC-MS/MS following oral gavage (i.g.) administration. The pharmacokinetic behaviours of the compounds of the present disclosure in rats were investigated, and their pharmacokinetic profiles were evaluated.

### 2. Experimental Protocol

### 2.1. Experimental compounds

### Compounds 2, 51A, 59 and 60A.

### 2.2. Experimental animals

Compound 2: eight rats, half male and half female, were provided by Beijing Vital River Laboratory Animal Technology Co., Ltd. (Production License Numbers: SCXK(Beijing) 2021-0006, SCXK(Zhejiang) 2024-0001). Compound 51A (20 mg/kg): two female rats, were provided by Beijing Vital River Laboratory Animal Technology Co., Ltd. (Production License Number: SCXK(Zhejiang) 2024-0001). Compound 51A (60 mg/kg): two female rats, were provided by Beijing Vital River Laboratory Animal Technology Co., Ltd. (Production License Number: SCXK(Zhejiang) 2024-0001). Compound 59: four rats, half male and half female, were provided by Beijing Vital River Laboratory Animal Technology Co., Ltd. (Production License Number: SCXK(Zhejiang) 2024-0001). Compound 60A: two female rats, were provided by Beijing Vital River Laboratory Animal Technology Co., Ltd. (Production License Number: SCXK(Zhejiang) 2024-0001).

### 2.3. Drug preparation

Compound 2: a given amount of the test compound was weighed and formulated with 5% DMSO + 10% HS15 + 17% (w/v) SBE-β-CD to prepare a 4.0 mg/mL (40 mpk) clear and colourless solution; same was formulated with 0.5% CMC-Na to prepare a 2.0 mg/mL (20 mpk) white suspension.

Compound 51A: a given amount of the test compound was weighed and formulated with 5% DMSO + 10% HS15 + 17% (w/v) SBE-β-CD to prepare 2.0 mg/mL and 6.0 mg/mL clear and colourless solutions.

Compound 59: a given amount of the test compound was weighed and formulated with 0.5% CMC-Na to prepare a 2.0 mg/mL white suspension. Compound 60A: a given amount of the test compound was weighed and formulated with 5% DMSO + 10% HS15 + 17% (w/v) SBE-β-CD to prepare a 2.0 mg/mL yellow suspension.

### 2.4. Administration

Compound 2: administration dose: 20.0 mg/kg and 40.0 mg/kg, with an administration volume of 10 mL/kg for both.

Compound 51A: administration dose: 20.0 mg/kg and 60 mg/kg, with an administration volume of 10 mL/kg for both.

Compound 59: administration dose: 20.0 mg/kg, with an administration volume of 10 mL/kg.

Compound 60A: administration dose: 20.0 mg/kg, with an administration volume of 10 mL/kg.

### 3. Procedures

Blood samples (0.2 mL) were collected from the orbit before administration and at 0.25, 0.5, 1.0, 2.0, 4.0, 6.0, 8.0, 11.0, and 24.0 hours after administration. The samples were placed in EDTA-K2 anticoagulant tubes, centrifuged at 10,000 rpm for 2 minute (4°C), and the plasma was separated within 1 hour and stored at -20°C or -80°C until analysis. The entire process from blood collection to centrifugation was performed in ice bath conditions.

Determination of test compound contents in rat plasma after administration of different compounds:
Compound 2 (20 mpk): 20 µL of the rat plasma sample collected at each time point after administration was taken, and 250 µL of acetonitrile solution (containing 100 ng/ml verapamil) was added. The mixture was vortexed and then centrifuged at 4000 rpm for 15 minutes; the supernatant was taken for LC-MS/MS analysis. Compound 2 (40 mpk): 50 µL of the rat plasma sample collected at each time point after administration was taken, and 25 µL of internal standard acetonitrile solution (containing 1000 ng/mL camptothecin) was added, followed by the addition of 450 µL of acetonitrile solution. The mixture was vortexed and then centrifuged at 3700 rpm for 10 minutes; the supernatant was taken for LC-MS/MS analysis.

For compound 51A (20 mpk), 25 µL of the rat plasma sample collected at each time point after administration was taken, and 200 µL of internal standard acetonitrile solution (containing 100 ng/mL camptothecin) was added. The mixture was vortexed and then centrifuged at 4000 rpm for 15 minutes; the supernatant was taken for LC-MS/MS analysis. For compound 51A (60 mpk), 20 µL of the rat plasma sample collected at each time point after administration was taken, and 250 µL of internal standard acetonitrile solution (containing 100 ng/mL camptothecin) was added. The mixture was vortexed and then centrifuged at 4000 rpm for 15 minutes; the supernatant was taken for LC-MS/MS analysis.

Compound 59: 20 µL of the rat plasma sample collected at each time point after administration was taken, and 250 µL of acetonitrile solution (containing 100 ng/ml camptothecin) was added. The mixture was vortexed and then centrifuged at 4000 rpm for 15 minutes; the supernatant was taken for LC-MS/MS analysis. Compound 60A: 20 µL of the rat plasma sample collected at each time point after administration was taken, and 250 µL of internal standard acetonitrile solution (containing 10 ng/mL verapamil) was added. The mixture was vortexed and then centrifuged at 4000 rpm for 15 minutes; the supernatant was taken for LC-MS/MS analysis.

### 4. Pharmacokinetic parameter results

**Table 3. Pharmacokinetic parameters of compounds of the present disclosure in rats**

| Compound No. | Administration dose (mg/kg) | Plasma concentration Cmax (ng/mL) | Area under the curve AUC₀₋ₜ (h*ng/mL) | Half life T1/2 (h) | Clearance CL/F (mL/min/kg) | Apparent volume of distribution Vz/F (mL/kg) |
|---|---|---|---|---|---|---|
| 2 | 20 | 377 | 5169 | 5.52 | 65.5 | 27995 |
| | 40 | 1168 | 17617 | 6.7 | 36.7 | 19641 |
| 51A | 20 | 495 | 5642 | 2.63 | 58.8 | 13427 |
| | 60 | 1873 | 25420 | / | / | / |
| 59 (reference) | 20 | 55.5 | 327 | 4.93 | 1037 | 438200 |
| 60A (reference) | 20 | 143 | 1818 | 3.53 | 178 | 54544 |

Conclusion: The compounds of the present disclosure exhibit higher plasma concentrations and greater exposure in rats, demonstrating pharmacokinetic advantages.

## Claims

1. A compound represented by general formula (I') or a pharmaceutically acceptable salt thereof: 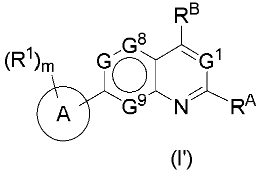 wherein:
R^{A} is selected from the group consisting of 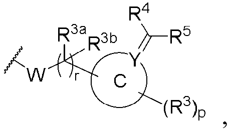 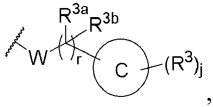 hydrogen atom, halogen, alkyl, alkoxy, haloalkyl, haloalkoxy, cyano, NR¹¹R¹², hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R³;
Y is carbon atom; ring C is selected from the group consisting of cycloalkyl, heterocyclyl, aryl and heteroaryl;
ring A is selected from the group consisting of cycloalkyl, heterocyclyl, aryl and heteroaryl;
G¹ is CR^{G1} or N; G is selected from the group consisting of C, CR^{G} and N;
G⁸ is CR⁷ or N; G⁹ is CR² or N;
R^{B} is selected from the group consisting of hydrogen atom, halogen, alkyl, alkoxy, haloalkyl, haloalkoxy, cyano, NR¹¹R¹², hydroxyl, hydroxyalkyl, alkenyl, alkynyl, OR¹⁴, C(O)OR¹⁴, S(O)ᵥR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁶;
R², R^{G1}, R^{G} and R⁷ are the same or different, and are each independently selected from the group consisting of hydrogen atom, halogen, alkyl, alkoxy, haloalkyl, haloalkoxy, cyano, hydroxyl, hydroxyalkyl, alkenyl, alkynyl, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR¹⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, G and R⁷, together with the carbon atoms to which they are attached, form ring B; ring B is optionally substituted with one or more R⁸;
ring B is selected from the group consisting of cycloalkyl, heterocyclyl, aryl and heteroaryl;
R⁴ and R⁵ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, NR¹¹R¹², C(O)R¹⁴, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R⁰¹;
or, R⁴ and R⁵, together with the carbon atoms to which they are attached, form cycloalkyl or heterocyclyl, wherein the cycloalkyl and heterocyclyl are each independently and optionally substituted with one or more R³;
W is selected from the group consisting of CR^{3a}R^{3b}, O, S and NR^{w};
R^{3a} and R^{3b} are the same or different, and are each independently selected from the group consisting of hydrogen atom, halogen, alkyl, alkoxy, haloalkyl, haloalkoxy, cyano, amino, hydroxyl and hydroxyalkyl; or, R^{3a} and R^{3b}, together with the carbon atoms to which they are attached, form cycloalkyl or heterocyclyl, wherein the cycloalkyl or heterocyclyl is optionally substituted with one or more R⁰;
R^{w} is selected from the group consisting of hydrogen atom, alkyl, alkoxy, haloalkyl, haloalkoxy, cyano, amino, hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R⁰;
each R¹, R³, R⁶, R⁰¹, R⁸ and R⁰ is the same or different, and is independently selected from the group consisting of oxo, halogen, alkyl, haloalkyl, alkoxy, haloalkoxy, hydroxyl, hydroxyalkyl, alkoxyalkyl, alkenyl, alkynyl, cyano, nitro, NR¹¹R¹², C(O)NR¹¹R¹², alkylene NR¹¹R¹², alkylene C(O)NR¹¹R¹², NR¹³C(O)R¹⁴, NR¹³C(O)NR¹¹R¹², C(O)R¹⁴, C(O)OR¹⁴, OC(O)R¹⁴, OC(O)OR¹⁴, S(O)ᵥR¹⁴, S(O)ᵥOR⁴, OS(O)ᵥR¹⁴, S(O)ᵥNR¹¹R¹², NR¹³S(O)ᵥR¹⁴, C(=NR¹³)R¹⁴, C(=NR¹³)NR¹¹R¹², S(=NR¹³)(O)R¹⁴, P(O)R¹¹R¹², OR¹⁴, =CR¹⁵R¹⁶, =NR¹³, cycloalkyl, heterocyclyl, aryl and heteroaryl, wherein the alkyl, alkoxy, alkenyl, alkynyl, alkylene, cycloalkyl, heterocyclyl, aryl and heteroaryl are each independently and optionally substituted with one or more R*;
each R¹¹, R¹², R¹³ and R¹⁴ is the same or different, and is independently selected from the group consisting of hydrogen atom, alkyl, alkoxy, hydroxyl, alkenyl, alkynyl, NR²⁰R²¹, C(O)NR²⁰R²¹, alkylene NR²⁰R²¹, alkylene C(O) NR²⁰R²¹, NR²²C(O)R²³, NR²²C(O)NR²⁰R²¹, C(O)R²³, C(O)OR²³, OC(O)R²³, OC(O)OR²³, OR²³, S(O)ᵥR²³, S(O)ᵥNR²⁰R²¹, cycloalkyl, heterocyclyl, aryl and heteroaryl, wherein the alkyl, alkylene, alkoxy, alkenyl, alkynyl, cycloalkyl, heterocyclyl, aryl and heteroaryl are each independently and optionally substituted with one or more R*; or, R¹¹ and R¹², together with the nitrogen atoms to which they are attached, form heterocyclyl, wherein the heterocyclyl is optionally substituted with one or more R*;
R¹⁵ and R¹⁶ are the same or different, and are each independently selected from the group consisting of hydrogen atom, halogen, alkyl, haloalkyl, alkoxy, haloalkoxy, hydroxyl, hydroxyalkyl, alkoxyalkyl, alkenyl, alkynyl, cyano, cycloalkyl, heterocyclyl, aryl and heteroaryl, wherein the alkyl, alkoxy, alkenyl, alkynyl, cycloalkyl, heterocyclyl, aryl and heteroaryl are each independently and optionally substituted with one or more R*;
each R²⁰, R²¹, R²² and R²³ is the same or different, and is independently selected from the group consisting of hydrogen atom, alkyl, alkoxy, haloalkyl, haloalkoxy, hydroxyl, hydroxyalkyl, alkoxyalkyl, alkenyl, alkynyl, NR^{a}R^{b}, C(O)NR^{a}R^{b}, C(O)R^{c}, cycloalkyl, heterocyclyl, cycloalkylalkyl, heterocyclylalkyl, cycloalkyloxy, heterocyclyloxy, aryl and heteroaryl, wherein the alkyl, alkoxy, alkenyl, alkynyl, cycloalkyl, heterocyclyl, aryl and heteroaryl are each independently and optionally substituted with one or more R*; or, R²⁰ and R²¹, together with the nitrogen atoms to which they are attached, form heterocyclyl, wherein the heterocyclyl is substituted with one or more R*;
each R* is the same or different, and is independently selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, C(O)NR^{a}R^{b}, C(O)R^{c}, alkylene NR^{a}R^{b}, alkylene C(O)NR^{a}R^{b}, nitro, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy, wherein the alkyl, alkoxy, alkenyl, alkynyl, alkylene, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy are each independently and optionally substituted with one or more substituents selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, NR^{a}OR^{c}, NR^{a}C(O)NR^{a}R^{b}, NR^{a}C(O)OR^{c}, C(O)NR^{a}R^{b}, C(O)R^{c}, C(O)OR^{c}, alkylene NR^{a}R^{b}, alkylene C(O)NR^{a}R^{b}, S(O)ᵥR^{c}, S(O)ᵥOR^{c}, S(O)ᵥNR^{a}R^{b}, cycloalkyl, heterocyclyl, cycloalkylalkyl, heterocyclylalkyl, cycloalkyloxy, heterocyclyloxy, aryl, heteroaryl, arylalkyl, heteroarylalkyl, aryloxy and heteroaryloxy;
R^{a}, R^{b} and R^{c} are the same or different, and are each independently selected from the group consisting of hydrogen atom, alkyl, cycloalkyl and heterocyclyl;
r is 0, 1, 2, 3, 4 or 5; v is 0, 1 or 2;
m is 0, 1, 2, 3, 4, 5 or 6;
p is 0, 1, 2, 3, 4, 5 or 6; and j is 0, 1, 2, 3, 4, 5 or 6.

2. The compound represented by general formula (I') or the pharmaceutically acceptable salt thereof according to claim 1, being a compound represented by general formula (I) or a pharmaceutically acceptable salt thereof: 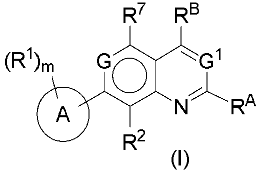
wherein, R^{A} is selected from the group consisting of 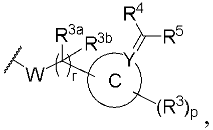 hydrogen atom, halogen, alkyl, alkoxy, haloalkyl, haloalkoxy, cyano, NR¹¹R¹², hydroxyl, hydroxyalkyl, cycloalkyl, heterocyclyl, aryl and heteroaryl, wherein the alkyl, alkoxy, cycloalkyl, heterocyclyl, aryl and heteroaryl are each independently and optionally substituted with one or more R³; each R* is the same or different, and is independently selected from the group consisting of oxo, halogen, alkyl, alkoxy, haloalkyl, haloalkoxy, hydroxyl, hydroxyalkyl, alkoxyalkyl, cyano, alkenyl, alkynyl, NR^{a}R^{b}, C(O)NR^{a}R^{b}, C(O)R^{c}, alkylene NR^{a}R^{b}, alkylene C(O)NR^{a}R^{b}, nitro, cycloalkyl, heterocyclyl, cycloalkylalkyl, heterocyclylalkyl, cycloalkyloxy, heterocyclyloxy, aryl and heteroaryl; ring A, R¹, m, G, G¹, R², R⁷, R^{B}, W, R^{3a}, R^{3b}, r, ring C, Y, R³, R⁴, R⁵, p, R¹¹, R¹², R^{a}, R^{b} and R^{c} are as defined in claim 1;
preferably, R^{A} is 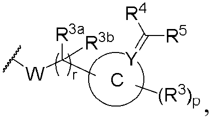
and W, R^{3a}, R^{3b}, r, Y, ring C, R³, R⁴, R⁵ and p are as defined in claim 1.

3. The compound represented by general formula (I') or the pharmaceutically acceptable salt thereof according to claim 1 or 2, being a compound represented by general formula (II) or a pharmaceutically acceptable salt thereof: 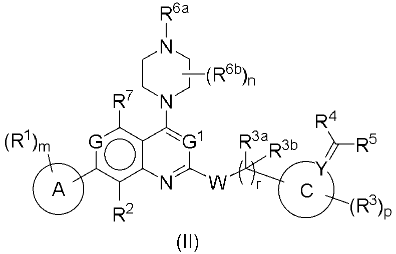
wherein, R^{6a} is hydrogen atom or R⁶;
R^{6b} is R⁶, or two R^{6b} groups, together with the carbon atoms to which they are attached, form CH(CR^{6c}R^{6d})ᵤCH;
R^{6c} and R^{6d} are the same or different, and are each independently hydrogen atom or R⁶;
n is 0, 1, 2, 3, 4, 5 or 6, and u is 0, 1 or 2;
ring A, ring C, R¹, m, G, G¹, R², R⁷, R⁶, W, R^{3a}, R^{3b}, r, Y, R³, R⁴, R⁵ and p are as defined in claim 1.

4. The compound represented by general formula (I') or the pharmaceutically acceptable salt thereof according to claim 1 or 2, being a compound represented by general formula (IN) or a pharmaceutically acceptable salt thereof: 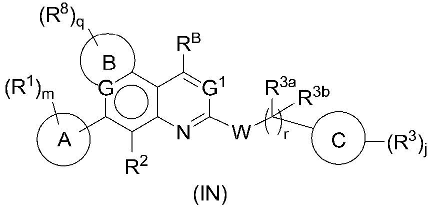
wherein, G is C or N; q is 0, 1, 2, 3 or 4;
ring A, ring B, ring C, R¹, m, R⁸, R², G¹, R^{B}, W, R^{3a}, R^{3b}, r, R³ and j are as defined in claim 1.

5. The compound represented by general formula (I') or the pharmaceutically acceptable salt thereof according to claim 1, 2 or 4, wherein: R^{B} is selected from the group consisting of hydrogen atom, 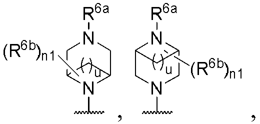 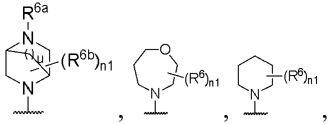 C₁₋₆ alkyl, C₂₋₆ alkenyl, C₂₋₆ alkynyl and 3- to 6-membered cycloalkyl, wherein the C₁₋₆ alkyl, C₂₋₆ alkenyl, C₂₋₆ alkynyl and 3- to 6-membered cycloalkyl are each independently and optionally substituted with one or more R⁶; R^{6a} is hydrogen atom or R⁶, R^{6b} is R⁶, and u is 0, 1 or 2; n1 is 0, 1, 2 or 3; R⁶ is as defined in claim 1; preferably, R^{B} is selected from the group consisting of hydrogen atom, 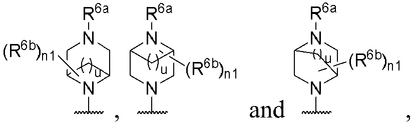 R^{6a} is hydrogen atom or R⁶, R^{6b} is R⁶, and u is 0, 1 or 2; n1 is 0, 1, 2 or 3; R⁶ is as defined in claim 1; more preferably, R^{B} is selected from the group consisting of hydrogen atom,

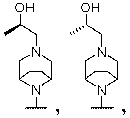

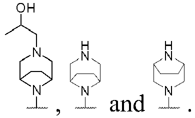

6. The compound represented by general formula (I') or the pharmaceutically acceptable salt thereof according to any one of claims 1 to 5, being a compound represented by general formula (V) or a pharmaceutically acceptable salt thereof: 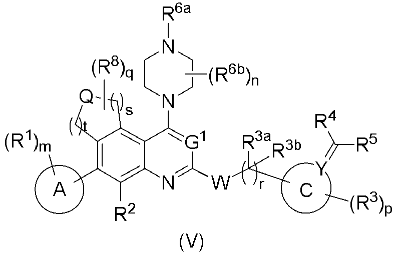 wherein
Q is selected from the group consisting of CR^{8a}R^{8b}, O, S, C(O), C(O)NR^{8c} and NR^{8c};
R^{8a} and R^{8b} are the same or different, and are each independently hydrogen atom or R⁸;
R^{8c} is selected from the group consisting of hydrogen atom, alkyl, haloalkyl, hydroxyalkyl, cycloalkyl and heterocyclyl;
s is 0, 1, 2 or 3; t is 0, 1, 2 or 3; q is 0, 1, 2, 3 or 4;
ring A, ring C, R¹, m, R², G¹, R⁸, R^{6a}, R^{6b}, n, W, R^{3a}, R^{3b}, r, Y, R³, R⁴, R⁵ and p are as defined in claim 3.

7. The compound represented by general formula (I') or the pharmaceutically acceptable salt thereof according to claim 1 or 2, being a compound represented by general formula (VII-1) or (VII-2), or a pharmaceutically acceptable salt thereof: 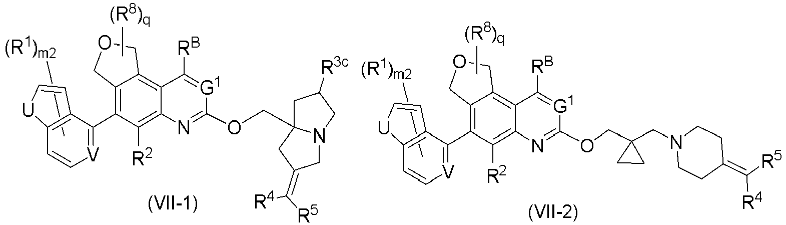 wherein
U is selected from the group consisting of NR^{1a}, O, S and Se; V is N or CR^{1b};
R^{1a} is hydrogen atom or R¹; R^{1b} is hydrogen atom or R¹; R^{3c} is hydrogen atom or R³;
m2 is 0, 1, 2, 3 or 4; q is 0, 1, 2, 3 or 4;
R¹, R⁸, R², G¹, R^{B}, R³, R⁴ and R⁵ are as defined in claim 1.

8. The compound represented by general formula (I') or the pharmaceutically acceptable salt thereof according to claim 3, 5 or 6, wherein 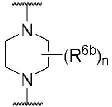 is selected from the group consisting of 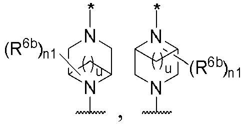 and 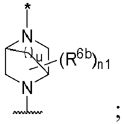 preferably 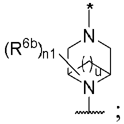 n1 is 0, 1, 2 or 3, R^{6b} is R⁶, and R⁶ and u are as defined in claim 3; more preferably 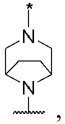 wherein the * end is connected to R^{6a}.

9. The compound represented by general formula (I') or the pharmaceutically acceptable salt thereof according to claim 3, 5, 6, 7 or 8, wherein R^{6a} is selected from the group consisting of hydrogen atom, C₁₋₆ alkyl and OR¹⁴, wherein the C₁₋₆ alkyl is optionally substituted with one or more substituents selected from the group consisting of halogen, hydroxyl, C₁₋₆ alkoxy and 3- to 6-membered heterocyclyl; R¹⁴ is selected from the group consisting of hydrogen atom, C₁₋₆ alkyl and 3- to 6-membered cycloalkyl; preferably, R^{6a} is hydrogen atom or C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; more preferably, R^{6a} is C₁₋₆ alkyl, wherein the C₁₋₆ alkyl is optionally substituted with one or more substituents selected from the group consisting of hydroxyl, C₁₋₆ alkoxy and 3- to 6-membered heterocyclyl; further preferably, R^{6a} is C₁₋₆ hydroxyalkyl.

10. The compound represented by general formula (I') or the pharmaceutically acceptable salt thereof according to any one of claims 1-3 or 5-9, wherein R⁴ and R⁵ are the same or different, and are each independently selected from the group consisting of hydrogen atom, halogen, C₁₋₆ alkyl and C₁₋₆ haloalkyl; preferably, R⁴ and R⁵ are the same or different, and are each independently hydrogen atom or halogen.

11. The compound represented by general formula (I') or the pharmaceutically acceptable salt thereof according to any one of claims 1-6 or 8-10, wherein ring C is 3- to 8-membered heterocyclyl; and/or each R³ is the same or different, and is independently halogen or =CR¹⁵R¹⁶, and R¹⁵ and R¹⁶ are the same or different, and are each independently hydrogen atom or halogen.

12. The compound represented by general formula (I') or the pharmaceutically acceptable salt thereof according to any one of claims 1-6 or 8-11, wherein W is O, and/or R^{3a} and R^{3b} are hydrogen atoms, or R^{3a} and R^{3b}, together with the carbon atoms to which they are attached, form cyclopropyl, and/or r is 1 or 3.

13. The compound represented by general formula (I') or the pharmaceutically acceptable salt thereof according to any one of claims 1-6 or 8-12, wherein ring A is 6- to 10-membered aryl or 5- to 14-membered heteroaryl; preferably, ring A is selected from the group consisting of naphthyl, phenyl, pyridyl, benzothienyl, benzothiazolyl, benzopyrazolyl, pyridothienyl, pyridothiazolyl, pyridopyrazolyl, benzoselenophenyl and pyridoselenophenyl; more preferably, ring A is selected from the group consisting of phenyl, benzothienyl and pyridothienyl.

14. The compound represented by general formula (I') or the pharmaceutically acceptable salt thereof according to any one of claims 1-6 or 8-13, wherein 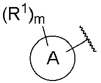 is selected from the group consisting of 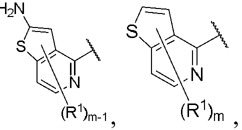 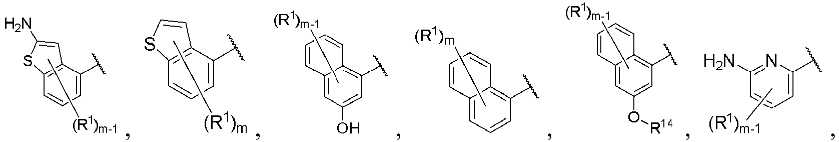 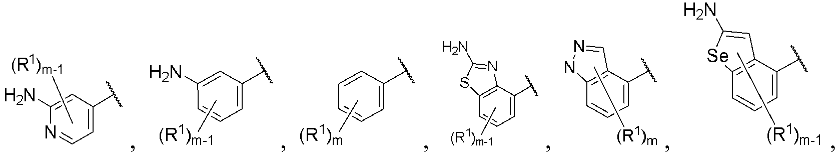 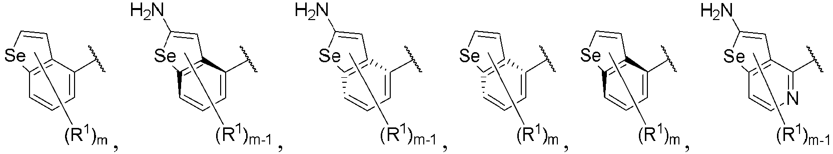 and 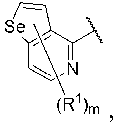 and R¹ and m are as defined in claim 1; preferably, 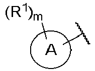 is selected from the group consisting of

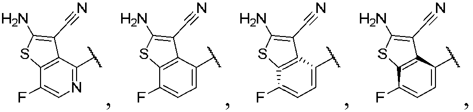

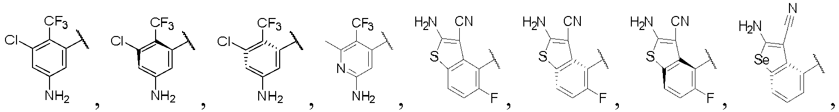

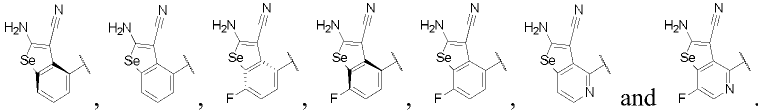

15. The compound represented by general formula (I') or the pharmaceutically acceptable salt thereof according to any one of claims 1-14, wherein each R¹ is the same or different, and is independently selected from the group consisting of halogen, C₁₋₆ alkyl, C₂₋₆ alkynyl, C₁₋₆ haloalkyl, hydroxyl, C₁₋₆ hydroxyalkyl, cyano, -NR¹¹R¹² and 3- to 8-membered cycloalkyl; preferably, each R¹ is the same or different, and is independently selected from the group consisting of halogen, C₁₋₆ haloalkyl, cyano and -NR¹¹R¹², R¹¹ and R¹² are as defined in claim 1, and/or m is 1, 2 or 3.

16. The compound represented by general formula (I') or the pharmaceutically acceptable salt thereof according to any one of claims 1-15, wherein G¹ is N, and/or R² is halogen.

17. The compound represented by general formula (I') or the pharmaceutically acceptable salt thereof according to any one of claims 1-16, wherein the compound is selected from the group consisting of the following compounds: 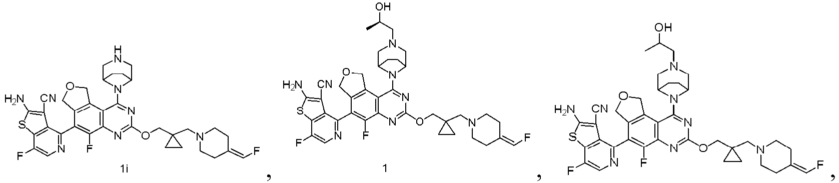 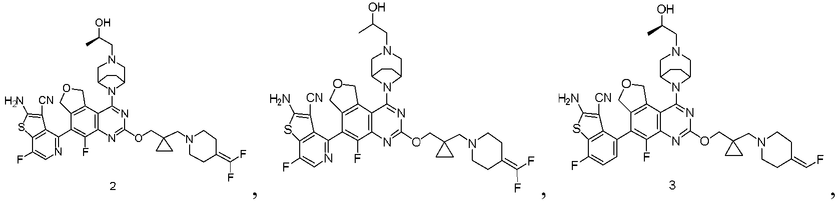 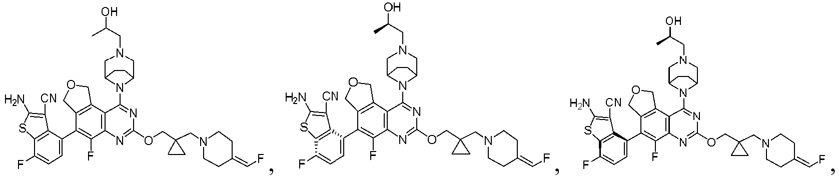 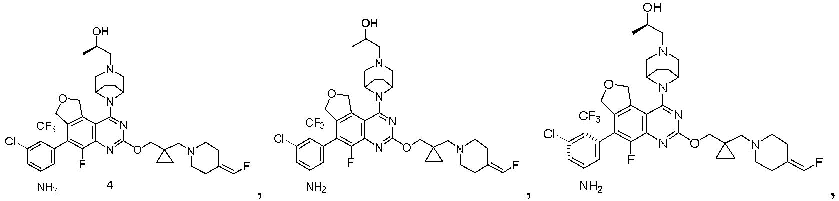 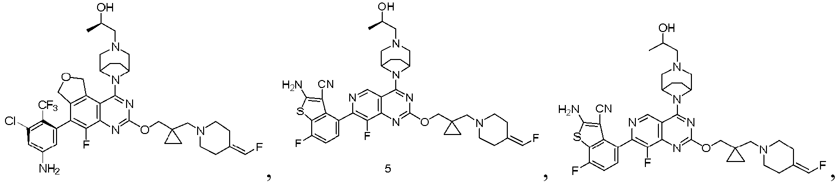 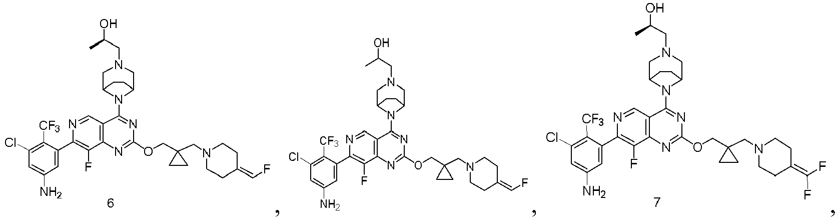 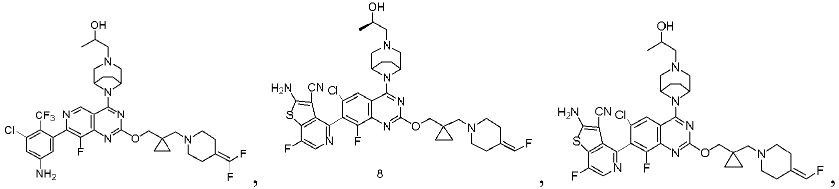 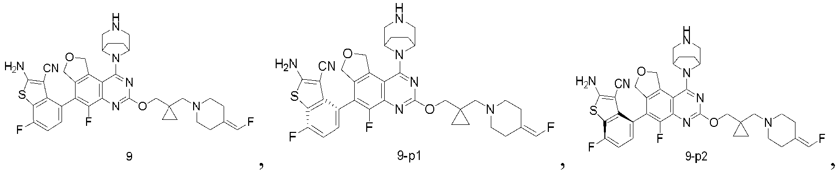 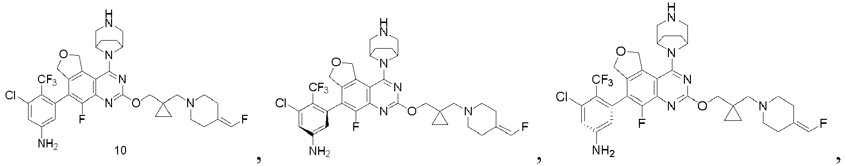 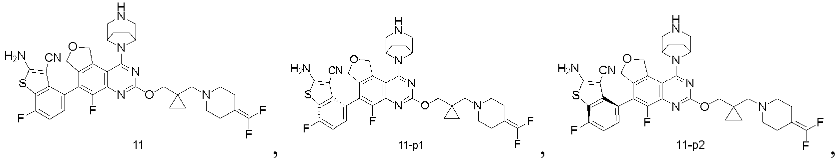 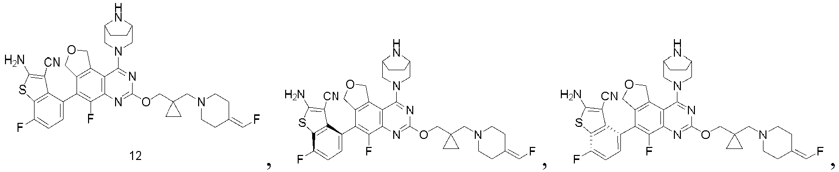 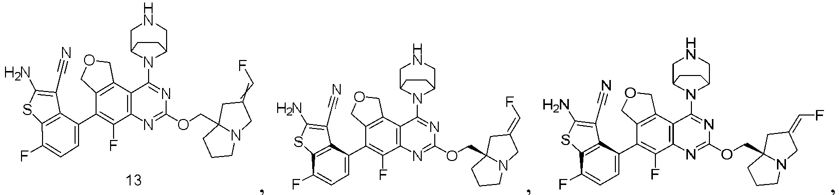 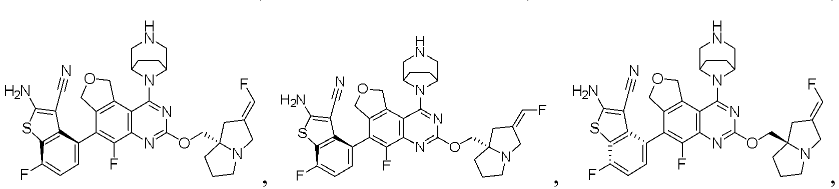 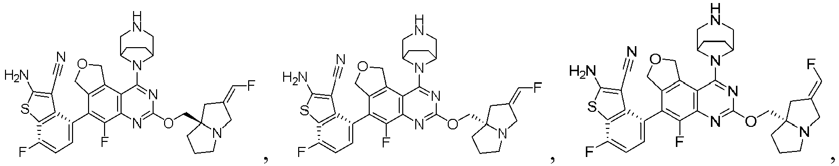 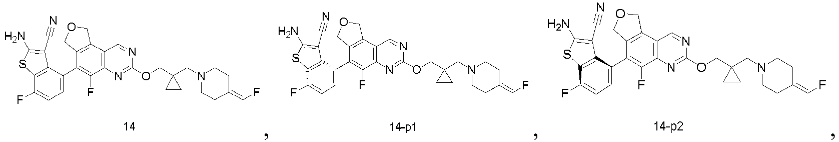 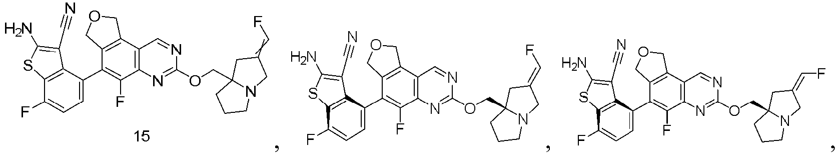 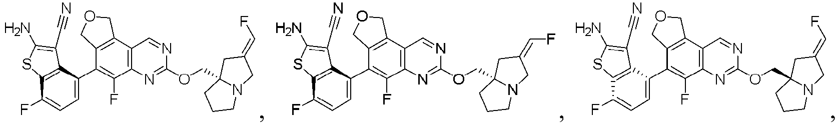 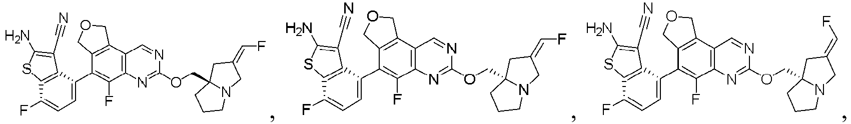 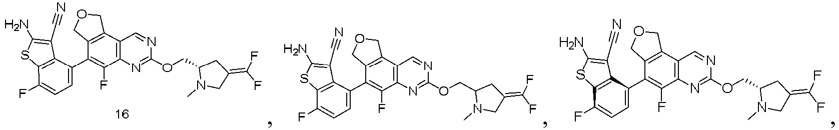 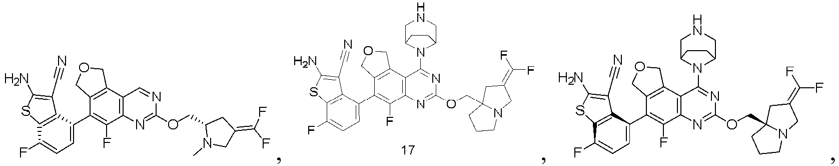 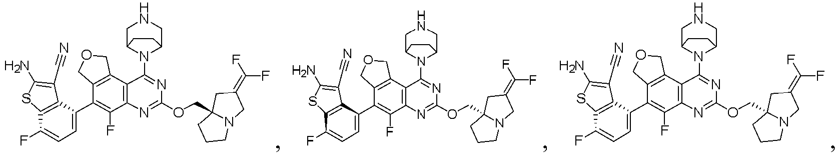 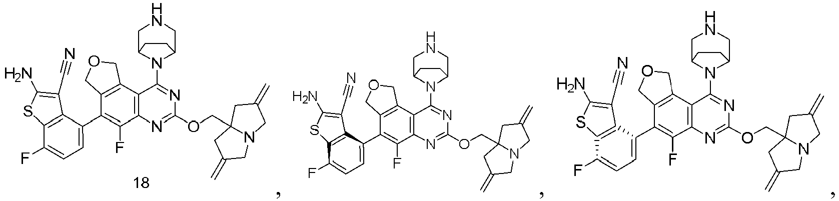 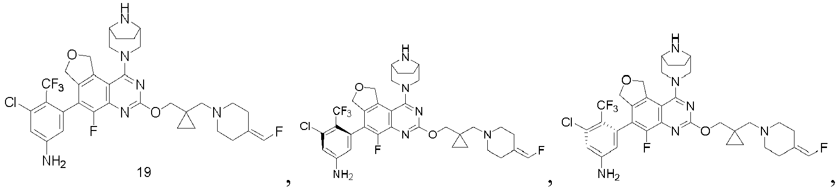 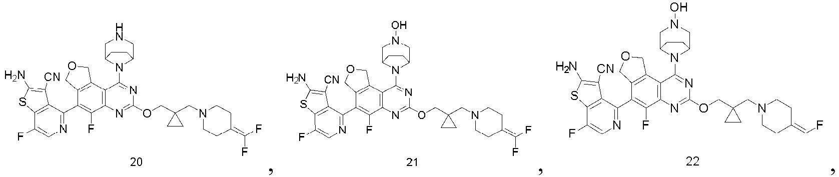 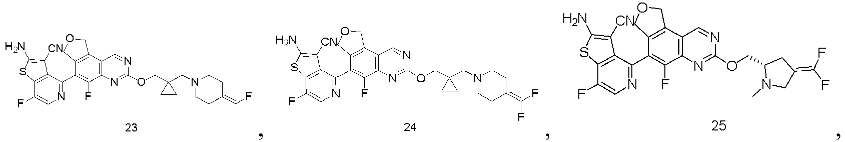 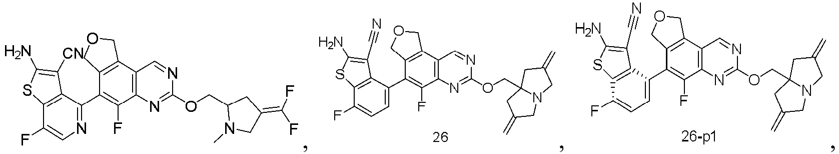 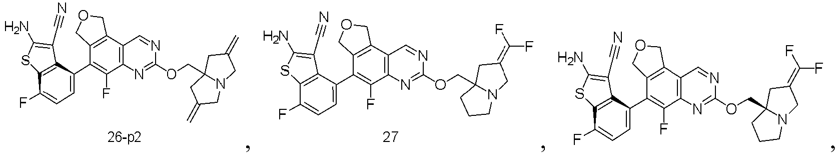 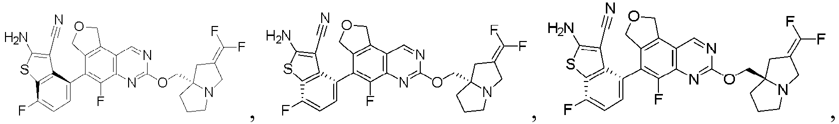 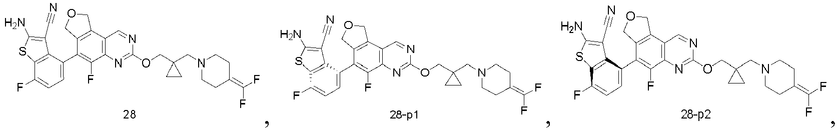 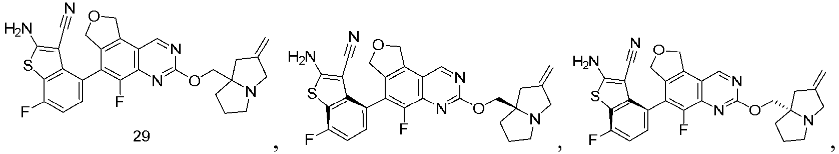 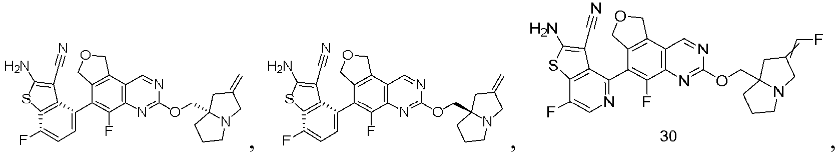 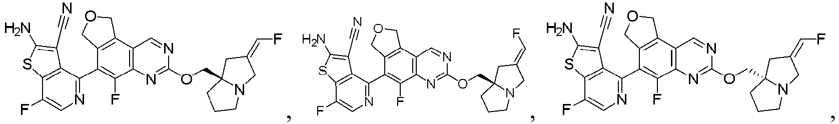 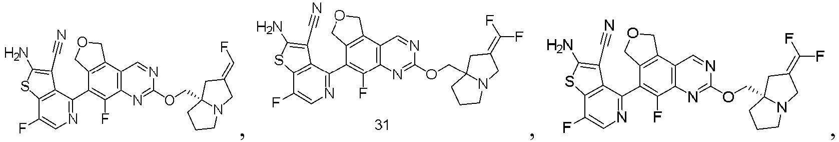 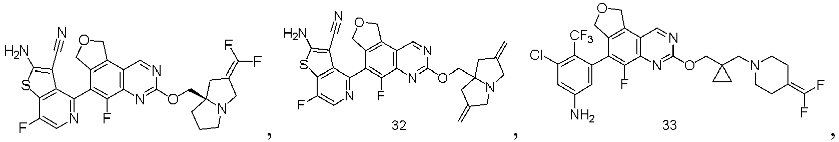 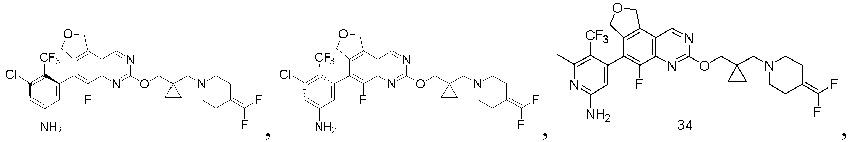 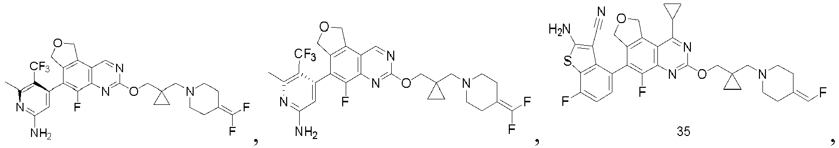 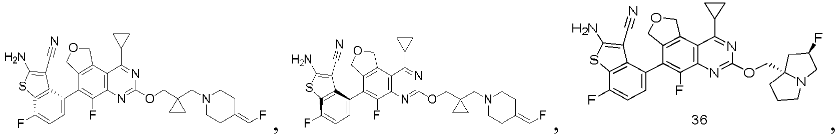 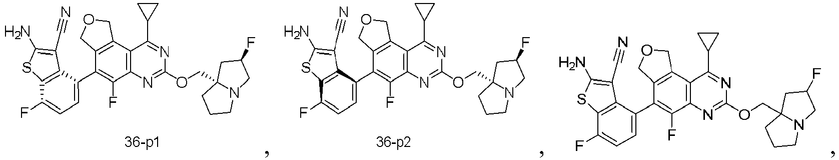 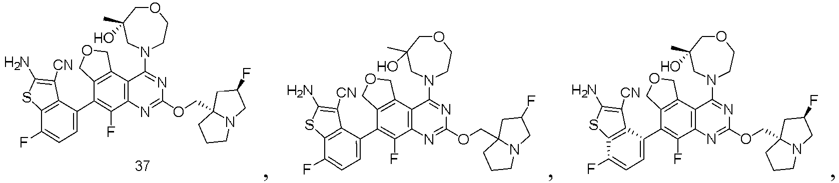 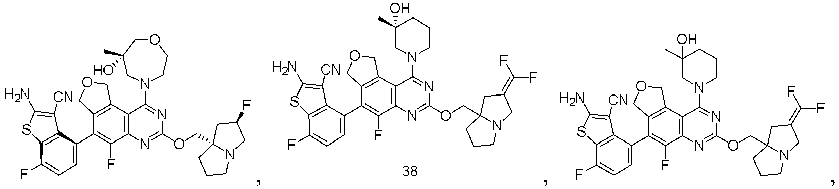 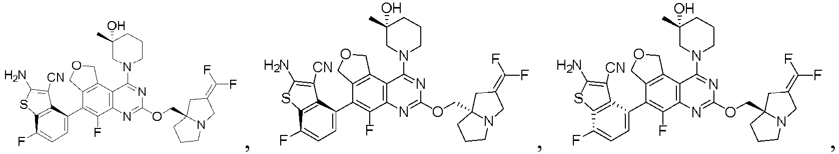 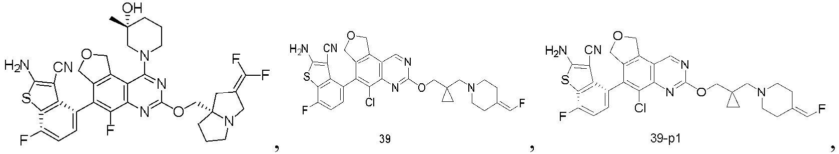 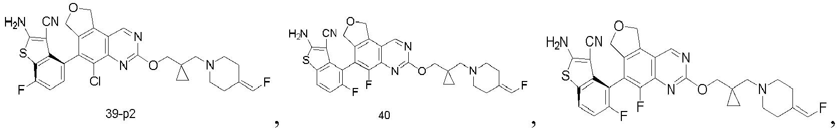 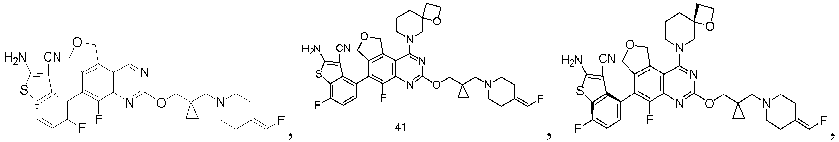 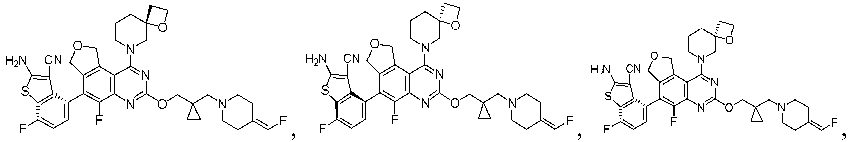 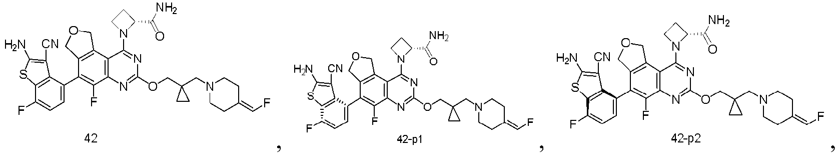 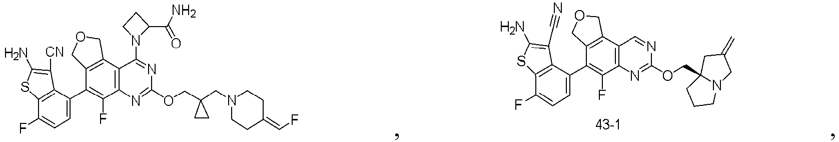 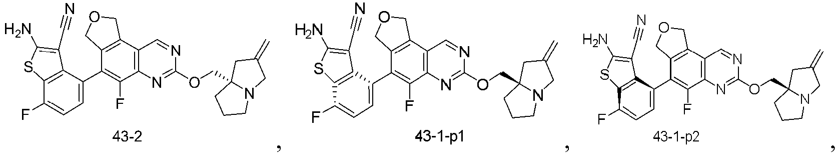 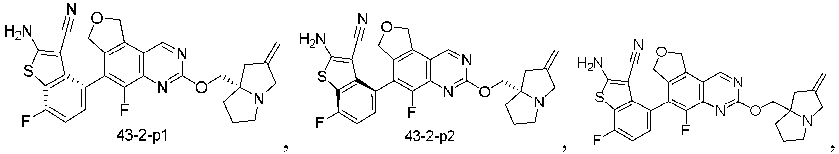 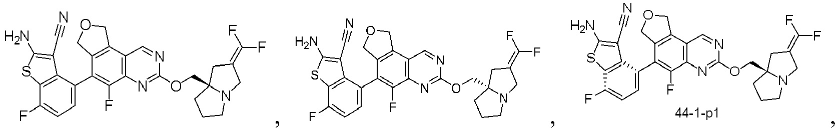 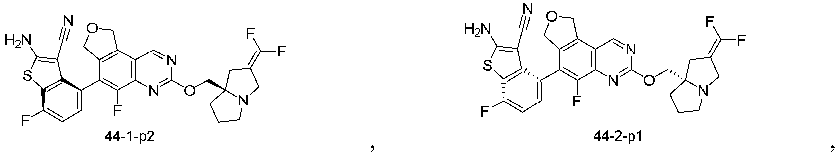 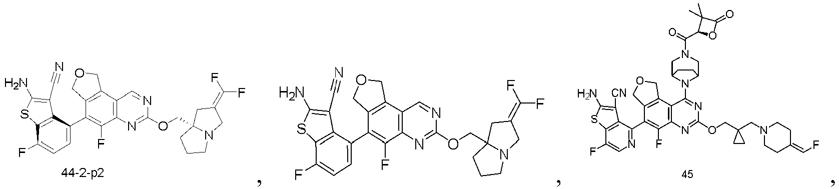 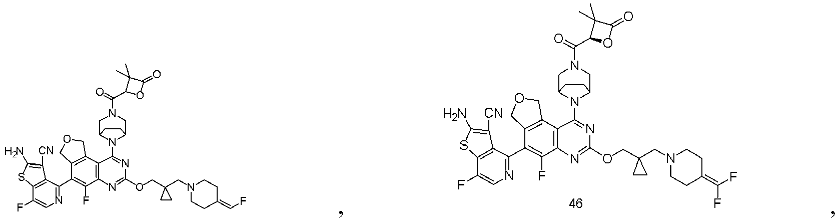 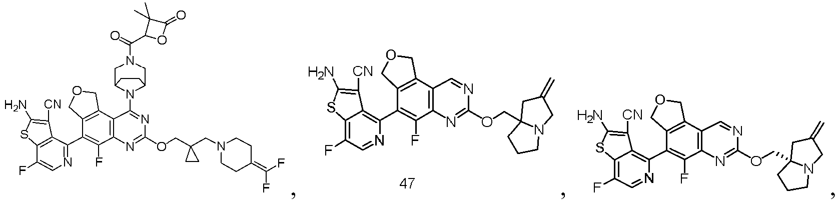 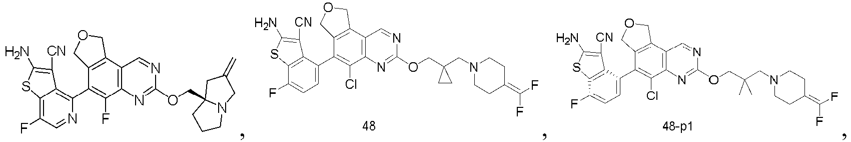 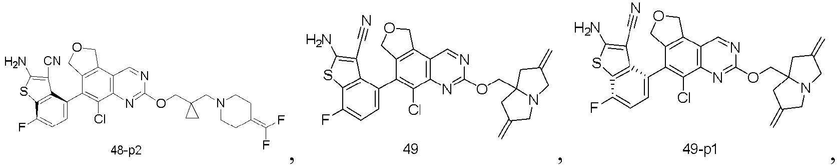 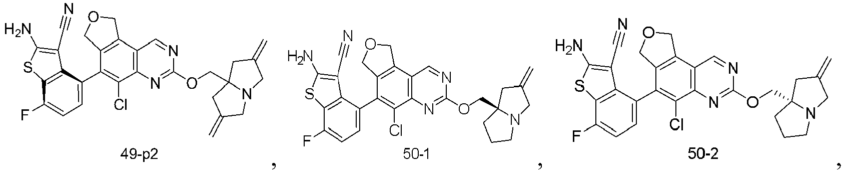 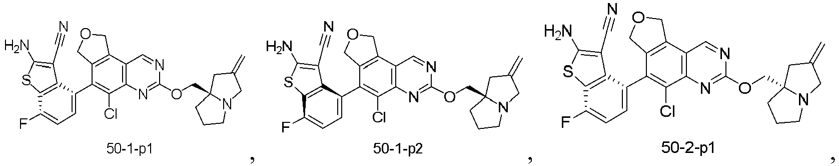 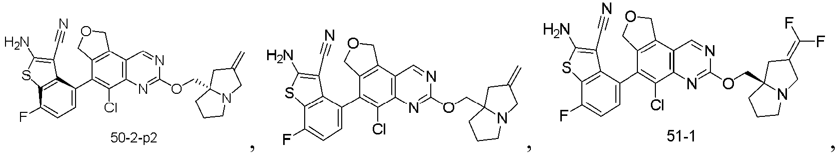 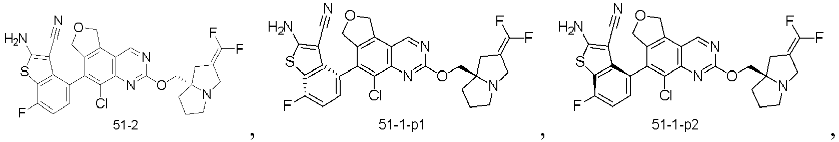 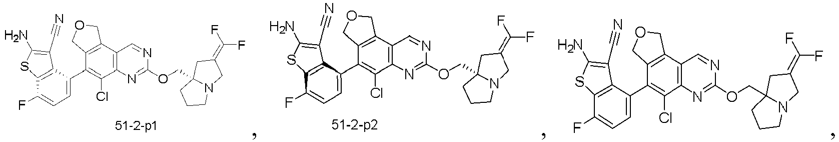 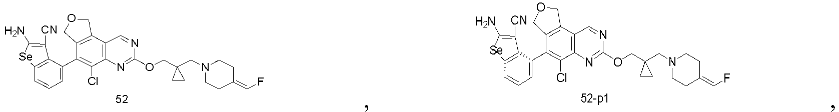 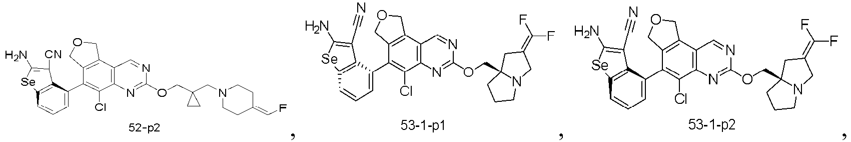 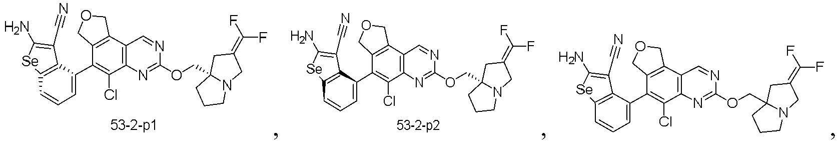 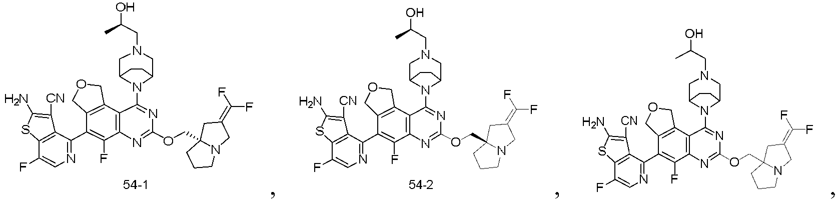 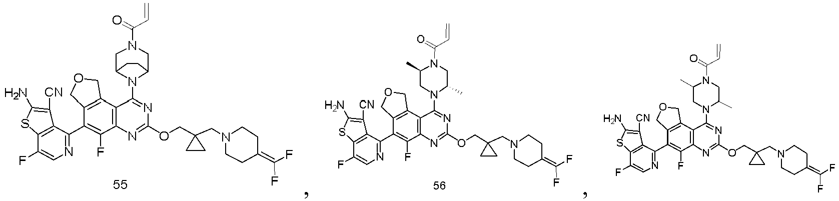 and

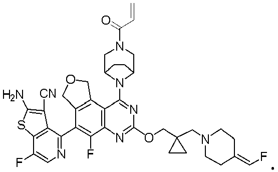

18. A compound represented by general formula (VA) or a salt thereof: 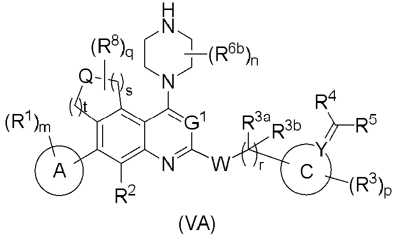 wherein ring A, ring C, R¹, m, Q, s, t, R⁸, q, R², G¹, R^{6b}, n, W, R^{3a}, R^{3b}, r, Y, R³, R⁴, R⁵ and p are as defined in claim 6.

19. The compound represented by general formula (I') or the pharmaceutically acceptable salt thereof according to claim 1, wherein the compound is selected from the group consisting of the following compounds:

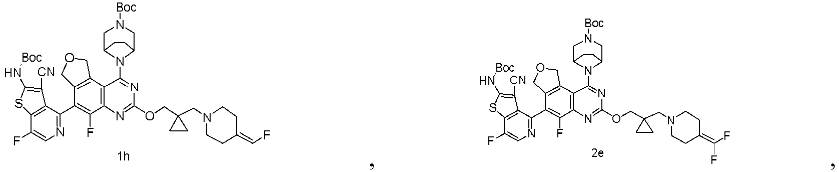

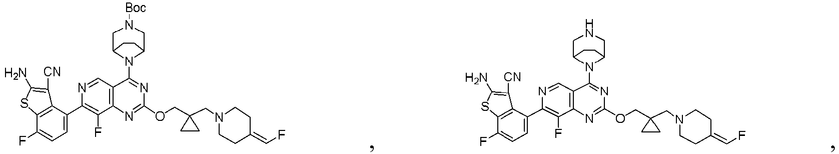

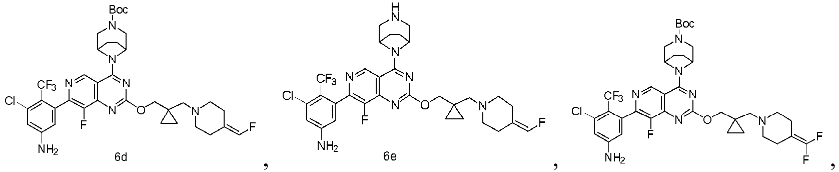

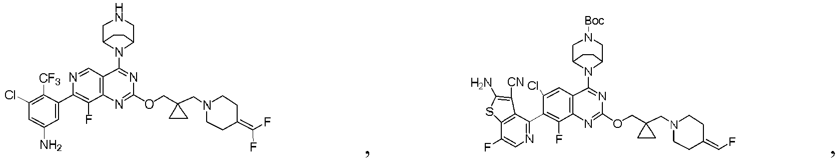

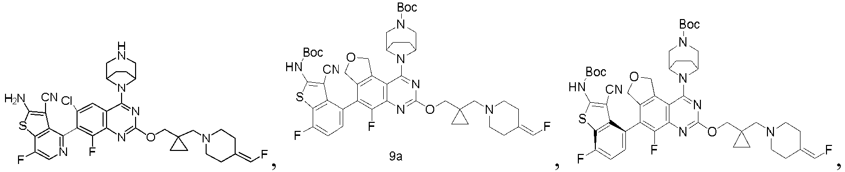

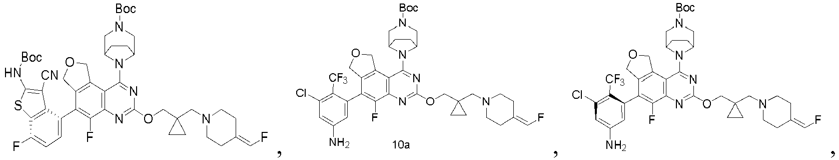

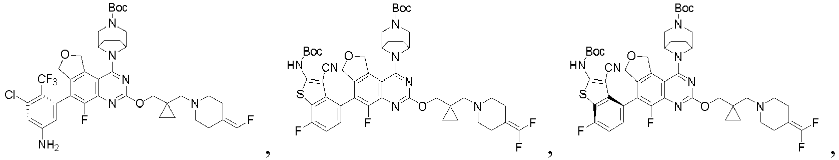

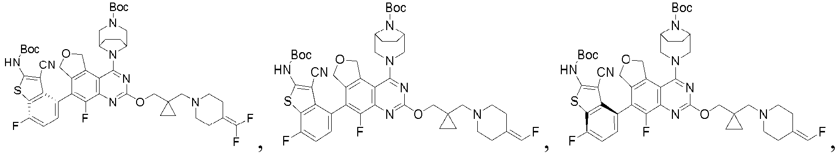

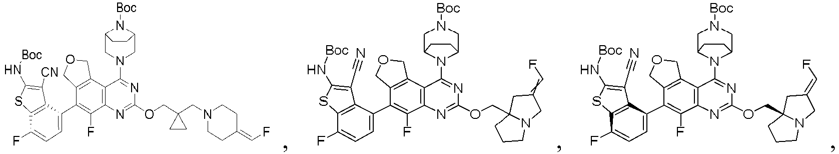

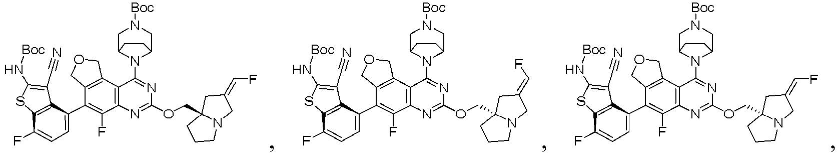

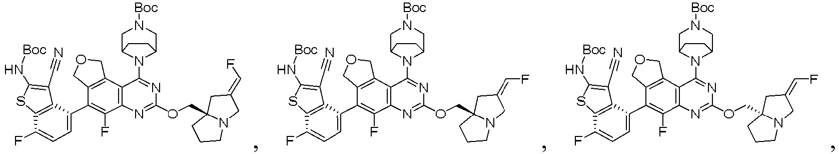

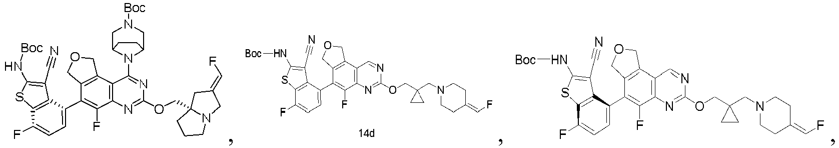

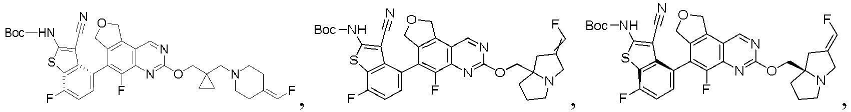

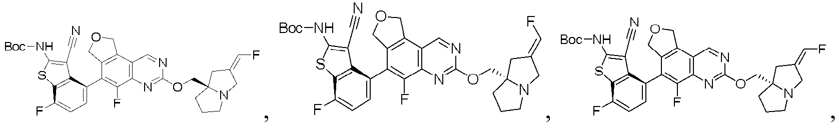

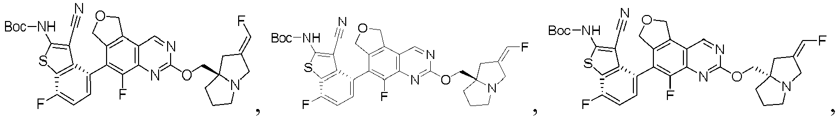

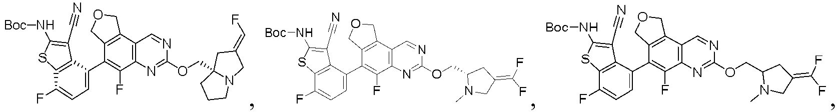

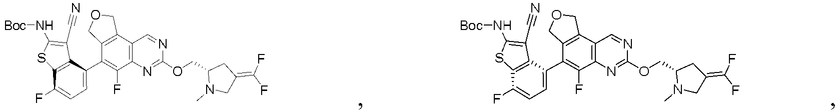

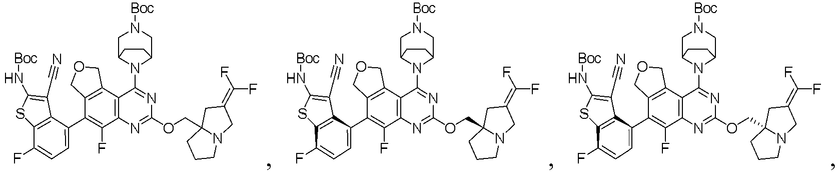

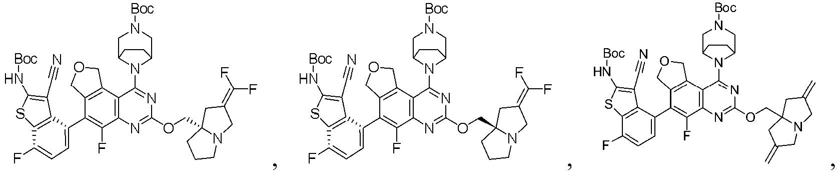

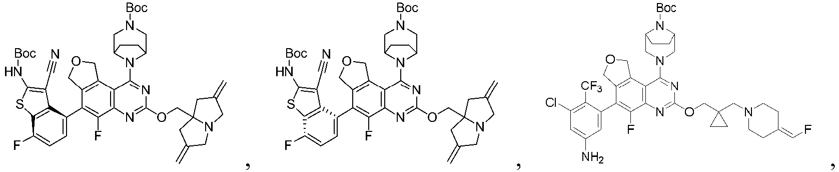

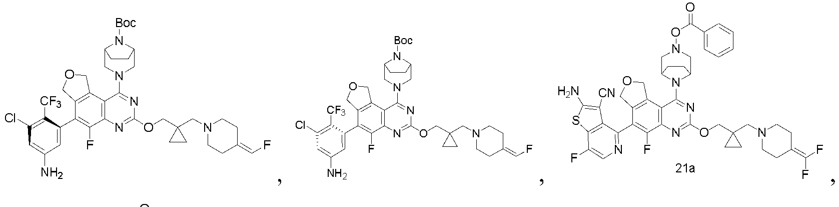

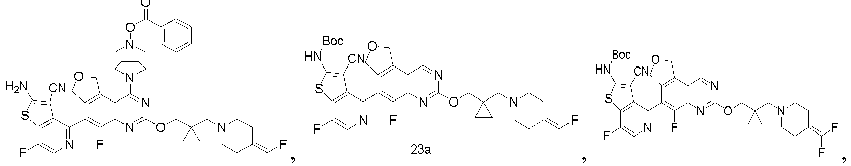

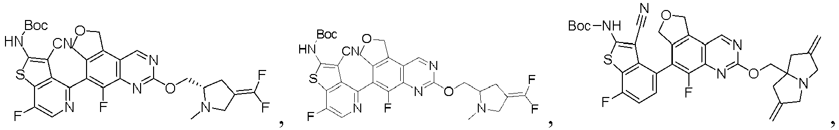

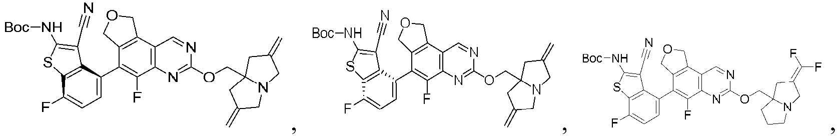

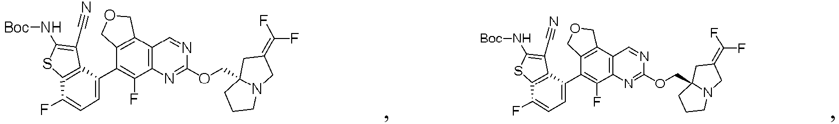

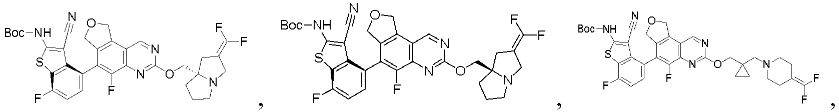

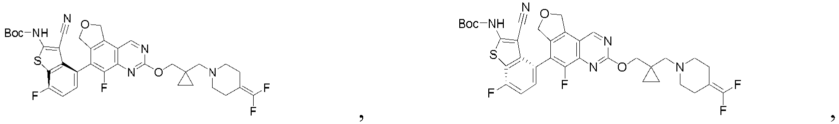

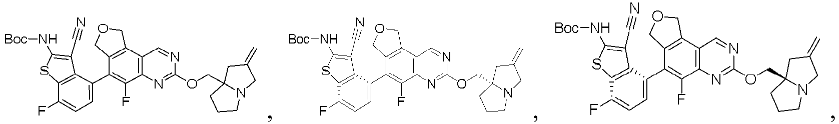

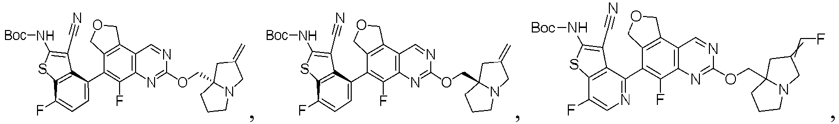

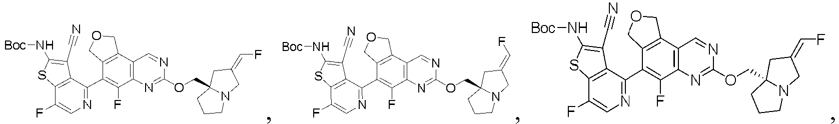

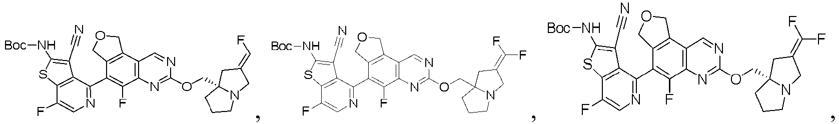

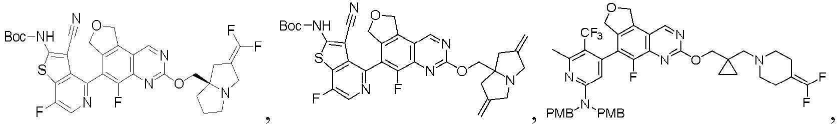

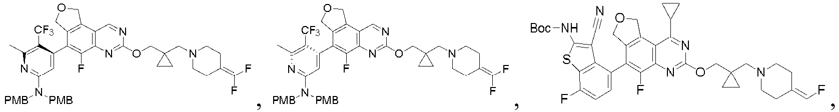

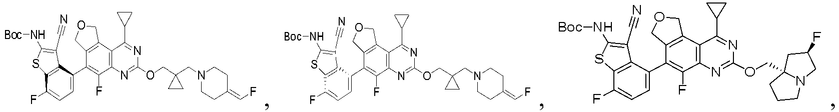

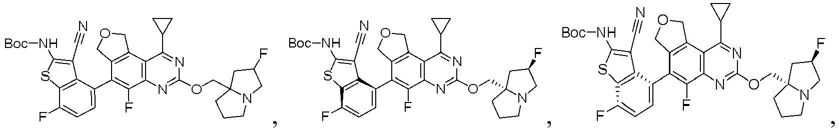

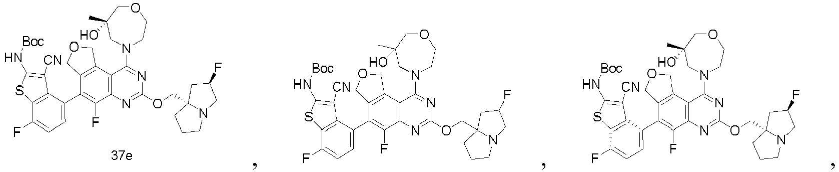

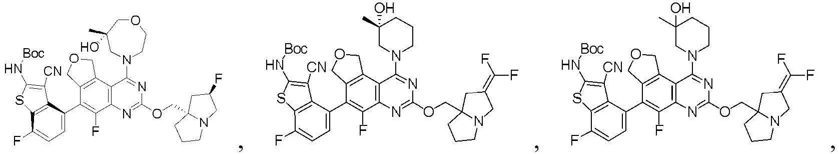

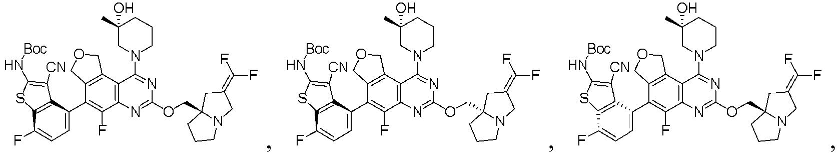

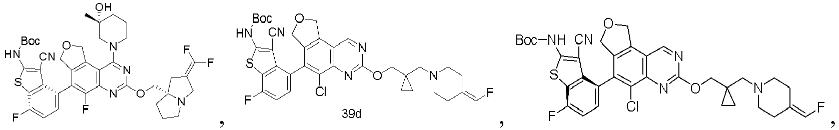

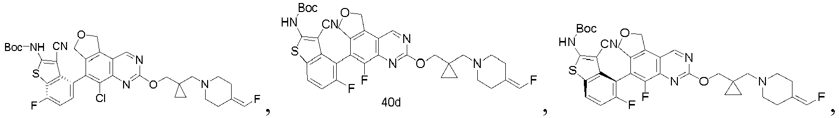

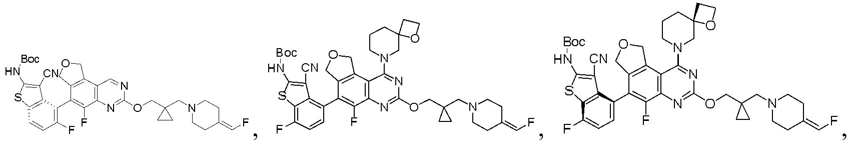

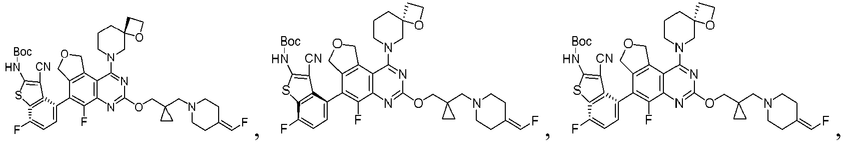

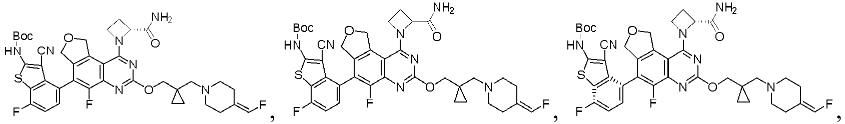

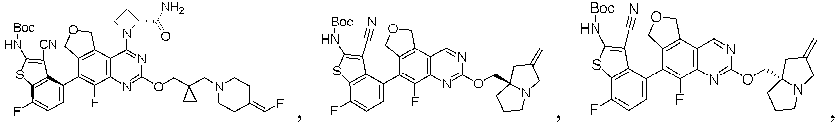

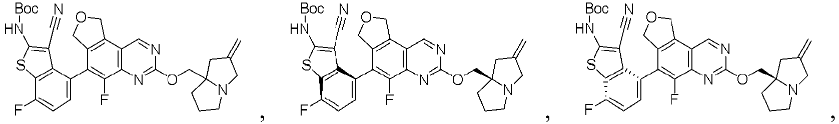

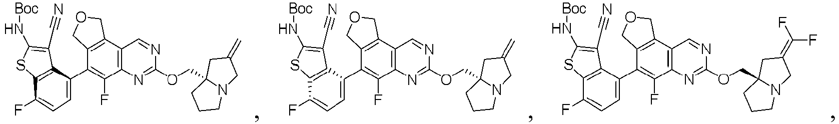

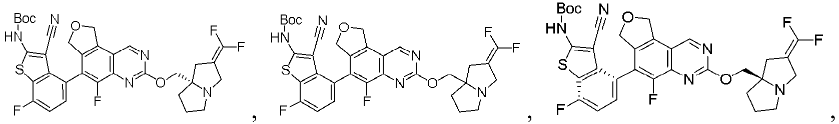

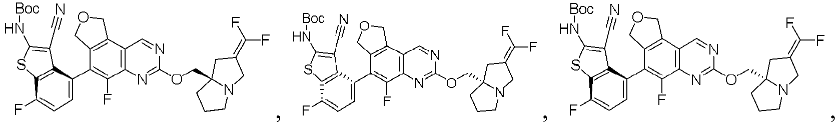

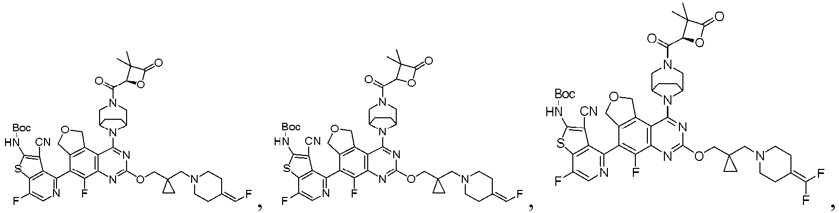

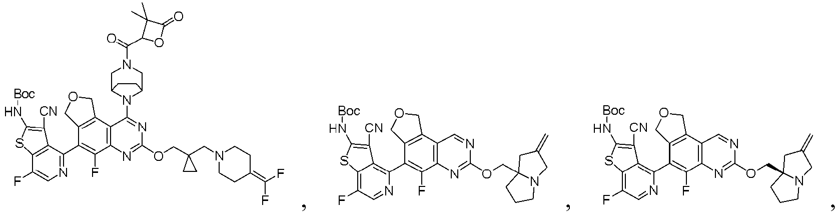

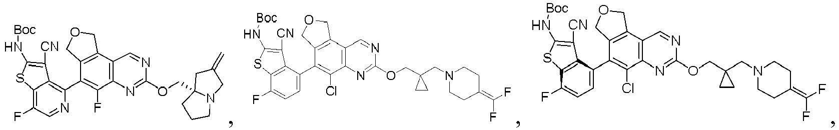

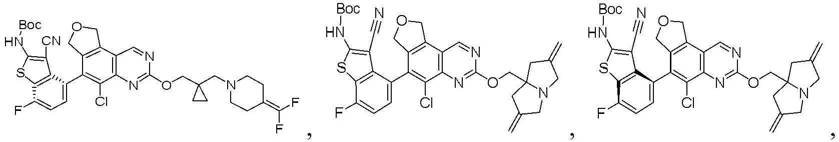

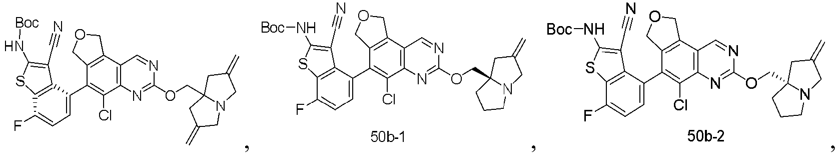

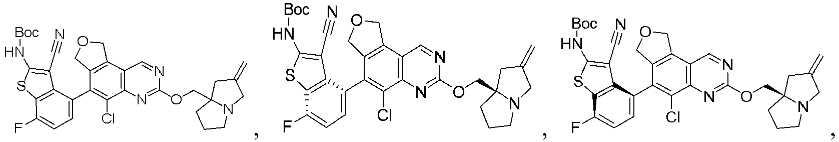

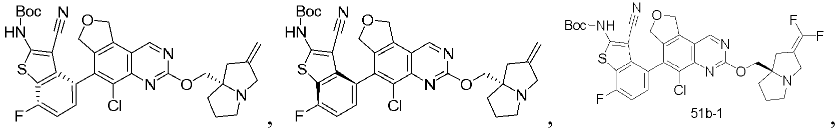

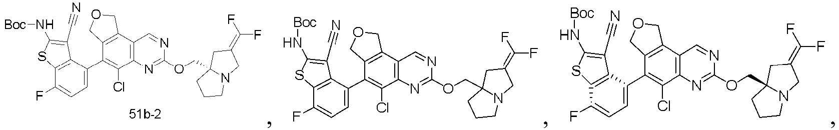

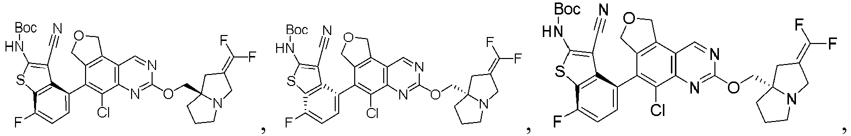

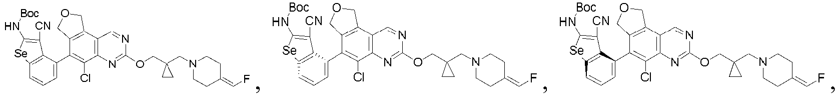

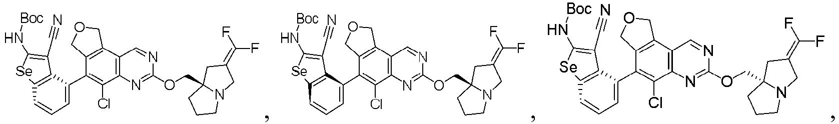

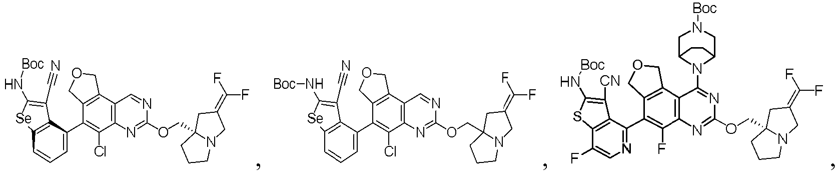

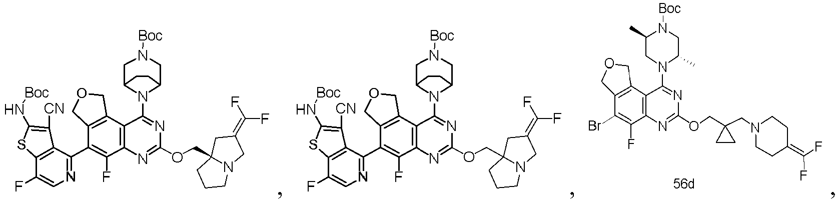

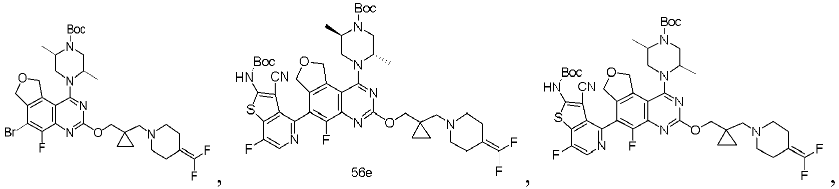

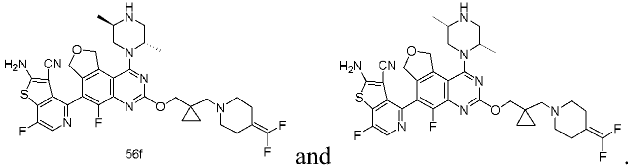

20. A method for preparing a compound represented by general formula (V) or a pharmaceutically acceptable salt thereof, wherein the method comprises: 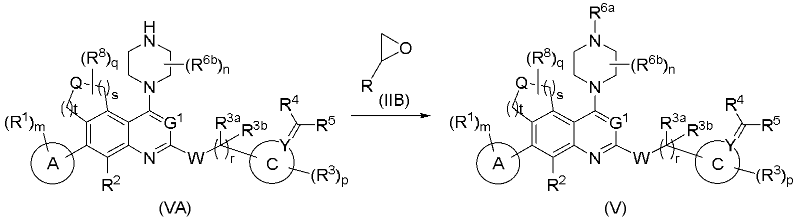
subjecting a compound represented by general formula (VA) or a salt thereof, and a compound represented by general formula (IIB) or a salt thereof to a ring-opening reaction to obtain the compound represented by general formula (V) or the pharmaceutically acceptable salt thereof,
wherein, R^{6a} is 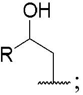 R is hydrogen atom or alkyl;
ring A, ring C, R¹, m, Q, s, t, R⁸, q, R², G¹, R^{6b}, n, W, R^{3a}, R^{3b}, r, Y, R³, R⁴, R⁵ and p are as defined in claim 6.

21. A pharmaceutical composition, comprising the compound or the pharmaceutically acceptable salt thereof according to any one of claims 1-17, and one or more pharmaceutically acceptable carriers, diluents or excipients.

22. Use of the compound or the pharmaceutically acceptable salt thereof according to any one of claims 1-17 or the pharmaceutical composition according to claim 21 in the preparation of a medicament for inhibiting KRAS amplification and/or KRAS mutant activity, wherein the KRAS mutant is preferably KRAS G12D and/or KRAS G12V mutation.

23. Use of the compound or the pharmaceutically acceptable salt thereof according to any one of claims 1-17 or the pharmaceutical composition according to claim 21 in the preparation of a medicament for treating and/or preventing a disease or condition mediated by KRAS amplification and/or KRAS mutant, wherein the KRAS mutant is preferably KRAS G12D and/or KRAS G12V mutation.

24. Use of the compound or the pharmaceutically acceptable salt thereof according to any one of claims 1-17 or the pharmaceutical composition according to claim 21 in the preparation of a medicament for treating and/or preventing cancer, wherein the cancer is preferably selected from the group consisitn of brain cancer, thyroid cancer, head and neck cancer, nasopharyngeal cancer, pharyngolaryngeal cancer, oral cancer, salivary gland cancer, oesophageal cancer, gastric cancer, lung cancer, liver cancer, kidney cancer, pancreatic cancer, gallbladder cancer, bile duct cancer, colourectal cancer, small intestine cancer, gastrointestinal stromal tumour, urothelial cancer, urethral cancer, bladder cancer, breast cancer, vaginal cancer, ovarian cancer, endometrial cancer, cervical cancer, fallopian tube cancer, testicular cancer, prostate cancer, haemangioma, leukaemia, lymphoma, myeloma, skin cancer, lipoma, bone cancer, soft tissue sarcoma, neurofibroma, glioma, neuroblastoma, and glioblastoma; more preferably, the cancer is selected from the group consisting of pancreatic cancer, colourectal cancer and non-small-cell lung cancer.
